# EUROPEAN PATENT APPLICATION

(11) **EP 4 295 861 A1**
(43) Date of publication of application: **27.12.2023**
(21) Application number: 22756238.6
(22) Date of filing: 16.02.2022
(51) Int. Cl.: A61K 39/04, A61K 31/713, A61K 39/00, A61K 39/002, A61K 39/02, A61K 39/12, A61K 39/145, A61K 39/155, A61K 39/215, A61K 39/235, A61K 39/39, A61K 45/00, A61P 31/00, A61P 31/04, A61P 31/12, A61P 31/14, A61P 37/04, A61P 37/06, A61P 37/08, A61P 43/00

(54) **NEW TREATMENT AND PREVENTION BASED ON NEW METHOD FOR CONTROLLING CELLULAR IMMUNITY**

(30) Priority: 17.02.2021 WO PCT/JP2021/005951; 17.02.2021 TW 110105403; 22.12.2021 JP 2021208598
(71) Applicant: Zeria Pharmaceutical Co., Ltd., Tokyo 103-8351 (JP); Ishii, Ken, Tokyo 113-8654 (JP)
(72) Inventor: TEMIZOZ, Burcu, Tokyo 113-8654 (JP); KATSUNUMA, Kokichi, Tokyo 103-8351 (JP); ISHII, Ken, Tokyo 113-8654 (JP)
(74) Representative: Engelhard, Markus
(86) International application number: PCT/JP2022/006212
(87) International publication number: WO 2022/176920

(57) **Abstract**

The present disclosure provides a composition for the prophylaxis or treatment of diseases (e.g., infectious disease, allergy or autoimmune disease). The present disclosure provides a composition, a medicament, a kit, and the like for the prophylaxis or treatment of the disease of a test subject. The composition, medicament, kit, and the like contain a non-causative factor antigen component that is neither derived from nor cross-reactive with the causative factor of the disease, and is administered under conditions where a causative factor antigen component derived from and/or cross-reactive with a causative factor of the target disease is present in the target.

## Description

### [Technical Field]

The present disclosure relates to novel disease treatment and prophylaxis, using novel combination components that regulate cellular immunity.

### [Background Art]

2019 Coronavirus disease (Covid-19) is a disease caused by severe acute respiratory syndrome coronavirus-2 (SARS CoV-2) and caused a worldwide pandemic in 2019 and thereafter. While clinical studies have been conducted on a number of coronavirus vaccines, a more effective therapeutic approach is urgently needed.

### [Summary of Invention]

### [Solution to Problem]

The present inventors have conducted intensive studies and developed new techniques for treating and preventing diseases using new combination components that regulate cellular immunity. In one embodiment of the present disclosure, it was found that cellular immune memory useful for preventing or treating a target disease in a subject can be freely controlled (e.g., induced, up-regulated, suppressed, downregulated, etc.) by combining a causative factor antigen component derived from and/or cross-reactive with a causative factor of the target disease and a non-causative factor antigen component that is neither derived from nor cross-reactive with the causative factor of the disease, and the disease can be specifically prevented or treated. In a preferred embodiment, it was found that the subject has immune memory for a non-causative factor antigen component, and humoral immunity to the causative factor antigen component is regulated to achieve the prophylaxis or cure of the target disease, by exposing the subject to a combination of a causative factor antigen component different from the non-causative factor antigen component.

As representative aspects, therefore, the present disclosure provides the following.
(item Y1) A composition for the prophylaxis or treatment of an infectious disease, an allergy, and/or an autoimmune disease in a subject, comprising a non-causative factor antigen component that is neither derived from nor cross-reactive with a causative factor of the infectious disease, the allergy, and/or the autoimmune disease, wherein
   the composition is characterized in that
   1) the subject is confirmed to have an immune response to the non-causative factor,
   2) the composition is administered to the subject when the subject has the immune response.
(item Y2) A composition for the prophylaxis or treatment of an infectious disease in a subject, comprising a non-causative factor antigen component that is neither derived from nor cross-reactive with a causative factor of the infectious disease, wherein
   the composition is characterized in that
   1) the subject is confirmed to have an immune response to the non-causative factor,
   2) the composition is administered to the subject when the subject has the immune response.
(item Y3) A composition for the prophylaxis or treatment of an infectious disease other than tuberculosis in a subject, comprising a Mycobacterium tuberculosis hot water extract or a part thereof, wherein
   the composition is characterized in that
   1) the subject is confirmed to have an immune response to tuberculosis,
   2) the composition is administered to the subject when the subject has the immune response.
(item Y4) The composition of any of the above-mentioned items, wherein the aforementioned Mycobacterium tuberculosis hot water extract or a part thereof is a hot water extract of Mycobacterium tuberculosis Aoyama B strain.
(item Y5) The composition of any of the above-mentioned items, wherein the aforementioned Mycobacterium tuberculosis hot water extract or a part thereof is Extract A.
(item Y6) The composition of any of the above-mentioned items, wherein the aforementioned composition is administered in an amount effective for the production of IL-10.
(item Y7) The composition of any of the above-mentioned items, wherein the aforementioned infectious disease is a viral infectious disease.
(item Y8) The composition of any of the above-mentioned items, wherein the aforementioned infectious disease is a viral infectious disease related to Coronavirus.
(item Y9) The composition of any of the above-mentioned items, wherein the aforementioned immune response is objectively confirmed.
(item Y10) The composition of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y11) The composition of any of the above-mentioned items, wherein the aforementioned immune response is tested by a tuberculin reaction test or an interferon γ release assay.
(item Y12) The composition of any of the above-mentioned items, wherein the aforementioned non-causative factor antigen component or a Mycobacterium tuberculosis hot water extract or a part thereof is pre-administered to the test subject when the aforementioned subject does not have the aforementioned immune response, and thereafter, the aforementioned composition is administered to the subject when the subject is confirmed to have the immune response.
(item Y13) The composition of any of the above-mentioned items, wherein the aforementioned pre-administration is repeated until the aforementioned subject has the aforementioned immune response.
(item Y14) The composition of any of the above-mentioned items, wherein the aforementioned pre-administration is administration before or after the onset of the aforementioned infectious disease.
(item Y15) The composition of any of the above-mentioned items, wherein the aforementioned pre-administration is administration after the onset of the aforementioned infectious disease.
(item Y16) The composition of any of the above-mentioned items, which is administered under conditions where a causative factor antigen component derived from and/or cross-reactive with the aforementioned causative factor is present.
(item Y17) A composition for the prophylaxis or treatment of a target disease in a subject, comprising a non-causative factor antigen component that is neither derived from nor cross-reactive with a causative factor of the disease, wherein the composition is administered under conditions where a causative factor antigen component derived from and/or cross-reactive with the causative factor of the disease is present.
(item Y18) The composition of any of the above-mentioned items, wherein the aforementioned causative factor comprises an exogenous substance for the aforementioned subject.
(item Y19) The composition of any of the above-mentioned items, wherein the aforementioned causative factor of the disease comprises a pathogenic factor of an infectious disease, a causative substance of an allergy, a causative substance of an autoimmune disease, or a toxic substance.
(item Y20) The composition of any of the above-mentioned items, wherein the aforementioned disease is an infectious disease.
(item Y21) The composition of any of the above-mentioned items, wherein the aforementioned subject has immune memory for the aforementioned non-causative factor antigen component.
(item Y22) The composition of any of the above-mentioned items, comprising the aforementioned causative factor antigen component.
(item Y23) The composition of any of the above-mentioned items, which is for the prophylaxis of the aforementioned disease.
(item Y24) The composition of any of the above-mentioned items, wherein the aforementioned composition prevents or treats the aforementioned disease without or with reduced immune hyperreactivity.
(item Y25) The composition of any of the above-mentioned items, wherein the aforementioned composition is administered in a form effective for regulating cellular immunity against the aforementioned causative factor of the disease.
(item Y26) The composition of any of the above-mentioned items, which is characterized in that whether a causative factor antigen component derived from and/or cross-reactive with the causative factor of the target disease is present in the aforementioned subject is confirmed, and when it is not present, the causative factor antigen component is administered together with the aforementioned non-causative factor antigen component.
(item Y27) The composition of any of the above-mentioned items, wherein the aforementioned disease is an infectious disease, an allergy, or an autoimmune disease.
(item Y28) The composition of any of the above-mentioned items, wherein the aforementioned disease is a viral infectious disease or a bacterium infectious disease.
(item Y29) The composition of any of the above-mentioned items, wherein the aforementioned disease is a viral infectious disease.
(item Y30) The composition of any of the above-mentioned items, wherein the aforementioned disease is an infectious disease related to Coronavirus.
(item Y31) The composition of any of the above-mentioned items, wherein the aforementioned causative factor antigen component is a molecule that appears at least partially on the surface of the causative factor of the target disease.
(item Y32) The composition of any of the above-mentioned items, wherein the aforementioned non-causative factor antigen component is a Mycobacterium tuberculosis hot water extract or a part thereof.
(item Y33) The composition of any of the above-mentioned items, wherein the aforementioned non-causative factor antigen component is a hot water extract of Mycobacterium tuberculosis Aoyama B strain or a part thereof.
(item Y34) The composition of any of the above-mentioned items, wherein the aforementioned non-causative factor antigen component is Extract A.
(item Y35) The composition of any of the above-mentioned items, wherein the aforementioned non-causative factor antigen component comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item Y36) The composition of any of the above-mentioned items, wherein the aforementioned non-causative factor antigen component comprises c-di-AMP, c-di-GMP, 3'3'-cGAMP, 2'3'-cGAMP, which are Mycobacterium tuberculosis-derived STING agonists, and/or dsDNA capable of producing 2'3'-cGAMP via cGAS in human cytoplasm.
(item Y37) The composition of any of the above-mentioned items, wherein the aforementioned non-causative factor antigen component is administered two times or more.
(item Y38) The composition of any of the above-mentioned items, which is characterized in that
   1) the aforementioned subject is confirmed to have an immune response to the aforementioned non-causative factor,
   2) the aforementioned composition is administered to the aforementioned subject when the subject has the aforementioned immune response.
(item Y39) The composition of any of the above-mentioned items, wherein the aforementioned immune response is objectively confirmed.
(item Y40) The composition of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y41) The composition of any of the above-mentioned items, wherein the aforementioned immune response is tested by a tuberculin reaction test or an interferon γ release assay.
(item Y42) The composition of any of the above-mentioned items, wherein the aforementioned non-causative factor antigen component or a Mycobacterium tuberculosis hot water extract or a part thereof is pre-administered to the test subject when the aforementioned subject does not have the aforementioned immune response, and thereafter, the aforementioned composition is administered to the subject when the subject is confirmed to have the immune response.
(item Y43) The composition of any of the above-mentioned items, wherein the aforementioned pre-administration is repeated until the aforementioned subject has the aforementioned immune response.
(item Y44) The composition of any of the above-mentioned items, wherein the aforementioned pre-administration is administration before or after the onset of the aforementioned infectious disease.
(item Y45) The composition of any of the above-mentioned items, wherein the aforementioned pre-administration is administration after the onset of the aforementioned infectious disease.
(item Y46) A combination of a causative factor antigen component derived from and/or cross-reactive with a causative factor of a target disease in a subject and a non-causative factor antigen component that is neither derived from nor cross-reactive with the causative factor of the disease.
(item Y47) The combination of any of the above-mentioned items, wherein the aforementioned causative factor antigen component comprises a pathogenic factor of an infectious disease, a causative substance of an allergy, a causative substance of an autoimmune disease, or a toxic substance.
(item Y48) The combination of any of the above-mentioned items, wherein the aforementioned causative factor antigen component is a bacterium, a virus or protozoa, or a part thereof.
(item Y49) The combination of any of the above-mentioned items, wherein the aforementioned causative factor antigen component is a virus or a part thereof.
(item Y50) The combination of any of the above-mentioned items, wherein the aforementioned causative factor antigen component is a virus belonging to the Coronaviridae family or a part thereof.
(item Y51) The combination of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item Y52) The combination of any of the above-mentioned items, wherein the aforementioned causative factor is a virus belonging to the genus Betacoronavirus.
(item Y53) The combination of any of the above-mentioned items, wherein the aforementioned causative factor is a virus selected from the group consisting of HCoV-HKU1, HCoV-OC43, SARS-CoV, MERS-CoV, and SARS-CoV-2.
(item Y54) The combination of any of the above-mentioned items, wherein the aforementioned causative factor is SARS-CoV-2 virus.
(item Y55) The combination of any of the above-mentioned items, wherein the aforementioned non-causative factor antigen component is a Mycobacterium tuberculosis hot water extract or a part thereof.
(item Y56) The combination of any of the above-mentioned items, wherein the aforementioned non-causative factor antigen component is a hot water extract of Mycobacterium tuberculosis Aoyama B strain or a part thereof.
(item Y57) The combination of any of the above-mentioned items, wherein the aforementioned non-causative factor antigen component is a Mycobacterium tuberculosis hot water extract or a part thereof.
(item Y58) The combination of any of the above-mentioned items, wherein the aforementioned non-causative factor antigen component is a hot water extract of Mycobacterium tuberculosis Aoyama B strain or a part thereof.
(item Y59) The combination of any of the above-mentioned items, wherein the aforementioned Mycobacterium tuberculosis hot water extract or a part thereof is Extract A.
(item Y60) The combination of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item Y61) The combination of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item Y62) The combination of any of the above-mentioned items, wherein the aforementioned non-causative factor antigen component is administered two times or more.
(item Y63) The combination of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item Y64) The combination of any of the above-mentioned items, wherein 1) the aforementioned subject is confirmed to have an immune response to the aforementioned non-causative factor, and 2) when the subject has the aforementioned immune response, one or both of the aforementioned combinations is/are administered to the subject.
(item Y65) The combination of any of the above-mentioned items, wherein the aforementioned immune response is objectively confirmed.
(item Y66) The combination of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y67) The combination of any of the above-mentioned items, wherein the aforementioned immune response is tested by a tuberculin reaction test or an interferon γ release assay.
(item Y68) The combination of any of the above-mentioned items, wherein the aforementioned non-causative factor antigen component or a Mycobacterium tuberculosis hot water extract or a part thereof is pre-administered to the test subject when the aforementioned subject does not have the aforementioned immune response, and thereafter, the aforementioned combination is administered to the subject when the subject is confirmed to have the immune response.
(item Y69) The combination of any of the above-mentioned items, wherein the aforementioned pre-administration is repeated until the aforementioned subject has the aforementioned immune response.
(item Y70) The combination of any of the above-mentioned items, wherein the aforementioned pre-administration is administration before or after the onset of the aforementioned infectious disease.
(item Y71) The combination of any of the above-mentioned items, wherein the aforementioned pre-administration is administration after the onset of the aforementioned infectious disease.
(item Y72) A medicament for the prophylaxis or treatment of a target disease in a subject, comprising a combination of a causative factor antigen component derived from and/or cross-reactive with a causative factor of the target disease and a non-causative factor antigen component that is neither derived from nor cross-reactive with the causative factor of the disease.
(item Y73) The medicament of any of the above-mentioned items, further comprising one or more features described in any of the above-mentioned items.
(item Y74) A kit for the prophylaxis or treatment of a target disease in a subject, comprising a causative factor antigen component derived from and/or cross-reactive with a causative factor of the target disease and a non-causative factor antigen component that is neither derived from nor cross-reactive with the causative factor of the disease.
(item Y75) The kit of any of the above-mentioned items, further comprising one or more features described in any of the above-mentioned items.
(item Y76) A method for preventing or treating a disease in a subject, comprising administering an effective amount of a non-causative factor antigen component that is neither derived from nor cross-reactive with the causative factor of the disease to the subject, under conditions where a causative factor antigen component derived from and/or cross-reactive with the causative factor of the disease is present.
(item Y77) The method of any of the above-mentioned items, further comprising one or more features described in any of the above-mentioned items.
(item Y78) A method for preventing or treating a disease in a subject, comprising administering an effective amount of a causative factor antigen component derived from and/or cross-reactive with the causative factor of the disease to the subject, and administering an effective amount of a non-causative factor antigen component that is neither derived from nor cross-reactive with the causative factor of the disease to the target.
(item Y79) The method of any of the above-mentioned items, further comprising one or more features described in any of the above-mentioned items.
(item Y80) A composition comprising a non-causative factor antigen component that is neither derived from nor cross-reactive with an infectious disease, an allergy, and/or an autoimmune disease to be the target, which removes or converts suppression on a regulatory T cell (Treg), wherein a test subject has immune memory for the antigen component, and, in the test subject, an activity of the regulatory T cell (Treg) against a causative factor of the infectious disease, the allergy, and/or the autoimmune disease, or a cell infected with the causative factor or containing the causative factor is suppressed.
(item Y81) The composition of any of the above-mentioned items, wherein the aforementioned Treg is converted to an effector T cell (Teff) for the causative factor of the disease or the cell containing the causative factor of the infectious disease in the aforementioned test subject, by further presence of a causative factor antigen component derived from and/or cross-reactive with the causative factor of the target disease in the aforementioned test subject.
(item Y82) The composition of any of the above-mentioned items, wherein the aforementioned test subject has immune memory for the aforementioned causative factor and/or a factor cross-reactive with the causative factor.
(item Y83) The method of any of the above-mentioned items, further comprising one or more features described in any of the above-mentioned items.
(item Y84) A composition for the prophylaxis or treatment of an infectious disease in a test subject, comprising an antigen component that is specific in the test subject for a component that is different from the causative factor of the infectious disease.
(item Y85) A composition for the prophylaxis or treatment of an infectious disease in a test subject, which is substantially free of a causative factor of the infectious disease or a part thereof and comprises an antigen component from a pathogen of a pre-existing infectious disease different from the infectious disease of the test subject.
(item Y86) A composition for the prophylaxis or treatment of an infectious disease other than tuberculosis, comprising a Mycobacterium tuberculosis extract.
(item Y87) A composition comprising a Mycobacterium tuberculosis extract for regulating immunity enhancement against a causative factor of an infectious disease other than tuberculosis in a subject when the subject is exposed to the causative agent.
(item Y88) A composition for the prophylaxis or treatment of an infectious disease, an allergy, or an autoimmune disease in a test subject, comprising a specific antigen component in the test subject that is neither derived from nor cross-reactive with a causative factor of the infectious disease, the allergy, or the autoimmune disease, wherein
   the antigen component is a protein or a part thereof, or a peptide, and capable of regulating an immune response via CD4-positive T cells,
   the infectious disease, the allergy, or the autoimmune disease comprises those that can be prevented or treated by an immune response via CD4-positive T cells, and wherein
   whether the test subject is capable of regulating a CD4-positive T cell-mediated immune response to the infectious disease, the allergy, or the autoimmune disease is confirmed, and the composition is administered when the test subject is capable of regulating a CD4-positive T cell-mediated immune response to the infectious disease, the allergy, or the autoimmune disease.
(item Y89) A composition for the prophylaxis or treatment of an infectious disease in a test subject, comprising a specific antigen component in the test subject for a component different from a causative factor of the infectious disease, wherein
   the infectious disease comprises a viral infectious disease,
   the antigen component is a protein or a part thereof, or a peptide, and capable of regulating an immune response via CD4-positive T cells,
   the viral infectious disease comprises those that can be prevented or treated by an immune response via CD4-positive T cells, and wherein
   whether the test subject is capable of regulating an antivirus immune response via CD4-positive T cells is confirmed, and when the test subject is capable of regulating an antivirus immune response via CD4-positive T cells, the composition is administered.
(item Y90) The composition of any of the above-mentioned items, further comprising one or more features described in any of the above-mentioned items.
(item Y91) A method for producing or providing in other manner a composition for the prophylaxis or treatment of an infectious disease, an allergy, or an autoimmune disease in a test subject, the method comprising:
   A) a step of specifying a specific antigen component for the test subject that is neither derived from nor cross-reactive with a causative factor of the infectious disease, the allergy, or the autoimmune disease,
   B) a step of specifying whether the test subject has immune memory for the antigen component and selecting one with the immune memory, and
   C) a step of producing or providing in other manner the selected antigen component.
(item Y92) A method for determining whether a specific antigen component for a test subject that is neither derived from nor cross-reactive with a causative factor of an infectious disease, an allergy, or an autoimmune disease can prevent or treat the infectious disease, the allergy, or the autoimmune disease in the test subject, the method comprising:
   B) a step of specifying whether the test subject has immune memory for the antigen component, and specifying that the infectious disease, the allergy, or the autoimmune disease in the test subject can be prevented or treated when the immune memory is present.
(item Y93) A method for the prophylaxis or treatment of an infectious disease, an allergy, or an autoimmune disease, comprising a) a step of obtaining an antigen responsiveness profile of a test subject,
   b) a step of specifying an antigen component or a combination of antigen components from the antigen responsiveness profile, wherein the antigen component or the combination of the antigen components is specified based on the immune responsiveness presented now or in the past by the test subject to the antigen component or the combination of the antigen components, and
   c) a step of administering the antigen component or the combination of the antigen components identified in step b) to the test subject in an amount sufficient to regulate an immune response in the test subject.
(item Y94) The method of any of the above-mentioned items, further comprising one or more features described in any of the above-mentioned items.
(item Y95) An adjuvant for a vaccine against an infectious disease, comprising a Mycobacterium tuberculosis hot water extract or a part thereof.
(item Y96) An adjuvant for a vaccine against an infectious disease, comprising a STING agonist.
(item Y97) An adjuvant for a vaccine against a viral infectious disease, comprising a STING agonist.
(item Y98) The adjuvant of any of the above-mentioned items, wherein the aforementioned Mycobacterium tuberculosis hot water extract or a part thereof is a hot water extract of Mycobacterium tuberculosis Aoyama B strain or a part thereof.
(item Y99) The adjuvant of any of the above-mentioned items, wherein the aforementioned Mycobacterium tuberculosis hot water extract or a part thereof is Extract A.
(item Y100) The adjuvant of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item Y101) The adjuvant of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item Y102) The adjuvant of any of the above-mentioned items, wherein a causative factor of the aforementioned infectious disease is a bacterium, a virus, or a protozoa.
(item Y103) The adjuvant of any of the above-mentioned items, wherein the aforementioned causative factor of the infectious disease is a virus.
(item Y104) The adjuvant of any of the above-mentioned items, wherein the aforementioned causative factor of the infectious disease is a virus belonging to the Coronaviridae family.
(item Y105) The adjuvant of any of the above-mentioned items, wherein the aforementioned causative factor of the infectious disease is a virus belonging to a family selected from the group consisting of Rhinovirus, adenovirus, Coronavirus, RS virus, influenza virus, parainfluenza virus, and Enterovirus.
(item Y106) The adjuvant of any of the above-mentioned items, wherein the aforementioned causative factor of the infectious disease is a virus belonging to the genus Betacoronavirus.
(item Y107) The adjuvant of any of the above-mentioned items, wherein the aforementioned causative factor of the infectious disease is a virus selected from the group consisting of HCoV-HKU1, HCoV-OC43, SARS-CoV, MERS-CoV, and SARS-CoV-2.
(item Y108) The adjuvant of any of the above-mentioned items, wherein the aforementioned causative factor of the infectious disease is a SARS-CoV-2 virus.
(item Y109) The adjuvant of any of the above-mentioned items, further comprising one or more features described in any of claims 64-71.
(item Y110) The adjuvant of any of the above-mentioned items, which converts innate immunity to acquired immunity.
(item Y1A) A method for preventing or treating an infectious disease, an allergy, and/or an autoimmune disease in a subject, comprising administering an effective amount of a non-causative factor antigen component that is neither derived from nor cross-reactive with a causative factor of the infectious disease, the allergy, and/or the autoimmune disease, wherein
   1) the subject is confirmed to have an immune response to the non-causative factor,
   2) the non-causative factor antigen component is administered to the subject when the subject has the immune response.
(item Y2A) A method for preventing or treating an infectious disease in a subject, comprising administering an effective amount of a non-causative factor antigen component that is neither derived from nor cross-reactive with a causative factor of the infectious disease, wherein
   1) the subject is confirmed to have an immune response to the non-causative factor,
   2) the non-causative factor antigen component is administered to the subject when the subject has the immune response.
(item Y3A) A method for preventing or treating an infectious disease other than tuberculosis in a subject, comprising administering an effective amount of a Mycobacterium tuberculosis hot water extract or a part thereof, wherein
   1) the subject is confirmed to have an immune response to tuberculosis,
   2) the non-causative factor antigen component is administered to the subject when the subject has the immune response.
(item Y4A) The method of any of the above-mentioned items, wherein the aforementioned Mycobacterium tuberculosis hot water extract or a part thereof is a hot water extract of Mycobacterium tuberculosis Aoyama B strain.
(item Y5A) The method of any of the above-mentioned items, wherein the aforementioned Mycobacterium tuberculosis hot water extract or a part thereof is Extract A.
(item Y6A) The method of any of the above-mentioned items, which is effective for the production of IL-10.
(item Y7A) The method of any of the above-mentioned items, wherein the aforementioned infectious disease is a viral infectious disease.
(item Y8A) The method of any of the above-mentioned items, wherein the aforementioned infectious disease is a viral infectious disease related to Coronavirus.
(item Y9A) The method of any of the above-mentioned items, wherein the aforementioned immune response is objectively confirmed.
(item Y10A) The method of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y11A) The method of any of the above-mentioned items, wherein the aforementioned immune response is tested by a tuberculin reaction test or an interferon γ release assay.
(item Y12A) The method of any of the above-mentioned items, wherein the aforementioned non-causative factor antigen component or a Mycobacterium tuberculosis hot water extract or a part thereof is pre-administered to the test subject when the aforementioned subject does not have the aforementioned immune response, and thereafter, the aforementioned non-causative factor antigen component is administered to the subject when the subject is confirmed to have the immune response.
(item Y13A) The method of any of the above-mentioned items, wherein the aforementioned pre-administration is repeated until the aforementioned subject has the aforementioned immune response.
(item Y14A) The method of any of the above-mentioned items, wherein the aforementioned pre-administration is administration before or after the onset of the aforementioned infectious disease.
(item Y15A) The method of any of the above-mentioned items, wherein the aforementioned pre-administration is administration after the onset of the aforementioned infectious disease.
(item Y16A) The method of any of the above-mentioned items, wherein the non-causative factor antigen component is administered under conditions where a causative factor antigen component derived from and/or cross-reactive with the aforementioned causative factor is present.
(item Y17A) A method for preventing or treating a target disease in a subject, comprising a non-causative factor antigen component that is neither derived from nor cross-reactive with a causative factor of the disease, wherein the method is administered under conditions where a causative factor antigen component derived from and/or cross-reactive with the causative factor of the disease is present.
(item Y18A) The method of any of the above-mentioned items, wherein the aforementioned causative factor comprises an exogenous substance for the aforementioned subject.
(item Y19A) The method of any of the above-mentioned items, wherein the aforementioned causative factor of the disease comprises a pathogenic factor of an infectious disease, a causative substance of an allergy, a causative substance of an autoimmune disease, or a toxic substance.
(item Y20A) The method of any of the above-mentioned items, wherein the aforementioned disease is an infectious disease.
(item Y21A) The method of any of the above-mentioned items, wherein the aforementioned subject has immune memory for the aforementioned non-causative factor antigen component.
(item Y22A) The method of any of the above-mentioned items, comprising the aforementioned causative factor antigen component.
(item Y23A) The method of any of the above-mentioned items, which is for the prophylaxis of the aforementioned disease.
(item Y24A) The method of any of the above-mentioned items, wherein the aforementioned method prevents or treats the aforementioned disease without or with reduced immune hyperreactivity.
(item Y25A) The method of any of the above-mentioned items, wherein the aforementioned method is administered in a form effective for regulating cellular immunity against the aforementioned causative factor of the disease.
(item Y26A) The method of any of the above-mentioned items, which is characterized in that whether a causative factor antigen component derived from and/or cross-reactive with the causative factor of the target disease is present in the aforementioned subject is confirmed, and when it is not present, the causative factor antigen component is administered together with the aforementioned non-causative factor antigen component.
(item Y27A) The method of any of the above-mentioned items, wherein the aforementioned disease is an infectious disease, an allergy, or an autoimmune disease.
(item Y28A) The method of any of the above-mentioned items, wherein the aforementioned disease is a viral infectious disease or a bacterium infectious disease.
(item Y29A) The method of any of the above-mentioned items, wherein the aforementioned disease is a viral infectious disease.
(item Y30A) The method of any of the above-mentioned items, wherein the aforementioned disease is an infectious disease related to Coronavirus.
(item Y31A) The method of any of the above-mentioned items, wherein the aforementioned causative factor antigen component is a molecule that appears at least partially on the surface of the causative factor of the target disease.
(item Y32A) The method of any of the above-mentioned items, wherein the aforementioned non-causative factor antigen component is a Mycobacterium tuberculosis hot water extract or a part thereof.
(item Y33A) The method of any of the above-mentioned items, wherein the aforementioned non-causative factor antigen component is a hot water extract of Mycobacterium tuberculosis Aoyama B strain or a part thereof.
(item Y34A) The method of any of the above-mentioned items, wherein the aforementioned non-causative factor antigen component is Extract A.
(item Y35A) The method of any of the above-mentioned items, wherein the aforementioned non-causative factor antigen component comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item Y36A) The method of any of the above-mentioned items, wherein the aforementioned non-causative factor antigen component comprises c-di-AMP, c-di-GMP, 3'3'-cGAMP, 2'3'-cGAMP, which are Mycobacterium tuberculosis-derived STING agonists, and/or dsDNA capable of producing 2'3'-cGAMP via cGAS in human cytoplasm.
(item Y37A) The method of any of the above-mentioned items, wherein the aforementioned non-causative factor antigen component is administered two times or more.
(item Y38A) The method of any of the above-mentioned items, wherein
   1) the aforementioned subject is confirmed to have an immune response to the aforementioned non-causative factor,
   2) the aforementioned composition is administered to the aforementioned subject when the subject has the aforementioned immune response.
(item Y39A) The method of any of the above-mentioned items, wherein the aforementioned immune response is objectively confirmed.
(item Y40A) The method of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y41A) The method of any of the above-mentioned items, wherein the aforementioned immune response is tested by a tuberculin reaction test or an interferon γ release assay.
(item Y42A) The method of any of the above-mentioned items, wherein the aforementioned non-causative factor antigen component or a Mycobacterium tuberculosis hot water extract or a part thereof is pre-administered to the test subject when the aforementioned subject does not have the aforementioned immune response, and thereafter, an effective amount of the aforementioned non-causative factor antigen component is administered to the subject when the subject is confirmed to have the immune response.
(item Y43A) The method of any of the above-mentioned items, wherein the aforementioned pre-administration is repeated until the aforementioned subject has the aforementioned immune response.
(item Y44A) The method of any of the above-mentioned items, wherein the aforementioned pre-administration is administration before or after the onset of the aforementioned infectious disease.
(item Y45A) The method of any of the above-mentioned items, wherein the aforementioned pre-administration is administration after the onset of the aforementioned infectious disease.
(item Y46A) A method for preventing or treating a target disease in a subject, comprising administering a combination of a causative factor antigen component derived from and/or cross-reactive with a causative factor of the disease and a non-causative factor antigen component that is neither derived from nor cross-reactive with the causative factor of the disease.
(item Y47A) The method of any of the above-mentioned items, wherein the aforementioned causative factor antigen component comprises a pathogenic factor of an infectious disease, a causative substance of an allergy, a causative substance of an autoimmune disease, or a toxic substance.
(item Y48A) The method of any of the above-mentioned items, wherein the aforementioned causative factor antigen component is a bacterium, a virus or protozoa, or a part thereof.
(item Y49A) The method of any of the above-mentioned items, wherein the aforementioned causative factor antigen component is a virus or a part thereof.
(item Y50A) The method of any of the above-mentioned items, wherein the aforementioned causative factor antigen component is a virus belonging to the Coronaviridae family or a part thereof.
(item Y51A) The method of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item Y52A) The method of any of the above-mentioned items, wherein the aforementioned causative factor is a virus belonging to the genus Betacoronavirus.
(item Y53A) The method of any of the above-mentioned items, wherein the aforementioned causative factor is a virus selected from the group consisting of HCoV-HKU1, HCoV-OC43, SARS-CoV, MERS-CoV, and SARS-CoV-2.
(item Y54A) The method of any of the above-mentioned items, wherein the aforementioned causative factor is SARS-CoV-2 virus.
(item Y55A) The method of any of the above-mentioned items, wherein the aforementioned non-causative factor antigen component is a Mycobacterium tuberculosis hot water extract or a part thereof.
(item Y56A) The method of any of the above-mentioned items, wherein the aforementioned non-causative factor antigen component is a hot water extract of Mycobacterium tuberculosis Aoyama B strain or a part thereof.
(item Y57A) The method of any of the above-mentioned items, wherein the aforementioned non-causative factor antigen component is a Mycobacterium tuberculosis hot water extract or a part thereof.
(item Y58A) The method of any of the above-mentioned items, wherein the aforementioned non-causative factor antigen component is a hot water extract of Mycobacterium tuberculosis Aoyama B strain or a part thereof.
(item Y59A) The method of any of the above-mentioned items, wherein the aforementioned Mycobacterium tuberculosis hot water extract or a part thereof is Extract A.
(item Y60A) The method of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item Y61A) The method of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item Y62A) The method of any of the above-mentioned items, wherein the aforementioned non-causative factor antigen component is administered two times or more.
(item Y63A) The method of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item Y64A) The method of any of the above-mentioned items, wherein 1) the aforementioned subject is confirmed to have an immune response to the aforementioned non-causative factor, and
   2) when the subject has the aforementioned immune response, one or both of the aforementioned combinations is/are administered to the subject.
(item Y65A) The method of any of the above-mentioned items, wherein the aforementioned immune response is objectively confirmed.
(item Y66A) The method of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y67A) The method of any of the above-mentioned items, wherein the aforementioned immune response is tested by a tuberculin reaction test or an interferon γ release assay.
(item Y68A) The method of any of the above-mentioned items, wherein the aforementioned non-causative factor antigen component or a Mycobacterium tuberculosis hot water extract or a part thereof is pre-administered to the test subject when the aforementioned subject does not have the aforementioned immune response, and thereafter, the aforementioned combination is administered to the subject when the subject is confirmed to have the immune response.
(item Y69A) The method of any of the above-mentioned items, wherein the aforementioned pre-administration is repeated until the aforementioned subject has the aforementioned immune response.
(item Y70A) The method of any of the above-mentioned items, wherein the aforementioned pre-administration is administration before or after the onset of the aforementioned infectious disease.
(item Y71A) The method of any of the above-mentioned items, wherein the aforementioned pre-administration is administration after the onset of the aforementioned infectious disease.
(item Y72A) A method for preventing or treating a target disease in a subject, comprising administering an effective amount of a combination of a causative factor antigen component derived from and/or cross-reactive with a causative factor of the target disease and a non-causative factor antigen component that is neither derived from nor cross-reactive with the causative factor of the disease.
(item Y73A) The method of any of the above-mentioned items, further comprising one or more features described in any of the above-mentioned items.
(item Y74A) A method for preventing or treating a target disease in a subject, comprising administering an effective amount of a causative factor antigen component derived from and/or cross-reactive with a causative factor of the target disease and a non-causative factor antigen component that is neither derived from nor cross-reactive with the causative factor of the disease.
(item Y75A) The method of any of the above-mentioned items, further comprising one or more features described in any of the above-mentioned items.
(item Y76A) A method for preventing or treating a disease in a subject, comprising administering an effective amount of a non-causative factor antigen component that is neither derived from nor cross-reactive with the causative factor of the disease to the subject, under conditions where a causative factor antigen component derived from and/or cross-reactive with the causative factor of the disease is present.
(item Y77A) The method of any of the above-mentioned items, further comprising one or more features described in any of the above-mentioned items.
(item Y78A) A method for preventing or treating a disease in a subject, comprising administering an effective amount of a causative factor antigen component derived from and/or cross-reactive with the causative factor of the disease to the subject, and administering an effective amount of a non-causative factor antigen component that is neither derived from nor cross-reactive with the causative factor of the disease to the target.
(item Y79A) The method of any of the above-mentioned items, further comprising one or more features described in any of the above-mentioned items.
(item Y80A) A method for removing or converting suppression on a regulatory T cell (Treg), comprising administering an effective amount of a non-causative factor antigen component that is neither derived from nor cross-reactive with an infectious disease, an allergy, and/or an autoimmune disease to be the target, wherein a test subject has immune memory for the antigen component, and, in the test subject, an activity of the regulatory T cell (Treg) against a causative factor of the infectious disease, the allergy, and/or the autoimmune disease, or a cell infected with the causative factor or containing the causative factor is suppressed.
(item Y81A) The method of any of the above-mentioned items, wherein the aforementioned Treg is converted to an effector T cell (Teff) for the causative factor of the disease or the cell containing the causative factor of the infectious disease in the aforementioned test subject, by further presence of a causative factor antigen component derived from and/or cross-reactive with the causative factor of the target disease in the aforementioned test subject.
(item Y82A) The method of any of the above-mentioned items, wherein the aforementioned test subject has immune memory for the aforementioned causative factor and/or a factor cross-reactive with the causative factor.
(item Y83A) The method of any of the above-mentioned items, further comprising one or more features described in any of the above-mentioned items.
(item Y84A) A method for preventing or treating an infectious disease in a test subject, comprising administering an effective amount of an antigen component that is specific in the test subject for a component that is different from the causative factor of the infectious disease.
(item Y85A) A method for preventing or treating an infectious disease in a test subject, which is substantially free of administration of a causative factor of the infectious disease or a part thereof and comprises administering an effective amount of an antigen component from a pathogen of a pre-existing infectious disease different from the infectious disease of the test subject.
(item Y86A) A method for preventing or treating an infectious disease other than tuberculosis, comprising administering an effective amount of a Mycobacterium tuberculosis extract.
(item Y87A) A method for regulating immunity enhancement against a causative factor of an infectious disease other than tuberculosis in a subject when the subject is exposed to the causative agent, comprising administering an effective amount of a Mycobacterium tuberculosis extract.
(item Y88A) A method for preventing or treating an infectious disease, an allergy, or an autoimmune disease in a test subject, comprising administering an effective amount of a specific antigen component in the test subject that is neither derived from nor cross-reactive with a causative factor of the infectious disease, the allergy, or the autoimmune disease, wherein
   the antigen component is a protein or a part thereof, or a peptide, and capable of regulating an immune response via CD4-positive T cells,
   the infectious disease, the allergy, or the autoimmune disease comprises those that can be prevented or treated by an immune response via CD4-positive T cells, and wherein
   whether the test subject is capable of regulating a CD4-positive T cell-mediated immune response to the infectious disease, the allergy, or the autoimmune disease is confirmed, and the antigen component is administered when the test subject is capable of regulating a CD4-positive T cell-mediated immune response to the infectious disease, the allergy, or the autoimmune disease.
(item Y89A) A method for preventing or treating an infectious disease in a test subject, comprising administering an effective amount of a specific antigen component in the test subject for a component different from a causative factor of the infectious disease, wherein
   the infectious disease comprises a viral infectious disease,
   the antigen component is a protein or a part thereof, or a peptide, and capable of regulating an immune response via CD4-positive T cells,
   the viral infectious disease comprises those that can be prevented or treated by an immune response via CD4-positive T cells, and wherein
   whether the test subject is capable of regulating an antivirus immune response via CD4-positive T cells is confirmed, and an effective amount of the antigen component is administered when the test subject is capable of regulating an antivirus immune response via CD4-positive T cells.
(item Y90A) The method of any of the above-mentioned items, further comprising one or more features described in any of the above-mentioned items.
(item Y91A) A method for producing or providing in other manner a composition for the prophylaxis or treatment of an infectious disease, an allergy, or an autoimmune disease in a test subject, the method comprising:
   A) a step of specifying a specific antigen component for the test subject that is neither derived from nor cross-reactive with a causative factor of the infectious disease, the allergy, or the autoimmune disease,
   B) a step of specifying whether the test subject has immune memory for the antigen component and selecting one with the immune memory, and
   C) a step of producing or providing in other manner the selected antigen component.
(item Y92A) A method for determining whether a specific antigen component for a test subject that is neither derived from nor cross-reactive with a causative factor of an infectious disease, an allergy, or an autoimmune disease can prevent or treat the infectious disease, the allergy, or the autoimmune disease in the test subject, the method comprising:
   B) a step of specifying whether the test subject has immune memory for the antigen component, and specifying that the infectious disease, the allergy, or the autoimmune disease in the test subject can be prevented or treated when the immune memory is present.
(item Y93A) A method for the prophylaxis or treatment of an infectious disease, an allergy, or an autoimmune disease, comprising a) a step of obtaining an antigen responsiveness profile of a test subject,
   b) a step of specifying an antigen component or a combination of antigen components from the antigen responsiveness profile, wherein the antigen component or the combination of the antigen components is specified based on the immune responsiveness presented now or in the past by the test subject to the antigen component or the combination of the antigen components, and
   c) a step of administering the antigen component or the combination of the antigen components identified in step b) to the test subject in an amount sufficient to regulate an immune response in the test subject.
(item Y94A) The method of any of the above-mentioned items, further comprising one or more features described in any of the above-mentioned items.
(item Y95A) An adjuvant for a vaccine against an infectious disease, comprising a Mycobacterium tuberculosis hot water extract or a part thereof.
(item Y96A) An adjuvant for a vaccine against an infectious disease, comprising a STING agonist.
(item Y97A) An adjuvant for a vaccine against a viral infectious disease, comprising a STING agonist.
(item Y98A) The adjuvant of any of the above-mentioned items, wherein the aforementioned Mycobacterium tuberculosis hot water extract or a part thereof is a hot water extract of Mycobacterium tuberculosis Aoyama B strain or a part thereof.
(item Y99A) The adjuvant of any of the above-mentioned items, wherein the aforementioned Mycobacterium tuberculosis hot water extract or a part thereof is Extract A.
(item Y100A) The adjuvant of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item Y101A) The adjuvant of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item Y102A) The adjuvant of any of the above-mentioned items, wherein a causative factor of the aforementioned infectious disease is a bacterium, a virus, or a protozoa.
(item Y103A) The adjuvant of any of the above-mentioned items, wherein the aforementioned causative factor of the infectious disease is a virus.
(item Y104A) The adjuvant of any of the above-mentioned items, wherein the aforementioned causative factor of the infectious disease is a virus belonging to the Coronaviridae family.
(item Y105A) The adjuvant of any of the above-mentioned items, wherein the aforementioned causative factor of the infectious disease is a virus belonging to a family selected from the group consisting of Rhinovirus, adenovirus, Coronavirus, RS virus, influenza virus, parainfluenza virus, and Enterovirus.
(item Y106A) The adjuvant of any of the above-mentioned items, wherein the aforementioned causative factor of the infectious disease is a virus belonging to the genus Betacoronavirus.
(item Y107A) The adjuvant of any of the above-mentioned items, wherein the aforementioned causative factor of the infectious disease is a virus selected from the group consisting of HCoV-HKU1, HCoV-OC43, SARS-CoV, MERS-CoV, and SARS-CoV-2.
(item Y108A) The adjuvant of any of the above-mentioned items, wherein the aforementioned causative factor of the infectious disease is a SARS-CoV-2 virus.
(item Y109A) The adjuvant of any of the above-mentioned items, further comprising one or more features described in any of the above-mentioned items.
(item Y110A) The adjuvant of any of the above-mentioned items, which converts innate immunity to acquired immunity.
(item Y1B) A non-causative factor antigen component for the prophylaxis or treatment of an infectious disease, an allergy, and/or an autoimmune disease in a subject, wherein the non-causative factor antigen component is neither derived from nor cross-reactive with a causative factor of the infectious disease, the allergy, and/or the autoimmune disease, and is characterized in that
   1) the subject is confirmed to have an immune response to the non-causative factor,
   2) the antigen component is administered to the subject when the subject has the immune response.
(item Y2B) A non-causative factor antigen component for the prophylaxis or treatment of an infectious disease in a subject, wherein the non-causative factor antigen component is neither derived from nor cross-reactive with a causative factor of the infectious disease, and is characterized in that
   1) the subject is confirmed to have an immune response to the non-causative factor,
   2) the antigen component is administered to the subject when the subject has the immune response.
(item Y3B) A Mycobacterium tuberculosis hot water extract or a part thereof for the prophylaxis or treatment of an infectious disease other than tuberculosis in a subject, wherein the Mycobacterium tuberculosis hot water extract or a part thereof is characterized in that
   1) the subject is confirmed to have an immune response to tuberculosis,
   2) the composition is administered to the subject when the subject has the immune response.
(item Y4B) The antigen component, or the Mycobacterium tuberculosis hot water extract or a part thereof of any of the above-mentioned items, wherein the aforementioned Mycobacterium tuberculosis hot water extract or a part thereof is a hot water extract of Mycobacterium tuberculosis Aoyama B strain.
(item Y5B) The antigen component, or the Mycobacterium tuberculosis hot water extract or a part thereof of any of the above-mentioned items, wherein the aforementioned Mycobacterium tuberculosis hot water extract or a part thereof is Extract A.
(item Y6B) The antigen component, or the Mycobacterium tuberculosis hot water extract or a part thereof of any of the above-mentioned items, wherein the aforementioned antigen component, or a Mycobacterium tuberculosis hot water extract or a part thereof is administered in an amount effective for the production of IL-10.
(item Y7B) The composition of any of the above-mentioned items, wherein the aforementioned infectious disease is a viral infectious disease.
(item Y8B) The antigen component, or the Mycobacterium tuberculosis hot water extract or a part thereof of any of the above-mentioned items, wherein the aforementioned infectious disease is a viral infectious disease related to Coronavirus.
(item Y9B) The antigen component, or the Mycobacterium tuberculosis hot water extract or a part thereof of any of the above-mentioned items, wherein the aforementioned immune response is objectively confirmed.
(item Y10B) The antigen component, or the Mycobacterium tuberculosis hot water extract or a part thereof of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y11B) The antigen component, or the Mycobacterium tuberculosis hot water extract or a part thereof of any of the above-mentioned items, wherein the aforementioned immune response is tested by a tuberculin reaction test or an interferon γ release assay.
(item Y12B) The antigen component, or the Mycobacterium tuberculosis hot water extract or a part thereof of any of the above-mentioned items, wherein the aforementioned antigen component, or a Mycobacterium tuberculosis hot water extract or a part thereof is pre-administered to the test subject when the aforementioned subject does not have the aforementioned immune response, and thereafter, the aforementioned antigen component, or a Mycobacterium tuberculosis hot water extract or a part thereof is administered to the subject when the subject is confirmed to have the immune response.
(item Y13B) The antigen component, or the Mycobacterium tuberculosis hot water extract or a part thereof of any of the above-mentioned items, wherein the aforementioned pre-administration is repeated until the aforementioned subject has the aforementioned immune response.
(item Y14B) The antigen component, or the Mycobacterium tuberculosis hot water extract or a part thereof of any of the above-mentioned items, wherein the aforementioned pre-administration is administration before or after the onset of the aforementioned infectious disease.
(item Y15B) The antigen component, or the Mycobacterium tuberculosis hot water extract or a part thereof of any of the above-mentioned items, wherein the aforementioned pre-administration is administration after the onset of the aforementioned infectious disease.
(item Y16B) The antigen component, or the Mycobacterium tuberculosis hot water extract or a part thereof of any of the above-mentioned items, which is administered under conditions where a causative factor antigen component derived from and/or cross-reactive with the aforementioned causative factor is present.
(item Y17B) A non-causative factor antigen component that is neither derived from nor cross-reactive with a causative factor of a target disease in a subject for the prophylaxis or treatment of the disease, which is administered under conditions where a causative factor antigen component derived from and/or cross-reactive with the causative factor of the disease is present.
(item Y18B) The antigen component of any of the above-mentioned items, wherein the aforementioned causative factor comprises an exogenous substance for the aforementioned subject.
(item Y19B) The antigen component of any of the above-mentioned items, wherein the aforementioned causative factor of the disease comprises a pathogenic factor of an infectious disease, a causative substance of an allergy, a causative substance of an autoimmune disease, or a toxic substance.
(item Y20B) The antigen component of any of the above-mentioned items, wherein the aforementioned disease is an infectious disease.
(item Y21B) The antigen component of any of the above-mentioned items, wherein the aforementioned subject has immune memory for the aforementioned non-causative factor antigen component.
(item Y22B) The antigen component of any of the above-mentioned items, comprising the aforementioned causative factor antigen component.
(item Y23B) The antigen component of any of the above-mentioned items, which is for the prophylaxis of the aforementioned disease.
(item Y24B) The antigen component of any of the above-mentioned items, wherein the aforementioned antigen component prevents or treats the aforementioned disease without or with reduced immune hyperreactivity.
(item Y25B) The antigen component of any of the above-mentioned items, wherein the aforementioned antigen component is administered in a form effective for regulating cellular immunity against the aforementioned causative factor of the disease.
(item Y26B) The antigen component of any of the above-mentioned items, which is characterized in that whether a causative factor antigen component derived from and/or cross-reactive with the causative factor of the target disease is present in the aforementioned subject is confirmed, and when it is not present, the causative factor antigen component is administered together with the aforementioned non-causative factor antigen component.
(item Y27B) The antigen component of any of the above-mentioned items, wherein the aforementioned disease is an infectious disease, an allergy, or an autoimmune disease.
(item Y28B) The antigen component of any of the above-mentioned items, wherein the aforementioned disease is a viral infectious disease or a bacterium infectious disease.
(item Y29B) The antigen component of any of the above-mentioned items, wherein the aforementioned disease is a viral infectious disease.
(item Y30B) The antigen component of any of the above-mentioned items, wherein the aforementioned disease is an infectious disease related to Coronavirus.
(item Y31B) The antigen component of any of the above-mentioned items, wherein the aforementioned causative factor antigen component is a molecule that appears at least partially on the surface of the causative factor of the target disease.
(item Y32B) The antigen component of any of the above-mentioned items, wherein the aforementioned non-causative factor antigen component is a Mycobacterium tuberculosis hot water extract or a part thereof.
(item Y33B) The antigen component of any of the above-mentioned items, wherein the aforementioned non-causative factor antigen component is a hot water extract of Mycobacterium tuberculosis Aoyama B strain or a part thereof.
(item Y34B) The antigen component of any of the above-mentioned items, wherein the aforementioned non-causative factor antigen component is Extract A.
(item Y35B) The antigen component of any of the above-mentioned items, wherein the aforementioned non-causative factor antigen component comprises a Mycobacterium tuberculosis-derived STING agonist, LpqH, Y1269, PPD, CMV pp65 overlapping peptide, Mtb-associated antigen, a component capable of biosynthesizing STING agonist in human cells, or a NOD2 agonist.
(item Y36B) The antigen component of any of the above-mentioned items, wherein the aforementioned non-causative factor antigen component comprises c-di-AMP, c-di-GMP, 3'3'-cGAMP, 2'3'-cGAMP, which are Mycobacterium tuberculosis-derived STING agonists, and/or dsDNA capable of producing 2'3'-cGAMP via cGAS in human cytoplasm.
(item Y37B) The antigen component of any of the above-mentioned items, wherein the aforementioned non-causative factor antigen component is administered two times or more.
(item Y38B) The antigen component of any of the above-mentioned items, which is characterized in that
   1) the aforementioned subject is confirmed to have an immune response to the aforementioned non-causative factor,
   2) the aforementioned composition is administered to the aforementioned subject when the subject has the aforementioned immune response.
(item Y39B) The antigen component of any of the above-mentioned items, wherein the aforementioned immune response is objectively confirmed.
(item Y40B) The antigen component of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y41B) The antigen component of any of the above-mentioned items, wherein the aforementioned immune response is tested by a tuberculin reaction test or an interferon γ release assay.
(item Y42B) The antigen component of any of the above-mentioned items, wherein the aforementioned non-causative factor antigen component or a Mycobacterium tuberculosis hot water extract or a part thereof is pre-administered to the test subject when the aforementioned subject does not have the aforementioned immune response, and thereafter, the aforementioned composition is administered to the subject when the subject is confirmed to have the immune response.
(item Y43B) The antigen component of any of the above-mentioned items, wherein the aforementioned pre-administration is repeated until the aforementioned subject has the aforementioned immune response.
(item Y44B) The antigen component of any of the above-mentioned items, wherein the aforementioned pre-administration is administration before or after the onset of the aforementioned infectious disease.
(item Y45B) The antigen component of any of the above-mentioned items, wherein the aforementioned pre-administration is administration after the onset of the aforementioned infectious disease.
(item Y46B) A combination of a causative factor antigen component derived from and/or cross-reactive with a causative factor of a target disease in a subject and a non-causative factor antigen component that is neither derived from nor cross-reactive with the causative factor of the disease.
(item Y47B) The combination of any of the above-mentioned items, wherein the aforementioned causative factor antigen component comprises a pathogenic factor of an infectious disease, a causative substance of an allergy, a causative substance of an autoimmune disease, or a toxic substance.
(item Y48B) The combination of any of the above-mentioned items, wherein the aforementioned causative factor antigen component is a bacterium, a virus or protozoa, or a part thereof.
(item Y49B) The combination of any of the above-mentioned items, wherein the aforementioned causative factor antigen component is a virus or a part thereof.
(item Y50B) The combination of any of the above-mentioned items, wherein the aforementioned causative factor antigen component is a virus belonging to the Coronaviridae family or a part thereof.
(item Y51B) The combination of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item Y52B) The combination of any of the above-mentioned items, wherein the aforementioned causative factor is a virus belonging to the genus Betacoronavirus.
(item Y53B) The combination of any of the above-mentioned items, wherein the aforementioned causative factor is a virus selected from the group consisting of HCoV-HKU1, HCoV-OC43, SARS-CoV, MERS-CoV, and SARS-CoV-2.
(item Y54B) The combination of any of the above-mentioned items, wherein the aforementioned causative factor is SARS-CoV-2 virus.
(item Y55B) The combination of any of the above-mentioned items, wherein the aforementioned non-causative factor antigen component is a Mycobacterium tuberculosis hot water extract or a part thereof.
(item Y56B) The combination of any of the above-mentioned items, wherein the aforementioned non-causative factor antigen component is a hot water extract of Mycobacterium tuberculosis Aoyama B strain or a part thereof.
(item Y57B) The combination of any of the above-mentioned items, wherein the aforementioned non-causative factor antigen component is a Mycobacterium tuberculosis hot water extract or a part thereof.
(item Y58B) The combination of any of the above-mentioned items, wherein the aforementioned non-causative factor antigen component is a hot water extract of Mycobacterium tuberculosis Aoyama B strain or a part thereof.
(item Y59B) The combination of any of the above-mentioned items, wherein the aforementioned Mycobacterium tuberculosis hot water extract or a part thereof is Extract A.
(item Y60B) The combination of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item Y61B) The combination of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item Y62B) The combination of any of the above-mentioned items, wherein the aforementioned non-causative factor antigen component is administered two times or more.
(item Y63B) The combination of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item Y64B) The combination of any of the above-mentioned items, wherein 1) the aforementioned subject is confirmed to have an immune response to the aforementioned non-causative factor, and
   2) when the subject has the aforementioned immune response, one or both of the aforementioned combinations is/are administered to the subject.
(item Y65B) The combination of any of the above-mentioned items, wherein the aforementioned immune response is objectively confirmed.
(item Y66B) The combination of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y67B) The combination of any of the above-mentioned items, wherein the aforementioned immune response is tested by a tuberculin reaction test or an interferon γ release assay.
(item Y68B) The combination of any of the above-mentioned items, wherein the aforementioned non-causative factor antigen component or Mycobacterium tuberculosis hot water extract or a part thereof is pre-administered to the test subject when the aforementioned subject does not have the aforementioned immune response, and thereafter, the aforementioned combination is administered to the subject when the subject is confirmed to have the immune response.
(item Y69B) The combination of any of the above-mentioned items, wherein the aforementioned pre-administration is repeated until the aforementioned subject has the aforementioned immune response.
(item Y70B) The combination of any of the above-mentioned items, wherein the aforementioned pre-administration is administration before or after the onset of the aforementioned infectious disease.
(item Y71B) The combination of any of the above-mentioned items, wherein the aforementioned pre-administration is administration after the onset of the aforementioned infectious disease.
(item Y72B) A medicament for the prophylaxis or treatment of a target disease in a subject, comprising a combination of a causative factor antigen component derived from and/or cross-reactive with a causative factor of the target disease and a non-causative factor antigen component that is neither derived from nor cross-reactive with the causative factor of the disease.
(item Y73B) The medicament of any of the above-mentioned items, further comprising one or more features described in any of the above-mentioned items.
(item Y74B) A kit for the prophylaxis or treatment of a target disease in a subject, comprising a causative factor antigen component derived from and/or cross-reactive with a causative factor of the target disease and a non-causative factor antigen component that is neither derived from nor cross-reactive with the causative factor of the disease.
(item Y75B) The kit of any of the above-mentioned items, further comprising one or more features described in any of the above-mentioned items.
(item Y76B) A non-causative factor antigen component for the prophylaxis or treatment of a disease in a subject, which is neither derived from nor cross-reactive with a causative factor of the disease, and administered to the subject under conditions where a causative factor antigen component derived from and/or cross-reactive with the causative factor of the disease is present.
(item Y77B) The antigen component of any of the above-mentioned items, further comprising one or more features described in any of the above-mentioned items.
(item Y78B) A combination for the prophylaxis or treatment of a disease in a subject, comprising a causative factor antigen component derived from and/or cross-reactive with the causative factor of the disease, and a non-causative factor antigen component that is neither derived from nor cross-reactive with the causative factor of the disease.
(item Y79B) The combination of any of the above-mentioned items, further comprising one or more features described in any of the above-mentioned items.
(item Y80B) A non-causative factor antigen component that is neither derived from nor cross-reactive with an infectious disease, an allergy, and/or an autoimmune disease to be the target, which removes or converts suppression on a regulatory T cell (Treg), wherein a test subject has immune memory for the antigen component, and, in the test subject, an activity of the regulatory T cell (Treg) against a causative factor of the infectious disease, the allergy, and/or the autoimmune disease, or a cell infected with the causative factor or containing the causative factor is suppressed.
(item Y81B) The antigen component of any of the above-mentioned items, wherein the aforementioned Treg is converted to an effector T cell (Teff) for the causative factor of the disease or the cell containing the causative factor of the infectious disease in the aforementioned test subject, by further presence of a causative factor antigen component derived from and/or cross-reactive with the causative factor of the target disease in the aforementioned test subject.
(item Y82B) The antigen component of any of the above-mentioned items, wherein the aforementioned test subject has immune memory for the aforementioned causative factor and/or a factor cross-reactive with the causative factor.
(item Y83B) The antigen component of any of the above-mentioned items, further comprising one or more features described in any of the above-mentioned items.
(item Y84B) An antigen component for the prophylaxis or treatment of an infectious disease in a test subject, which is specific in the test subject for a component that is different from the causative factor of the infectious disease.
(item Y85B) An antigen component for the prophylaxis or treatment of an infectious disease in a test subject, which is from a pathogen of a pre-existing infectious disease different from the infectious disease of the test subject and is substantially free of a causative factor of the infectious disease or a part thereof.
(item Y86B) A Mycobacterium tuberculosis extract for the prophylaxis or treatment of an infectious disease other than tuberculosis.
(item Y87B) A Mycobacterium tuberculosis extract for regulating immunity enhancement against a causative factor of an infectious disease other than tuberculosis in a subject when the subject is exposed to the causative agent.
(item Y88B) An antigen component for the prophylaxis or treatment of an infectious disease, an allergy, or an autoimmune disease in a test subject, that is neither derived from nor cross-reactive with a causative factor of the infectious disease, the allergy, or the autoimmune disease, wherein
   the antigen component is a protein or a part thereof, or a peptide, and capable of regulating an immune response via CD4-positive T cells,
   the infectious disease, the allergy, or the autoimmune disease comprises those that can be prevented or treated by an immune response via CD4-positive T cells, and wherein
   whether the test subject is capable of regulating a CD4-positive T cell-mediated immune response to the infectious disease, the allergy, or the autoimmune disease is confirmed, and the antigen component is administered when the test subject is capable of regulating a CD4-positive T cell-mediated immune response to the infectious disease, the allergy, or the autoimmune disease.
(item Y89B) An antigen component for the prophylaxis or treatment of an infectious disease in a test subject, which is a specific antigen component in the test subject for a component different from a causative factor of the infectious disease, wherein
   the infectious disease comprises a viral infectious disease,
   the antigen component is a protein or a part thereof, or a peptide, and capable of regulating an immune response via CD4-positive T cells,
   the viral infectious disease comprises those that can be prevented or treated by an immune response via CD4-positive T cells, and wherein
   whether the test subject is capable of regulating an antivirus immune response via CD4-positive T cells is confirmed, and when the test subject is capable of regulating an antivirus immune response via CD4-positive T cells, the antigen component is administered.
(item Y90B) The antigen component of any of the above-mentioned items, further comprising one or more features described in any of the above-mentioned items.
(item Y91B) A method for producing or providing in other manner a composition for the prophylaxis or treatment of an infectious disease, an allergy, or an autoimmune disease in a test subject, the method comprising:
   A) a step of specifying a specific antigen component for the test subject that is neither derived from nor cross-reactive with a causative factor of the infectious disease, the allergy, or the autoimmune disease,
   B) a step of specifying whether the test subject has immune memory for the antigen component and selecting one with the immune memory, and
   C) a step of producing or providing in other manner the selected antigen component.
(item Y92B) A method for determining whether a specific antigen component for a test subject that is neither derived from nor cross-reactive with a causative factor of an infectious disease, an allergy, or an autoimmune disease can prevent or treat the infectious disease, the allergy, or the autoimmune disease in the test subject, the method comprising:
   B) a step of specifying whether the test subject has immune memory for the antigen component, and specifying that the infectious disease, the allergy, or the autoimmune disease in the test subject can be prevented or treated when the immune memory is present.
(item Y93B) An antigen component for use in a method for the prophylaxis or treatment of an infectious disease, an allergy, or an autoimmune disease, wherein the method comprises a) a step of obtaining an antigen responsiveness profile of a test subject,
   b) a step of specifying an antigen component or a combination of antigen components from the antigen responsiveness profile, wherein the antigen component or the combination of the antigen components is specified based on the immune responsiveness presented now or in the past by the test subject to the antigen component or the combination of the antigen components, and
   c) a step of administering the antigen component or the combination of the antigen components identified in step b) to the test subject in an amount sufficient to regulate an immune response in the test subject.
(item Y94B) The antigen component of any of the above-mentioned items, further comprising one or more features described in any of the above-mentioned items.
(item Y95B) An adjuvant for a vaccine against an infectious disease, comprising a Mycobacterium tuberculosis hot water extract or a part thereof.
(item Y96B) An adjuvant for a vaccine against an infectious disease, comprising a STING agonist.
(item Y97B) An adjuvant for a vaccine against a viral infectious disease, comprising a STING agonist.
(item Y98B) The adjuvant of any of the above-mentioned items, wherein the aforementioned Mycobacterium tuberculosis hot water extract or a part thereof is a hot water extract of Mycobacterium tuberculosis Aoyama B strain or a part thereof. (item Y99B) The adjuvant of any of the above-mentioned items, wherein the aforementioned Mycobacterium tuberculosis hot water extract or a part thereof is Extract A.
(item Y100B) The adjuvant of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item Y101B) The adjuvant of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item Y102B) The adjuvant of any of the above-mentioned items, wherein a causative factor of the aforementioned infectious disease is a bacterium, a virus, or a protozoa.
(item Y103B) The adjuvant of any of the above-mentioned items, wherein the aforementioned causative factor of the infectious disease is a virus.
(item Y104B) The adjuvant of any of the above-mentioned items, wherein the aforementioned causative factor of the infectious disease is a virus belonging to the Coronaviridae family.
(item Y105B) The adjuvant of any of the above-mentioned items, wherein the aforementioned causative factor of the infectious disease is a virus belonging to a family selected from the group consisting of Rhinovirus, adenovirus, Coronavirus, RS virus, influenza virus, parainfluenza virus, and Enterovirus. (item Y106B) The adjuvant of any of the above-mentioned items, wherein the aforementioned causative factor of the infectious disease is a virus belonging to the genus Betacoronavirus. (item Y107B) The adjuvant of any of the above-mentioned items, wherein the aforementioned causative factor of the infectious disease is a virus selected from the group consisting of HCoV-HKU1, HCoV-OC43, SARS-CoV, MERS-CoV, and SARS-CoV-2.
(item Y108B) The adjuvant of any of the above-mentioned items, wherein the aforementioned causative factor of the infectious disease is a SARS-CoV-2 virus.
(item Y109B) The adjuvant of any of the above-mentioned items, further comprising one or more features described in any of claims 64-71.
(item Y110B) The adjuvant of any of the above-mentioned items, which converts innate immunity to acquired immunity.
(item Y1C) Use of a non-causative factor antigen component in the production of a medicament for the prophylaxis or treatment of an infectious disease, an allergy, and/or an autoimmune disease in a subject, wherein the non-causative factor antigen component is neither derived from nor cross-reactive with a causative factor of the infectious disease, the allergy, and/or the autoimmune disease, and is characterized in that
   1) the subject is confirmed to have an immune response to the non-causative factor, and
   2) the antigen component is administered to the subject when the subject has the immune response.
(item Y2C) Use of a non-causative factor antigen component in the production of a medicament for the prophylaxis or treatment of an infectious disease in a subject, wherein the non-causative factor antigen component is neither derived from nor cross-reactive with a causative factor of the infectious disease, and is characterized in that
   1) the subject is confirmed to have an immune response to the non-causative factor, and
   2) the antigen component is administered to the subject when the subject has the immune response.
(item Y3C) Use of a Mycobacterium tuberculosis hot water extract or a part thereof for the prophylaxis or treatment of an infectious disease other than tuberculosis in a subject, wherein the Mycobacterium tuberculosis hot water extract or a part thereof is characterized in that
   1) the subject is confirmed to have an immune response to tuberculosis, and
   2) the Mycobacterium tuberculosis hot water extract or a part thereof is administered to the subject when the subject has the immune response.
(item Y4C) The use of any of the above-mentioned items, wherein the aforementioned Mycobacterium tuberculosis hot water extract or a part thereof is a hot water extract of Mycobacterium tuberculosis Aoyama B strain.
(item Y5C) The use of any of the above-mentioned items, wherein the aforementioned Mycobacterium tuberculosis hot water extract or a part thereof is Extract A.
(item Y6C) The use of any of the above-mentioned items, wherein the aforementioned composition is administered in an amount effective for the production of IL-10.
(item Y7C) The use of any of the above-mentioned items, wherein the aforementioned infectious disease is a viral infectious disease.
(item Y8C) The use of any of the above-mentioned items, wherein the aforementioned infectious disease is a viral infectious disease related to Coronavirus.
(item Y9C) The use of any of the above-mentioned items, wherein the aforementioned immune response is objectively confirmed.
(item Y10C) The use of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y11C) The use of any of the above-mentioned items, wherein the aforementioned immune response is tested by a tuberculin reaction test or an interferon γ release assay.
(item Y12C) The use of any of the above-mentioned items, wherein the aforementioned antigen component, or a Mycobacterium tuberculosis hot water extract or a part thereof is pre-administered to the test subject when the aforementioned subject does not have the aforementioned immune response, and thereafter, the aforementioned antigen component, or a Mycobacterium tuberculosis hot water extract or a part thereof is administered to the subject when the subject is confirmed to have the immune response.
(item Y13C) The use of any of the above-mentioned items, wherein the aforementioned pre-administration is repeated until the aforementioned subject has the aforementioned immune response.
(item Y14C) The use of any of the above-mentioned items, wherein the aforementioned pre-administration is administration before or after the onset of the aforementioned infectious disease.
(item Y15C) The use of any of the above-mentioned items, wherein the aforementioned pre-administration is administration after the onset of the aforementioned infectious disease.
(item Y16C) The use of any of the above-mentioned items, which is administered under conditions where a causative factor antigen component derived from and/or cross-reactive with the aforementioned causative factor is present.
(item Y17C) Use of a non-causative factor antigen component in the production of a medicament for the prophylaxis or treatment of a disease, wherein the antigen component is neither derived from nor cross-reactive with a causative factor of the disease, which is administered under conditions where a causative factor antigen component derived from and/or cross-reactive with the causative factor of the disease is present.
(item Y18C) The use of any of the above-mentioned items, wherein the aforementioned causative factor comprises an exogenous substance for the aforementioned subject.
(item Y19C) The use of any of the above-mentioned items, wherein the aforementioned causative factor of the disease comprises a pathogenic factor of an infectious disease, a causative substance of an allergy, a causative substance of an autoimmune disease, or a toxic substance.
(item Y20C) The use of any of the above-mentioned items, wherein the aforementioned disease is an infectious disease.
(item Y21C) The use of any of the above-mentioned items, wherein the aforementioned subject has immune memory for the aforementioned non-causative factor antigen component.
(item Y22C) The use of any of the above-mentioned items, comprising the aforementioned causative factor antigen component.
(item Y23C) The use of any of the above-mentioned items, which is for the prophylaxis of the aforementioned disease.
(item Y24C) The use of any of the above-mentioned items, wherein the aforementioned antigen component prevents or treats the aforementioned disease without or with reduced immune hyperreactivity.
(item Y25C) The use of any of the above-mentioned items, wherein the aforementioned antigen component is administered in a form effective for regulating cellular immunity against the aforementioned causative factor of the disease.
(item Y26C) The use of any of the above-mentioned items, which is characterized in that whether a causative factor antigen component derived from and/or cross-reactive with the causative factor of the target disease is present in the aforementioned subject is confirmed, and when it is not present, the causative factor antigen component is administered together with the aforementioned non-causative factor antigen component.
(item Y27C) The use of any of the above-mentioned items, wherein the aforementioned disease is an infectious disease, an allergy, or an autoimmune disease.
(item Y28C) The use of any of the above-mentioned items, wherein the aforementioned disease is a viral infectious disease or a bacterium infectious disease.
(item Y29C) The use of any of the above-mentioned items, wherein the aforementioned disease is a viral infectious disease.
(item Y30C) The use of any of the above-mentioned items, wherein the aforementioned disease is an infectious disease related to Coronavirus.
(item Y31C) The use of any of the above-mentioned items, wherein the aforementioned causative factor antigen component is a molecule that appears at least partially on the surface of the causative factor of the target disease.
(item Y32C) The use of any of the above-mentioned items, wherein the aforementioned non-causative factor antigen component is a Mycobacterium tuberculosis hot water extract or a part thereof.
(item Y33C) The use of any of the above-mentioned items, wherein the aforementioned non-causative factor antigen component is a hot water extract of Mycobacterium tuberculosis Aoyama B strain or a part thereof.
(item Y34C) The use of any of the above-mentioned items, wherein the aforementioned non-causative factor antigen component is Extract A.
(item Y35C) The use of any of the above-mentioned items, wherein the aforementioned non-causative factor antigen component comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item Y36C) The use of any of the above-mentioned items, wherein the aforementioned non-causative factor antigen component comprises c-di-AMP, c-di-GMP, 3'3'-cGAMP, 2'3'-cGAMP, which are Mycobacterium tuberculosis-derived STING agonists, and/or dsDNA capable of producing 2'3'-cGAMP via cGAS in human cytoplasm.
(item Y37C) The use of any of the above-mentioned items, wherein the aforementioned non-causative factor antigen component is administered two times or more.
(item Y38C) The use of any of the above-mentioned items, which is characterized in that
   1) the aforementioned subject is confirmed to have an immune response to the aforementioned non-causative factor, and
   2) the aforementioned antigen component is administered to the aforementioned subject when the subject has the aforementioned immune response.
(item Y39C) The use of any of the above-mentioned items, wherein the aforementioned immune response is objectively confirmed.
(item Y40C) The use of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y41C) The use of any of the above-mentioned items, wherein the aforementioned immune response is tested by a tuberculin reaction test or an interferon γ release assay.
(item Y42C) The use of any of the above-mentioned items, wherein the aforementioned non-causative factor antigen component or a Mycobacterium tuberculosis hot water extract or a part thereof is pre-administered to the test subject when the aforementioned subject does not have the aforementioned immune response, and thereafter, the aforementioned composition is administered to the subject when the subject is confirmed to have the immune response.
(item Y43C) The use of any of the above-mentioned items, wherein the aforementioned pre-administration is repeated until the aforementioned subject has the aforementioned immune response.
(item Y44C) The use of any of the above-mentioned items, wherein the aforementioned pre-administration is administration before or after the onset of the aforementioned infectious disease.
(item Y45C) The use of any of the above-mentioned items, wherein the aforementioned pre-administration is administration after the onset of the aforementioned infectious disease.
(item Y46C) Use of a combination of a causative factor antigen component derived from and/or cross-reactive with a causative factor of a target disease in a subject and a non-causative factor antigen component that is neither derived from nor cross-reactive with the causative factor of the disease in the production of a medicament for the prophylaxis or treatment of the disease.
(item Y47C) The use of any of the above-mentioned items, wherein the aforementioned causative factor antigen component comprises a pathogenic factor of an infectious disease, a causative substance of an allergy, a causative substance of an autoimmune disease, or a toxic substance.
(item Y48C) The use of any of the above-mentioned items, wherein the aforementioned causative factor antigen component is a bacterium, a virus or protozoa, or a part thereof.
(item Y49C) The use of any of the above-mentioned items, wherein the aforementioned causative factor antigen component is a virus or a part thereof.
(item Y50C) The use of any of the above-mentioned items, wherein the aforementioned causative factor antigen component is a virus belonging to the Coronaviridae family or a part thereof.
(item Y51C) The use of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item Y52C) The use of any of the above-mentioned items, wherein the aforementioned causative factor is a virus belonging to the genus Betacoronavirus.
(item Y53C) The use of any of the above-mentioned items, wherein the aforementioned causative factor is a virus selected from the group consisting of HCoV-HKU1, HCoV-OC43, SARS-CoV, MERS-CoV, and SARS-CoV-2.
(item Y54C) The use of any of the above-mentioned items, wherein the aforementioned causative factor is SARS-CoV-2 virus.
(item Y55C) The use of any of the above-mentioned items, wherein the aforementioned non-causative factor antigen component is a Mycobacterium tuberculosis hot water extract or a part thereof.
(item Y56C) The use of any of the above-mentioned items, wherein the aforementioned non-causative factor antigen component is a hot water extract of Mycobacterium tuberculosis Aoyama B strain or a part thereof.
(item Y57C) The use of any of the above-mentioned items, wherein the aforementioned non-causative factor antigen component is a Mycobacterium tuberculosis hot water extract or a part thereof.
(item Y58C) The use of any of the above-mentioned items, wherein the aforementioned non-causative factor antigen component is a hot water extract of Mycobacterium tuberculosis Aoyama B strain or a part thereof.
(item Y59C) The use of any of the above-mentioned items, wherein the aforementioned Mycobacterium tuberculosis hot water extract or a part thereof is Extract A.
(item Y60C) The use of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item Y61C) The use of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item Y62C) The use of any of the above-mentioned items, wherein the aforementioned non-causative factor antigen component is administered two times or more.
(item Y63C) The use of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item Y64C) The use of any of the above-mentioned items, wherein 1) the aforementioned subject is confirmed to have an immune response to the aforementioned non-causative factor, and
   2) when the subject has the aforementioned immune response, one or both of the aforementioned combinations is/are administered to the subject.
(item Y65C) The use of any of the above-mentioned items, wherein the aforementioned immune response is objectively confirmed.
(item Y66C) The use of any of the above-mentioned items, wherein the aforementioned immune response is objectively confirmed by a tuberculin reaction test or interferon γ release assay, or by objective information contained in the mother and child health handbook or its equivalent, clinical records, or other medical information.
(item Y67C) The use of any of the above-mentioned items, wherein the aforementioned immune response is tested by a tuberculin reaction test or an interferon γ release assay.
(item Y68C) The use of any of the above-mentioned items, wherein the aforementioned non-causative factor antigen component or Mycobacterium tuberculosis hot water extract or a part thereof is pre-administered to the test subject when the aforementioned subject does not have the aforementioned immune response, and thereafter, the aforementioned combination is administered to the subject when the subject is confirmed to have the immune response.
(item Y69C) The use of any of the above-mentioned items, wherein the aforementioned pre-administration is repeated until the aforementioned subject has the aforementioned immune response.
(item Y70C) The use of any of the above-mentioned items, wherein the aforementioned pre-administration is administration before or after the onset of the aforementioned infectious disease.
(item Y71C) The use of any of the above-mentioned items, wherein the aforementioned pre-administration is administration after the onset of the aforementioned infectious disease.
(item Y72C) Use of a combination of a causative factor antigen component derived from and/or cross-reactive with a causative factor of a target disease and a non-causative factor antigen component that is neither derived from nor cross-reactive with the causative factor of the disease, in the production of a medicament for the prophylaxis or treatment of the disease.
(item Y73C) The use of any of the above-mentioned items, further comprising one or more features described in any of the above-mentioned items.
(item Y74C) Use of a causative factor antigen component derived from and/or cross-reactive with a causative factor of a target disease and a non-causative factor antigen component that is neither derived from nor cross-reactive with the causative factor of the disease, in the production of a kit for the prophylaxis or treatment of the disease.
(item Y75C) The use of any of the above-mentioned items, further comprising one or more features described in any of the above-mentioned items.
(item Y76C) Use of a non-causative factor antigen component which is neither derived from nor cross-reactive with a causative factor of a disease in a subject, in the production of a medicament for the prophylaxis or treatment of the disease, wherein an effective amount of the non-causative factor antigen component is administered to the subject under conditions where a causative factor antigen component derived from and/or cross-reactive with the causative factor of the disease is present.
(item Y77C) The use of any of the above-mentioned items, further comprising one or more features described in any of the above-mentioned items.
(item Y78C) Use in a method for the prophylaxis or treatment of a disease in a subject, which is use of a causative factor antigen component derived from and/or cross-reactive with the causative factor of the disease, and a non-causative factor antigen component that is neither derived from nor cross-reactive with the causative factor of the disease.
(item Y79C) The use of any of the above-mentioned items, further comprising one or more features described in any of the above-mentioned items.
(item Y80C) Use of a non-causative factor antigen component that is neither derived from nor cross-reactive with an infectious disease, an allergy, and/or an autoimmune disease to be the target, in the production of a medicament for removing or converting suppression on a regulatory T cell (Treg), wherein a test subject has immune memory for the antigen component, and, in the test subject, an activity of the regulatory T cell (Treg) against a causative factor of the infectious disease, the allergy, and/or the autoimmune disease, or a cell infected with the causative factor or containing the causative factor is suppressed.
(item Y81C) The use of any of the above-mentioned items, wherein the aforementioned Treg is converted to an effector T cell (Teff) for the causative factor of the disease or the cell containing the causative factor of the infectious disease in the aforementioned test subject, by further presence of a causative factor antigen component derived from and/or cross-reactive with the causative factor of the target disease in the aforementioned test subject.
(item Y82C) The use of any of the above-mentioned items, wherein the aforementioned test subject has immune memory for the aforementioned causative factor and/or a factor cross-reactive with the causative factor.
(item Y83C) The use of any of the above-mentioned items, further comprising one or more features described in any of the above-mentioned items.
(item Y84C) Use of an antigen component in the production of a medicament for the prophylaxis or treatment of an infectious disease in a test subject, which is specific in the test subject for a component that is different from the causative factor of the infectious disease.
(item Y85C) Use of an antigen component in the production of a medicament for the prophylaxis or treatment of an infectious disease in a test subject, which is from a pathogen of a pre-existing infectious disease different from the infectious disease of the test subject and is substantially free of a causative factor of the infectious disease or a part thereof.
(item Y86C) Use of a Mycobacterium tuberculosis extract in the production of a medicament for the prophylaxis or treatment of an infectious disease other than tuberculosis.
(item Y87C) Use of a Mycobacterium tuberculosis extract in the production of a medicament for regulating immunity enhancement against a causative factor of an infectious disease other than tuberculosis in a subject when the subject is exposed to the causative agent.
(item Y88C) Use of an antigen component in the production of a medicament for the prophylaxis or treatment of an infectious disease, an allergy, or an autoimmune disease in a test subject, that is neither derived from nor cross-reactive with a causative factor of the infectious disease, the allergy, or the autoimmune disease, wherein
   the antigen component is a protein or a part thereof, or a peptide, and capable of regulating an immune response via CD4-positive T cells,
   the infectious disease, the allergy, or the autoimmune disease comprises those that can be prevented or treated by an immune response via CD4-positive T cells, and wherein
   whether the test subject is capable of regulating a CD4-positive T cell-mediated immune response to the infectious disease, the allergy, or the autoimmune disease is confirmed, and the antigen component is administered when the test subject is capable of regulating a CD4-positive T cell-mediated immune response to the infectious disease, the allergy, or the autoimmune disease.
(item Y89C) Use of an antigen component in the production of a medicament for the prophylaxis or treatment of an infectious disease in a test subject, which is a specific antigen component in the test subject for a component different from a causative factor of the infectious disease, wherein
   the infectious disease comprises a viral infectious disease,
   the antigen component is a protein or a part thereof, or a peptide, and capable of regulating an immune response via CD4-positive T cells,
   the viral infectious disease comprises those that can be prevented or treated by an immune response via CD4-positive T cells, and wherein
   whether the test subject is capable of regulating an antivirus immune response via CD4-positive T cells is confirmed, and when the test subject is capable of regulating an antivirus immune response via CD4-positive T cells, the antigen component is administered.
(item Y90C) Use of any of the above-mentioned items, further comprising one or more features described in any of the above-mentioned items.
(item Y91C) A method for producing or providing in other manner a composition for the prophylaxis or treatment of an infectious disease, an allergy, or an autoimmune disease in a test subject, the method comprising:
   A) a step of specifying a specific antigen component for the test subject that is neither derived from nor cross-reactive with a causative factor of the infectious disease, the allergy, or the autoimmune disease,
   B) a step of specifying whether the test subject has immune memory for the antigen component and selecting one with the immune memory, and
   C) a step of producing or providing in other manner the selected antigen component.
(item Y92C) A method for determining whether a specific antigen component for a test subject that is neither derived from nor cross-reactive with a causative factor of an infectious disease, an allergy, or an autoimmune disease can prevent or treat the infectious disease, the allergy, or the autoimmune disease in the test subject, the method comprising:
   B) a step of specifying whether the test subject has immune memory for the antigen component, and specifying that the infectious disease, the allergy, or the autoimmune disease in the test subject can be prevented or treated when the immune memory is present.
(item Y93C) Use of an antigen component or a combination of antigen components in the production of a medicament for use in a method for the prophylaxis or treatment of an infectious disease, an allergy, or an autoimmune disease, wherein the method comprises
   a) a step of obtaining an antigen responsiveness profile of a test subject,
   b) a step of specifying an antigen component or a combination of antigen components from the antigen responsiveness profile, wherein the antigen component or the combination of the antigen components is specified based on the immune responsiveness presented now or in the past by the test subject to the antigen component or the combination of the antigen components,
   c) a step of administering the antigen component or the combination of the antigen components identified in step b) to the test subject in an amount sufficient to regulate an immune response in the test subject.
(item Y94C) The use of any of the above-mentioned items, further comprising one or more features described in any of the above-mentioned items.
(item Y95C) Use of a Mycobacterium tuberculosis hot water extract or a part thereof in the production of an adjuvant for a vaccine against an infectious disease.
(item Y96C) Use of a STING agonist in the production of an adjuvant for a vaccine against an infectious disease.
(item Y97C) Use of a STING agonist in the production of an adjuvant for a vaccine against a viral infectious disease.
(item Y98C) The use of any of the above-mentioned items, wherein the aforementioned Mycobacterium tuberculosis hot water extract or a part thereof is a hot water extract of Mycobacterium tuberculosis Aoyama B strain or a part thereof.
(item Y99C) The use of any of the above-mentioned items, wherein the aforementioned Mycobacterium tuberculosis hot water extract or a part thereof is Extract A.
(item Y100C) The use of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item Y101C) The use of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item Y102C) The use of any of the above-mentioned items, wherein a causative factor of the aforementioned infectious disease is a bacterium, a virus, or a protozoa.
(item Y103C) The use of any of the above-mentioned items, wherein the aforementioned causative factor of the infectious disease is a virus.
(item Y104C) The use of any of the above-mentioned items, wherein the aforementioned causative factor of the infectious disease is a virus belonging to the Coronaviridae family.
(item Y105C) The use of any of the above-mentioned items, wherein the aforementioned causative factor of the infectious disease is a virus belonging to a family selected from the group consisting of Rhinovirus, adenovirus, Coronavirus, RS virus, influenza virus, parainfluenza virus, and Enterovirus.
(item Y106C) The use of any of the above-mentioned items, wherein the aforementioned causative factor of the infectious disease is a virus belonging to the genus Betacoronavirus.
(item Y107C) The use of any of the above-mentioned items, wherein the aforementioned causative factor of the infectious disease is a virus selected from the group consisting of HCoV-HKU1, HCoV-OC43, SARS-CoV, MERS-CoV, and SARS-CoV-2.
(item Y108C) The use of any of the above-mentioned items, wherein the aforementioned causative factor of the infectious disease is a SARS-CoV-2 virus.
(item Y109C) The use of any of the above-mentioned items, further comprising one or more features described in any of the above-mentioned items.
(item Y110C) The use of any of the above-mentioned items, which converts innate immunity to acquired immunity.
(item X1) A composition for the prophylaxis or treatment of a target disease in a subject, comprising a non-causative factor antigen component that is neither derived from nor cross-reactive with a causative factor of the disease, wherein the composition is administered under conditions where a causative factor antigen component derived from and/or cross-reactive with the causative factor of the disease is present.
(item X2) The composition of any of the above-mentioned items, wherein the aforementioned causative factor comprises an exogenous substance for the aforementioned subject.
(item X3) The composition of any of the above-mentioned items, wherein the aforementioned causative factor of the disease comprises a pathogenic factor of an infectious disease, a causative substance of an allergy, a causative substance of an autoimmune disease, or a toxic substance.
(item X4) The composition of any of the above-mentioned items, wherein the aforementioned disease is an infectious disease.
(item X5) The composition of any of the above-mentioned items, wherein the aforementioned subject has immune memory for the aforementioned non-causative factor antigen component.
(item X6) The composition of any of the above-mentioned items, comprising the aforementioned causative factor antigen component.
(item X7) The composition of any of the above-mentioned items, which is for the prophylaxis of the aforementioned disease.
(item X8) The composition of any of the above-mentioned items, wherein the aforementioned composition prevents or treats the aforementioned disease without or with reduced immune hyperreactivity.
(item X9) The composition of any of the above-mentioned items, wherein the aforementioned composition is administered in a form effective for regulating cellular immunity against the aforementioned causative factor of the disease.
(item X10) The composition of any of the above-mentioned items, which is characterized in that whether a causative factor antigen component derived from and/or cross-reactive with the causative factor of the target disease is present in the aforementioned subject is confirmed, and when it is not present, the causative factor antigen component is administered together with the aforementioned non-causative factor antigen component.
(item X11) The composition of any of the above-mentioned items, wherein the aforementioned disease is an infectious disease, an allergy, or an autoimmune disease.
(item X12) The composition of any of the above-mentioned items, wherein the aforementioned disease is a viral infectious disease or a bacterium infectious disease.
(item X13) The composition of any of the above-mentioned items, wherein the aforementioned disease is a viral infectious disease.
(item X14) The composition of any of the above-mentioned items, wherein the aforementioned disease is an infectious disease related to Coronavirus.
(item X15) The composition of any of the above-mentioned items, wherein the aforementioned causative factor antigen component is a molecule that appears at least partially on the surface of the causative factor of the target disease.
(item X15A) The composition of any of the above-mentioned items, wherein the aforementioned non-causative factor antigen component is a Mycobacterium tuberculosis hot water extract or a part thereof.
(item X15B) The composition of any of the above-mentioned items, wherein the aforementioned non-causative factor antigen component is a hot water extract of Mycobacterium tuberculosis Aoyama B strain or a part thereof.
(item X15C) The composition of any of the above-mentioned items, wherein the aforementioned hot water extract of Mycobacterium tuberculosis or a part thereof is Extract A.
(item X15D) The composition of any of the above-mentioned items, wherein the aforementioned hot water extract of Mycobacterium tuberculosis or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item X15E) The composition of any of the above-mentioned items, wherein the aforementioned hot water extract of Mycobacterium tuberculosis Aoyama B strain or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X15F) The composition of any of the above-mentioned items, wherein the aforementioned non-causative factor antigen component is administered two times or more.
(item X16) A combination of a causative factor antigen component derived from and/or cross-reactive with a causative factor of a target disease in a subject and a non-causative factor antigen component that is neither derived from nor cross-reactive with the causative factor of the disease.
(item X17) The combination of any of the above-mentioned items, wherein the aforementioned causative factor antigen component comprises a pathogenic factor of an infectious disease, a causative substance of an allergy, a causative substance of an autoimmune disease, or a toxic substance.
(item X18) The combination of any of the above-mentioned items, wherein the aforementioned causative factor antigen component is a bacterium, a virus or protozoa, or a part thereof.
(item X19) The combination of any of the above-mentioned items, wherein the aforementioned causative factor antigen component is a virus or a part thereof.
(item X20) The combination of any of the above-mentioned items, wherein the aforementioned causative factor antigen component is a virus belonging to the Coronaviridae family or a part thereof.
(item X21) The combination of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X22) The combination of any of the above-mentioned items, wherein the aforementioned causative factor is a virus belonging to the genus Betacoronavirus.
(item X23) The combination of any of the above-mentioned items, wherein the aforementioned causative factor is a virus selected from the group consisting of HCoV-HKU1, HCoV-OC43, SARS-CoV, MERS-CoV, and SARS-CoV-2.
(item X24) The combination of any of the above-mentioned items, wherein the aforementioned causative factor is SARS-CoV-2 virus.
(item X25) The combination of any of the above-mentioned items, wherein the aforementioned non-causative factor antigen component is a Mycobacterium tuberculosis hot water extract or a part thereof.
(item X26) The combination of any of the above-mentioned items, wherein the aforementioned non-causative factor antigen component is a hot water extract of Mycobacterium tuberculosis Aoyama B strain or a part thereof.
(item X26A) The combination of any of the above-mentioned items, wherein the aforementioned non-causative factor antigen component is a Mycobacterium tuberculosis hot water extract or a part thereof.
(item X26B) The combination of any of the above-mentioned items, wherein the aforementioned non-causative factor antigen component is a hot water extract of Mycobacterium tuberculosis Aoyama B strain or a part thereof.
(item X26C) The combination of any of the above-mentioned items, wherein the aforementioned Mycobacterium tuberculosis hot water extract or a part thereof is Extract A.
(item X26D) The combination of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, STING agonist, or a NOD2 agonist.
(item X26E) The combination of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X26F) The combination of any of the above-mentioned items, wherein the aforementioned non-causative factor antigen component is administered two times or more.
(item X26G) The combination of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X27) A medicament for the prophylaxis or treatment of a target disease in a subject, comprising a combination of a causative factor antigen component derived from and/or cross-reactive with a causative factor of the target disease and a non-causative factor antigen component that is neither derived from nor cross-reactive with the causative factor of the disease.
(item X27A) The medicament of any of the above-mentioned items, wherein the aforementioned causative factor comprises an exogenous substance for the aforementioned subject.
(item X27B) The medicament of any of the above-mentioned items, wherein the aforementioned causative factor of the disease comprises a pathogenic factor of an infectious disease, a causative substance of an allergy, a causative substance of an autoimmune disease, or a toxic substance.
(item X27C) The medicament of any of the above-mentioned items, wherein the aforementioned disease is an infectious disease.
(item X27D) The medicament of any of the above-mentioned items, wherein the aforementioned subject has immune memory for the aforementioned non-causative factor antigen component.
(item X27E) The medicament of any of the above-mentioned items, which is for the prophylaxis of the aforementioned disease.
(item X27F) The medicament of any of the above-mentioned items, wherein the aforementioned antigen component prevents or treats the aforementioned disease without or with reduced immune hyperreactivity.
(item X27G) The medicament of any of the above-mentioned items, wherein the aforementioned antigen component is administered in a form effective for regulating cellular immunity against the aforementioned causative factor of the disease.
(item X27H) The medicament of any of the above-mentioned items, wherein the aforementioned disease is an infectious disease, an allergy, or an autoimmune disease.
(item X27I) The medicament of any of the above-mentioned items, wherein the aforementioned disease is a viral infectious disease or a bacterium infectious disease.
(item X27J) The medicament of any of the above-mentioned items, wherein the aforementioned disease is a viral infectious disease.
(item X27K) The medicament of any of the above-mentioned items, wherein the aforementioned disease is an infectious disease related to Coronavirus.
(item X27L) The medicament of any of the above-mentioned items, wherein the aforementioned causative factor antigen component is a molecule that appears at least partially on a membrane surface.
(item X27AA) The medicament of any of the above-mentioned items, wherein the aforementioned non-causative factor antigen component is a Mycobacterium tuberculosis hot water extract or a part thereof.
(item X27AB) The medicament of any of the above-mentioned items, wherein the aforementioned non-causative factor antigen component is a hot water extract of Mycobacterium tuberculosis Aoyama B strain or a part thereof.
(item X27AC) The medicament of any of the above-mentioned items, wherein the aforementioned hot water extract of Mycobacterium tuberculosis or a part thereof is Extract A.
(item X27AD) The medicament of any of the above-mentioned items, wherein the aforementioned hot water extract of Mycobacterium tuberculosis or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item X27AE) The medicament of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X27AF) The medicament of any of the above-mentioned items, wherein the aforementioned non-causative factor antigen component is administered two times or more.
(item X27AG) The medicament of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X28) A kit for the prophylaxis or treatment of a target disease in a subject, comprising a causative factor antigen component derived from and/or cross-reactive with a causative factor of the target disease and a non-causative factor antigen component that is neither derived from nor cross-reactive with the causative factor of the disease.
(item X28A) The kit of any of the above-mentioned items, wherein the aforementioned causative factor comprises an exogenous substance for the aforementioned subject.
(item X28B) The kit of any of the above-mentioned items, wherein the aforementioned causative factor of the disease comprises a pathogenic factor of an infectious disease, a causative substance of an allergy, a causative substance of an autoimmune disease, or a toxic substance.
(item X28C) The kit of any of the above-mentioned items, wherein the aforementioned disease is an infectious disease.
(item X28D) The kit of any of the above-mentioned items, wherein the aforementioned subject has immune memory for the aforementioned non-causative factor antigen component.
(item X28E) The kit of any of the above-mentioned items, which is for the prophylaxis or treatment of the aforementioned disease.
(item X28F) The kit of any of the above-mentioned items, wherein the aforementioned antigen component prevents or treats the aforementioned disease without or with reduced immune hyperreactivity.
(item X28G) The kit of any of the above-mentioned items, wherein the aforementioned antigen component is administered in a form effective for regulating cellular immunity against the aforementioned causative factor of the disease.
(item X28H) The kit of any of the above-mentioned items, wherein the aforementioned disease is an infectious disease, an allergy, or an autoimmune disease.
(item X28I) The kit of any of the above-mentioned items, wherein the aforementioned disease is a viral infectious disease or a bacterium infectious disease.
(item X28J) The kit of any of the above-mentioned items, wherein the aforementioned disease is a viral infectious disease.
(item X28K) The kit of any of the above-mentioned items, wherein the aforementioned disease is an infectious disease related to Coronavirus.
(item X28L) The kit of any of the above-mentioned items, wherein the aforementioned causative factor antigen component is a molecule that appears at least partially on a membrane surface.
(item X28AA) The kit of any of the above-mentioned items, wherein the aforementioned non-causative factor antigen component is a Mycobacterium tuberculosis hot water extract or a part thereof.
(item X28AB) The kit of any of the above-mentioned items, wherein the aforementioned non-causative factor antigen component is a hot water extract of Mycobacterium tuberculosis Aoyama B strain or a part thereof.
(item X28AC) The kit of any of the above-mentioned items, wherein the aforementioned hot water extract of Mycobacterium tuberculosis or a part thereof is Extract A.
(item X28AD) The kit of any of the above-mentioned items, wherein the aforementioned hot water extract of Mycobacterium tuberculosis or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item X28AE) The kit of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X28AF) The kit of any of the above-mentioned items, wherein the aforementioned non-causative factor antigen component is administered two times or more.
(item X28AG) The kit of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X29) A method for preventing or treating a disease in a subject, comprising administering an effective amount of a non-causative factor antigen component that is neither derived from nor cross-reactive with the causative factor of the disease to the subject, under conditions where a causative factor antigen component derived from and/or cross-reactive with the causative factor of the disease is present.
(item X30) A method for preventing or treating a disease in a subject, comprising administering an effective amount of a causative factor antigen component derived from and/or cross-reactive with the causative factor of the disease to the subject, and administering an effective amount of a non-causative factor antigen component that is neither derived from nor cross-reactive with the causative factor of the disease to the target.
(item X30A) The method of any of the above-mentioned items, wherein the aforementioned causative factor comprises an exogenous substance for the aforementioned subject.
(item X30B) The method of any of the above-mentioned items, wherein the aforementioned causative factor of the disease comprises a pathogenic factor of an infectious disease, a causative substance of an allergy, a causative substance of an autoimmune disease, or a toxic substance.
(item X30C) The method of any of the above-mentioned items, wherein the aforementioned disease is an infectious disease.
(item X30D) The method of any of the above-mentioned items, wherein the aforementioned subject has immune memory for the aforementioned non-causative factor antigen component.
(item X30E) The method of any of the above-mentioned items, which is for the prophylaxis of the aforementioned disease.
(item X30F) The method of any of the above-mentioned items, which is for the prophylaxis or treatment of the aforementioned disease without or with reduced immune hyperreactivity.
(item X30G) The method of any of the above-mentioned items, wherein the aforementioned non-causative factor antigen component is administered in a form effective for regulating cellular immunity against the aforementioned causative factor of the disease.
(item X30H) The method of any of the above-mentioned items, wherein the aforementioned disease is an infectious disease, an allergy, or an autoimmune disease.
(item X30I) The method of any of the above-mentioned items, wherein the aforementioned disease is a viral infectious disease or a bacterium infectious disease.
(item X30J) The method of any of the above-mentioned items, wherein the aforementioned disease is a viral infectious disease.
(item X30K) The method of any of the above-mentioned items, wherein the aforementioned disease is an infectious disease related to Coronavirus.
(item X30L) The method of any of the above-mentioned items, wherein the aforementioned causative factor antigen component is a molecule that appears at least partially on a membrane surface.
(item X30AA) The method of any of the above-mentioned items, wherein the aforementioned non-causative factor antigen component is a Mycobacterium tuberculosis hot water extract or a part thereof.
(item X30AB) The method of any of the above-mentioned items, wherein the aforementioned non-causative factor antigen component is a hot water extract of Mycobacterium tuberculosis Aoyama B strain or a part thereof.
(item X30AC) The method of any of the above-mentioned items, wherein the aforementioned Mycobacterium tuberculosis hot water extract or a part thereof is Extract A.
(item X30AD) The method of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, STING agonist, or a NOD2 agonist.
(item X30AE) The method of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X30AF) The method of any of the above-mentioned items, wherein the aforementioned non-causative factor antigen component is administered two times or more.
(item X30AG) The method of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X31) A composition comprising a non-causative factor antigen component that is neither derived from nor cross-reactive with an infectious disease, an allergy, and/or an autoimmune disease to be the target, which removes or converts suppression on a regulatory T cell (Treg), wherein a test subject has immune memory for the antigen component, and, in the test subject, an activity of the regulatory T cell (Treg) against a causative factor of the infectious disease, the allergy, and/or the autoimmune disease, or a cell infected with the causative factor or containing the causative factor is suppressed.
(item X32) The composition of any of the above-mentioned items, wherein the aforementioned Treg is converted to an effector T cell (Teff) for the causative factor of the disease (infectious disease, allergy, and/or autoimmune disease) or the cell containing the causative factor of the disease in the aforementioned test subject, by further presence of a causative factor antigen component derived from and/or cross-reactive with the causative factor of the target disease in the aforementioned test subject.
(item X33) The composition of any of the above-mentioned items, wherein the aforementioned test subject has immune memory for the aforementioned causative factor and/or a factor cross-reactive with the causative factor.
(item X33A) The composition of any of the above-mentioned items, wherein removal or conversion of the suppression on the aforementioned Treg imparts the ability to kill a causative factor of the aforementioned infectious disease, allergy, and/or autoimmune disease or an immunostimulatory action a cell infected with the causative factor or containing the causative factor, or an immunostimulatory action on a causative factor of the aforementioned infectious disease, allergy and/or autoimmune disease or a cell infected with the causative factor or containing the causative factor.
(item X33B) The composition of any of the above-mentioned items, wherein the aforementioned causative factor comprises at least one selected from the group consisting of malaria, yellow fever virus, smallpox virus, smallpox vaccination, measles/rubella, polio, epidemic parotitis (mumps)/MUMPS, rotaviral infectious disease, varicella (possum), yellow fever, Ebola, West Nile fever, hib infection, pneumococcul infection, pertussis, Japanese encephalitis, meningococcal infection, Salmonella infection, pathogenic Escherichia coli, Toxoplasma, Zika virus, herpes virus type 1, EBV/Epstein-Barr virus (herpes virus type 4), CMV/cytomegalovirus (herpes virus type 5), influenza, MARS, rabies, and diphtheria.
(item X33C) The composition of any of the above-mentioned items, wherein the aforementioned causative factor is a virus.
(item X33D) The composition of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X33E) The composition of any of the above-mentioned items, wherein the aforementioned causative factor is a virus belonging to the genus Betacoronavirus.
(item X33F) The composition of any of the above-mentioned items, wherein the aforementioned causative factor is a virus selected from the group consisting of HCoV-HKU1, HCoV-OC43, SARS-CoV, MERS-CoV, and SARS-CoV-2.
(item X33G) The composition of any of the above-mentioned items, wherein the aforementioned causative factor is SARS-CoV-2 virus.
(item X33H) The composition of any of the above-mentioned items, wherein the aforementioned antigen component comprises a protein, a part thereof, or a peptide.
(item X33I) The composition of any of the above-mentioned items, wherein the aforementioned antigen component comprises an antigen selected from the group consisting of a pathogen of an infectious disease or a part thereof, an antigen related to a medical history, and an antigen related to a vaccination history.
(item X33J) The composition of any of the above-mentioned items, wherein the aforementioned antigen component comprises an antigen from a pathogen of a pre-existing infectious disease.
(item X33K) The composition of any of the above-mentioned items, wherein the aforementioned antigen component comprises one capable of regulating an immune response via CD4-positive T cells.
(item X33L) The composition of any of the above-mentioned items, wherein the aforementioned antigen component comprises a human type Mycobacterium tuberculosis hot water extract.
(item X33M) The composition of any of the above-mentioned items, wherein the aforementioned test subject has a history of BCG vaccination, a history of tuberculosis infection, or an antigen responsiveness to Mycobacterium tuberculosis, and the aforementioned antigen component comprises a human type Mycobacterium tuberculosis hot water extract.
(item X33N) The composition of any of the above-mentioned items, wherein the aforementioned Treg is CD4-positive.
(item X330) The composition of any of the above-mentioned items, wherein the aforementioned Treg removes suppression of activation of killer T cells specific for the aforementioned causative factor of the infectious disease.
(item X33P) The composition of any of the above-mentioned items, which prevents the aforementioned infectious disease.
(item X33Q) The composition of any of the above-mentioned items, for removing suppression of activation of a killer cell that kills a cell infected with the aforementioned causative factor.
(item X33R) The composition of any of the above-mentioned items, which is used in a method comprising examining whether the aforementioned antigen component has immune memory of Treg in the test subject, and administering the antigen component to the test subject when the test subject has immune memory for the antigen component.
(item X33S) The composition of any of the above-mentioned items, wherein whether the aforementioned test subject is capable of regulating an antivirus immune response via CD4-positive T cells is confirmed, and when the test subject is capable of regulating an antivirus immune response via CD4-positive T cells, the aforementioned composition is administered.
(item X33AA) The composition of any of the above-mentioned items, wherein the aforementioned non-causative factor antigen component is a Mycobacterium tuberculosis hot water extract or a part thereof.
(item X33AB) The composition of any of the above-mentioned items, wherein the aforementioned non-causative factor antigen component is a hot water extract of Mycobacterium tuberculosis Aoyama B strain or a part thereof.
(item X33AC) The composition of any of the above-mentioned items, wherein the aforementioned hot water extract of Mycobacterium tuberculosis or a part thereof is Extract A.
(item X33AD) The composition of any of the above-mentioned items, wherein the aforementioned hot water extract of Mycobacterium tuberculosis or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item X33AE) The composition of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X33AF) The composition of any of the above-mentioned items, wherein the aforementioned non-causative factor antigen component is administered two times or more.
(item X33AG) The composition of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X34) A composition for the prophylaxis or treatment of an infectious disease in a test subject, comprising an antigen component that is specific in the test subject for a component that is different from the causative factor of the infectious disease.
(item X35) A composition for the prophylaxis or treatment of an infectious disease in a test subject, which is substantially free of a causative factor of the infectious disease or a part thereof and comprises an antigen component from a pathogen of a pre-existing infectious disease different from the infectious disease of the test subject.
(item X35A) The composition of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X35B) The composition of any of the above-mentioned items, wherein the aforementioned causative factor is a virus belonging to the genus Betacoronavirus.
(item X35C) The composition of any of the above-mentioned items, wherein the aforementioned causative factor is a virus selected from the group consisting of HCoV-HKU1, HCoV-OC43, SARS-CoV, MERS-CoV, and SARS-CoV-2.
(item X35D) The composition of any of the above-mentioned items, wherein the aforementioned causative factor is SARS-CoV-2 virus.
(item X35E) The composition of any of the above-mentioned items, wherein the aforementioned antigen component is specific for memory T cell of the aforementioned test subject.
(item X35F) The composition of any of the above-mentioned items, wherein the aforementioned memory T cell is a memory-type regulatory T cell (IL-2-producing).
(item X35G) The composition of any of the above-mentioned items, wherein the aforementioned antigen component has an immunostimulatory action.
(item X35H) The composition of any of the above-mentioned items, wherein the aforementioned antigen component acts on memory CD4-positive T cells in an antigen-dependent manner.
(item X35I) The composition of any of the above-mentioned items, wherein the aforementioned antigen component has an activity to bias the existence ratio of Foxp3-positive Treg cells and IFN-γ-producing T cells.
(item X35J) The composition of any of the above-mentioned items, wherein the aforementioned bias is to increase IFN-γ-producing T cells as compared with Foxp3-positive Treg cells.
(item X35K) The composition of any of the above-mentioned items, wherein the aforementioned antigen component has an activity to bias the existence ratio of Foxp3-positive Treg cells and type 1 helper T cells.
(item X35L) The composition of any of the above-mentioned items, wherein the aforementioned IFN-γ-producing T cell comprises a type 1 helper T cell.
(item X35M) The composition of any of the above-mentioned items, wherein the aforementioned antigen component has an activity to bias the existence ratio of Th1 cells.
(item X35N) The composition of any of the above-mentioned items, wherein the aforementioned IFN-γ-producing T cell is a T-bet-positive Th1 cell.
(item X35O) The composition of any of the above-mentioned items, wherein the aforementioned specific antigen component has the ability to enhance at least one selected from the group consisting of IFN-γ-producing ability, IL-2-producing ability, and TNF-α-producing ability in a sample derived from the aforementioned test subject.
(item X35P) The composition of any of the above-mentioned items, wherein the aforementioned antigen component is a protein.
(item X36) A composition for the prophylaxis or treatment of an infectious disease other than tuberculosis, comprising a Mycobacterium tuberculosis extract.
(item X37) A composition comprising a Mycobacterium tuberculosis extract for regulating immunity enhancement against a causative factor of an infectious disease other than tuberculosis in a subject when the subject is exposed to the causative agent.
(item X37A) The composition of any of the above-mentioned items, wherein the aforementioned immune enhancement includes enhancement of cytokines and/or enhancement of immune cells related to immunity enhancement.
(item X37B) The composition of any of the above-mentioned items, wherein the aforementioned immune cell includes IL-10-producing cells.
(item X38) A composition for the prophylaxis or treatment of an infectious disease, an allergy, or an autoimmune disease in a test subject, comprising a specific antigen component in the test subject that is neither derived from nor cross-reactive with a causative factor of the infectious disease, the allergy, and/or the autoimmune disease, wherein
   the antigen component is a protein or a part thereof, or a peptide, and capable of regulating an immune response via CD4-positive T cells,
   the infectious disease, the allergy, or the autoimmune disease comprises those that can be prevented or treated by an immune response via CD4-positive T cells, and wherein
   whether the test subject is capable of regulating a CD4-positive T cell-mediated immune response to the infectious disease, the allergy, or the autoimmune disease is confirmed, and the composition is administered when the test subject is capable of regulating a CD4-positive T cell-mediated immune response to the infectious disease, the allergy, or the autoimmune disease.
(item X39) A composition for the prophylaxis or treatment of an infectious disease in a test subject, comprising a specific antigen component in the test subject for a component different from a causative factor of the infectious disease, wherein
   the infectious disease comprises a viral infectious disease,
   the antigen component is a protein or a part thereof, or a peptide, and capable of regulating an immune response via CD4-positive T cells,
   the viral infectious disease comprises those that can be prevented or treated by an immune response via CD4-positive T cells, and wherein
   whether the test subject is capable of regulating an antivirus immune response via CD4-positive T cells is confirmed, and when the test subject is capable of regulating an antivirus immune response via CD4-positive T cells, the composition is administered.
(item X40) A method for producing or providing in other manner a composition for the prophylaxis or treatment of an infectious disease, an allergy, or an autoimmune disease in a test subject, the method comprising:
   A) a step of specifying a specific antigen component for the test subject that is neither derived from nor cross-reactive with a causative factor of the infectious disease, the allergy, and/or the autoimmune disease,
   B) a step of specifying whether the test subject has immune memory for the antigen component and selecting one with the immune memory, and
   C) a step of producing or providing in other manner the selected antigen component.
(item X41) A method for determining whether a specific antigen component for a test subject that is neither derived from nor cross-reactive with a causative factor of an infectious disease, an allergy, and/or an autoimmune disease can prevent or treat the infectious disease, the allergy, or the autoimmune disease in the test subject, the method comprising:
   B) a step of specifying whether the test subject has immune memory for the antigen component and selecting one with the immune memory, and specifying that the infectious disease, the allergy, or the autoimmune disease in the test subject can be prevented or treated when the immune memory is present.
(item X42) A method for the prophylaxis or treatment of an infectious disease, an allergy, or an autoimmune disease, comprising a) a step of obtaining an antigen responsiveness profile of a test subject,
   b) a step of specifying an antigen component or a combination of antigen components from the antigen responsiveness profile, wherein the antigen component or the combination of the antigen components is specified based on the immune responsiveness presented now or in the past by the test subject to the antigen component or the combination of the antigen components, and
   c) a step of administering the antigen component or the combination of the antigen components identified in step b) to the test subject in an amount sufficient to regulate an immune response in the test subject.
(item X42A) The method of any of the above-mentioned items, wherein obtaining the aforementioned antigen responsiveness profile includes confirming past physical condition of the subject, confirming whether one or more antigen candidates are responsive in a sample derived from a test subject, or both of them.
(item X42B) The method of any of the above-mentioned items, wherein obtaining the aforementioned antigen responsiveness profile comprises specifying an antigen component or a combination of antigen components that regulates an immune response via CD4-positive T cells.
(item X42C) The method of any of the above-mentioned items, wherein i) the aforementioned antigen responsiveness profile includes at least one selected from the group consisting of a medical interview, a medical history based on the mother and child health handbook or its equivalent and the like, a vaccination history, and combination of these, and/or ii) confirmation of the aforementioned responsive includes collecting a body fluid (e.g., blood) from a test subject, separating peripheral blood cells, and measuring whether the peripheral blood cells produce cytokines in response to antigens related to the antigen profile, or other biomarkers.
(item X42D) The method of any of the above-mentioned items, further comprising a step of periodically inspecting the responsiveness of the aforementioned antigen and confirming the maintenance of the responsiveness.
(item X42E) The method of any of the above-mentioned items, wherein the aforementioned a) and b) are performed by the following steps
   i) a step of obtaining the physical condition in the past of the test subject,
   ii) a step of collecting blood from the test subject, separating peripheral blood cells, and measuring whether the peripheral blood cells produce cytokines in response to the antigens corresponding to physical condition, or other biomarkers, and
   iii) a step of specifying an appropriate antigen component or a combination of antigen components from the results of ii) .
(item X42F) The method of any of the above-mentioned items, further comprising one or more features described in any of the above-mentioned items.
(item X42G) The method of any of the above-mentioned items, wherein the aforementioned physical condition in the past comprises a medical history and a vaccination history.
(item X42H) The method of any of the above-mentioned items, wherein the aforementioned physical condition comprises a history of a non-coronaviral infectious disease and the aforementioned antigen component comprises an antigen component or a combination of antigen components against the non-coronaviral infectious disease.
(item X42I) The method of any of the above-mentioned items, wherein the aforementioned physical condition comprises a history of BCG vaccination, a history of tuberculosis infection, or an antigen responsiveness to Mycobacterium tuberculosis, and the aforementioned antigen component or the combination of antigen components comprises a human type Mycobacterium tuberculosis hot water extract.
(item X42J) The method of any of the above-mentioned items, wherein the aforementioned administration comprises subcutaneous administration, intradermal administration, or intramuscular administration.
(item X42K) The method of any of the above-mentioned items, wherein the aforementioned step ii) comprises measuring the IFN-γ production, IL-2 production, TNF-α production, or cells that simultaneously produce a plurality of these cytokines.
(item X42L) The method of any of the above-mentioned items, wherein the aforementioned antigen responsiveness profile is obtained by prior companion diagnosis based on medical history and vaccination history.
(item X42M) The method of any of the above-mentioned items, wherein the aforementioned physical condition in the past and the aforementioned antigen component or the combination of antigen components include a history of tuberculosis infection and a human type Mycobacterium tuberculosis hot water extract.
(item X42N) The method of any of the above-mentioned items, wherein the aforementioned test subject is a test subject whose history of BCG vaccination, history of tuberculosis infection, or antigen responsiveness could be confirmed,
   the aforementioned Mycobacterium tuberculosis extract is administered prophylactically before the onset of the infectious disease or at the early stage of the onset of the infectious disease, and an extract from Mycobacterium tuberculosis is administered subcutaneously, intradermally, or intramuscularly at the early stage of the onset of the infectious disease by a conventional method.
(item X42O) The method for the prophylaxis or treatment of a viral infectious disease based on any of the above-mentioned items, comprising revaccination with the aforementioned antigen component or the combination of antigen components.
(item X42P) A method of preventing or treating an infectious disease in a subject by an antigen component different from the causative agent of the infectious disease, wherein the antigen component is an antigen or extract identified by a medical interview and/or with reference to the test subject's medical history and vaccination history,
   the test subject is a person who has a history of infection with an infectious disease related to the antigen component or a person who has a history of vaccination,
   and the component is administered to the test subject for reinfection after treatment, prophylactic administration before onset, or at the early stage of the onset of the infectious disease, and as necessary at the early stage of the onset of the infectious disease.
(item X42Q) The method of any of the above-mentioned items, wherein the aforementioned responsiveness is specified by previous infection history, a vaccination history, and by measuring the IFN-γ production, IL-2 production, TNF-α production, or cells that simultaneously produce a plurality of these cytokines using peripheral blood.
(item X42R) The method of any of the above-mentioned items, wherein the aforementioned Mycobacterium tuberculosis extract is a human type Mycobacterium tuberculosis hot water extract or an extract (extract with high safety) from, other Mycobacterium tuberculosis.
(item X42S) The method of any of the above-mentioned items, wherein the aforementioned antigen component is a protein.
(item X42T) A vaccine preparation comprising the antigen component of any of the above-mentioned items and an adjuvant base.
(item X42U) The vaccine preparation of any of the above-mentioned items, wherein the aforementioned adjuvant base comprises a substance that promotes Th1-type immune response.
(item X42V) A method for preventing or treating an infectious disease, an allergy, or an autoimmune disease in a test subject, comprising
   a) a step of specifying, based on an antigen responsiveness profile, an antigen component specific for the test subject that is neither derived from nor cross-reactive with a causative factor of the infectious disease, the allergy, and/or the autoimmune disease,
   b) a step of specifying whether the test subject has immune memory for the antigen component and specifying a test subject having the immune memory, and
   c) a step of administering the antigen component to the test subject specified to have the immune memory.
(item X42W) The method of any of the above-mentioned items, wherein the aforementioned antigen responsiveness profile comprises a vaccination history and/or an infection history.
(item X43) An adjuvant for a vaccine against an infectious disease, comprising a Mycobacterium tuberculosis hot water extract or a part thereof.
(item X43A) An adjuvant for a vaccine against an infectious disease, comprising a STING agonist.
(item X43B) An adjuvant for a vaccine against a viral infectious disease, comprising a STING agonist.
(item X44) The adjuvant of any of the above-mentioned items, wherein the aforementioned Mycobacterium tuberculosis hot water extract or a part thereof is a hot water extract of Mycobacterium tuberculosis Aoyama B strain or a part thereof.
(item X45) The adjuvant of any of the above-mentioned items, wherein the aforementioned Mycobacterium tuberculosis hot water extract or a part thereof is Extract A.
(item X46) The adjuvant of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, metabolites such as STING agonist c-di-AMP, and the like, NOD2 agonist, or other moiety.
(item X47) The adjuvant of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X48) The adjuvant of any of the above-mentioned items, wherein the aforementioned causative factor of the infectious disease is a bacterium, a virus, or a protozoa.
(item X49) The adjuvant of any of the above-mentioned items, wherein the aforementioned causative factor of the infectious disease is a virus.
(item X50) The adjuvant of any of the above-mentioned items, wherein the aforementioned causative factor of the infectious disease is a virus belonging to the Coronaviridae family.
(item X51) The adjuvant of any of the above-mentioned items, wherein the aforementioned causative factor of the infectious disease is a virus belonging to a family selected from the group consisting of Rhinovirus, adenovirus, Coronavirus, RS virus, influenza virus, parainfluenza virus, and Enterovirus.
(item X52) The adjuvant of any of the above-mentioned items, wherein the aforementioned causative factor of the infectious disease is a virus belonging to the genus Betacoronavirus.
(item X53) The adjuvant of any of the above-mentioned items, wherein the aforementioned causative factor of the infectious disease is a virus selected from the group consisting of HCoV-HKU1, HCoV-OC43, SARS-CoV, MERS-CoV, and SARS-CoV-2.
(item X54) The adjuvant of any of the above-mentioned items, wherein the aforementioned causative factor of the infectious disease is a SARS-CoV-2 virus.
(item X1A) A method for preventing or treating a target disease in a subject, comprising administering an effective amount of a non-causative factor antigen component that is neither derived from nor cross-reactive with a causative factor of the disease, under conditions where a causative factor antigen component derived from and/or cross-reactive with the causative factor of the disease is present.
(item X2A) The method of any of the above-mentioned items, wherein the aforementioned causative factor comprises an exogenous substance for the aforementioned subject.
(item X3A) The method of any of the above-mentioned items, wherein the aforementioned causative factor of the disease comprises a pathogenic factor of an infectious disease, a causative substance of an allergy, a causative substance of an autoimmune disease, or a toxic substance.
(item X4A) The method of any of the above-mentioned items, wherein the aforementioned disease is an infectious disease.
(item X5A) The method of any of the above-mentioned items, wherein the aforementioned subject has immune memory for the aforementioned non-causative factor antigen component.
(item X6A) The method of any of the above-mentioned items, comprising the aforementioned causative factor antigen component.
(item X7A) The method of any of the above-mentioned items for preventing the aforementioned disease.
(item X8A) The method of any of the above-mentioned items for preventing or treating the aforementioned disease without or with reduced immune hyperreactivity.
(item X9A) The method of any of the above-mentioned items, wherein the aforementioned administration is performed in a form effective for regulating cellular immunity against the aforementioned causative factor of the disease.
(item X10A) The method of any of the above-mentioned items, wherein whether a causative factor antigen component derived from and/or cross-reactive with the causative factor of the target disease is present in the aforementioned subject is confirmed, and when it is not present, the causative factor antigen component is administered together with the aforementioned non-causative factor antigen component.
(item X11A) The method of any of the above-mentioned items, wherein the aforementioned disease is an infectious disease, an allergy, or an autoimmune disease.
(item X12A) The method of any of the above-mentioned items, wherein the aforementioned disease is a viral infectious disease or a bacterium infectious disease.
(item X13A) The method of any of the above-mentioned items, wherein the aforementioned disease is a viral infectious disease.
(item X14A) The method of any of the above-mentioned items, wherein the aforementioned disease is an infectious disease related to Coronavirus.
(item X15A) The method of any of the above-mentioned items, wherein the aforementioned causative factor antigen component is a molecule that appears at least partially on the surface of the causative factor of the target disease.
(item X15AA) The method of any of the above-mentioned items, wherein the aforementioned non-causative factor antigen component is a Mycobacterium tuberculosis hot water extract or a part thereof.
(item X15AB) The method of any of the above-mentioned items, wherein the aforementioned non-causative factor antigen component is a hot water extract of Mycobacterium tuberculosis Aoyama B strain or a part thereof.
(item X15AC) The method of any of the above-mentioned items, wherein the aforementioned Mycobacterium tuberculosis hot water extract or a part thereof is Extract A.
(item X15AD) The method of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item X15AE) The method of any of the above-mentioned items, wherein the aforementioned Mycobacterium tuberculosis hot water extract or a part thereof comprises c-di-AMP, c-di-GMP, 3'3'-cGAMP, 2'3'-cGAMP and/or dsDNA capable of producing these via cGAS in human cytoplasm, which are Mycobacterium tuberculosis-derived STING agonists.
(item X15AF) The method of any of the above-mentioned items, wherein the aforementioned non-causative factor antigen component is administered two times or more.
(item X16A) A method comprising administering a combination of a causative factor antigen component derived from and/or cross-reactive with a causative factor of a target disease in a subject, and a non-causative factor antigen component that is neither derived from nor cross-reactive with the causative factor of the disease.
(item X17A) The method of any of the above-mentioned items, wherein the aforementioned causative factor antigen component comprises a pathogenic factor of an infectious disease, a causative substance of an allergy, a causative substance of an autoimmune disease, or a toxic substance.
(item X18A) The method of any of the above-mentioned items, wherein the aforementioned causative factor antigen component is a bacterium, a virus or protozoa, or a part thereof.
(item X19A) The method of any of the above-mentioned items, wherein the aforementioned causative factor antigen component is a virus or a part thereof.
(item X20A) The method of any of the above-mentioned items, wherein the aforementioned causative factor antigen component is a virus belonging to the Coronaviridae family or a part thereof.
(item X21A) The method of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X22A) The method of any of the above-mentioned items, wherein the aforementioned causative factor is a virus belonging to the genus Betacoronavirus.
(item X23A) The method of any of the above-mentioned items, wherein the aforementioned causative factor is a virus selected from the group consisting of HCoV-HKU1, HCoV-OC43, SARS-CoV, MERS-CoV, and SARS-CoV-2.
(item X24A) The method of any of the above-mentioned items, wherein the aforementioned causative factor is a SARS-CoV-2 virus.
(item X25A) The method of any of the above-mentioned items, wherein the aforementioned non-causative factor antigen component is a Mycobacterium tuberculosis hot water extract or a part thereof.
(item X26A) The method of any of the above-mentioned items, wherein the aforementioned non-causative factor antigen component is a hot water extract of Mycobacterium tuberculosis Aoyama B strain or a part thereof.
(item X26AA) The method of any of the above-mentioned items, wherein the aforementioned non-causative factor antigen component is a Mycobacterium tuberculosis hot water extract or a part thereof.
(item X26AB) The method of any of the above-mentioned items, wherein the aforementioned non-causative factor antigen component is a hot water extract of Mycobacterium tuberculosis Aoyama B strain or a part thereof.
(item X26AC) The method of any of the above-mentioned items, wherein the aforementioned Mycobacterium tuberculosis hot water extract or a part thereof is Extract A.
(item X26AD) The method of any of the above-mentioned items, wherein the aforementioned Mycobacterium tuberculosis hot water extract or a part thereof comprises a Mycobacterium tuberculosis-derived STING agonist, LpqH, Y126A9, PPD, CMV pp65 overlapping peptide, a Mtb-associated antigen, STING agonist, or a NOD2 agonist.
(item X26AE) The method of any of the above-mentioned items, wherein the aforementioned Mycobacterium tuberculosis hot water extract or a part thereof is c-di-AMP, c-di-GMP, 3'3'-cGAMP, 2'3'-cGAMP and/or dsDNA capable of producing these via cGAS in human cytoplasm, which are Mycobacterium tuberculosis-derived STING agonists.
(item X26AF) The method of any of the above-mentioned items, wherein the aforementioned non-causative factor antigen component is administered two times or more.
(item X26AG) The method of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X27A) A method for preventing or treating a target disease in a subject, comprising administering a combination of a causative factor antigen component derived from and/or cross-reactive with a causative factor of a disease in a subject, and a non-causative factor antigen component that is neither derived from nor cross-reactive with the causative factor of the disease.
(item X27AA) The method of any of the above-mentioned items, wherein the aforementioned causative factor comprises an exogenous substance for the aforementioned subject.
(item X27BA) The method of any of the above-mentioned items, wherein the aforementioned causative factor of the disease comprises a pathogenic factor of an infectious disease, a causative substance of an allergy, a causative substance of an autoimmune disease, or a toxic substance.
(item X27CA) The method of any of the above-mentioned items, wherein the aforementioned disease is an infectious disease.
(item X27DA) The method of any of the above-mentioned items, wherein the aforementioned subject has immune memory for the aforementioned non-causative factor antigen component.
(item X27EA) The method of any of the above-mentioned items for preventing the aforementioned disease.
(item X27FA) The method of any of the above-mentioned items, wherein for preventing or treating the aforementioned disease without or with reduced immune hyperreactivity.
(item X27GA) The method of any of the above-mentioned items, wherein the aforementioned administration is performed in a form effective for regulating cellular immunity against the aforementioned causative factor of the disease.
(item X27HA) The method of any of the above-mentioned items, wherein the aforementioned disease is an infectious disease, an allergy, or an autoimmune disease.
(item X27IA) The method of any of the above-mentioned items, wherein the aforementioned disease is a viral infectious disease or a bacterium infectious disease.
(item X27JA) The method of any of the above-mentioned items, wherein the aforementioned disease is a viral infectious disease.
(item X27KA) The method of any of the above-mentioned items, wherein the aforementioned disease is an infectious disease related to Coronavirus.
(item X28LA) The method of any of the above-mentioned items, wherein the aforementioned causative factor antigen component is a molecule that appears at least partially on a membrane surface.
(item X28AA) The method of any of the above-mentioned items, wherein the aforementioned non-causative factor antigen component is a Mycobacterium tuberculosis hot water extract or a part thereof.
(item X28AB) The method of any of the above-mentioned items, wherein the aforementioned non-causative factor antigen component is a hot water extract of Mycobacterium tuberculosis Aoyama B strain or a part thereof.
(item X28AC) The method of any of the above-mentioned items, wherein the aforementioned Mycobacterium tuberculosis hot water extract or a part thereof is Extract A.
(item X28AD) The method of any of the above-mentioned items, wherein the aforementioned Mycobacterium tuberculosis hot water extract or a part thereof comprises LpqH, Y1269, PPD, CMV pp65 overlapping peptide, a Mtb-associated antigen, STING agonist, or a NOD2 agonist, which are Mycobacterium tuberculosis-derived STING agonists.
(item X28AE) The method of any of the above-mentioned items, wherein the aforementioned Mycobacterium tuberculosis hot water extract or a part thereof comprises c-di-AMP, c-di-GMP, 3'3'-cGAMP, 2'3'-cGAMP and/or dsDNA capable of producing these via cGAS in human cytoplasm, which are Mycobacterium tuberculosis-derived STING agonists.
(item X28AF) The method of any of the above-mentioned items, wherein the aforementioned non-causative factor antigen component is administered two times or more.
(item X28AG) The method of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X31A) A method for removing or converting suppression on a regulatory T cell (Treg), comprising administering an effective amount of a non-causative factor antigen component that is neither derived from nor cross-reactive with an infectious disease, an allergy, and/or an autoimmune disease to be the target, wherein a test subject has immune memory for the antigen component, and, in the test subject, an activity of the regulatory T cell (Treg) against a causative factor of the infectious disease, the allergy, and/or the autoimmune disease, or a cell infected with the causative factor or containing the causative factor is suppressed.
(item X32A) The method of any of the above-mentioned items, wherein the aforementioned Treg is converted to an effector T cell (Teff) for the causative factor of the disease (infectious disease, allergy and/or autoimmune disease) or the cell containing the causative factor of the infectious disease in the aforementioned test subject, by further presence of a causative factor antigen component derived from and/or cross-reactive with the causative factor of the target disease in the aforementioned test subject.
(item X33A) The method of any of the above-mentioned items, wherein the aforementioned test subject has immune memory for the aforementioned causative factor and/or a factor cross-reactive with the causative factor.
(item X33AA) The method of any of the above-mentioned items, wherein removal or conversion of the suppression on the aforementioned Treg imparts the ability to kill a causative factor of the aforementioned infectious disease, allergy, and/or autoimmune disease or an immunostimulatory action a cell infected with the causative factor or containing the causative factor, or an immunostimulatory action on a causative factor of the aforementioned infectious disease, allergy and/or autoimmune disease or a cell infected with the causative factor or containing the causative factor.
(item X33BA) The method of any of the above-mentioned items, wherein the aforementioned causative factor comprises at least one selected from the group consisting of malaria, yellow fever virus, smallpox virus, smallpox vaccination, measles/rubella, polio, epidemic parotitis (mumps)/MUMPS, rotaviral infectious disease, varicella (possum), yellow fever, Ebola, West Nile fever, hib infection, pneumococcul infection, pertussis, Japanese encephalitis, meningococcal infection, Salmonella infection, pathogenic Escherichia coli, Toxoplasma, Zika virus, herpes virus type 1, EBV/Epstein-Barr virus (herpes virus type 4), CMV/cytomegalovirus (herpes virus type 5), influenza, MARS, rabies, and diphtheria.
(item X33CA) The method of any of the above-mentioned items, wherein the aforementioned causative factor is a virus.
(item X33DA) The method of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X33EA) The method of any of the above-mentioned items, wherein the aforementioned causative factor is a virus belonging to the genus Betacoronavirus.
(item X33FA) The method of any of the above-mentioned items, wherein the aforementioned causative factor is a virus selected from the group consisting of HCoV-HKU1, HCoV-OC43, SARS-CoV, MERS-CoV, and SARS-CoV-2.
(item X33GA) The method of any of the above-mentioned items, wherein the aforementioned causative factor is a SARS-CoV-2 virus.
(item X33HA) The method of any of the above-mentioned items, wherein the aforementioned antigen component comprises a protein, a part thereof, or a peptide.
(item X33IA) The method of any of the above-mentioned items, wherein the aforementioned antigen component comprises an antigen selected from the group consisting of a pathogen of an infectious disease or a part thereof, an antigen related to medical history, and an antigen related to vaccination history.
(item X33JA) The method of any of the above-mentioned items, wherein the aforementioned antigen component comprises an antigen from a pathogen of a pre-existing infectious disease.
(item X33KA) The method of any of the above-mentioned items, wherein the aforementioned antigen component comprises one capable of regulating an immune response via CD4-positive T cells.
(item X33LA) The method of any of the above-mentioned items, wherein the aforementioned antigen component comprises a human type Mycobacterium tuberculosis hot water extract.
(item X33MA) The method of any of the above-mentioned items, wherein the aforementioned test subject has a history of BCG vaccination, a history of tuberculosis infection, or an antigen responsiveness to Mycobacterium tuberculosis, and the aforementioned antigen component comprises a human type Mycobacterium tuberculosis hot water extract.
(item X33NA) The method of any of the above-mentioned items, wherein the aforementioned Treg is CD4-positive.
(item X33OA) The method of any of the above-mentioned items, wherein the aforementioned Treg removes suppression of activation of killer T cells specific to the aforementioned causative factor of the infectious disease.
(item X33PA) The method of any of the above-mentioned items, for preventing the aforementioned infectious disease.
(item X33QA) The method of any of the above-mentioned items, for removing suppression of activation of killer cells that kill cells infected with the aforementioned causative factor.
(item X33RA) The method of any of the above-mentioned items, comprising examining whether the aforementioned antigen component has Treg immune memory in the test subject, and administering the antigen component to the test subject when the test subject has immune memory for the antigen component.
(item X33SA) The method of any of the above-mentioned items, comprising confirming whether the aforementioned test subject is capable of regulating an antivirus immune response via CD4-positive T cells, and administering the aforementioned composition when the test subject is capable of regulating an antivirus immune response via CD4-positive T cells.
(item X33XAA) The method of any of the above-mentioned items, wherein the aforementioned non-causative factor antigen component is a Mycobacterium tuberculosis hot water extract or a part thereof.
(item X33XAB) The method of any of the above-mentioned items, wherein the aforementioned non-causative factor antigen component is a hot water extract of Mycobacterium tuberculosis Aoyama B strain or a part thereof.
(item X33XAC) The method of any of the above-mentioned items, wherein the aforementioned Mycobacterium tuberculosis hot water extract or a part thereof is Extract A.
(item X33XAD) The method of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item X33XAE) The method of any of the above-mentioned items, wherein the aforementioned Mycobacterium tuberculosis hot water extract or a part thereof comprises c-di-AMP, c-di-GMP, 3'3'-cGAMP, 2'3'-cGAMP and/or dsDNA capable of producing these via cGAS in human cytoplasm, which are Mycobacterium tuberculosis-derived STING agonists.
(item X33XAF) The method of any of the above-mentioned items, wherein the aforementioned non-causative factor antigen component is administered two times or more.
(item X33XAG) The method of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X34A) A method for preventing or treating an infectious disease in a test subject, comprising administering an effective amount of an antigenic component specific in the test subject to a component different from the causative factor of the infectious disease.
(item X35A) A method for preventing or treating an infectious disease in a test subject, which is substantially free of a step of administering a causative factor of the infectious disease or a part thereof and comprises administering an effective amount of an antigen component from a pathogen of a pre-existing infectious disease different from the infectious disease of the test subject.
(item X35AA) The method of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X35BA) The method of any of the above-mentioned items, wherein the aforementioned causative factor is a virus belonging to the genus Betacoronavirus.
(item X35CA) The method of any of the above-mentioned items, wherein the aforementioned causative factor is a virus selected from the group consisting of HCoV-HKU1, HCoV-OC43, SARS-CoV, MERS-CoV, and SARS-CoV-2.
(item X35DA) The method of any of the above-mentioned items, wherein the aforementioned causative factor is a SARS-CoV-2 virus.
(item X35EA) The method of any of the above-mentioned items, wherein the aforementioned antigen component is specific for memory T cell of the aforementioned test subject.
(item X35FA) The method of any of the above-mentioned items, wherein the aforementioned memory T cell is a memory-type regulatory T cell (IL-2-producing).
(item X35GA) The method of any of the above-mentioned items, wherein the aforementioned antigen component has an immunostimulatory action.
(item X35HA) The method of any of the above-mentioned items, wherein the aforementioned antigen component acts on memory CD4-positive T cells in an antigen-dependent manner.
(item X35IA) The method of any of the above-mentioned items, wherein the aforementioned antigen component has an activity to bias the existence ratio of Foxp3-positive Treg cells and IFN-γ-producing T cells.
(item X35JA) The method of any of the above-mentioned items, wherein the aforementioned bias is to increase IFN-γ-producing T cells as compared with Foxp3-positive Treg cells.
(item X35KA) The method of any of the above-mentioned items, wherein the aforementioned antigen component has an activity to bias the existence ratio of Foxp3-positive Treg cells and type 1 helper T cells.
(item X35LA) The method of any of the above-mentioned items, wherein the aforementioned IFN-γ-producing T cell comprises type 1 helper T cells.
(item X35MA) The method of any of the above-mentioned items, wherein the aforementioned antigen component has an activity to bias the existence ratio of Th1 cells.
(item X35NA) The method of any of the above-mentioned items, wherein the aforementioned IFN-γ-producing T cell is a T-bet-positive Th1 cell.
(item X350A) The method of any of the above-mentioned items, wherein the aforementioned specific antigen component has the ability to enhance at least one selected from the group consisting of IFN-γ-producing ability, IL-2-producing ability, and TNF-α-producing ability, in a sample derived from the aforementioned test subject.
(item X35PA) The method of any of the above-mentioned items, wherein the aforementioned antigen component is a protein.
(item X36A) A method for preventing or treating an infectious disease other than tuberculosis, comprising administering an effective amount of a Mycobacterium tuberculosis extract.
(item X37A) A method comprising an effective amount of a Mycobacterium tuberculosis extract for regulating immunity enhancement against a causative factor of an infectious disease other than tuberculosis in a subject when the subject is exposed to the causative agent.
(item X37AA) The method of any of the above-mentioned items, wherein the aforementioned immunity enhancement comprises enhancement of cytokines relating to immunity enhancement and/or enhancement of immune cells.
(item X37BA) The method of any of the above-mentioned items, wherein the aforementioned immune cell comprises IL-10-producing cells.
(item X38A) A method for preventing or treating an infectious disease, an allergy, or an autoimmune disease in a test subject, comprising administering an effective amount of a specific antigen component in the test subject that is neither derived from nor cross-reactive with a causative factor of the infectious disease, the allergy, and/or the autoimmune disease, wherein
   the antigen component is a protein or a part thereof, or a peptide, and capable of regulating an immune response via CD4-positive T cells,
   the infectious disease, the allergy, or the autoimmune disease comprises those that can be prevented or treated by an immune response via CD4-positive T cells, and wherein
   whether the test subject is capable of regulating a CD4-positive T cell-mediated immune response to the infectious disease, the allergy, or the autoimmune disease is confirmed, and the antigen component is administered when the test subject is capable of regulating a CD4-positive T cell-mediated immune response to the infectious disease, the allergy, or the autoimmune disease.
(item X39A) A method for preventing or treating an infectious disease in a test subject, comprising administering a specific antigen component in the test subject for a component different from a causative factor of the infectious disease, wherein
   the infectious disease comprises a viral infectious disease,
   the antigen component is a protein or a part thereof, or a peptide, and capable of regulating an immune response via CD4-positive T cells,
   the viral infectious disease comprises those that can be prevented or treated by an immune response via CD4-positive T cells, and wherein
   whether the test subject is capable of regulating an antivirus immune response via CD4-positive T cells is confirmed, and an effective amount of the antigen component is administered when the test subject is capable of regulating an antivirus immune response via CD4-positive T cells.
(item X40A) A method for producing or providing in other manner a composition for the prophylaxis or treatment of an infectious disease, an allergy, or an autoimmune disease in a test subject, the method comprising:
   A) a step of specifying a specific antigen component for the test subject that is neither derived from nor cross-reactive with a causative factor of the infectious disease, the allergy, and/or the autoimmune disease,
   B) a step of specifying whether the test subject has immune memory for the antigen component and selecting one with the immune memory, and
   C) a step of producing or providing in other manner the selected antigen component.
(item X41A) A method for determining whether a specific antigen component for a test subject that is neither derived from nor cross-reactive with a causative factor of an infectious disease, an allergy, and/or an autoimmune disease can prevent or treat the infectious disease, the allergy, or the autoimmune disease in the test subject, the method comprising:
   B) a step of specifying whether the test subject has immune memory for the antigen component and selecting one with the immune memory, and specifying that the infectious disease, the allergy, or the autoimmune disease in the test subject can be prevented or treated when the immune memory is present.
(item X42A) A method for the prophylaxis or treatment of an infectious disease, an allergy, or an autoimmune disease, comprising a) a step of obtaining an antigen responsiveness profile of a test subject,
   b) a step of specifying an antigen component or a combination of antigen components from the antigen responsiveness profile, wherein the antigen component or the combination of the antigen components is specified based on the immune responsiveness presented now or in the past by the test subject to the antigen component or the combination of the antigen components, and
   c) a step of administering the antigen component or the combination of the antigen components identified in step b) to the test subject in an amount sufficient to regulate an immune response in the test subject.
(item X42AA) The method of any of the above-mentioned items, wherein obtaining the aforementioned antigen responsiveness profile includes confirming past physical condition of the subject, confirming whether one or more antigen candidates are responsive in a sample derived from a test subject, or both of them.
(item X42BA) The method of any of the above-mentioned items, wherein obtaining the aforementioned antigen responsiveness profile comprises specifying an antigen component or a combination of antigen components that regulates an immune response via CD4-positive T cells.
(item X42CA) The method of any of the above-mentioned items, wherein i) the aforementioned antigen responsiveness profile includes at least one selected from the group consisting of a medical interview, a medical history based on the mother and child health handbook or its equivalent and the like, a vaccination history, and combination of these, and/or ii) confirmation of the aforementioned responsive includes collecting a body fluid (e.g., blood) from a test subject, separating peripheral blood cells, and measuring whether the peripheral blood cells produce cytokines in response to antigens related to the antigen profile, or other biomarkers.
(item X42DA) The method of any of the above-mentioned items, further comprising a step of periodically inspecting the responsiveness of the aforementioned antigen and confirming the maintenance of the responsiveness.
(item X42EA) The method of any of the above-mentioned items, wherein the aforementioned a) and b) are performed by the following steps
   i) a step of obtaining the physical condition in the past of the test subject,
   ii) a step of collecting blood from the test subject, separating peripheral blood cells, and measuring whether the peripheral blood cells produce cytokines in response to the antigens corresponding to physical condition, or other biomarkers, and
   iii) a step of specifying an appropriate antigen component or a combination of antigen components from the results of ii).
(item X42FA) The method of any of the above-mentioned items, further comprising one or more features described in any of the above-mentioned items.
(item X42GA) The method of any of the above-mentioned items, wherein the aforementioned physical condition in the past comprises a medical history and a vaccination history.
(item X42HA) The method of any of the above-mentioned items, wherein the aforementioned physical condition comprises a history of a non-coronaviral infectious disease and the aforementioned antigen component comprises an antigen component or a combination of antigen components against the non-coronaviral infectious disease.
(item X42IA) The method of any of the above-mentioned items, wherein the aforementioned physical condition comprises a history of BCG vaccination, a history of tuberculosis infection, or an antigen responsiveness to Mycobacterium tuberculosis, and the aforementioned antigen component or the combination of antigen components comprises a human type Mycobacterium tuberculosis hot water extract.
(item X42JA) The method of any of the above-mentioned items, wherein the aforementioned administration comprises subcutaneous administration, intradermal administration, or intramuscular administration.
(item X42KA) The method of any of the above-mentioned items, wherein the aforementioned step ii) comprises measuring the IFN-γ production, IL-2 production, TNF-α production, or cells that simultaneously produce a plurality of these cytokines.
(item X42LA) The method of any of the above-mentioned items, wherein the aforementioned antigen responsiveness profile is obtained by prior companion diagnosis based on medical history and vaccination history.
(item X42MA) The method of any of the above-mentioned items, wherein the aforementioned physical condition in the past and the aforementioned antigen component or the combination of antigen components include a history of tuberculosis infection and a human type Mycobacterium tuberculosis hot water extract.
(item X42NA) The method of any of the above-mentioned items, wherein the aforementioned test subject is a test subject whose history of BCG vaccination, history of tuberculosis infection, or antigen responsiveness could be confirmed,
   the aforementioned Mycobacterium tuberculosis extract is administered prophylactically before the onset of the infectious disease or at the early stage of the onset of the infectious disease, and an extract from Mycobacterium tuberculosis is administered subcutaneously, intradermally, or intramuscularly at the early stage of the onset of the infectious disease by a conventional method.
(item X420A) The method for the prophylaxis or treatment of a viral infectious disease based on any of the above-mentioned items, comprising revaccination with the aforementioned antigen component or the combination of antigen components.
(item X42PA) A method of preventing or treating an infectious disease in a subject by an antigen component different from the causative agent of the infectious disease, wherein the antigen component is an antigen or extract identified by a medical interview and/or with reference to the test subject's medical history and vaccination history,
   the test subject is a person who has a history of infection with an infectious disease related to the antigen component or a person who has a history of vaccination,
   and the component is administered to the test subject for reinfection after treatment, prophylactic administration before onset, or at the early stage of the onset of the infectious disease, and as necessary at the early stage of the onset of the infectious disease.
(item X42QA) The method of any of the above-mentioned items, wherein the aforementioned responsiveness is specified by previous infection history, a vaccination history, and by measuring the IFN-γ production, IL-2 production, TNF-α production, or cells that simultaneously produce a plurality of these cytokines using peripheral blood.
(item X42RA) The method of any of the above-mentioned items, wherein the aforementioned Mycobacterium tuberculosis extract is a human type Mycobacterium tuberculosis hot water extract or an extract (extract with high safety) from, other Mycobacterium tuberculosis.
(item X42SA) The method of any of the above-mentioned items, wherein the aforementioned antigen component is a protein.
(item X42TA) A vaccine preparation comprising the antigen component of any of the above-mentioned items and an adjuvant base.
(item X42UA) The vaccine preparation of any of the above-mentioned items, wherein the aforementioned adjuvant base comprises a substance that promotes Th1-type immune response.
(item X42VA) A method for preventing or treating an infectious disease, an allergy, or an autoimmune disease in a test subject, comprising
   a) a step of specifying, based on an antigen responsiveness profile, an antigen component specific for the test subject that is neither derived from nor cross-reactive with a causative factor of the infectious disease, the allergy, and/or the autoimmune disease,
   b) a step of specifying whether the test subject has immune memory for the antigen component and specifying a test subject having the immune memory, and
   c) a step of administering the antigen component to the test subject specified to have the immune memory.
(item X42WA) The method of any of the above-mentioned items, wherein the aforementioned antigen responsiveness profile comprises a vaccination history and/or an infection history.
(item X43A) A method for treating or preventing an infectious disease in a test subject, comprising an effective amount of a Mycobacterium tuberculosis hot water extract or a part thereof to the test subject together with a vaccine against the infectious disease.
(item X43AA) A method for treating or preventing an infectious disease in a test subject, comprising an effective amount of a STING agonist to the test subject together with a vaccine against the infectious disease.
(item X43AB) A method for treating or preventing an infectious disease in a test subject, comprising an effective amount of a STING agonist to the test subject together with a vaccine against a viral infectious disease.
(item X44A) The method of any of the above-mentioned items, wherein the aforementioned Mycobacterium tuberculosis hot water extract or a part thereof is a hot water extract of Mycobacterium tuberculosis Aoyama B strain or a part thereof.
(item X45A) The method of any of the above-mentioned items, wherein the aforementioned Mycobacterium tuberculosis hot water extract or a part thereof is Extract A.
(item X46A) The method of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, metabolites such as STING agonist c-di-AMP, and the like, NOD2 agonist, or other moiety.
(item X47A) The method of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X48A) The method of any of the above-mentioned items, wherein the aforementioned causative factor of the infectious disease is a bacterium, a virus, or a protozoa.
(item X49A) The method of any of the above-mentioned items, wherein the aforementioned causative factor of the infectious disease is a virus.
(item X50A) The method of any of the above-mentioned items, wherein the aforementioned causative factor of the infectious disease is a virus belonging to the Coronaviridae family.
(item X51A) The method of any of the above-mentioned items, wherein the aforementioned causative factor of the infectious disease is a virus belonging to a family selected from the group consisting of Rhinovirus, adenovirus, Coronavirus, RS virus, influenza virus, parainfluenza virus, and Enterovirus.
(item X52A) The method of any of the above-mentioned items, wherein the aforementioned causative factor of the infectious disease is a virus belonging to the genus Betacoronavirus.
(item X53A) The method of any of the above-mentioned items, wherein the aforementioned causative factor of the infectious disease is a virus selected from the group consisting of HCoV-HKU1, HCoV-OC43, SARS-CoV, MERS-CoV, and SARS-CoV-2.
(item X54A) The method of any of the above-mentioned items, wherein the aforementioned causative factor of the infectious disease is a SARS-CoV-2 virus.
(item X1B) A non-causative factor antigen component for preventing or treating a disease, which is administered under conditions where a causative factor antigen component derived from and/or cross-reactive with a causative factor of a target disease in a subject is present, and is not derived from or cross-reactive with the causative factor of the disease.
(item X2B) The antigen component of any of the above-mentioned items, wherein the aforementioned causative factor comprises an exogenous substance for the aforementioned subject.
(item X3B) The antigen component of any of the above-mentioned items, wherein the aforementioned causative factor of the disease comprises a pathogenic factor of an infectious disease, a causative substance of an allergy, a causative substance of an autoimmune disease, or a toxic substance.
(item X4B) The antigen component of any of the above-mentioned items, wherein the aforementioned disease is an infectious disease.
(item X5B) The antigen component of any of the above-mentioned items, wherein the aforementioned subject has immune memory for the aforementioned non-causative factor antigen component.
(item X6B) The antigen component of any of the above-mentioned items, wherein the aforementioned causative factor antigen component is further administered.
(item X7B) The antigen component of any of the above-mentioned items, for the prophylaxis of the aforementioned disease.
(item X8B) The antigen component of any of the above-mentioned items, for preventing or treating the aforementioned disease without or with reduced immune hyperreactivity.
(item X9B) The antigen component of any of the above-mentioned items, wherein the aforementioned antigen component is administered in a form effective for regulating cellular immunity against the aforementioned causative factor of the disease.
(item X10B) The antigen component of any of the above-mentioned items, which is characterized in that whether a causative factor antigen component derived from and/or cross-reactive with the causative factor of the target disease is present in the aforementioned subject is confirmed, and when it is not present, the causative factor antigen component is administered together with the aforementioned non-causative factor antigen component.
(item X11B) The antigen component of any of the above-mentioned items, wherein the aforementioned disease is an infectious disease, an allergy, or an autoimmune disease.
(item X12B) The antigen component of any of the above-mentioned items, wherein the aforementioned disease is a viral infectious disease or a bacterium infectious disease.
(item X13B) The antigen component of any of the above-mentioned items, wherein the aforementioned disease is a viral infectious disease.
(item X14B) The antigen component of any of the above-mentioned items, wherein the aforementioned disease is an infectious disease related to Coronavirus.
(item X15B) The antigen component of any of the above-mentioned items, wherein the aforementioned causative factor antigen component is a molecule that appears at least partially on the surface of the causative factor of the target disease.
(item X15BA) The antigen component of any of the above-mentioned items, wherein the aforementioned non-causative factor antigen component is a Mycobacterium tuberculosis hot water extract or a part thereof.
(item X15BB) The antigen component of any of the above-mentioned items, wherein the aforementioned non-causative factor antigen component is a hot water extract of Mycobacterium tuberculosis Aoyama B strain or a part thereof.
(item X15BC) The antigen component of any of the above-mentioned items, wherein the aforementioned Mycobacterium tuberculosis hot water extract or a part thereof is Extract A.
(item X15BD) The antigen component of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.
(item X15BE) The antigen component of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X15BF) The antigen component of any of the above-mentioned items, wherein the aforementioned non-causative factor antigen component is administered two times or more.
(item X16B) A combination of a causative factor antigen component derived from and/or cross-reactive with a causative factor of a target disease in a subject and a non-causative factor antigen component that is neither derived from nor cross-reactive with the causative factor of the disease.
(item X17B) The combination of any of the above-mentioned items, wherein the aforementioned causative factor antigen component comprises a pathogenic factor of an infectious disease, a causative substance of an allergy, a causative substance of an autoimmune disease, or a toxic substance.
(item X18B) The combination of any of the above-mentioned items, wherein the aforementioned causative factor antigen component is a bacterium, a virus, or a protozoa, or a part thereof.
(item X19B) The combination of any of the above-mentioned items, wherein the aforementioned causative factor antigen component is a virus or a part thereof.
(item X20B) The combination of any of the above-mentioned items, wherein the aforementioned causative factor antigen component is a virus belonging to the Coronaviridae family or a part thereof.
(item X21B) The combination of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X22B) The combination of any of the above-mentioned items, wherein the aforementioned causative factor is a virus belonging to the genus Betacoronavirus.
(item X23B) The combination of any of the above-mentioned items, wherein the aforementioned causative factor is a virus selected from the group consisting of HCoV-HKU1, HCoV-OC43, SARS-CoV, MERS-CoV, and SARS-CoV-2.
(item X24B) The combination of any of the above-mentioned items, wherein the aforementioned causative factor is a SARS-CoV-2 virus.
(item X25B) The combination of any of the above-mentioned items, wherein the aforementioned non-causative factor antigen component is a Mycobacterium tuberculosis hot water extract or a part thereof.
(item X26B) The combination of any of the above-mentioned items, wherein the aforementioned non-causative factor antigen component is a hot water extract of Mycobacterium tuberculosis Aoyama B strain or a part thereof.
(item X26BA) The combination of any of the above-mentioned items, wherein the aforementioned non-causative factor antigen component is a Mycobacterium tuberculosis hot water extract or a part thereof.
(item X26BB) The combination of any of the above-mentioned items, wherein the aforementioned non-causative factor antigen component is a hot water extract of Mycobacterium tuberculosis Aoyama B strain or a part thereof.
(item X26BC) The combination of any of the above-mentioned items, wherein the aforementioned Mycobacterium tuberculosis hot water extract or a part thereof is Extract A.
(item X26BD) The combination of any of the above-mentioned items, wherein the aforementioned Mycobacterium tuberculosis hot water extract or a part thereof comprises Mycobacterium tuberculosis-derived Mycobacterium tuberculosis-derived STING agonist, LpqH, Y1269, PPD, CMV pp65 overlapping peptide, a Mtb-associated antigen, a component capable of biosynthesizing a STING agonist in human cells, or a NOD2 agonist.
(item X26BE) The combination of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X26BF) The combination of any of the above-mentioned items, wherein the aforementioned non-causative factor antigen component is administered two times or more.
(item X26BG) The combination of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X27B) A medicament for the prophylaxis or treatment of a target disease in a subject, comprising a combination of a causative factor antigen component derived from and/or cross-reactive with a causative factor of the target disease and a non-causative factor antigen component that is neither derived from nor cross-reactive with the causative factor of the disease.
(item X27AB) The medicament of any of the above-mentioned items, wherein the aforementioned causative factor comprises an exogenous substance for the aforementioned subject.
(item X27BB) The medicament of any of the above-mentioned items, wherein the aforementioned causative factor of the disease comprises a pathogenic factor of an infectious disease, a causative substance of an allergy, a causative substance of an autoimmune disease, or a toxic substance.
(item X27CB) The medicament of any of the above-mentioned items, wherein the aforementioned disease is an infectious disease.
(item X27DB) The medicament of any of the above-mentioned items, wherein the aforementioned subject has immune memory for the aforementioned non-causative factor antigen component.
(item X27EB) The medicament of any of the above-mentioned items, wherein the aforementioned medicament is for the prophylaxis of the aforementioned disease.
(item X27FB) The medicament of any of the above-mentioned items, wherein the aforementioned medicament prevents or treats the aforementioned disease without or with reduced immune hyperreactivity.
(item X27GB) The medicament of any of the above-mentioned items, wherein the aforementioned medicament is administered in a form effective for regulating cellular immunity against the aforementioned causative factor of the disease.
(item X27HB) The medicament of any of the above-mentioned items, wherein the aforementioned disease is an infectious disease, an allergy, or an autoimmune disease.
(item X27IB) The medicament of any of the above-mentioned items, wherein the aforementioned disease is a viral infectious disease or a bacterium infectious disease.
(item X27JB) The medicament of any of the above-mentioned items, wherein the aforementioned disease is a viral infectious disease.
(item X27KB) The medicament of any of the above-mentioned items, wherein the aforementioned disease is an infectious disease related to Coronavirus.
(item X27LB) The medicament of any of the above-mentioned items, wherein the aforementioned causative factor antigen component is a molecule that appears at least partially on a membrane surface.
(item X27BAA) The medicament of any of the above-mentioned items, wherein the aforementioned non-causative factor antigen component is a Mycobacterium tuberculosis hot water extract or a part thereof.
(item X27BAB) The medicament of any of the above-mentioned items, wherein the aforementioned non-causative factor antigen component is a hot water extract of Mycobacterium tuberculosis Aoyama B strain or a part thereof.
(item X27BAC) The medicament of any of the above-mentioned items, wherein the aforementioned Mycobacterium tuberculosis hot water extract or a part thereof is Extract A.
(item X27BAD) The medicament of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a component capable of biosynthesizing a STING agonist in human cells, or a NOD2 agonist.
(item X27BAE) The medicament of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X27BAF) The medicament of any of the above-mentioned items, wherein the aforementioned non-causative factor antigen component is administered two times or more.
(item X27BAG) The medicament of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X28B) A kit for preventing or treating a disease, comprising a causative factor antigen component derived from and/or cross-reactive with a causative factor of a target disease in a subject, and a non-causative factor antigen component that is neither derived from nor cross-reactive with the causative factor of the disease.
(item X28AB) The kit of any of the above-mentioned items, wherein the aforementioned causative factor comprises an exogenous substance for the aforementioned subject.
(item X28BB) The kit of any of the above-mentioned items, wherein the aforementioned causative factor of the disease comprises a pathogenic factor of an infectious disease, a causative substance of an allergy, a causative substance of an autoimmune disease, or a toxic substance.
(item X28CB) The kit of any of the above-mentioned items, wherein the aforementioned disease is an infectious disease.
(item X28DB) The kit of any of the above-mentioned items, wherein the aforementioned subject has immune memory for the aforementioned non-causative factor antigen component.
(item X28EB) The kit of any of the above-mentioned items, which is for the prophylaxis or treatment of the aforementioned disease.
(item X28FB) The kit of any of the above-mentioned items, wherein the aforementioned kit prevents or treats the aforementioned disease without or with reduced immune hyperreactivity.
(item X28GB) The kit of any of the above-mentioned items, wherein the aforementioned kit is administered in a form effective for regulating cellular immunity against the aforementioned causative factor of the disease.
(item X28HB) The kit of any of the above-mentioned items, wherein the aforementioned disease is an infectious disease, an allergy, or an autoimmune disease.
(item X28IB) The kit of any of the above-mentioned items, wherein the aforementioned disease is a viral infectious disease or a bacterium infectious disease.
(item X28JB) The kit of any of the above-mentioned items, wherein the aforementioned disease is a viral infectious disease.
(item X28KB) The kit of any of the above-mentioned items, wherein the aforementioned disease is an infectious disease related to Coronavirus.
(item X28LB) The kit of any of the above-mentioned items, wherein the aforementioned causative factor antigen component is a molecule that appears at least partially on a membrane surface.
(item X28BAA) The kit of any of the above-mentioned items, wherein the aforementioned non-causative factor antigen component is a Mycobacterium tuberculosis hot water extract or a part thereof.
(item X28BAB) The kit of any of the above-mentioned items, wherein the aforementioned non-causative factor antigen component is a hot water extract of Mycobacterium tuberculosis Aoyama B strain or a part thereof.
(item X28BAC) The kit of any of the above-mentioned items, wherein the aforementioned Mycobacterium tuberculosis hot water extract or a part thereof is Extract A.
(item X28BAD) The kit of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item X28BAE) The kit of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X28BAF) The kit of any of the above-mentioned items, wherein the aforementioned non-causative factor antigen component is administered two times or more.
(item X28BAG) The kit of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X31B) An antigen component comprising a non-causative factor antigen component that is neither derived from nor cross-reactive with an infectious disease, an allergy, and/or an autoimmune disease to be the target, which removes or converts suppression on a regulatory T cell (Treg), wherein a test subject has immune memory for the antigen component, and, in the test subject, an activity of the regulatory T cell (Treg) against a causative factor of the infectious disease, the allergy, and/or the autoimmune disease, or a cell infected with the causative factor or containing the causative factor is suppressed.
(item X32B) The antigen component of any of the above-mentioned items, wherein the aforementioned Treg is converted to an effector T cell (Teff) for the causative factor of the disease (infectious disease, allergy, and/or autoimmune disease) or the cell containing the causative factor of the infectious disease in the aforementioned test subject, by further presence of a causative factor antigen component derived from and/or cross-reactive with the causative factor of the target disease in the aforementioned test subject.
(item X33B) The antigen component of any of the above-mentioned items, wherein the aforementioned test subject has immune memory for the aforementioned causative factor and/or a factor cross-reactive with the causative factor.
(item X33AB) The antigen component of any of the above-mentioned items, wherein removal or conversion of the suppression on the aforementioned Treg imparts the ability to kill a causative factor of the aforementioned infectious disease, allergy, and/or autoimmune disease or an immunostimulatory action a cell infected with the causative factor or containing the causative factor, or an immunostimulatory action on a causative factor of the aforementioned infectious disease, allergy and/or autoimmune disease or a cell infected with the causative factor or containing the causative factor.
(item X33BB) The antigen component of any of the above-mentioned items, wherein the aforementioned causative factor comprises at least one selected from the group consisting of malaria, yellow fever virus, smallpox virus, smallpox vaccination, measles/rubella, polio, epidemic parotitis (mumps)/MUMPS, rotaviral infectious disease, varicella (possum), yellow fever, Ebola, West Nile fever, hib infection, pneumococcul infection, pertussis, Japanese encephalitis, meningococcal infection, Salmonella infection, pathogenic Escherichia coli, Toxoplasma, Zika virus, herpes virus type 1, EBV/Epstein-Barr virus (herpes virus type 4), CMV/cytomegalovirus (herpes virus type 5), influenza, MARS, rabies, and diphtheria.
(item X33CB) The antigen component of any of the above-mentioned items, wherein the aforementioned causative factor is a virus.
(item X33DB) The antigen component of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X33EB) The antigen component of any of the above-mentioned items, wherein the aforementioned causative factor is a virus belonging to the genus Betacoronavirus.
(item X33FB) The antigen component of any of the above-mentioned items, wherein the aforementioned causative factor is a virus selected from the group consisting of HCoV-HKU1, HCoV-OC43, SARS-CoV, MERS-CoV, and SARS-CoV-2.
(item X33GB) The antigen component of any of the above-mentioned items, wherein the aforementioned causative factor is a SARS-CoV-2 virus.
(item X33HB) The antigen component of any of the above-mentioned items, wherein the aforementioned antigen component comprises a protein, a part thereof, or a peptide.
(item X33IB) The antigen component of any of the above-mentioned items, wherein the aforementioned antigen component comprises an antigen selected from the group consisting of a pathogen of an infectious disease or a part thereof, an antigen related to medical history and an antigen related to vaccination history.
(item X33JB) The antigen component of any of the above-mentioned items, wherein the aforementioned antigen component comprises an antigen from a pathogen of a pre-existing infectious disease.
(item X33KB) The antigen component of any of the above-mentioned items, wherein the aforementioned antigen component comprises one capable of regulating an immune response via CD4-positive T cells.
(item X33LB) The antigen component of any of the above-mentioned items, wherein the aforementioned antigen component comprises a human type Mycobacterium tuberculosis hot water extract.
(item X33MB) The antigen component of any of the above-mentioned items, wherein the aforementioned test subject has a history of BCG vaccination, a history of tuberculosis infection, or antigen responsiveness to Mycobacterium tuberculosis, and the aforementioned antigen component comprises a human type Mycobacterium tuberculosis hot water extract.
(item X33NB) The antigen component of any of the above-mentioned items, wherein the aforementioned Treg is CD4-positive.
(item X330B) The antigen component of any of the above-mentioned items, wherein the aforementioned Treg removes suppression of activation of killer T cells specific for the aforementioned causative factor of the infectious disease.
(item X33PB) The antigen component of any of the above-mentioned items, for preventing the aforementioned infectious disease.
(item X33QB) The antigen component of any of the above-mentioned items, for removing suppression of activation of a killer cell that kills a cell infected with the aforementioned causative factor.
(item X33RB) The antigen component of any of the above-mentioned items, which is used in a method comprising examining whether the aforementioned antigen component has Treg immune memory in the test subject, and administering the antigen component to the test subject when the test subject has immune memory for the antigen component.
(item X33SB) The antigen component of any of the above-mentioned items, characterized by confirming whether the aforementioned test subject is capable of regulating an antivirus immune response via CD4-positive T cells, and administering the aforementioned composition when the test subject is capable of regulating an antivirus immune response via CD4-positive T cells.
(item X33XAA) The antigen component of any of the above-mentioned items, wherein the aforementioned non-causative factor antigen component is a Mycobacterium tuberculosis hot water extract or a part thereof.
(item X33XAB) The antigen component of any of the above-mentioned items, wherein the aforementioned non-causative factor antigen component is a hot water extract of Mycobacterium tuberculosis Aoyama B strain or a part thereof.
(item X33XAC) The antigen component of any of the above-mentioned items, wherein the aforementioned Mycobacterium tuberculosis hot water extract or a part thereof is Extract A.
(item X33XAD) The antigen component of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item X33XAE) The antigen component of any of the above-mentioned items, wherein the aforementioned Mycobacterium tuberculosis hot water extract or a part thereof comprises a Mycobacterium tuberculosis-derived STING agonist c-di-AMP, c-di-GMP, 3'3'-cGAMP, 2'3'-cGAMP, and/or dsDNA capable of producing these via cGAS in human cytoplasm.
(item X33XAF) The antigen component of any of the above-mentioned items, wherein the aforementioned non-causative factor antigen component is administered two times or more.
(item X33XAG) The antigen component of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X34B) An antigen component for preventing or treating an infectious disease in a test subject, comprising an antigen component that is specific in the test subject for a component that is different from the causative factor of the infectious disease.
(item X35B) An antigen component for preventing or treating an infectious disease in a test subject, which is substantially free of a causative factor of the infectious disease or a part thereof, and comprises an antigen component from a pathogen of a pre-existing infectious disease different from the infectious disease of the test subject.
(item X35AB) The antigen component of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X35BB) The antigen component of any of the above-mentioned items, wherein the aforementioned causative factor is a virus belonging to the genus Betacoronavirus.
(item X35CB) The antigen component of any of the above-mentioned items, wherein the aforementioned causative factor is a virus selected from the group consisting of HCoV-HKU1, HCoV-OC43, SARS-CoV, MERS-CoV, and SARS-CoV-2.
(item X35DB) The antigen component of any of the above-mentioned items, wherein the aforementioned causative factor is a SARS-CoV-2 virus.
(item X35EB) The antigen component of any of the above-mentioned items, wherein the aforementioned antigen component is specific for memory T cell of the aforementioned test subject.
(item X35FB) The antigen component of any of the above-mentioned items, wherein the aforementioned memory T cell is a memory-type regulatory T cell (IL-2-producing).
(item X35GB) The antigen component of any of the above-mentioned items, wherein the aforementioned antigen component has an immunostimulatory action.
(item X35HB) The antigen component of any of the above-mentioned items, wherein the aforementioned antigen component acts on memory CD4-positive T cells in an antigen-dependent manner.
(item X35IB) The antigen component of any of the above-mentioned items, wherein the aforementioned antigen component has an activity to bias the existence ratio of Foxp3-positive Treg cells and IFN-γ-producing T cells.
(item X35JB) The antigen component of any of the above-mentioned items, wherein the aforementioned bias is to increase IFN-γ-producing T cells as compared with Foxp3-positive Treg cells.
(item X35KB) The antigen component of any of the above-mentioned items, wherein the aforementioned antigen component an activity to bias the existence ratio of Foxp3-positive Treg cells and type 1 helper T cells.
(item X35LB) The antigen component of any of the above-mentioned items, wherein the aforementioned IFN-γ-producing T cells comprises type 1 helper T cells.
(item X35MB) The antigen component of any of the above-mentioned items, wherein the aforementioned antigen component has an activity to bias the existence ratio of Th1 cells.
(item X35NB) The antigen component of any of the above-mentioned items, wherein The composition of any of the above-mentioned items, wherein the aforementioned IFN-γ-producing T cell is a T-bet-positive Th1 cell.
(item X350B) The antigen component of any of the above-mentioned items, wherein the aforementioned specific antigen component has the ability to enhance at least one selected from the group consisting of IFN-γ-producing ability, IL-2-producing ability, and TNF-α-producing ability in a sample derived from the aforementioned test subject.
(item X35PB) The antigen component of any of the above-mentioned items, wherein the aforementioned antigen component is a protein.
(item X36B) An antigen component for the prophylaxis or treatment of an infectious disease other than tuberculosis, comprising a Mycobacterium tuberculosis extract.
(item X37B) An antigen component comprising a Mycobacterium tuberculosis extract for regulating immunity enhancement against a causative factor of an infectious disease other than tuberculosis in a subject when the subject is exposed to the causative agent.
(item X37AB) The antigen component of any of the above-mentioned items, wherein the aforementioned immune enhancement includes enhancement of cytokines and/or enhancement of immune cells related to immunity enhancement.
(item X37BB) The antigen component of any of the above-mentioned items, wherein the aforementioned immune cell comprises an IL-10-producing cell.
(item X38B) An antigen component for the prophylaxis or treatment of an infectious disease, an allergy, or an autoimmune disease in a test subject, comprising a specific antigen component in the test subject that is neither derived from nor cross-reactive with a causative factor of the infectious disease, the allergy, and/or the autoimmune disease, wherein
   the antigen component is a protein or a part thereof, or a peptide, and capable of regulating an immune response via CD4-positive T cells,
   the infectious disease, the allergy, or the autoimmune disease comprises those that can be prevented or treated by an immune response via CD4-positive T cells, and wherein
   whether the test subject is capable of regulating a CD4-positive T cell-mediated immune response to the infectious disease, the allergy, or the autoimmune disease is confirmed, and the antigen component is administered when the test subject is capable of regulating a CD4-positive T cell-mediated immune response to the infectious disease, the allergy, or the autoimmune disease.
(item X39B) An antigen component for the prophylaxis or treatment of an infectious disease in a test subject, comprising a specific antigen component in the test subject for a component different from a causative factor of the infectious disease, wherein
   the infectious disease comprises a viral infectious disease,
   the antigen component is a protein or a part thereof, or a peptide, and capable of regulating an immune response via CD4-positive T cells,
   the viral infectious disease comprises those that can be prevented or treated by an immune response via CD4-positive T cells, and wherein
   whether the test subject is capable of regulating an antivirus immune response via CD4-positive T cells is confirmed, and when the test subject is capable of regulating an antivirus immune response via CD4-positive T cells, the antigen component is administered.
(item X40B) A method for producing or providing in other manner an antigen component for the prophylaxis or treatment of an infectious disease, an allergy, or an autoimmune disease in a test subject, the method comprising:
   A) a step of specifying a specific antigen component for the test subject that is neither derived from nor cross-reactive with a causative factor of the infectious disease, the allergy, and/or the autoimmune disease,
   B) a step of specifying whether the test subject has immune memory for the antigen component and selecting one with the immune memory, and
   C) a step of producing or providing in other manner the selected antigen component.
   an infectious disease, an allergy, or an autoimmune disease in a test subject
(item X41B) A method for determining whether a specific antigen component for a test subject that is neither derived from nor cross-reactive with a causative factor of an infectious disease, an allergy, and/or an autoimmune disease can prevent or treat the infectious disease, the allergy, or the autoimmune disease in the test subject, the method comprising:
   B) a step of specifying whether the test subject has immune memory for the antigen component and selecting one with the immune memory, and specifying that the infectious disease, the allergy, or the autoimmune disease in the test subject can be prevented or treated when the immune memory is present.
(item X42B) An antigen component for use in a method for the prophylaxis or treatment of an infectious disease, an allergy, or an autoimmune disease, wherein the method comprises
   a) a step of obtaining an antigen responsiveness profile of a test subject,
   b) a step of specifying an antigen component or a combination of antigen components from the antigen responsiveness profile, wherein the antigen component or the combination of the antigen components is specified based on the immune responsiveness presented now or in the past by the test subject to the antigen component or the combination of the antigen components, and
   c) a step of administering the antigen component or the combination of the antigen components identified in step b) to the test subject in an amount sufficient to regulate an immune response in the test subject.
(item X42AB) The antigen component of any of the above-mentioned items, wherein obtaining the aforementioned antigen responsiveness profile includes confirming past physical condition of the subject, confirming whether one or more antigen candidates are responsive in a sample derived from a test subject, or both of them.
(item X42BB) The antigen component of any of the above-mentioned items, wherein obtaining the aforementioned antigen responsiveness profile comprises specifying an antigen component or a combination of antigen components that regulates an immune response via CD4-positive T cells.
(item X42CB) The antigen component of any of the above-mentioned items, wherein i) the aforementioned antigen responsiveness profile includes at least one selected from the group consisting of a medical interview, a medical history based on the mother and child health handbook or its equivalent and the like, a vaccination history, and combination of these, and/or ii) confirmation of the aforementioned responsive includes collecting a body fluid (e.g., blood) from a test subject, separating peripheral blood cells, and measuring whether the peripheral blood cells produce cytokines in response to antigens related to the antigen profile, or other biomarkers.
(item X42DB) The antigen component of any of the above-mentioned items, wherein the aforementioned method further comprises a step of periodically inspecting the responsiveness of the aforementioned antigen and confirming the maintenance of the responsiveness.
(item X42EB) The antigen component of any of the above-mentioned items, wherein the aforementioned a) and b) are performed by the following steps
   i) a step of obtaining the physical condition in the past of the test subject,
   ii) a step of collecting blood from the test subject, separating peripheral blood cells, and measuring whether the peripheral blood cells produce cytokines in response to the antigens corresponding to physical condition, or other biomarkers, and
   iii) a step of specifying an appropriate antigen component or a combination of antigen components from the results of ii).
(item X42FB) The antigen component of any of the above-mentioned items, further comprising one or more features described in any of the above-mentioned items.
(item X42GB) The antigen component of any of the above-mentioned items, wherein the aforementioned physical condition in the past comprises a medical history and a vaccination history.
(item X42HB) The antigen component of any of the above-mentioned items, wherein the aforementioned physical condition comprises a history of a non-coronaviral infectious disease and the aforementioned antigen component comprises an antigen component or a combination of antigen components against the non-coronaviral infectious disease.
(item X42IB) The antigen component of any of the above-mentioned items, wherein the aforementioned physical condition comprises a history of BCG vaccination, a history of tuberculosis infection, or an antigen responsiveness to Mycobacterium tuberculosis, and the aforementioned antigen component or the combination of antigen components comprises a human type Mycobacterium tuberculosis hot water extract.
(item X42JB) The antigen component of any of the above-mentioned items, wherein the aforementioned administration comprises subcutaneous administration, intradermal administration, or intramuscular administration.
(item X42KB) The antigen component of any of the above-mentioned items, wherein the aforementioned step ii) comprises measuring the IFN-γ production, IL-2 production, TNF-α production, or cells that simultaneously produce a plurality of these cytokines.
(item X42LB) The antigen component of any of the above-mentioned items, wherein the aforementioned antigen responsiveness profile is obtained by prior companion diagnosis based on medical history and vaccination history.
(item X42MB) The antigen component of any of the above-mentioned items, wherein the aforementioned physical condition in the past and the aforementioned antigen component or the combination of antigen components include a history of tuberculosis infection and a human type Mycobacterium tuberculosis hot water extract.
(item X42NB) The antigen component of any of the above-mentioned items, wherein the aforementioned test subject is a test subject whose history of BCG vaccination, history of tuberculosis infection, or antigen responsiveness could be confirmed,
   the aforementioned Mycobacterium tuberculosis extract is administered prophylactically before the onset of the infectious disease or at the early stage of the onset of the infectious disease, and an extract from Mycobacterium tuberculosis is administered subcutaneously, intradermally, or intramuscularly at the early stage of the onset of the infectious disease by a conventional method.
(item X42OB) An antigen component for the prophylaxis or treatment of a viral infectious disease based on any of the above-mentioned items, comprising revaccination with the aforementioned antigen component or the combination of antigen components.
(item X42PB) An antigen component for preventing or treating an infectious disease in a subject by an antigen component different from the causative agent of the infectious disease, wherein the antigen component is an antigen or extract identified by a medical interview and/or with reference to the test subject's medical history and vaccination history,
   the test subject is a person who has a history of infection with an infectious disease related to the antigen component or a person who has a history of vaccination,
   and the antigen component is administered to the test subject for reinfection after treatment, prophylactic administration before onset, or at the early stage of the onset of the infectious disease, and as necessary at the early stage of the onset of the infectious disease.
(item X42QB) The antigen component of any of the above-mentioned items, wherein the aforementioned responsiveness is specified by previous infection history, a vaccination history, and by measuring the IFN-γ production, IL-2 production, TNF-α production, or cells that simultaneously produce a plurality of these cytokines using peripheral blood.
(item X42RB) The antigen component of any of the above-mentioned items, wherein the aforementioned Mycobacterium tuberculosis extract is a human type Mycobacterium tuberculosis hot water extract or an extract (extract with high safety) from, other Mycobacterium tuberculosis.
(item X42SB) The antigen component of any of the above-mentioned items, wherein the aforementioned antigen component is a protein.
(item X42TB) A vaccine preparation comprising the antigen component of any of the above-mentioned items and an adjuvant base.
(item X42UB) The vaccine preparation of any of the above-mentioned items, wherein the aforementioned adjuvant base comprises a substance that promotes Th1-type immune response.
(item X42VB) An antigen component for use in a method for preventing or treating an infectious disease, an allergy, or an autoimmune disease in a test subject, wherein the method comprises
   a) a step of specifying, based on an antigen responsiveness profile, an antigen component specific for the test subject that is neither derived from nor cross-reactive with a causative factor of the infectious disease, the allergy, and/or the autoimmune disease,
   b) a step of specifying whether the test subject has immune memory for the antigen component and specifying a test subject having the immune memory, and
   c) a step of administering the antigen component to the test subject specified to have the immune memory.
(item X42WB) The antigen component of any of the above-mentioned items, wherein the aforementioned antigen responsiveness profile comprises a vaccination history, and/or an infection history.
(item X43B) A Mycobacterium tuberculosis hot water extract or a part thereof for use as an adjuvant for a vaccine against an infectious disease.
(item X43AB) A STING agonist for use as an adjuvant for a vaccine against an infectious disease.
(item X43BB) A STING agonist for use as an adjuvant for a vaccine against a viral infectious disease.
(item X44B) The Mycobacterium tuberculosis hot water extract or a part thereof or the agonist of any of the above-mentioned items, wherein the aforementioned Mycobacterium tuberculosis hot water extract or a part thereof is a hot water extract of Mycobacterium tuberculosis Aoyama B strain or a part thereof.
(item X45B) The Mycobacterium tuberculosis hot water extract or a part thereof or the agonist of any of the above-mentioned items, wherein the aforementioned Mycobacterium tuberculosis hot water extract or a part thereof is Extract A.
(item X46B) The Mycobacterium tuberculosis hot water extract or a part thereof or the agonist of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist, or other moiety.
(item X47B) The Mycobacterium tuberculosis hot water extract or a part thereof or the agonist of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X48B) The Mycobacterium tuberculosis hot water extract or a part thereof or the agonist of any of the above-mentioned items, wherein the aforementioned causative factor of the infectious disease is a bacterium, a virus, or a protozoa..
(item X49B) The Mycobacterium tuberculosis hot water extract or a part thereof or the agonist of any of the above-mentioned items, wherein the aforementioned causative factor of the infectious disease is a virus.
(item X50B) The Mycobacterium tuberculosis hot water extract or a part thereof or the agonist of any of the above-mentioned items, wherein the aforementioned causative factor of the infectious disease is a virus belonging to the Coronaviridae family.
(item X51B) The Mycobacterium tuberculosis hot water extract or a part thereof or the agonist of any of the above-mentioned items, wherein the aforementioned causative factor of the infectious disease is a virus belonging to a family selected from the group consisting of Rhinovirus, adenovirus, Coronavirus, RS virus, influenza virus, parainfluenza virus, and Enterovirus.
(item X52B) The Mycobacterium tuberculosis hot water extract or a part thereof or the agonist of any of the above-mentioned items, wherein the aforementioned causative factor of the infectious disease is a virus belonging to the genus Betacoronavirus.
(item X53B) The Mycobacterium tuberculosis hot water extract or a part thereof or the agonist of any of the above-mentioned items, wherein the aforementioned causative factor of the infectious disease is a virus selected from the group consisting of HCoV-HKU1, HCoV-OC43, SARS-CoV, MERS-CoV, and SARS-CoV-2.
(item X54B) The Mycobacterium tuberculosis hot water extract or a part thereof or the agonist of any of the above-mentioned items, wherein the aforementioned causative factor of the infectious disease is a SARS-CoV-2 virus.
(item X1C) Use of a non-causative factor antigen component that is neither derived from nor cross-reactive with the causative factor in the production of a medicament for preventing or treating a disease, wherein the antigen component is administered under conditions where a causative factor antigen component derived from and/or cross-reactive with a causative factor of a target disease in a subject is present.
(item X2C) The use of any of the above-mentioned items, wherein the aforementioned causative factor comprises an exogenous substance for the aforementioned subject.
(item X3C) The use of any of the above-mentioned items, wherein the aforementioned causative factor of the disease comprises a pathogenic factor of an infectious disease, a causative substance of an allergy, a causative substance of an autoimmune disease, or a toxic substance.
(item X4C) The use of any of the above-mentioned items, wherein the aforementioned disease is an infectious disease.
(item X5C) The use of any of the above-mentioned items, wherein the aforementioned subject has immune memory for the aforementioned non-causative factor antigen component,.
(item X6C) The use of any of the above-mentioned items, comprising the aforementioned causative factor antigen component.
(item X7C) The use of any of the above-mentioned items, wherein the aforementioned medicament is for the prophylaxis of the aforementioned disease.
(item X8C) The use of any of the above-mentioned items, wherein the aforementioned medicament prevents or treats the aforementioned disease without or with reduced immune hyperreactivity.
(item X9C) The use of any of the above-mentioned items, wherein the aforementioned medicament is administered in a form effective for regulating cellular immunity against the aforementioned causative factor of the disease.
(item X10C) The use of any of the above-mentioned items, wherein, in the aforementioned medicament, whether a causative factor antigen component derived from and/or cross-reactive with the causative factor of the target disease is present in the aforementioned subject is confirmed, and when it is not present, the causative factor antigen component is administered together with the aforementioned non-causative factor antigen component.
(item X11C) The use of any of the above-mentioned items, wherein the aforementioned disease is an infectious disease, an allergy, or an autoimmune disease.
(item X12C) The use of any of the above-mentioned items, wherein the aforementioned disease is a viral infectious disease or a bacterium infectious disease.
(item X13C) The use of any of the above-mentioned items, wherein the aforementioned disease is a viral infectious disease.
(item X14C) The use of any of the above-mentioned items, wherein the aforementioned disease is an infectious disease related to Coronavirus.
(item X15C) The use of any of the above-mentioned items, wherein the aforementioned causative factor antigen component is a molecule that appears at least partially on the surface of the causative factor of the target disease.
(item X15CA) The use of any of the above-mentioned items, wherein the aforementioned non-causative factor antigen component is a Mycobacterium tuberculosis hot water extract or a part thereof.
(item X15CB) The use of any of the above-mentioned items, wherein the aforementioned non-causative factor antigen component is a hot water extract of Mycobacterium tuberculosis Aoyama B strain or a part thereof.
(item X15CC) The use of any of the above-mentioned items, wherein the aforementioned Mycobacterium tuberculosis hot water extract or a part thereof is Extract A.
(item X15CD) The use of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item X15CE) The use of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X15CF) The use of any of the above-mentioned items, wherein the aforementioned non-causative factor antigen component is administered two times or more.
(item X16C) Use of a combination of a causative factor antigen component derived from and/or cross-reactive with a causative factor of a target disease in a subject and a non-causative factor antigen component that is neither derived from nor cross-reactive with the causative factor of the disease, in the production of a medicament.
(item X17C) The use of any of the above-mentioned items, wherein the aforementioned causative factor antigen component comprises a pathogenic factor of an infectious disease, a causative substance of an allergy, a causative substance of an autoimmune disease, or a toxic substance.
(item X18C) The use of any of the above-mentioned items, wherein the aforementioned causative factor antigen component is a bacterium, a virus, or a protozoa, or a part thereof.
(item X19C) The use of any of the above-mentioned items, wherein the aforementioned causative factor antigen component is a virus or a part thereof.
(item X20C) The use of any of the above-mentioned items, wherein the aforementioned causative factor antigen component is a virus belonging to the Coronaviridae family or a part thereof.
(item X21C) The use of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X22C) The use of any of the above-mentioned items, wherein the aforementioned causative factor is a virus belonging to the genus Betacoronavirus.
(item X23C) The use of any of the above-mentioned items, wherein the aforementioned causative factor is a virus selected from the group consisting of HCoV-HKU1, HCoV-OC43, SARS-CoV, MERS-CoV, and SARS-CoV-2.
(item X24C) The use of any of the above-mentioned items, wherein the aforementioned causative factor is a SARS-CoV-2 virus.
(item X25C) The use of any of the above-mentioned items, wherein the aforementioned non-causative factor antigen component is a Mycobacterium tuberculosis hot water extract or a part thereof.
(item X26C) The use of any of the above-mentioned items, wherein the aforementioned non-causative factor antigen component is a hot water extract of Mycobacterium tuberculosis Aoyama B strain or a part thereof.
(item X26CA) The use of any of the above-mentioned items, wherein the aforementioned non-causative factor antigen component is a Mycobacterium tuberculosis hot water extract or a part thereof.
(item X26CB) The use of any of the above-mentioned items, wherein the aforementioned non-causative factor antigen component is a hot water extract of Mycobacterium tuberculosis Aoyama B strain or a part thereof.
(item X26CC) The use of any of the above-mentioned items, wherein the aforementioned Mycobacterium tuberculosis hot water extract or a part thereof is Extract A.
(item X26CD) The use of any of the above-mentioned items, wherein the aforementioned Mycobacterium tuberculosis hot water extract or a part thereof comprises a Mycobacterium tuberculosis-derived STING agonist, LpqH, Y126C9, PPD, CMV pp65 overlapping peptide, a Mtb-associated antigen, a STING agonist, or a NOD2 agonist.
(item X26CE) The use of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X26CF) The use of any of the above-mentioned items, wherein the aforementioned non-causative factor antigen component is administered two times or more.
(item X26CG) The use of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X27C) Use of a combination of a causative factor antigen component derived from and/or cross-reactive with a causative factor of a target disease in a subject and a non-causative factor antigen component that is neither derived from nor cross-reactive with the causative factor of the disease, in the production of a medicament for preventing or treating the disease.
(item X27AC) The use of any of the above-mentioned items, wherein the aforementioned causative factor comprises an exogenous substance for the aforementioned subject.
(item X27BC) The use of any of the above-mentioned items, wherein the aforementioned causative factor of the disease comprises a pathogenic factor of an infectious disease, a causative substance of an allergy, a causative substance of an autoimmune disease, or a toxic substance.
(item X27CC) The use of any of the above-mentioned items, wherein the aforementioned disease is an infectious disease.
(item X27DC) The use of any of the above-mentioned items, wherein the aforementioned subject has immune memory for the aforementioned non-causative factor antigen component.
(item X27EC) The use of any of the above-mentioned items, wherein the aforementioned medicament is for the prophylaxis of the aforementioned disease.
(item X27FC) The use of any of the above-mentioned items, wherein the aforementioned medicament prevents or treats the aforementioned disease without or with reduced immune hyperreactivity.
(item X27GC) The use of any of the above-mentioned items, wherein the aforementioned medicament is administered in a form effective for regulating cellular immunity against the aforementioned causative factor of the disease.
(item X27HC) The use of any of the above-mentioned items, wherein the aforementioned disease is an infectious disease, an allergy, or an autoimmune disease.
(item X27IC) The use of any of the above-mentioned items, wherein the aforementioned disease is a viral infectious disease or a bacterium infectious disease.
(item X27JC) The use of any of the above-mentioned items, wherein the aforementioned disease is a viral infectious disease.
(item X27KC) The use of any of the above-mentioned items, wherein the aforementioned disease is an infectious disease related to Coronavirus.
(item X27LC) The use of any of the above-mentioned items, wherein the aforementioned causative factor antigen component is a molecule that appears at least partially on a membrane surface.
(item X27CAA) The use of any of the above-mentioned items, wherein the aforementioned non-causative factor antigen component is a Mycobacterium tuberculosis hot water extract or a part thereof.
(item X27CAB) The use of any of the above-mentioned items, wherein the aforementioned non-causative factor antigen component is a hot water extract of Mycobacterium tuberculosis Aoyama B strain or a part thereof.
(item X27CAC) The use of any of the above-mentioned items, wherein the aforementioned Mycobacterium tuberculosis hot water extract or a part thereof is Extract A.
(item X27CAD) The use of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item X27CAE) The use of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X27CAF) The use of any of the above-mentioned items, wherein the aforementioned non-causative factor antigen component is administered two times or more.
(item X27CAG) The use of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X28C) Use of a causative factor antigen component derived from and/or cross-reactive with a causative factor of a target disease in a subject and a non-causative factor antigen component that is neither derived from nor cross-reactive with the causative factor of the disease, in the production of a kit for preventing or treating the disease.
(item X28AC) The use of any of the above-mentioned items, wherein the aforementioned causative factor comprises an exogenous substance for the aforementioned subject.
(item X28BC) The use of any of the above-mentioned items, wherein the aforementioned causative factor of the disease comprises a pathogenic factor of an infectious disease, a causative substance of an allergy, a causative substance of an autoimmune disease, or a toxic substance.
(item X28CC) The use of any of the above-mentioned items, wherein the aforementioned disease is an infectious disease.
(item X28DC) The use of any of the above-mentioned items, wherein the aforementioned subject has immune memory for the aforementioned non-causative factor antigen component.
(item X28EC) The use of any of the above-mentioned items, wherein the aforementioned kit is for preventing or treating the aforementioned disease.
(item X28FC) The use of any of the above-mentioned items, wherein the aforementioned kit prevents or treats the aforementioned disease without or with reduced immune hyperreactivity.
(item X28GC) The use of any of the above-mentioned items, wherein the aforementioned kit is administered in a form effective for regulating cellular immunity against the aforementioned causative factor of the disease.
(item X28HC) The use of any of the above-mentioned items, wherein the aforementioned disease is an infectious disease, an allergy, or an autoimmune disease.
(item X28IC) The use of any of the above-mentioned items, wherein the aforementioned disease is a viral infectious disease or a bacterium infectious disease.
(item X28JC) The use of any of the above-mentioned items, wherein the aforementioned disease is a viral infectious disease.
(item X28KC) The use of any of the above-mentioned items, wherein the aforementioned disease is an infectious disease related to Coronavirus.
(item X28LC) The use of any of the above-mentioned items, wherein the aforementioned causative factor antigen component is a molecule that appears at least partially on a membrane surface.
(item X28CAA) The use of any of the above-mentioned items, wherein the aforementioned non-causative factor antigen component is a Mycobacterium tuberculosis hot water extract or a part thereof.
(item X28CAB) The use of any of the above-mentioned items, wherein the aforementioned non-causative factor antigen component is a hot water extract of Mycobacterium tuberculosis Aoyama B strain or a part thereof.
(item X28CAC) The use of any of the above-mentioned items, wherein the aforementioned Mycobacterium tuberculosis hot water extract or a part thereof is Extract A.
(item X28CAD) The use of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item X28CAE) The use of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X28CAF) The use of any of the above-mentioned items, wherein the aforementioned non-causative factor antigen component is administered two times or more.
(item X28CAG) The use of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X31C) Use of a non-causative factor antigen component that is neither derived from nor cross-reactive with an infectious disease, an allergy, and/or an autoimmune disease to be the target, in the production of a medicament for removing or converting suppression on a regulatory T cell (Treg), wherein a test subject has immune memory for the antigen component, and, in the test subject, an activity of the regulatory T cell (Treg) against a causative factor of the infectious disease, the allergy, and/or the autoimmune disease, or a cell infected with the causative factor or containing the causative factor is suppressed.
(item X32C) The use of any of the above-mentioned items, wherein the aforementioned Treg is converted to an effector T cell (Teff) for the causative factor of the disease (infectious disease, allergy, and/or autoimmune disease) or the cell containing the causative factor of the infectious disease in the aforementioned test subject, by further presence of a causative factor antigen component derived from and/or cross-reactive with the causative factor of the target disease in the aforementioned test subject.
(item X33C) The use of any of the above-mentioned items, wherein the aforementioned test subject has immune memory for the aforementioned causative factor and/or a factor cross-reactive with the causative factor.
(item X33AC) The use of any of the above-mentioned items, wherein removal or conversion of the suppression on the aforementioned Treg imparts the ability to kill a causative factor of the aforementioned infectious disease, allergy, and/or autoimmune disease or an immunostimulatory action a cell infected with the causative factor or containing the causative factor, or an immunostimulatory action on a causative factor of the aforementioned infectious disease, allergy and/or autoimmune disease or a cell infected with the causative factor or containing the causative factor.
(item X33BC) The use of any of the above-mentioned items, wherein the aforementioned causative factor comprises at least one selected from the group consisting of malaria, yellow fever virus, smallpox virus, smallpox vaccination, measles/rubella, polio, epidemic parotitis (mumps)/MUMPS, rotaviral infectious disease, varicella (possum), yellow fever, Ebola, West Nile fever, hib infection, pneumococcul infection, pertussis, Japanese encephalitis, meningococcal infection, Salmonella infection, pathogenic Escherichia coli, Toxoplasma, Zika virus, herpes virus type 1, EBV/Epstein-Barr virus (herpes virus type 4), CMV/cytomegalovirus (herpes virus type 5), influenza, MARS, rabies, and diphtheria.
(item X33CC) The use of any of the above-mentioned items, wherein the aforementioned causative factor is a virus.
(item X33DC) The use of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X33EC) The use of any of the above-mentioned items, wherein the aforementioned causative factor is a virus belonging to the genus Betacoronavirus.
(item X33FC) The use of any of the above-mentioned items, wherein the aforementioned causative factor is a virus selected from the group consisting of HCoV-HKU1, HCoV-OC43, SARS-CoV, MERS-CoV, and SARS-CoV-2.
(item X33GC) The use of any of the above-mentioned items, wherein the aforementioned causative factor is a SARS-CoV-2 virus.
(item X33HC) The use of any of the above-mentioned items, wherein the aforementioned antigen component comprises a protein, a part thereof, or a peptide.
(item X33IC) The use of any of the above-mentioned items, wherein the aforementioned antigen component comprises an antigen selected from the group consisting of a pathogen of an infectious disease or a part thereof, an antigen related to medical history, and an antigen related to vaccination history.
(item X33JC) The use of any of the above-mentioned items, wherein the aforementioned antigen component comprises an antigen from a pathogen of a pre-existing infectious disease.
(item X33KC) The use of any of the above-mentioned items, wherein the aforementioned antigen component comprises one capable of regulating an immune response via CD4-positive T cells.
(item X33LC) The use of any of the above-mentioned items, wherein the aforementioned antigen component comprises a human type Mycobacterium tuberculosis hot water extract.
(item X33MC) The use of any of the above-mentioned items, wherein the aforementioned test subject has a history of BCG vaccination, a history of tuberculosis infection, or an antigen responsiveness to Mycobacterium tuberculosis, and the aforementioned antigen component comprises a human type Mycobacterium tuberculosis hot water extract.
(item X33NC) The use of any of the above-mentioned items, wherein the aforementioned Treg is CD4-positive.
(item X33OC) The use of any of the above-mentioned items, wherein the aforementioned Treg removes suppression of activation of killer T cells specific to the aforementioned causative factor of the infectious disease.
(item X33PC) The use of any of the above-mentioned items, wherein the aforementioned medicament is for preventing the aforementioned infectious disease.
(item X33QC) The use of any of the above-mentioned items, for removing suppression of activation of killer cells that kill cells infected with the aforementioned causative factor.
(item X33RC) The use of any of the above-mentioned items, which is used in a method comprising examining whether the aforementioned medicament has immune memory of Treg in the test subject, and administering the antigen component to the test subject when the test subject has immune memory for the antigen component.
(item X33SC) The use of any of the above-mentioned items, wherein whether the aforementioned test subject is capable of regulating an antivirus immune response via CD4-positive T cells is confirmed, and when the test subject is capable of regulating an antivirus immune response via CD4-positive T cells, the aforementioned medicament is administered.
(item X33CAA) The use of any of the above-mentioned items, wherein the aforementioned non-causative factor antigen component is a Mycobacterium tuberculosis hot water extract or a part thereof.
(item X33CAB) The use of any of the above-mentioned items, wherein the aforementioned non-causative factor antigen component is a hot water extract of Mycobacterium tuberculosis Aoyama B strain or a part thereof.
(item X33CAC) The use of any of the above-mentioned items, wherein the aforementioned Mycobacterium tuberculosis hot water extract or a part thereof is Extract A.
(item X33CAD) The use of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.
(item X33CAE) The use of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X33CAF) The use of any of the above-mentioned items, wherein the aforementioned non-causative factor antigen component is administered two times or more.
(item X33CAG) The use of any of the above-mentioned items, wherein the aforementioned causative factor antigen component is a pathogenic factor of an infectious disease, and the aforementioned non-causative factor antigen component functions as an adjuvant when the aforementioned causative factor antigen component is used as a vaccine.
(item X34C) Use of an antigen component in the production of a medicament for the prophylaxis or treatment of an infectious disease in a test subject, which is specific in the test subject for a component that is different from the causative factor of the infectious disease.
(item X35C) Use of an antigen component in the production of a medicament for the prophylaxis or treatment of an infectious disease in a test subject, which is from a pathogen of a pre-existing infectious disease different from the infectious disease of the test subject and is substantially free of a causative factor of an infectious disease or a part thereof.
(item X35AC) The use of any of the above-mentioned items, wherein the aforementioned causative factor is a virus belonging to a family selected from the group consisting of Rhinovirus, adenovirus, Coronavirus, RS virus, influenza virus, parainfluenza virus, and Enterovirus.
(item X35BC) The use of any of the above-mentioned items, wherein the aforementioned causative factor is a virus belonging to the genus Betacoronavirus.
(item X35CC) The use of any of the above-mentioned items, wherein the aforementioned causative factor is a virus selected from the group consisting of HCoV-HKU1, HCoV-OC43, SARS-CoV, MERS-CoV, and SARS-CoV-2.
(item X35DC) The use of any of the above-mentioned items, wherein the aforementioned causative factor is a SARS-CoV-2 virus.
(item X35EC) The use of any of the above-mentioned items, wherein the aforementioned antigen component is specific for memory T cell of the aforementioned test subject.
(item X35FC) The use of any of the above-mentioned items, wherein the aforementioned memory T cell is a memory-type regulatory T cell (IL-2-producing).
(item X35GC) The use of any of the above-mentioned items, wherein the aforementioned antigen component has an immunostimulatory action.
(item X35HC) The use of any of the above-mentioned items, wherein the aforementioned antigen component acts on memory CD4-positive T cells in an antigen-dependent manner.
(item X35IC) The use of any of the above-mentioned items, wherein the aforementioned antigen component has an activity to bias the existence ratio of Foxp3-positive Treg cells and IFN-γ-producing T cells.
(item X35JC) The use of any of the above-mentioned items, wherein the aforementioned bias is to increase IFN-γ-producing T cells as compared with Foxp3-positive Treg cells.
(item X35KC) The use of any of the above-mentioned items, wherein the aforementioned antigen component has an activity to bias the existence ratio of Foxp3-positive Treg cells and type 1 helper T cells.
(item X35LC) The use of any of the above-mentioned items, wherein the aforementioned IFN-γ-producing T cell comprises a type 1 helper T cell.
(item X35MC) The use of any of the above-mentioned items, wherein the aforementioned antigen component has an activity to bias the existence ratio of Th1 cells.
(item X35NC) The use of any of the above-mentioned items, wherein the aforementioned IFN-γ-producing T cell is T-bet-positive Th1 cell.
(item X35OC) The use of any of the above-mentioned items, wherein the aforementioned specific antigen component has the ability to enhance at least one selected from the group consisting of IFN-γ-producing ability, IL-2-producing ability, and TNF-α-producing ability, in a sample derived from the aforementioned test subject.
(item X35PC) The use of any of the above-mentioned items, wherein the aforementioned antigen component is a protein.
(item X36C) Use of Mycobacterium tuberculosis extract in production of a medicament for the prophylaxis or treatment of an infectious disease other than tuberculosis,.
(item X37C) Use of a Mycobacterium tuberculosis extract for regulating immunity enhancement against a causative factor of an infectious disease other than tuberculosis in a subject when the subject is exposed to the causative agent.
(item X37AC) The use of any of the above-mentioned items, wherein the aforementioned immunity enhancement comprises enhancement of cytokines relating to immunity enhancement and/or enhancement of immune cells.
(item X37BC) The use of any of the above-mentioned items, wherein the aforementioned immune cell comprises an IL-10-producing cell.
(item X38C) Use of a specific antigen component in a test subject that is neither derived from nor cross-reactive with a causative factor of an infectious disease, an allergy, or an autoimmune disease, in the production of a medicament for the prophylaxis or treatment of the infectious disease, the allergy, or the autoimmune disease in the test subject, wherein
   the antigen component is a protein or a part thereof, or a peptide, and capable of regulating an immune response via CD4-positive T cells,
   the infectious disease, the allergy, or the autoimmune disease comprises those permitting prophylaxis or treatment by an immune response via CD4-positive T cells, and wherein
   whether the test subject is capable of regulating an immune response to CD4-positive T cell-mediated infectious disease, allergy, or autoimmune disease is confirmed, and when the test subject is capable of regulating an immune response to CD4-positive T cell-mediated infectious disease, allergy, or autoimmune disease, the medicament is administered.
(item X39C) Use of an antigen component in the production of a medicament for the prophylaxis or treatment of an infectious disease in a test subject, which is a specific antigen component in the test subject for a component different from a causative factor of the infectious disease, wherein
   the infectious disease comprises a viral infectious disease,
   the antigen component is a protein or a part thereof, or a peptide, and capable of regulating an immune response via CD4-positive T cells,
   the viral infectious disease comprises those that can be prevented or treated by an immune response via CD4-positive T cells, and wherein
   whether the test subject is capable of regulating an antivirus immune response via CD4-positive T cells is confirmed, and when the test subject is capable of regulating an antivirus immune response via CD4-positive T cells, the composition is administered.
(item X40C) A method for producing or providing in other manner a composition for the prophylaxis or treatment of an infectious disease, an allergy, or an autoimmune disease in a test subject, the method comprising:
   A) a step of specifying a specific antigen component for the test subject that is neither derived from nor cross-reactive with a causative factor of the infectious disease, the allergy, or the autoimmune disease,
   B) a step of specifying whether the test subject has immune memory for the antigen component and selecting one with the immune memory, and
   C) a step of producing or providing in other manner the selected antigen component.
(item X41C) A method for determining whether a specific antigen component for a test subject that is neither derived from nor cross-reactive with a causative factor of an infectious disease, an allergy, or an autoimmune disease can prevent or treat the infectious disease, the allergy, or the autoimmune disease in the test subject, the method comprising:
   B) a step of specifying whether the test subject has immune memory for the antigen component, and specifying that the infectious disease, the allergy, or the autoimmune disease in the test subject can be prevented or treated when the immune memory is present.
(item X42C) Use of an antigen component in the production of a medicament for use in a method for the prophylaxis or treatment of an infectious disease, an allergy, or an autoimmune disease, wherein the method comprises
   a) a step of obtaining an antigen responsiveness profile of a test subject,
   b) a step of specifying an antigen component or a combination of antigen components from the antigen responsiveness profile, wherein the antigen component or the combination of the antigen components is specified based on the immune responsiveness presented now or in the past by the test subject to the antigen component or the combination of the antigen components, and
   c) a step of administering the antigen component or the combination of the antigen components identified in step b) to the test subject in an amount sufficient to regulate an immune response in the test subject.
(item X42AC) The use of any of the above-mentioned items, wherein obtaining the aforementioned antigen responsiveness profile includes confirming past physical condition of a test subject, confirming whether one or more antigen candidates are responsive in a sample derived from the test subject, or both of them.
(item X42BC) The use of any of the above-mentioned items, wherein obtaining the aforementioned antigen responsiveness profile comprises specifying an antigen component or a combination of antigen components that regulates an immune response via CD4-positive T cells.
(item X42CC) The use of any of the above-mentioned items, wherein i) the aforementioned antigen responsiveness profile includes at least one selected from the group consisting of a medical interview, a medical history based on the mother and child health handbook or its equivalent and the like, a vaccination history, and combination of these, and/or ii) confirmation of the aforementioned responsiveness includes collecting a body fluid (e.g., blood) from a test subject, separating peripheral blood cells, and measuring whether the peripheral blood cells produce cytokines in response to antigens related to the antigen profile, or other biomarkers.
(item X42DC) The use of any of the above-mentioned items, wherein the aforementioned method further comprises a step of periodically inspecting the responsiveness of the aforementioned antigen and confirming the maintenance of the responsiveness.
(item X42EC) The use of any of the above-mentioned items, wherein the aforementioned a) and b) are performed by the following steps
   i) a step of obtaining the physical condition in the past of the test subject,
   ii) a step of collecting blood from the test subject, separating peripheral blood cells, and measuring whether the peripheral blood cells produce cytokines in response to the antigens corresponding to physical condition, or other biomarkers, and
   iii) a step of specifying an appropriate antigen component or a combination of antigen components from the results of ii).
(item X42FC) The use of any of the above-mentioned items, wherein further comprising one or more features described in any of the above-mentioned items.
(item X42GC) The use of any of the above-mentioned items, wherein the aforementioned physical condition in the past comprises a medical history and a vaccination history.
(item X42HC) The use of any of the above-mentioned items, wherein the aforementioned physical condition comprises a history of a non-coronaviral infectious disease and the aforementioned antigen component comprises an antigen component or a combination of antigen components against the non-coronaviral infectious disease.
(item X42IC) The use of any of the above-mentioned items, wherein the aforementioned physical condition comprises a history of BCG vaccination, a history of tuberculosis infection, or an antigen responsiveness to Mycobacterium tuberculosis, and the aforementioned antigen component or the combination of antigen components comprises a human type Mycobacterium tuberculosis hot water extract.
(item X42JC) The use of any of the above-mentioned items, wherein the aforementioned administration comprises subcutaneous administration, intradermal administration, or intramuscular administration.
(item X42KC) The use of any of the above-mentioned items, wherein the aforementioned step ii) comprises measuring the IFN-γ production, IL-2 production, TNF-α production, or induction of cells that simultaneously produce a plurality of these cytokines.
(item X42LC) The use of any of the above-mentioned items, wherein the aforementioned antigen responsiveness profile is obtained by prior companion diagnosis based on medical history and vaccination history.
(item X42MC) The use of any of the above-mentioned items, wherein the aforementioned physical condition in the past and the aforementioned antigen component or the combination of antigen components include a history of tuberculosis infection and a human type Mycobacterium tuberculosis hot water extract.
(item X42NC) The use of any of the above-mentioned items, wherein the aforementioned test subject is a test subject whose history of BCG vaccination, history of tuberculosis infection, or antigen responsiveness could be confirmed,
   the aforementioned Mycobacterium tuberculosis extract is administered prophylactically before the onset of the infectious disease or at the early stage of the onset of the infectious disease, and an extract from Mycobacterium tuberculosis is administered subcutaneously, intradermally, or intramuscularly at the early stage of the onset of the infectious disease by a conventional method.
(item X42OC) The use of any of the above-mentioned items in the production of a medicament for use in preventing or treating a viral infectious disease, wherein the aforementioned antigen component or the combination of antigen components is revaccinated.
(item X42PC) Use of an antigen component different from a causative factor of an infectious disease in a test subject, in the production of a medicament for preventing or treating the infectious disease, wherein the antigen component is an antigen or extract identified by a medical interview and/or with reference to the test subject's medical history and vaccination history,
   the test subject is a person who has a history of infection with an infectious disease related to the antigen component or a person who has a history of vaccination,
   and the component is administered to the test subject for reinfection after treatment, prophylactic administration before onset, or at the early stage of the onset of the infectious disease, and as necessary at the early stage of the onset of the infectious disease.
(item X42QC) The use of any of the above-mentioned items, wherein the aforementioned responsiveness is specified by previous infection history, a vaccination history, and by measuring the IFN-γ production, IL-2 production, TNF-α production, or induction of cells that simultaneously produce a plurality of these cytokines using peripheral blood.
(item X42RC) The use of any of the above-mentioned items, wherein the aforementioned Mycobacterium tuberculosis extract is a human type Mycobacterium tuberculosis hot water extract or an extract (extract with high safety) from other Mycobacterium tuberculosis.
(item X42SC) The use of any of the above-mentioned items, wherein the aforementioned antigen component is a protein.
(item X42TC) The use of any of the above-mentioned items, wherein the aforementioned medicament is a vaccine preparation comprising an adjuvant base.
(item X42UC) The use of any of the above-mentioned items, wherein the aforementioned adjuvant base comprises a substance that promotes Th1-type immune response.
(item X42VC) Use of an antigen component in the production of a medicament for use in a method for the prophylaxis or treatment of an infectious disease, an allergy, or an autoimmune disease in a test subject, wherein the method comprises
   a) a step of specifying, based on an antigen responsiveness profile, an antigen component specific for the test subject that is neither derived from nor cross-reactive with a causative factor of the infectious disease, the allergy, or the autoimmune disease,
   b) a step of specifying whether the test subject has immune memory for the antigen component and specifying a test subject having the immune memory, and
   c) a step of administering the antigen component to the test subject specified to have the immune memory.
(item X42WC) The use of any of the above-mentioned items, wherein the aforementioned antigen responsiveness profile comprises a vaccination history, and/or an infection history.
(item X43C) Use of a Mycobacterium tuberculosis hot water extract or a part thereof in the production of a medicament for an adjuvant in a vaccine against an infectious disease.
(item X43CA) Use of a STING agonist in the production of a medicament for an adjuvant for a vaccine against an infectious disease.
(item X43CB) Use of a STING agonist in the production of a medicament for an adjuvant for a vaccine against a viral infectious disease.
(item X44C) The use of any of the above-mentioned items, wherein the aforementioned Mycobacterium tuberculosis hot water extract or a part thereof is a hot water extract of Mycobacterium tuberculosis Aoyama B strain or a part thereof.
(item X45C) The use of any of the above-mentioned items, wherein the aforementioned Mycobacterium tuberculosis hot water extract or a part thereof is Extract A.
(item X46C) The use of any of the above-mentioned items, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a component capable of biosynthesizing STING agonist in human cells, NOD2 agonist, or other moiety.
(item X47C) The use of any of the above-mentioned items, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.
(item X48C) The use of any of the above-mentioned items, wherein the aforementioned causative factor of the infectious disease is a bacterium, a virus, or a protozoa.
(item X49C) The use of any of the above-mentioned items, wherein the aforementioned causative factor of the infectious disease is a virus.
(item X50C) The use of any of the above-mentioned items, wherein the aforementioned causative factor of the infectious disease is a virus belonging to the Coronaviridae family.
(item X51C) The use of any of the above-mentioned items, wherein the aforementioned causative factor of the infectious disease is a virus belonging to a family selected from the group consisting of Rhinovirus, adenovirus, Coronavirus, RS virus, influenza virus, parainfluenza virus, and Enterovirus.
(item X52C) The use of any of the above-mentioned items, wherein the aforementioned causative factor of the infectious disease is a virus belonging to the genus Betacoronavirus.
(item X53C) The use of any of the above-mentioned items, wherein the aforementioned causative factor of the infectious disease is a virus selected from the group consisting of HCoV-HKU1, HCoV-OC43C, SARS-CoV, MERS-CoV, and SARS-CoV-2.
(item X54C) The use of any of the above-mentioned items, wherein the aforementioned causative factor of the infectious disease is a SARS-CoV-2 virus.

The present disclosure is intended so that one or more of the aforementioned features can be provided not only as the explicitly disclosed combinations, but also as other combinations thereof. Additional embodiments and advantages of the present disclosure are recognized by those skilled in the art by reading and understanding the following detailed description as needed.

### [Advantageous Effects of Invention]

The present disclosure provides techniques that can effectively prevent or treat diseases such as infectious disease, allergy, autoimmune disease, and the like.

In one embodiment, specific and solid usefulness in the prophylaxis and treatment of infectious diseases was surprisingly found in the present disclosure, by combining a causative factor antigen of an infectious disease with a non-causative factor antigen different from the cause of the infectious disease. The immunotherapy with non-causative factor antigen in the present disclosure has conventionally been considered a "non-specific" immunotherapy (i.e., innate immunity). In the present disclosure, an aspect thereof as a "specific" immunotherapy was found, and a novel immunotherapy using this mechanism is provided.

### [Brief Description of Drawings]

[Fig. 1]

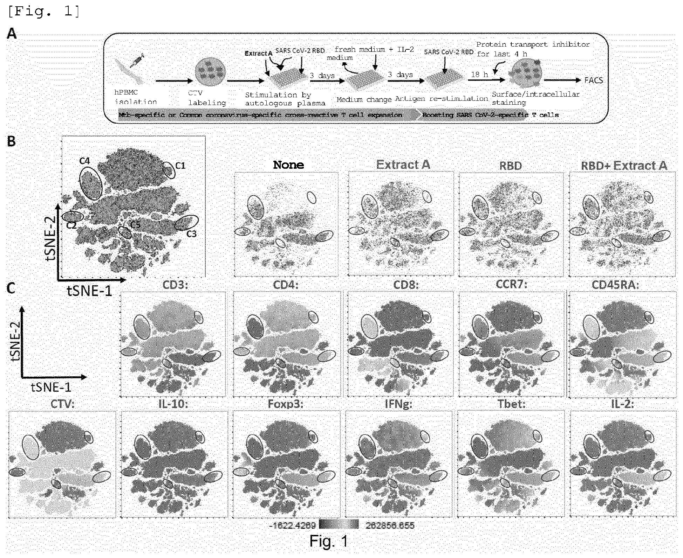

   Fig. 1 shows that Extract A and SARS CoV2 antigen combination induces distinct T cell population in uninfected peripheral blood mononuclear cells (PBMC). Fig. 1A shows human PBMC culture protocol. In Fig. 1B, human PBMCs derived from three healthy individuals were labeled with Cell Trace Violet (CTV) and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 6, the cells were re-stimulated with SARS CoV-2 receptor binding domain (RBD) antigen for 18 h, during which a protein transport inhibitor was added to the medium for the final 4 h. Intracellular staining was performed, and cell proliferation, intracellular cytokine, and transcription factor were analyzed by FACS. After concatenation of all samples derived from three individuals, tSNE (t-Distributed Stochastic Neighbor Embedding) analysis of survived CD14/19/56⁻ cells was performed by FlowJo^{™}. Representative tSNE plots from one individual are shown together with the tSNE plot of all concatenated samples. Fig. 1C shows heatmaps showing the expression of surface and intracellular markers including cytokines and transcription factors in each specific population. Scale bar shows expression levels of each marker from low (dark blue) to high (red).
[Fig. 2]

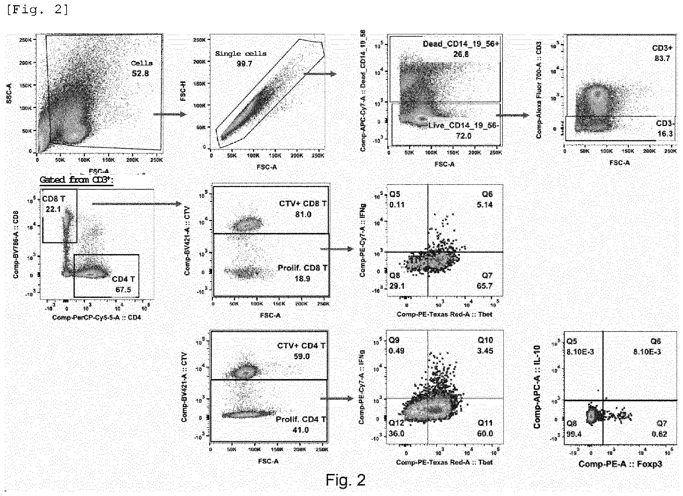

   Fig. 2 shows the gating strategy for intracellular staining of transcription factors and cytokines at day 7. After cells were gated based on forward scatter light (FSC) and side scatter light (SSC), viable CD14/19/56- cells were gated from the single cell gate. CD4 and CD8-positive T cells were gated from CD3⁺ cells and expanded CD4 and CD8 T cells were gated based on CTV intensity (e.g., CTV diluted cells represent expanded cells). Tbet⁺ cells and IFNγ⁺ cells were gated from expanded CD4 and CD8 T cells. Foxp3 and IL-10 were gated from expanded CD4 T cells.
[Fig. 3]

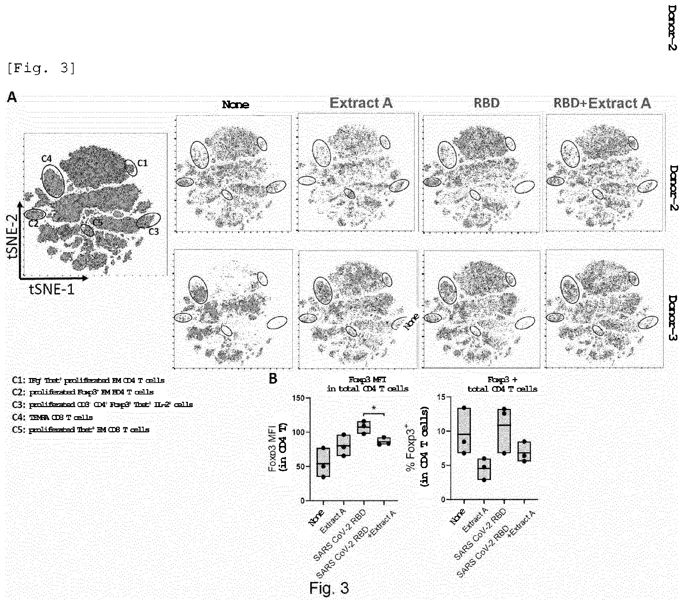

   Fig. 3 shows unique T cell population expansion proliferation (tSNE plot of donors 2 and 3) induced by Extract A and SARS CoV-2 antigen combination in uninfected PBMC. In Fig. 3A, human PBMCs derived from three healthy individuals were labeled with CTV and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 6, the cells were re-stimulated with SARS CoV-2 RBD antigen for 18 h, during which a protein transport inhibitor was added to the medium for the final 4 h. Intracellular staining was performed, and cell proliferation, intracellular cytokine, and transcription factor were analyzed by FACS. After concatenation of all samples derived from three individuals, tSNE analysis of survived CD14/19/56⁻ cells was performed by FlowJo. Representative tSNE plots from donor 2 and donor 3 are shown together with the tSNE plot of all concatenated samples (Fig. 3A). Fig. 3B is a diagram showing the abundance ratio of Foxp3 MFI and Foxp3+ cells in total CD4 T cells as a box-and-whisker plot using data from three individuals. *p<0.05.
[Fig. 4]

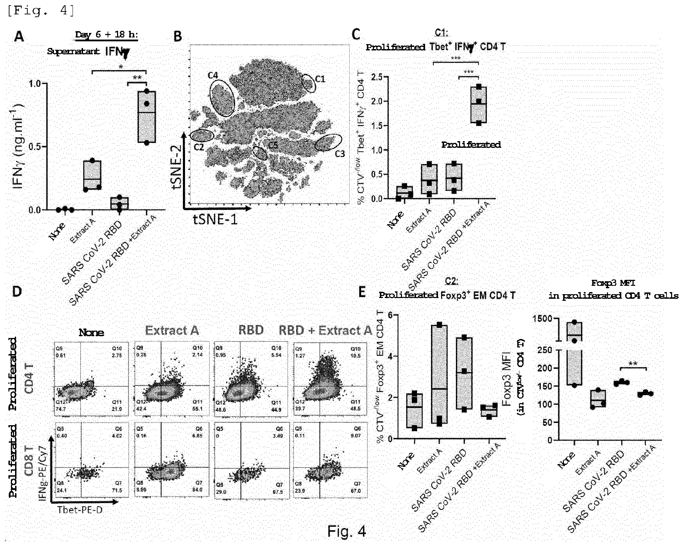

   Fig. 4 shows that Extract A adjusts Treg/Th1 balance to accelerate anti-SARS CoV-2 specific T cell response in uninfected PBMC. In Fig. 4A, human PBMCs derived from three healthy individuals were labeled with CTV and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 6, the cells were re-stimulated with SARS CoV-2 RBD antigen for 18 h, during which a protein transport inhibitor was added to the medium for the final 4 h. The IFNγ level in the supernatant 6 days after antigen restimulation was measured by ELISA, and the data thereof is shown as a box-and-whisker plot. *p<0.05, **p<0.01. In Fig. 4B, intracellular staining was performed, and cell proliferation, intracellular cytokine, and transcription factor were analyzed by FACS. After concatenation of all samples derived from three individuals, tSNE analysis of survived CD14/19/56⁻ cells was performed by FlowJo. Representative tSNE plots from one individual are shown together with the tSNE plot of all concatenated samples. Fig. 4C is a diagram showing the abundance ratio of C1 population from tSNE analysis as a box-and-whisker plot using data from all three individuals. *p<0.05, **p<0.01, ***p<0.001. Fig. 4D is a diagram showing FACS plot of Tbet expression and IFNγ production from expanded CD4 and CD8T cells (CTV^{low/negative} gate) . Fig. 4E is a diagram showing the abundance ratio of C2 population from tSNE analysis and Foxp3 MFI in expanded CD4 T cells (CTV^{low/negative} gate) as a box-and-whisker plot using data from all three individuals. *p<0.05, **p<0.01.
[Fig. 5]

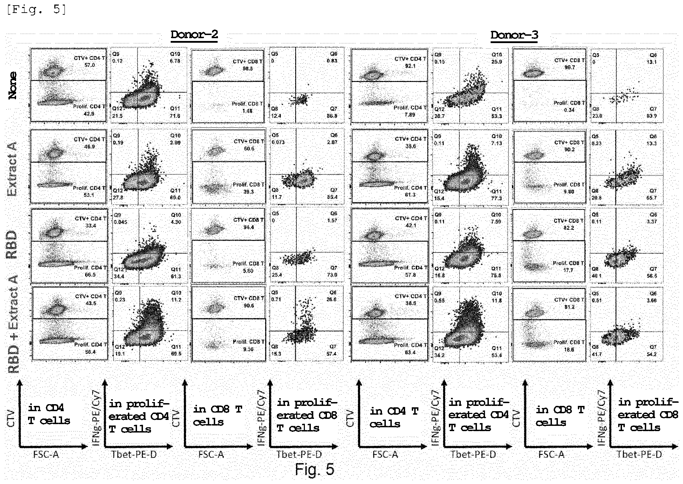

   Fig. 5 shows that Extract A accelerate anti-SARS CoV-2 specific T cell response via adjustment of Treg/Th1 balance in uninfected PBMC. In Fig. 5A, human PBMCs derived from three healthy individuals were labeled with CTV and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 6, the cells were re-stimulated with SARS CoV-2 RBD antigen for 18 h, during which a protein transport inhibitor was added to the medium for the final 4 h. Intracellular staining was performed, and cell proliferation, intracellular cytokine, and transcription factor were analyzed by FACS. The FACS plot shows CD4 T cell and CD8 T cell proliferation, and Tbet expression and IFNγ production (CTV^{low/negative gate}) from proliferated CD4 T cells and CD8 T cells.
[Fig. 6]

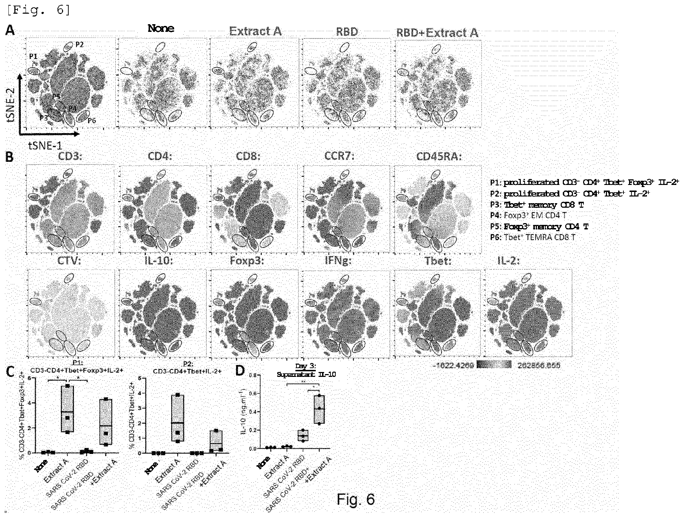

   Fig. 6 shows that at earlier time points, Extract A induces proliferation of MDSC-like innate immune cells with suppressive effects capable of producing IL-10. In Fig. 6A, human PBMCs derived from three healthy individuals were labeled with CTV and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 3, the cells were re-stimulated with SARS CoV-2 RBD antigen for 6 h in the presence of a protein transport inhibitor. Intracellular staining was performed, and cell proliferation, intracellular cytokine, and transcription factor were analyzed by FACS. After concatenation of all samples derived from three individuals, tSNE analysis of survived CD14/19/56⁻ cells was performed by FlowJo. Representative tSNE plots from one individual are shown together with the tSNE plot of all concatenated samples. In Fig. 6B, heatmaps show the expression of surface and intracellular markers including cytokines and transcription factors in each specific population. Scale bar shows expression levels of each marker from low (dark blue) to high (red). Fig. 6C shows frequencies of P1 and P2 population from the tSNE analysis, plotted in box-and-whisker plots by using the data from all three individuals. *p<0.05, **p<0.01. Fig. 6D shows IL-10 levels of supernatants of day 3 as measured by ELISA wherein the data is represented as box-and-whisker plot. SD. *p<0.05, **p<0.01.
[Fig. 7]

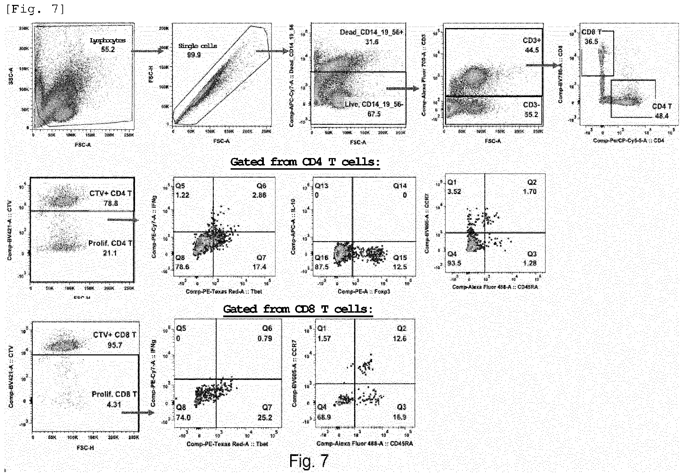

   Fig. 7 shows gating strategy for intracellular staining of transcription factors and cytokines. After cells were gated based on FSC and SSC, viable CD14/19/56⁻ cells were gated from the single cell gate. CD4 and CD8 T cells were gated from CD3⁺ cells and expanded CD4 and CD8 T cells were gated based on CTV intensity (e.g., CTV diluted cells represent expanded cells). Tbet⁺ cells and IFNγ⁺ cells were gated from expanded CD4 and CD8 T cells. Foxp3 and IL-10 were gated from expanded CD4 T cells. In addition, the memory phenotype was determined by CCR7 and CD45RA expression in expanded CD4 and CD8 T cells.
[Fig. 8A]

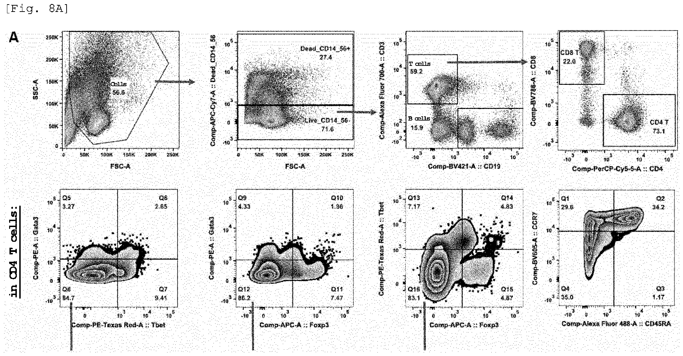

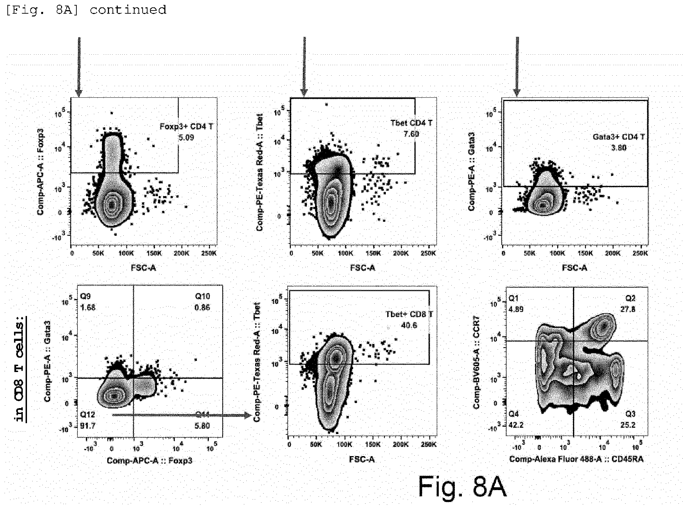

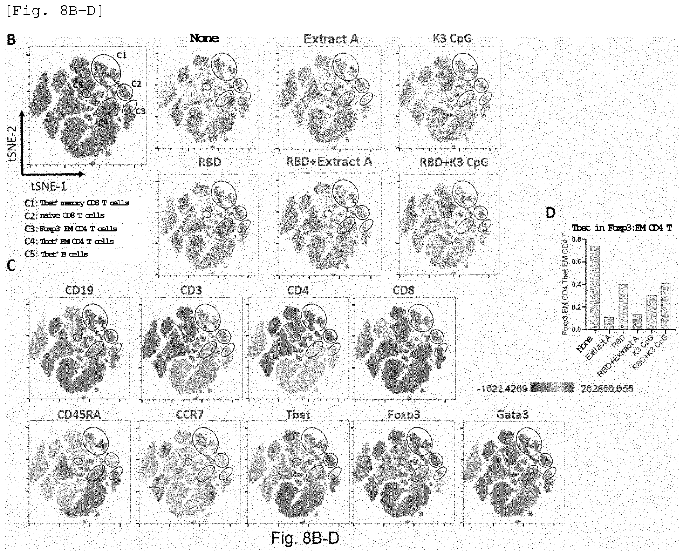

   Fig. 8 shows that Extract A-mediated SARS CoV-2-specific T cell enhancement cannot simply be attributed to the adjuvant effect. In Fig. 8A, human PBMCs derived from healthy individuals were labeled with CTV and stimulated with EXTRACT A (30 pg/ml), SARS CoV-2 RBD (1 pg/ml), or their combination for 3 days, after which the media was replaced with a fresh medium containing IL-2, and the cells were cultured for additional 3 days. On day 6, the cells were re-stimulated with SARS CoV-2 RBD antigen for 18 h. Transcription factors were analyzed by FACS upon intracellular staining. After gating the cells based on FSC and SSC, viable CD14/56⁻ cells were gated from the single cell gate. T cells and B cells were gated from CD3⁺ cells based on CD3 and CD19 expression from live/CD14/56⁻ gate. CD4 T cells and CD8 T cells were gated from CD3⁺ cells. Tbet⁺ cells, IFNγ⁺ cells, and Gata3⁺ cells were gated from CD4 T cells or CD8 T cells negative for the other two transcription factors (e.g., Tbet⁺ cells were gated from Gata3⁻Foxp3⁻ cells). In addition, the memory phenotype was determined by CCR7 and CD45RA expression in expanded CD4 and CD8 T cells.
[Fig. 8B-D]
   In Fig. 8B, tSNE analysis was performed with live, CD14/56- cells by FlowJo^{™} after concatenation of all samples, and tSNE plots together with the tSNE plot of all concatenated samples are shown. In Fig. 8C, heatmaps show the expression of surface makers and transcription factors in each specific population. Scale bar shows the expression level of each marker from low (dark blue) to high (red). In Fig. 8D, Foxp3/Tbet ratios in effector memory CD4 T cells are shown in bar graphs.
[Fig. 9A-D]

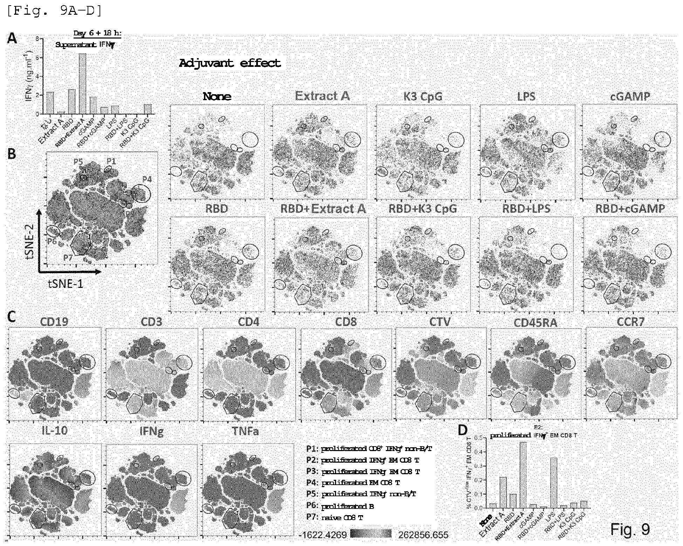

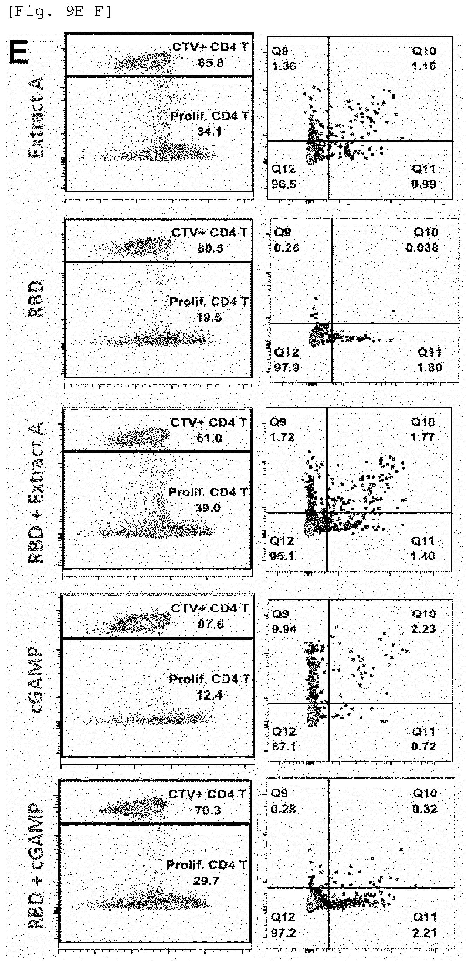

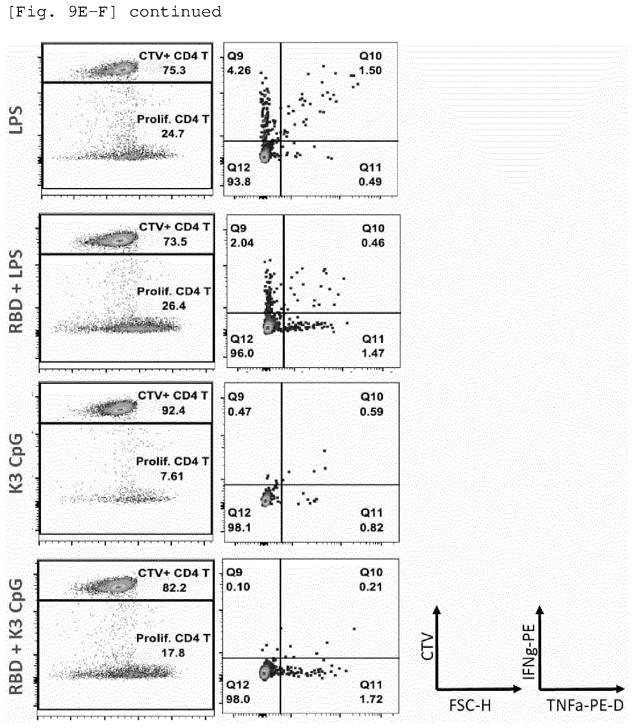

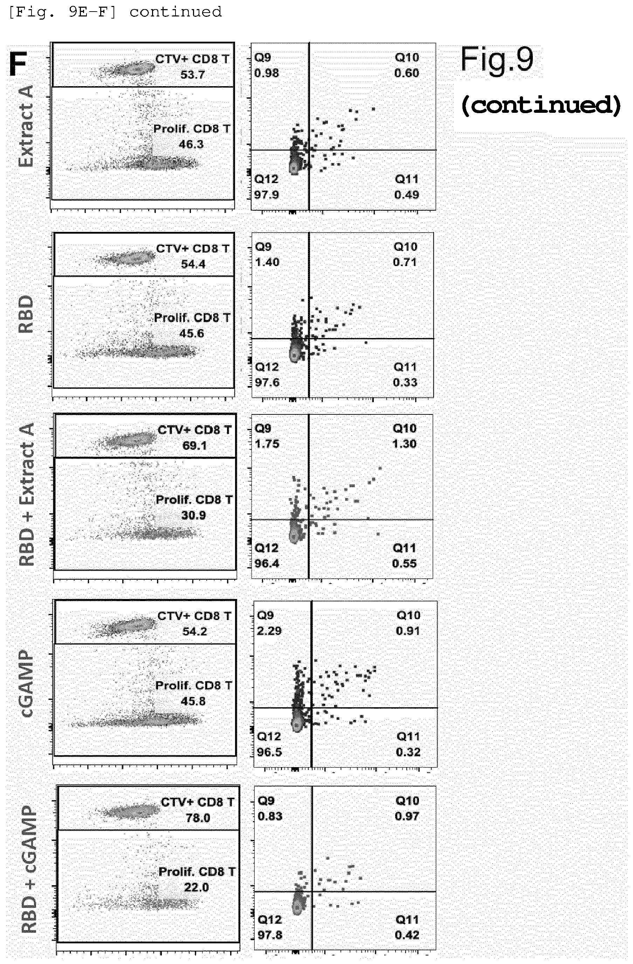

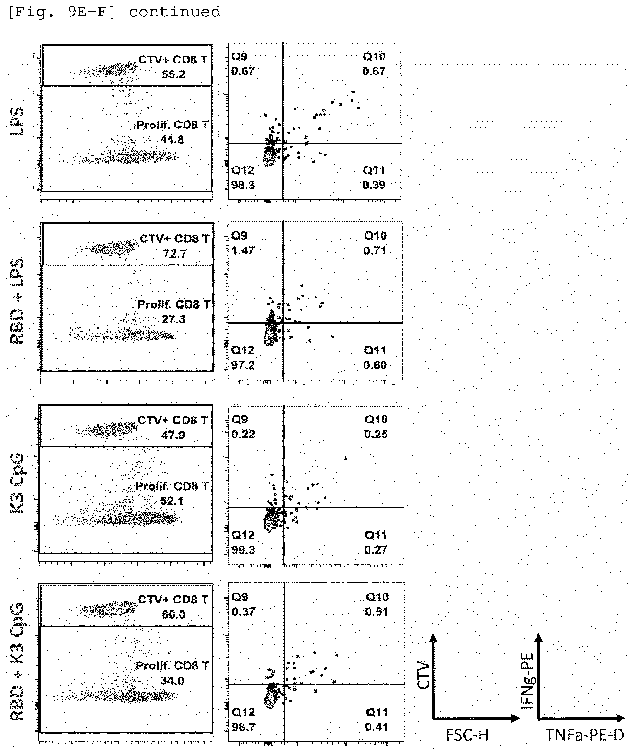

   Fig. 9 shows that Extract A-mediated SARS CoV-2-specific T cell enhancement cannot simply be attributed to the adjuvant effect. In Fig. 9A, human PBMCs from two healthy individuals were labeled and stimulated with Extract A (30 pg/ml), SARS CoV-2 RBD (1 pg/ml), or their combination for 3 days after which the media was replaced with a fresh medium containing IL-2, and the cells were cultured for additional three days. On day 6, the cells were re-stimulated with SARS CoV-2 RBD antigen for 18 h. Supernatant IFN-γ levels were measured by ELISA. In Fig. 9B, cytokine production was analyzed by FACS upon intracellular staining. tSNE analysis was performed with live, CD14/56- cells using FlowJo^{™} after concatenation of all samples from two individuals, and representative tSNE plots from one individual together with the tSNE plot of all concatenated samples are shown. In Fig. 9C, heatmaps show the expression of surface makers and transcription factors in each population. Scale bar shows expression levels of each marker from low (dark blue) to high (red). In Fig. 9D, frequencies of P1 and P2 populations from tSNE analysis of the representative healthy control were plotted in bar graphs.
[Fig. 9E-F]
   Fig. 9E shows FACS plots showing CD4 T cell population by CTV dye dilution and TNFα and IFN-γ production from the proliferated CD4 T cells (CTV^{low/negative} gate) . Fig. 9F shows FACS plots showing CD8 T cell population by CTV dye dilution and TNFα and IFN-γ production from the proliferated CD8 T cells.
[Fig. 10]

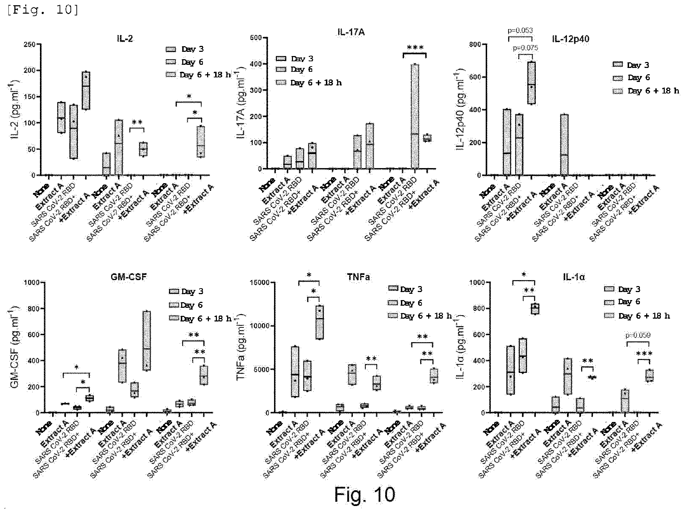

   Fig. 10 shows Bio-Plex analysis of PBMC stimulation over time. Human PBMCs derived from three healthy individuals were labeled with CTV and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 6, the cells were re-stimulated with SARS CoV-2 RBD antigen for 18 h (together with protein transport inhibitor for the final 4 h), the supernatants before and after restimulation were collected, and cytokine levels were measured by 48-plex Bio-Plex analysis.
[Fig. 11]

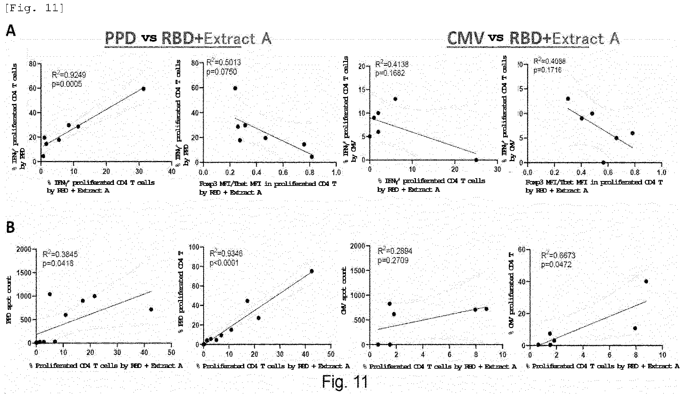

   Fig. 11 shows that pre-existing Mtb-specific T cell memory is required for Extract A-mediated promotion of anti-SARS CoV-2-specific T cell responses. In Fig. 11A, human PBMC from healthy individuals with high/low PPD/CMV reactivity were labeled with CTV, stimulated with CMVpp65, PPD (1 µg/ml), Extract A (30 µg/ml), SARS CoV-2 RBD (1 µg/ml), or a combination of these for 3 days, the medium was replaced with a fresh medium containing IL-2 and the cells were cultured for additional 3 days. On day 6, the cells were re-stimulated with SARS CoV-2 RBD antigen for 18 h, during which a protein transport inhibitor was added to the medium for the final 4 h. The IFNγ level in the supernatant was measured by ELISA, intracellular staining was performed, and cell proliferation, intracellular cytokine, and transcription factor were analyzed by FACS. On day 6, the cells were re-stimulated with SARS CoV-2 RBD antigen for 18 h, during which a protein transport inhibitor was added to the medium for the final 4 h. IFNγ levels in supernatants were measured by ELISA and intracellular staining was performed to analyze cell proliferation, intracellular cytokines and transcription factors by FACS. The percentage of IFNγ-producing RBD/CMV-expanded CD4 T cells was plotted against the percentage of IFNγ-producing RBD+Extract A-expanded CD4 T cells or the Foxp3:Tbet ratio in RBD+Extract A-expanded CD4 T cells. R² and p-values are shown on linear regression plots. *p<0.05, **p<0.01. Fig. 11B shows a plot of PPD/CMV spot counts against percent of IFNγ-producing RBD+ Extract A expanded CD4 or CD8 T cells. R² and p-values are shown on linear regression plots. *p<0.05.
[Fig. 12]

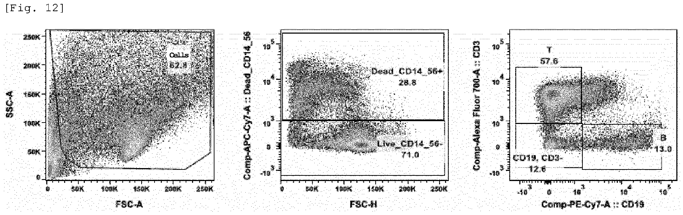

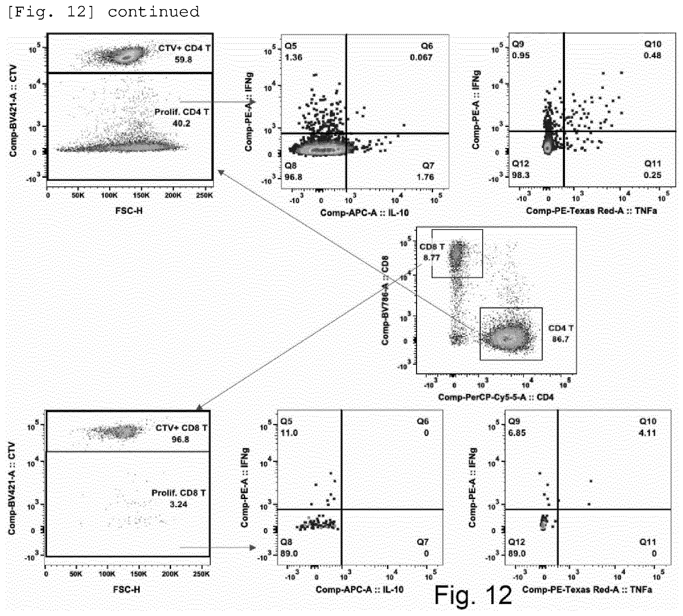

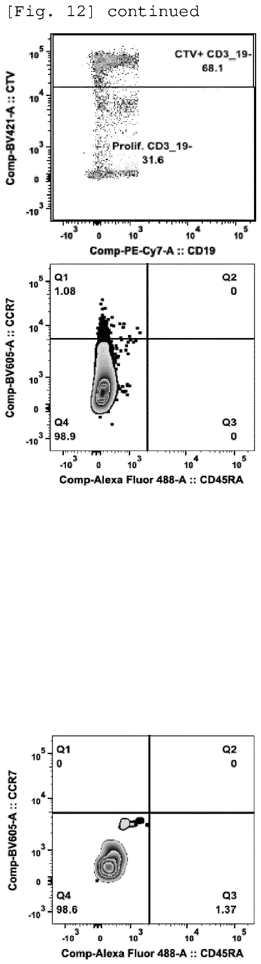

   Fig. 12 shows gating strategy for intracellular staining of cytokines. After gating cells based on FSC and SSC, live CD14/56- cells were gated. T cells and B cells were gated based on CD3 and CD19 expression from live/CD14/56- gate. CD4 and CD8 T cells were gated from CD3+CD19- cells, upon which proliferated CD4 and CD8 T cells were gated based on the CTV intensity (e.g., CTV-diluted cells represent proliferated cells). IFN-γ+, IL-10+ or TNFα+ cells were gated from proliferated CD4 or CD8 T cells. In addition, memory phenotype was determined by CCR7 and CD45RA expression in proliferated CD4 and CD8 T cells.
[Fig. 13]

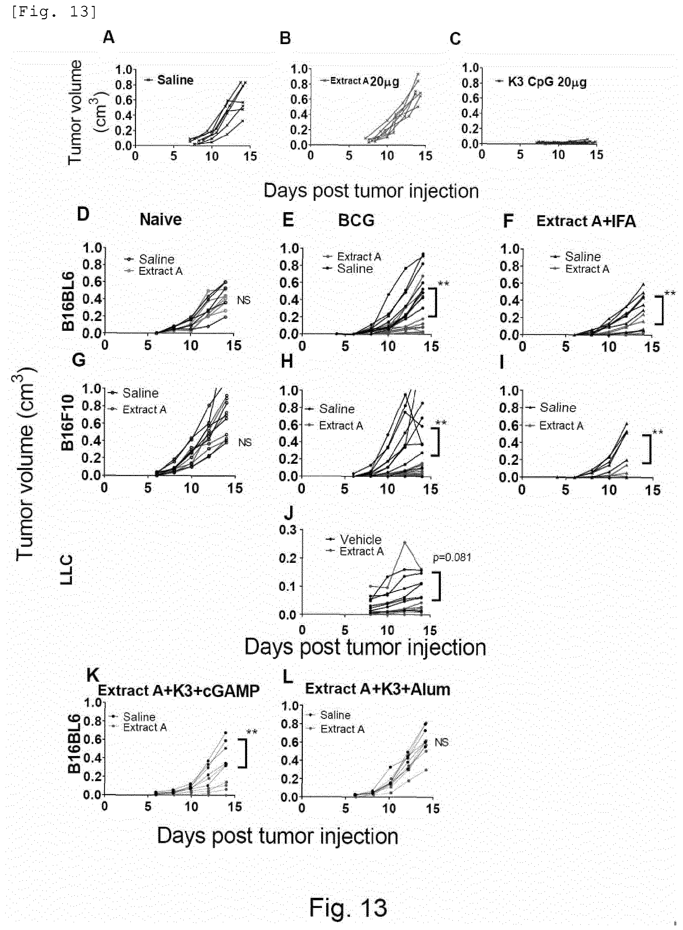

   Fig. 13 shows that Extract A exerts anti-tumor effect only in pre-immunized mice. B16-BL6 cells were subcutaneously inoculated into the lower back of naive mice on day 0. Starting from day 2, mice were administered intratumorally of saline (Fig. 13A), Extract A (20 pg) (Fig. 13B) or K3 CpG (20 pg) (Fig. 13C) every other day, and the tumor growth was measured by using digital calipers for two weeks. B16-BL6 (Fig. 13D)or B16-F10 (Fig. 13G) cells were subcutaneously inoculated into the lower back of the naive mice on day 0. Starting from day 2, mice were administered by subcutaneous injections with saline or Extract A (20 pg) every other day, and the tumor growth was measured using digital calipers for 2 weeks. B16-BL6)Fig. 13E), B16-F10 (Fig. 13H) or LLC (Fig. 13J) cells were subcutaneously inoculated into the lower back of mice that were immunized with BCG 5 or two weeks prior to tumor inoculation. Extract A was administrated subcutaneously starting from 8 days before tumor injection for 2 weeks every other day, and the tumor growth was measured using digital calipers for 2 weeks. On day 0, B16-BL6 (Fig. 13F) or B16-F10 (Fig. 13I) cells were subcutaneously inoculated into the lower back of the mice, which were immunized with Extract A emulsified in incomplete Freund's adjuvant (IFA) administered intradermally 4 weeks and 2 weeks prior to tumor inoculation at base of their tail. Then Extract A or saline was injected into the mice starting from 8 days before tumor inoculation until euthanization, and the tumor growth was measured using digital calipers for 2 weeks. On day 0, B16-BL6 cells were subcutaneously inoculated into the lower back of the mice, which were immunized with Extract A + K3 + cGAMP (Fig. 13K), or Extract A + K3 + Alum Extract A (Fig. 13L) 4 weeks and 2 weeks prior to tumor inoculation at base of their tail. Then, Extract A or saline was injected into the mice starting from 8 days before tumor inoculation until euthanization, and the tumor growth was measured using digital calipers for 2 weeks. Data are shown as mean ± SE. * p < 0.05, ** p < 0.01 (student's t-test).
[Fig. 14]

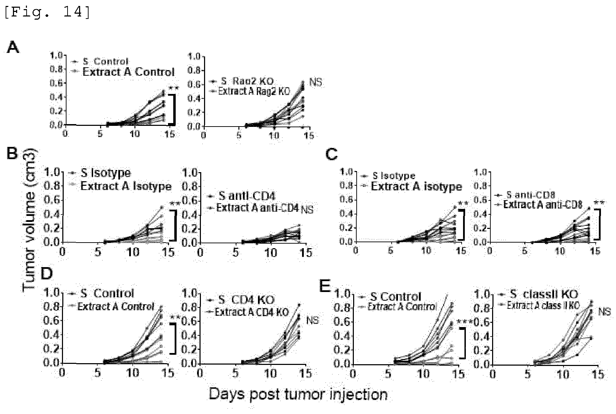

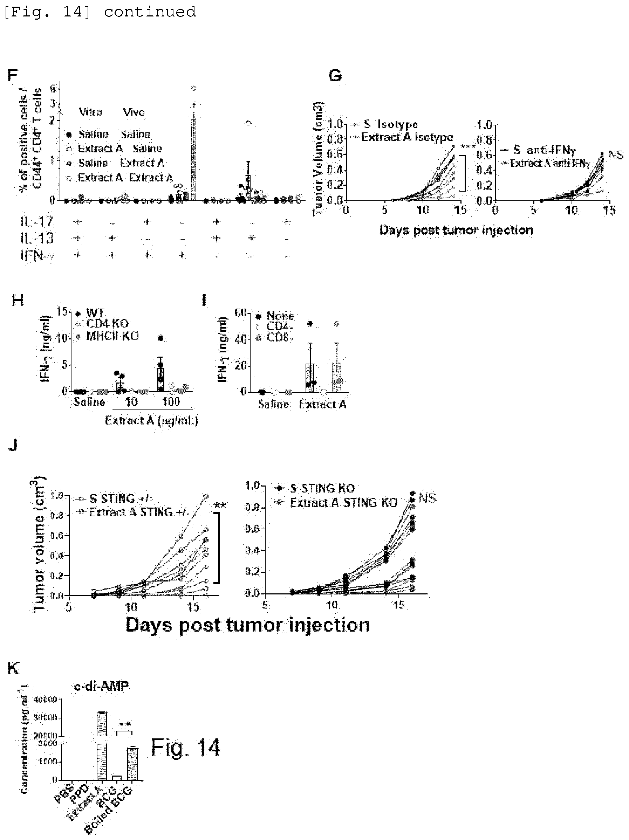

   Fig. 14 shows that anti-tumor immunity via interferone (IFN)-γ-secreting CD4 T cells and STING pathway activation induced by Mycobacterium tuberculosis (Mtb)-derived STING agonists in BCG-immunized tumor-bearing mice is induced. B16-BL6 cells were subcutaneously inoculated into the lower back of mice that were immunized with BCG 5 and two weeks prior to tumor inoculation. Extract A was administrated subcutaneously every other day starting from 8 days before tumor injection, and the tumor growth was measured in wild type (WT), isotype control antibody-treated mice, Rag2 knock-out (KO) mice (Fig. 14A), CD4+cell-depleted mice (Fig. 14B), CD8+cell-depleted (Fig. 14C), CD4+ KO (Fig. 14D), MHC class II KO (Fig. 14E), IFN-γ-depleted mice (Fig. 14G), or STING heterozygous or KO mice (Fig. 14J), respectively. (Fig. 14F) Splenocytes isolated from WT tumor-bearing BCG-immunized mice and Extract A- or saline-treated mice were stimulated with saline or Extract A, and intracellular staining for IFN-γ, IL-13 and IL-17 was performed. The percentages of CD44+CD4+ cells producing each cytokine were plotted as bar graph, in which each dot corresponds to individual mice. Splenocytes isolated from WT, CD4 KO or MHC class II KO tumor-bearing BCG-immunized mice and Extract A-treated mice were stimulated with saline or indicated doses of Extract A. IFN-γ levels in the supernatant were measured by ELISA. (Fig. 14I) Splenocytes were isolated from WT tumor-bearing BCG-immunized mice and Extract A-treated mice that were depleted of CD4+ or CD8+ cells using AUTOMACS and stimulated with saline or indicated doses of Extract A. IFN-γ levels in the supernatant were measured by ELISA. Data are plotted as bar graphs, in which each dot corresponds to individual mice. (Fig. 14K) BCG vaccine resuspended in PBS was boiled for 30 min at 95 °C to lyse and release the contents of live-attenuated M. bovis bacteria of BCG vaccine. Concentrations of c-di-AMP in the Extract A solution, PPD antigen solution, or BCG vaccine solution with or without boiling were measured by ELISA. Data are shown as mean ± SE. * p < 0.05, ** p < 0.01 (student's t-test).
[Fig. 15]

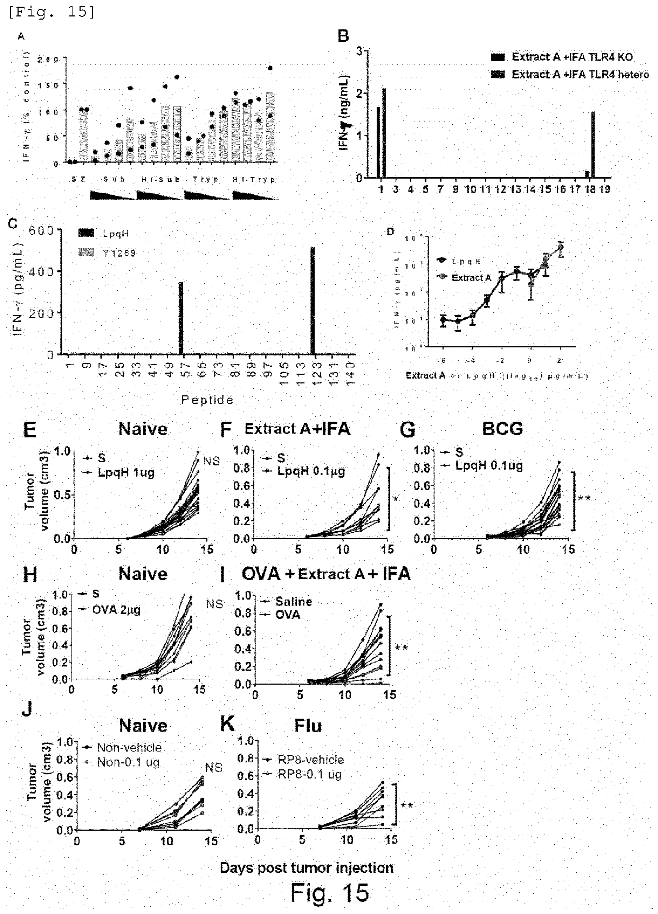

   Fig. 15 shows that Injection of tumor-unrelated protein present in Extract A suppresses tumor growth in BCG-immunized mice. (Fig. 15A) Splenocytes derived from BCG-immunized mice and Extract A-treated mice were stimulated with saline (S), Extract A (Z), subtilisin-treated Extract A (Sub), heat-inactivated subtilisin-treated Extract A (HI-Sub), trypsin-treated Extract A (Trp), or heat-inactivated trypsin-treated Extract A (HI-Trp). IFN-γ production was measured by ELISA. The triangle indicates the concentrations of proteases used in the experiments. Initial Extract A-induced IFN-γ production was considered 1000 (% control), and the percentage values for each treatment were calculated based on the initial levels of Extract A-induced expression of IFN-γ. (Fig. 15B) Splenic IFN-γ secretion from IFA-emulsified Extract A-immunized WT or TLR4 KO mice in response to antigens present in Extract A (Table 1) was measured by ELISA. (Fig. 15C) Splenic IFN-γ secretion from IFA-emulsified Extract A-immunized mice in response to overlapping peptides of LpqH or Y1269 was measured by ELISA. (Fig. 15D) Dose response of splenic IFN-γ secretion in response to Extract A or LpqH in IFA-emulsified Extract A-immunized mice. (Fig. 15E) B16-BL6 cells were subcutaneously inoculated into the lower back of the naive mice on day 0. LpqH (Fig. 15E) or OVA (Fig. 15H) was administrated subcutaneously every other day starting from 8 days before tumor inoculation, and the tumor growth was measured using digital calipers for 2 weeks. (Fig. 15F) On day 0, B16-BL6 cells were subcutaneously inoculated into the lower back of mice immunized with IFA-emulsified Extract A. Mice were stimulated with subcutaneous injections of Lpqh (Fig. 15F) or OVA (Fig. 15I) every other day starting from 12 (Lpqh) or 8 days (OVA) before tumor inoculation. Tumor growth was measured using digital calipers for 2 weeks. (Fig. 15G) B16-BL6 cells were subcutaneously inoculated to the lower back of the BCG-immunized mice on day 0. LpqH was administrated subcutaneously every other day starting from 12 days before tumor inoculation and the tumor growth was measured by using digital calipers for 2 weeks. Influenza (Flu) virus (PR8 strain) was administrated by intra-nasal route and B16-BL6 was inoculated 5 weeks after infection. Virion Flu vaccine was administrated to (Fig. 15J) naive mice, or (Fig. 15K)Flu-immunized mice subcutaneously twice a week starting from 8 days before tumor inoculation. Tumor growth was measured by using digital calipers for 2 weeks. Data are shown as mean ± SE. * p < 0.05, ** p < 0.01 (student's t-test).
[Fig. 16]

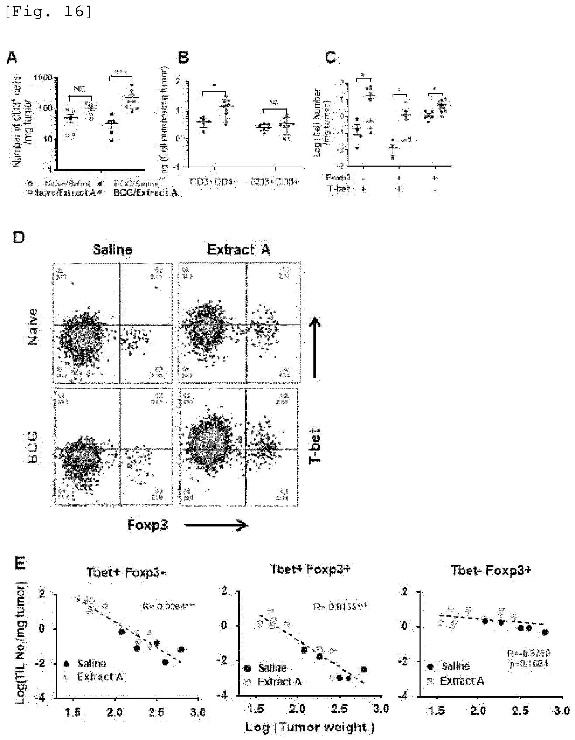

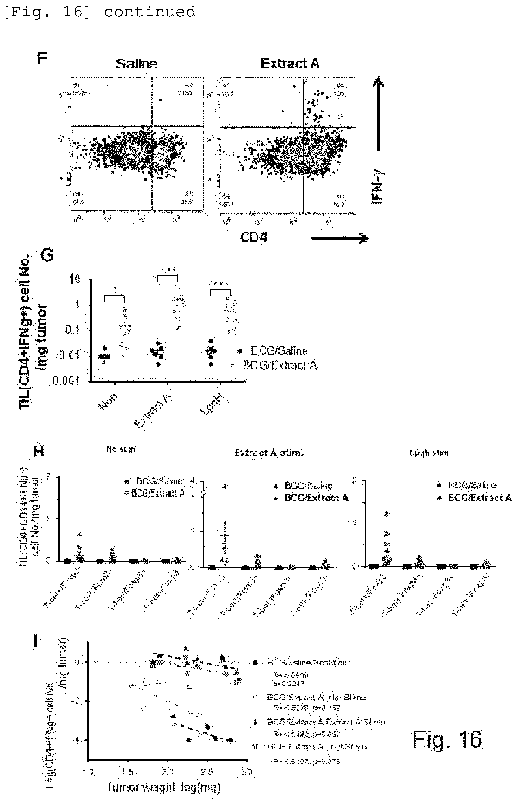

   Fig. 16 shows that Extract A increases IFN-γ-producing Th1 cells in tumor microenvironment. Extract A was administrated to BCG-immunized mice via subcutaneous route every other day starting from 8 days before B16-BL6 tumor inoculation. After 14 days, tumor-bearing mice were euthanized, and the tumor tissues were harvested. The TILs (abbreviation of tumor infiltrating lymphocyte, tumor-reactive lymphocytes for lymphocytes that infiltrate tumors) purified from the tumor tissues were analyzed by intracellular staining and FACS. (Fig. 16A) Number of TILs (CD45+CD3+) per mg of tumor tissues, (Fig. 16B) Number of CD4+ and CD8+ TILs per mg of tumor tissues, or (Fig. 16C) number of T-bet and Foxp3 positive or negative TILs per mg of tumor tissues were plotted. (Fig. 16D) Representative FACS plots showing TIL gating. (Fig. 16E) Correlation between tumor weight and the number of T-bet+Foxp3- (left), T-bet+Foxp3+ (center), T-bet-Foxp3+ (right) TILs per mg of tumor tissues was plotted. (Fig. 16F) Representative FACS plots showing IFN-γ-producing CD4 T cells in TILs derived from saline- or Extract A-treated mice. (Fig. 16G) IFN-γ producing CD4 T cells in the TILs stimulated with control, Extract A or Lpqh are plotted. (Fig. 16H) IFN-γ producing CD44+ memory CD4 T cells in the TILs expressing T-bet and/or Foxp3 after stimulation with control, Extract A, or Lpqh are plotted. (Fig. 16I) Correlation between tumor weight and number of IFN-γ+CD4 T cells per mg of tumor after stimulation with Extract A, or Lpqh was plotted. Data are shown as mean ± SE. * p < 0.05, ** p < 0.01 (student's t-test).
[Fig. 17]

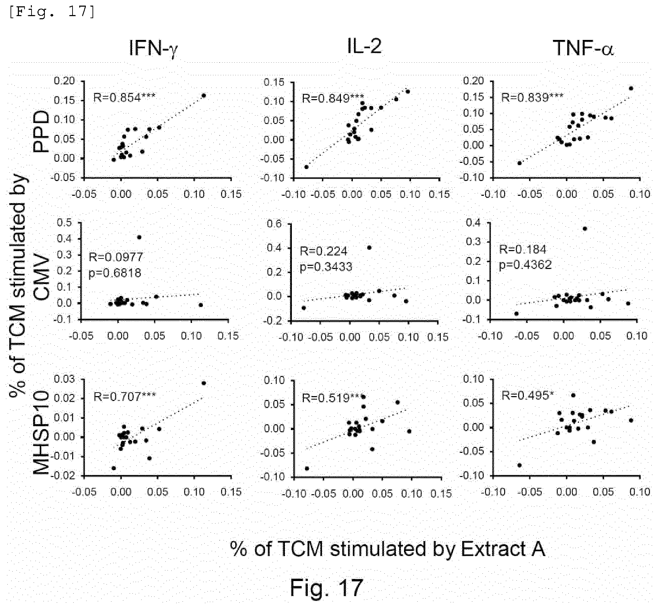

   Fig. 17 shows that Human PBMCs with memory T cells against PPD respond to Extract A. Human PBMCs were cultured in 96-well plates at a density of 1 × 10⁶ cells/well and rested in the CO₂ incubator overnight. Then, Extract A (100 µg/mL), PPD (3 µg/mL), CMV pp65 overlapping peptides (3 µg/mL) or recombinant Mtb proteins (each 10 µg/mL) were added. A total of 1 ug/mL anti-CD28 (CD28.2), Golgi Plug, and Golgi Stop were also added at the same time as the stimulants, and the cells were stimulated for 6 h, after which the PBMCs were stained for intracellular molecules and analyzed by FACS. Correlation graphs showing IFN-γ, IL-2, or TNF-α produced by TCMs stimulated with PPD (top)/CMV (middle)/MHSP10 (bottom) versus TCMs stimulated with Extract A were plotted.
[Fig. 18]

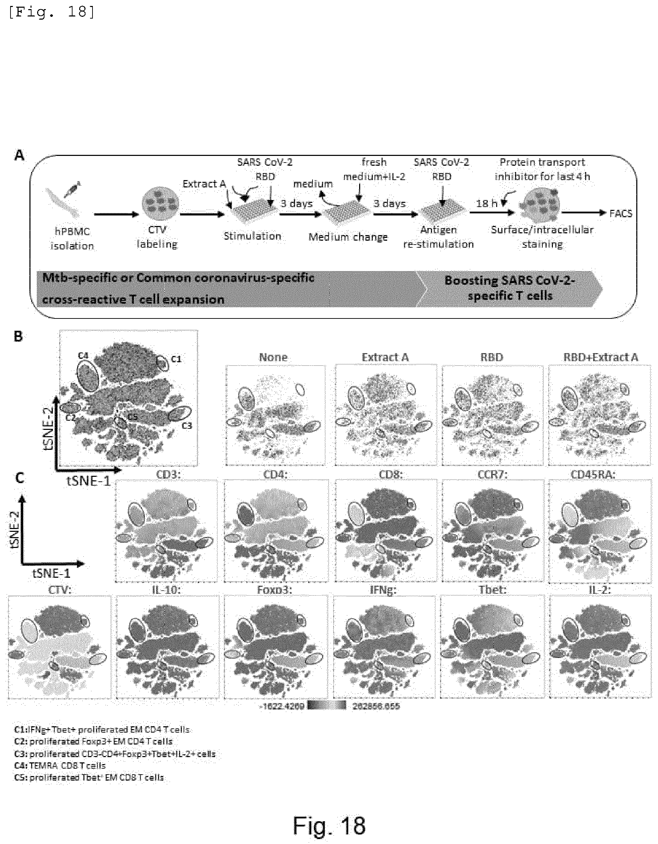

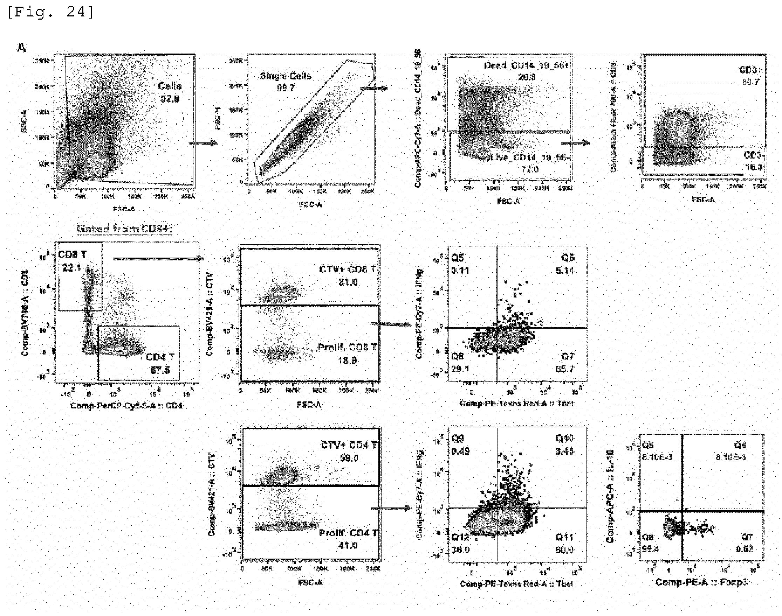

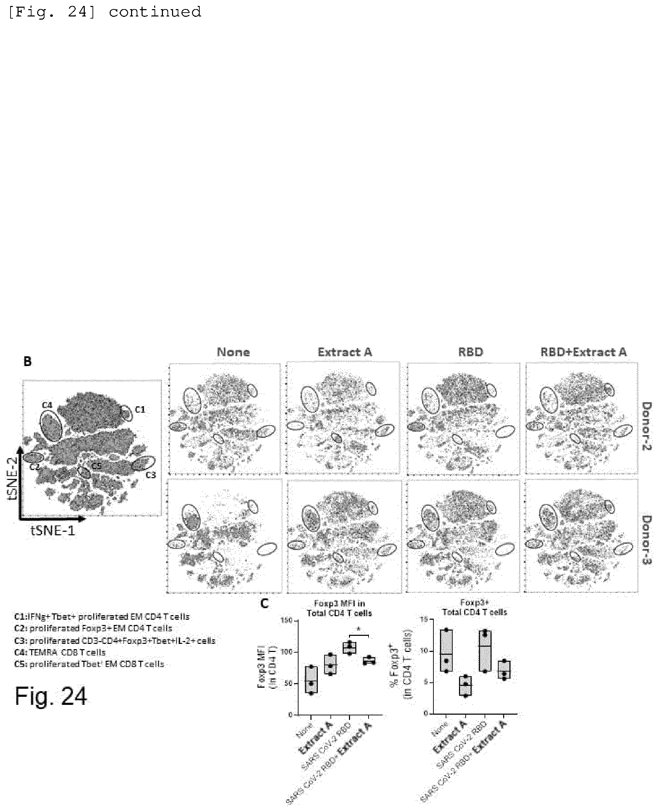

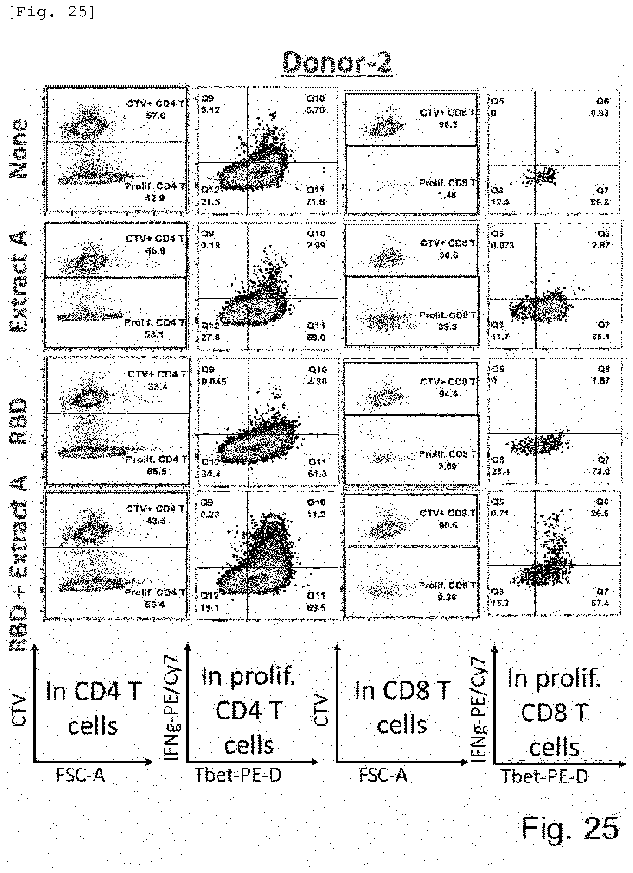

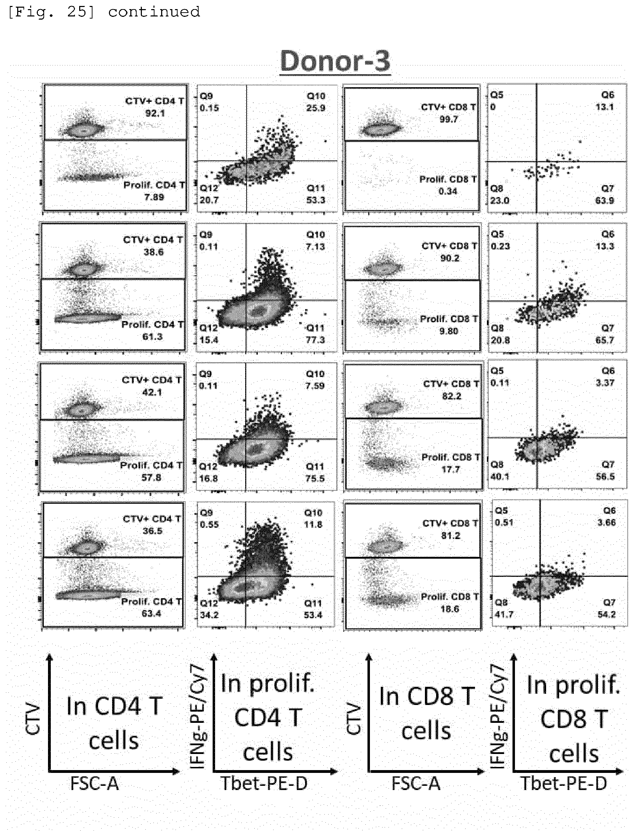

   Fig. 18 shows that Extract A and SARS CoV-2 antigen combination induces expansion proliferation of unique T cell populations in unimmunized PBMCs. (Fig. 18A) Human PBMC culturing protocol. (Fig. 18B) Human PBMCs derived from three healthy individuals were labeled with CTV and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 6, the cells were re-stimulated with SARS CoV-2 RBD antigen for 18 h, during which the protein transport inhibitor was added to the culture for the last 4 h. Cell proliferation, intracellular cytokines, and transcription factors were analyzed by FACS upon intracellular staining. tSNE analysis was performed with live, CD14/19/56- cells by FlowJo after concentration of all samples from three individuals, and representative tSNE plots from one individual together with the tSNE plot of all concentrated samples are shown. (Fig. 18C) Heatmaps show the expression of surface and intracellular markers including cytokines and transcription factors in each specific population. Scale bar shows the expression levels of each marker from low (dark blue) to high (red). See also Figs. 24 and 25.
[Fig. 19]

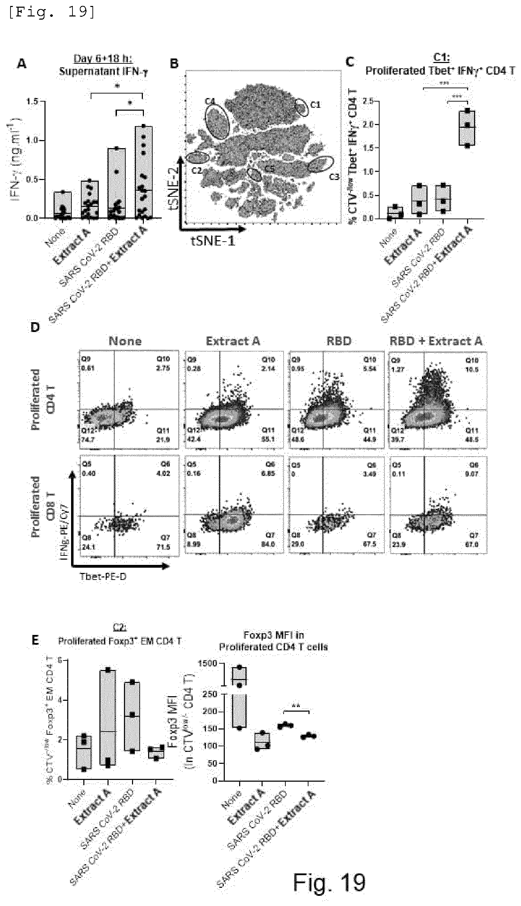

   Fig. 19 shows that Extract A boosts anti-SARS-CoV-2-specific T cell responses in unimmunized PBMCs via modulation of Treg/Th1 balance. (Fig. 19A) Human PBMCs from 21 healthy individuals were labeled with CTV and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 6, the cells were re-stimulated with SARS CoV-2 RBD antigen for 18 h, during which a protein transport inhibitor was added to the culture at last for 4 h. IFN-γ levels of supernatants after antigen re-stimulation on day 6 were measured by ELISA, and the data is represented as box-and-whisker plot. SD. * p<0.05; ** p<0.01. (Fig. 19B) Human PBMCs derived from three healthy individuals were labeled with CTV and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 6, the cells were re-stimulated with SARS CoV-2 RBD antigen for 18 h, during which a protein transport inhibitor was added to the culture for the last 4 h. Cell proliferation, intracellular cytokines, and transcription factors were analyzed by FACS upon intracellular staining. tSNE analysis was performed on live, CD14/19/56- cells by FlowJo^{™} after concentration of all samples from three individuals, and the tSNE plot of all concentrated samples is shown. (Fig. 19C) Frequencies of C1 population derived from the tSNE analysis were plotted in box-and-whisker plots using the data from all three individuals. * p<0.05; ** p<0.01; *** p<0.001. (Fig. 19D) FACS plots show Tbet expression and IFN-γ production from the proliferated CD4 and CD8 T cells (CTVlow/negative gate). (Fig. 19E) Frequencies of C2 population from the tSNE analysis and Foxp3 MFI in the proliferated CD4 T cells (CTVlow/negative gate) were plotted in box-and-whisker plots using the data from all three individuals. * p<0.05; ** p<0.01. See also Figs. 24 and 25.
[Fig. 20]

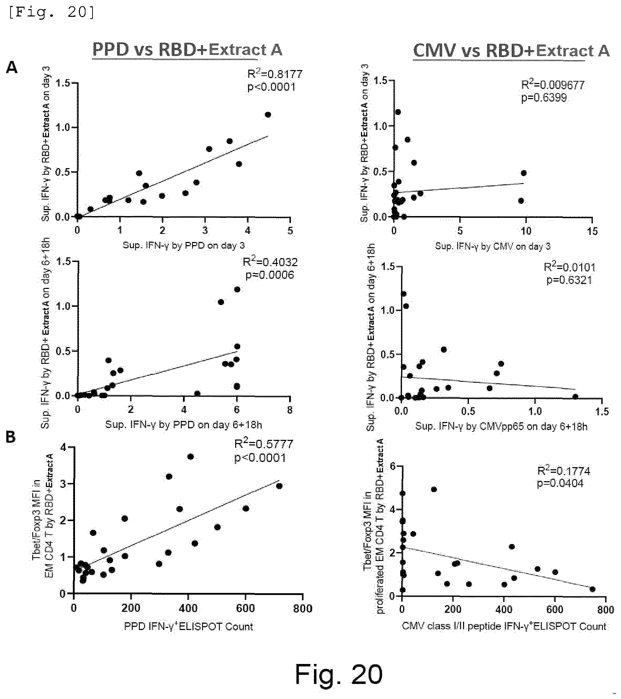

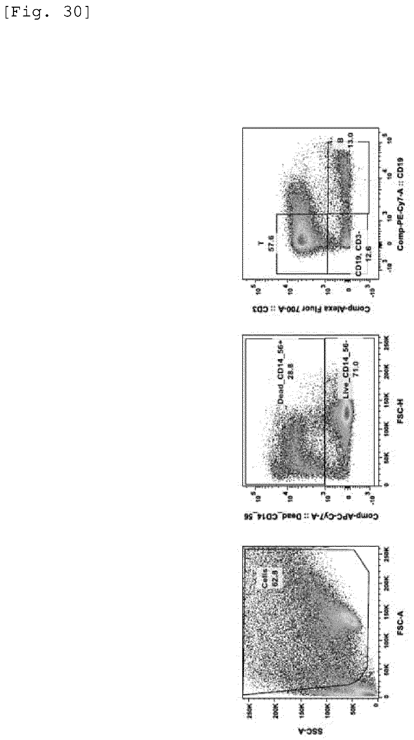

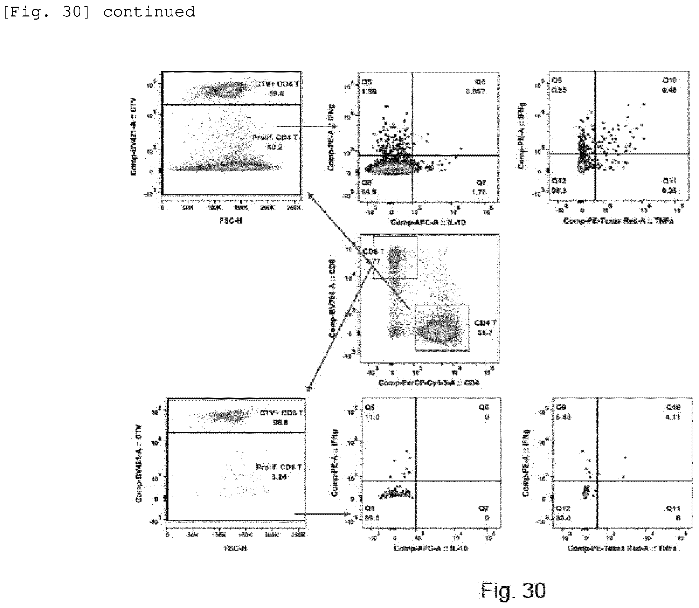

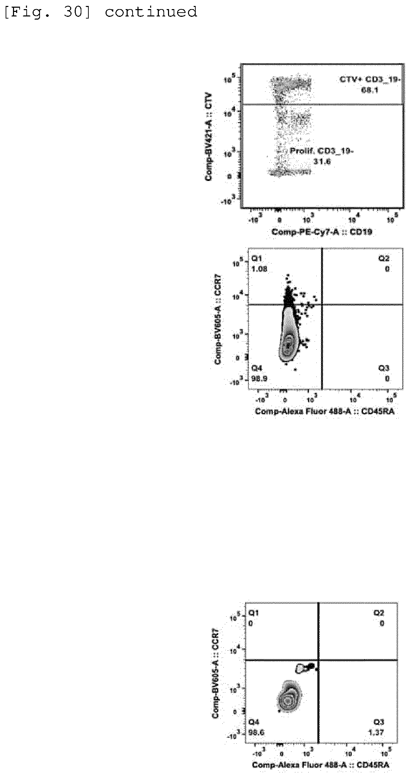

   Fig. 20 shows that Pre-existing Mtb-specific T cell memory is required for Extract A-mediated promotion of anti-SARS-CoV-2-specific T cell responses. (Fig. 20A) Human PBMCs derived from healthy individuals with high/low PPD/CMV reactivity were labeled with CTV and stimulated with CMVpp65, PPD (1 pg/ml), Extract A (30 pg/ml), SARS CoV-2 RBD (1 pg/ml), or combination of RBD+Extract A for 3 days, after which the media was replaced with a fresh medium containing IL-2, and the cells were cultured for additional 3 days. On day 6, the cells were re-stimulated with SARS CoV-2 RBD antigen for 18 h, during which a protein transport inhibitor was added to the medium for the final 4 h. Supernatant IFN-γ levels were measured by ELISA, and cell proliferation, intracellular cytokines and transcription factors were analyzed by FACS upon intracellular staining. (Fig. 20A) IFN-γ production induced by RBD+PPD was plotted against IFN-γ production induced by PPD or CMV on days 3 and 6 after restimulation. R² and p values are shown on the linear regression plots. * p<0.05; ** p<0.01. (Fig. 20B) Tbet/Foxp3 MFI ratio in RBD+Extract A-proliferated EM CD4 T cells was plotted against PPD ELISPOT count values. R² and p values are shown on the linear regression plots. * p<0.05. See also Fig. 30 and Tables 3A-B.
[Fig. 21]

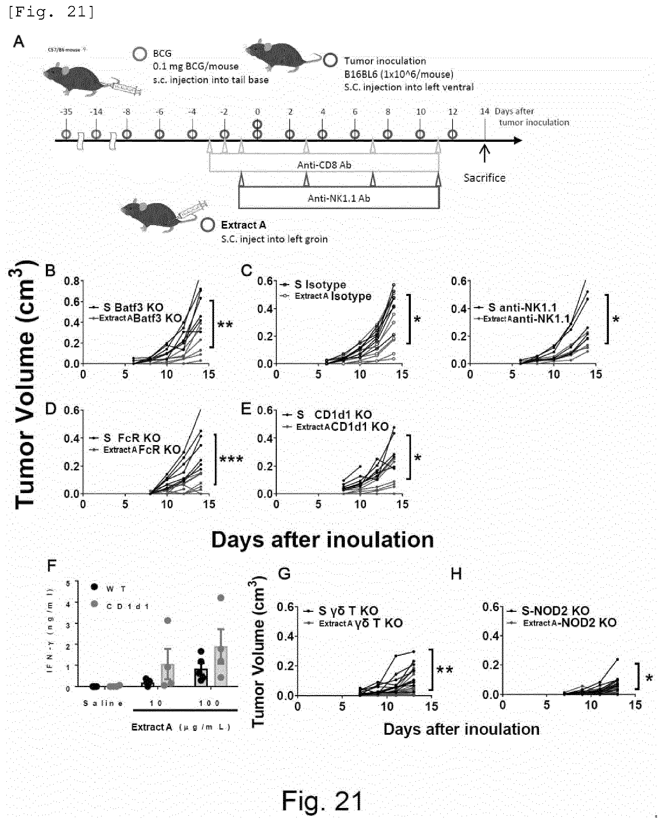

   (Fig. 21A) Experimental procedure for cell depletion in BCG-immunized and Extract A-immunized mice. BCG was subcutaneously injected to the mice twice before tumor inoculation, and B16-BL6 cells were inoculated into the mice on day 0. Extract A was administrated subcutaneously every other day starting from 8 days before tumor injection. Depletion antibodies were injected on the days shown in the figure. (Fig. 21B-H) Experiments were performed as described in Fig. 21A, and (Fig. 21B-E) Batf3 KO, NK-1.1- depleted, FcγR KO, CD1d1 KO, (Fig. 21G-H) γδ T cell KO, or NOD2 KO mice were used. (Fig. 21F) IFN-γ production after stimulation with saline or Extract A in the BCG-immunized mice and Extract A-treated mice splenocytes was measured by ELISA. Data are shown as mean ± SE. * p < 0.05, ** p < 0.01 (student's t-test).
[Fig. 22]

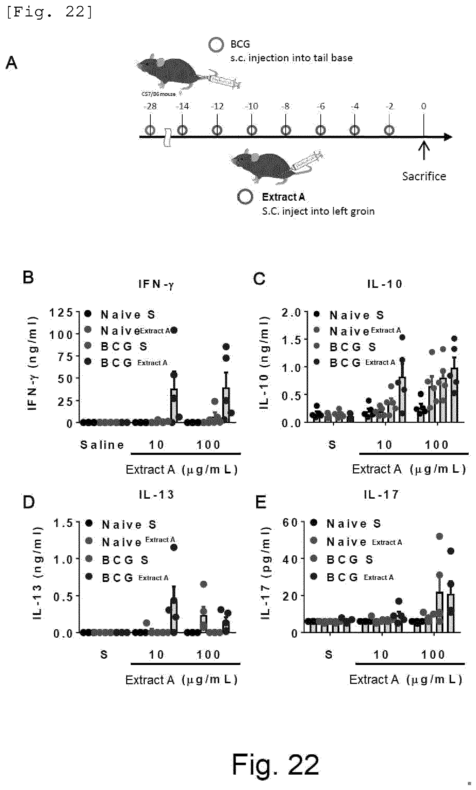

   Fig. 22 shows Extract A-induced cytokine secretion from splenocytes of BCG-immunized mice. (Fig. 22A) Experimental Procedure; mice were immunized with BCG four weeks before euthanization. Saline or Extract A was subcutaneously injected into the mice every other day starting from 2 weeks before euthanization. Splenocytes were prepared and stimulated with Extract A (10 or 100 µg/m). (Fig. 22B-E) Concentrations of cytokines were quantified with ELISA. Data are shown as mean ± SE. * p < 0.05, ** p < 0.01 (student's t-test).
[Fig. 23]

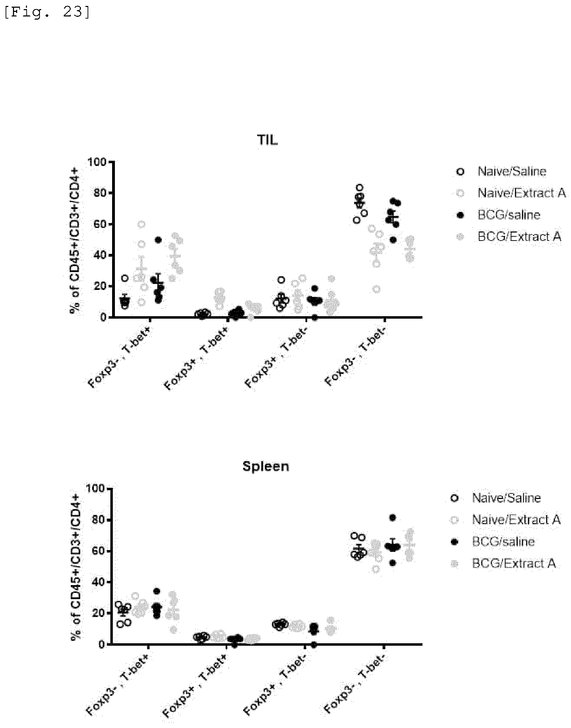

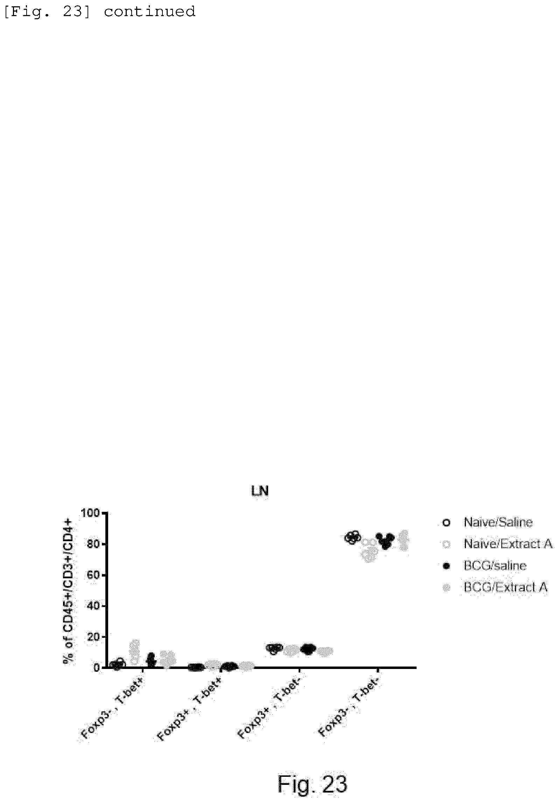

   Fig. 23 shows that Extract A modulates Treg/Th1 balance in the tumor microenvironment but not systemically. Extract A was administrated to BCG-immunized mice via subcutaneous route every other day starting from 8 days before B16-BL6 tumor inoculation. After 14 days, the tumor-bearing mice were euthanized, and the tumor tissues were harvested. The TILs purified from the tumor tissues were analyzed by intracellular staining and FACS. Percentages of CD45+CD3+CD4+ cells in TILs expressing T-bet and Foxp3 were plotted.
[Fig. 24]
   Fig. 24 shows Extract A and SARS CoV-2 antigen combination-induced expansion of unique T cell populations and tSNE plots of donor-2 and-3 in unimmunized PBMCs. (Fig. 24A) Gating strategy for the intracellular staining of transcription factors and cytokines on day seven. After gating cells based on FSC and SSC, live CD14/19/56- cells were gated from the single cell gate. CD4 and CD8 T cells were gated from CD3+ cells, upon which the CD4 or CD8 T cells were gated based on the CTV intensity (e.g., CTV diluted cells represent proliferated cells). Tbet+ and IFN-γ+ cells were gated from proliferated CD4 or CD8 T cells. Foxp3 and IL-10 was gated from proliferated CD4 T cells. (Fig. 24B) Human PBMCs derived from three healthy individuals were labeled with CTV and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 6, the cells were cultured with SARS CoV-2 RBD antigen for 18 h, during which a protein transport inhibitor was added to the culture for the final 4 h. Cell proliferation, intracellular cytokines and transcription factors were analyzed by FACS upon intracellular staining. The tSNE analysis was performed with live, CD14/19/56- cells by FlowJo after concentration of all samples from three individuals, and representative tSNE plots from donor-2 and -3 together with the tSNE plot of all concentrated samples are shown. (Fig. 24C) Foxp3 MFI and frequencies of Foxp3+ in total CD4 T cells were plotted in box-and-whisker plots using the data derived from all three individuals. * p<0.05.
[Fig. 25]
   Fig. 25 shows that Extract A boosts anti-SARS-CoV-2-specific T cell responses in unimmunized PBMCs via modulation of Treg/Th1 balance. Human PBMCs from three healthy individuals were labeled with CTV and stimulated with Extract A (30 pg/ml), SARS CoV-2 RBD (1 pg/ml), or their combination for 3 days, after which the media was replaced with a fresh medium containing IL-2, and the cells were cultured for additional 3 days. On day 6, the cells were re-stimulated with SARS CoV-2 RBD antigen for 18 h, during which a protein transport inhibitor was added to the culture for the final 4 h. Cell proliferation, intracellular cytokines, and transcription factors were analyzed by FACS upon intracellular staining. FACS plots show CD4 and CD8 T cell proliferation as well as Tbet expression and IFN-γ production from the proliferated CD4 and CD8 T cells (CTV^{low/negative} gate) .
[Fig. 26]

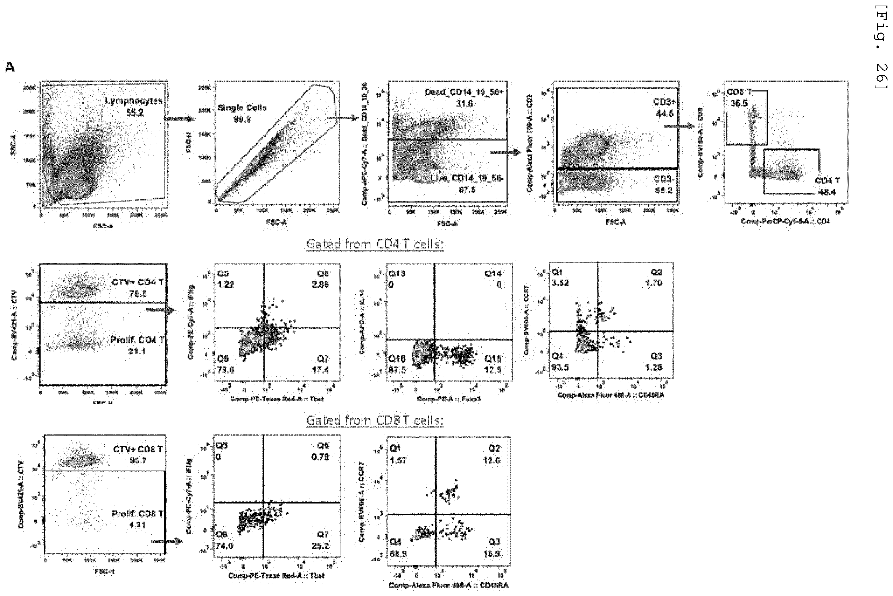

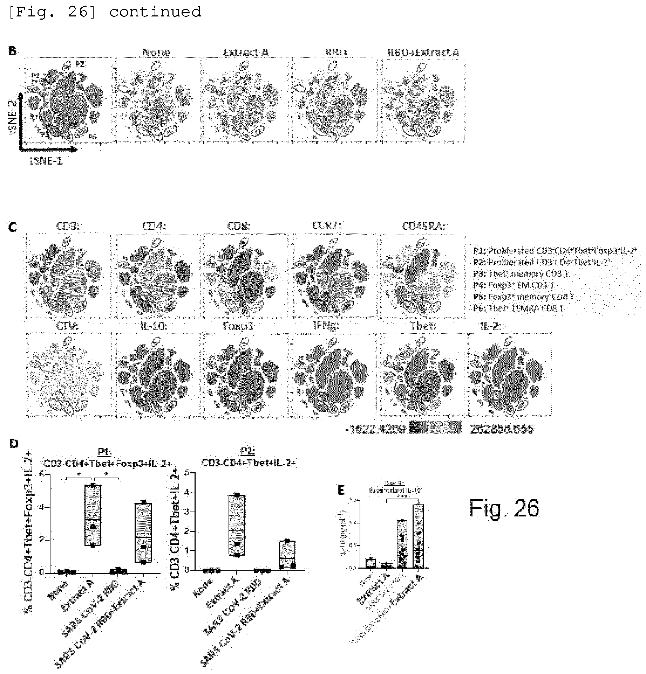

   Fig. 26 shows that at earlier time points, Extract A induces the proliferation of innate immune cells with suppressive function capable of producing IL-10. (Fig. 26A) Gating strategy for intracellular staining of transcription factors and cytokines. After gating cells based on FSC and SSC, live CD14/19/56- cells were gated from single cells gate. CD4 and CD8 T cells were gated from CD3+ cells, upon which CD4 or CD8 T cells were gated based on CTV intensity (e.g. CTV diluted cells represent proliferated cells). Tbet+ and IFN-γ+ cells were gated from proliferated CD4 or CD8 T cells. Foxp3 and IL-10 were gated from proliferated CD4 T cells. In addition, memory phenotype was determined by CCR7 and CD45RA expression in proliferated CD4 and CD8 T cells. (Fig. 26B) Human PBMCs from three healthy individuals were labeled with CTV and stimulated with Extract A (30 pg/ml), SARS CoV-2 RBD (1 pg/ml), or their combination for 3 days after which the media was replaced with a fresh medium containing IL-2 and cultured for additional 3 days. On day 3, cells were re-stimulated with SARS CoV-2 RBD antigen for 6 h in the presence of protein transport inhibitor. Cell proliferation, intracellular cytokines and transcription factors were analyzed by FACS upon intracellular staining. tSNE analysis was performed on live, CD14/19/56-cells by FlowJo after concatenation of all samples from three individuals and representative tSNE plots from one individual together with the tSNE plot of all concatenated samples are shown. (Fig. 26C) Heatmaps show the expression of surface and intracellular markers including cytokines and transcription factors in each specific population. Scale bar shows expression levels of each marker from low (dark blue) to high (red). (Fig. 26D) Frequencies of P1 and P2 population from the tSNE analysis were plotted in box-and-whisker plots by using the data from all three individuals. * p<0.05; ** p<0.01. (Fig. 26E) Human PBMCs from 21 healthy individuals were labeled with CTV and stimulated with Extract A (30 pg/ml), SARS CoV-2 RBD (1 pg/ml), or their combination for 3 days after which the media was replaced with a fresh medium containing IL-2 and cultured for additional 3 days. On day 6, cells were re-stimulated with SARS CoV-2 RBD antigen for 18 h, during which protein transport inhibitor was added to the culture for the final 4h. IL-10 levels of supernatants of day 3 were measured by ELISA and the data was represented as box-and-whisker plot. SD. * p<0.05; ** p<0.01.
[Fig. 27]

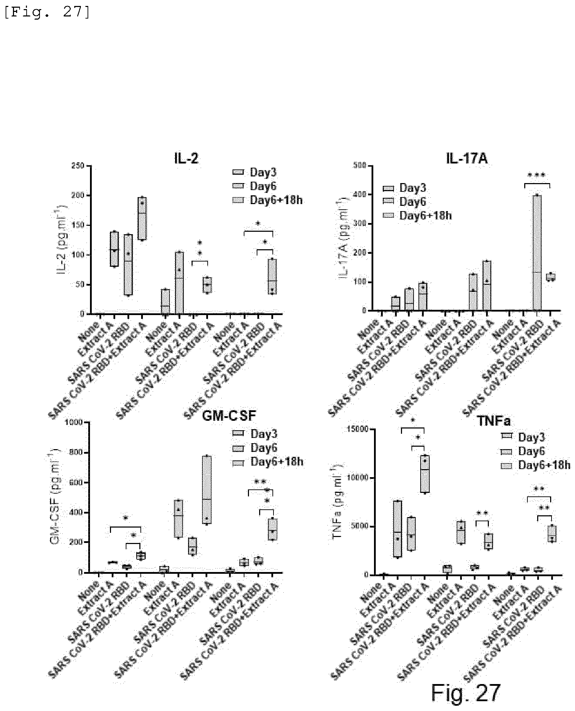

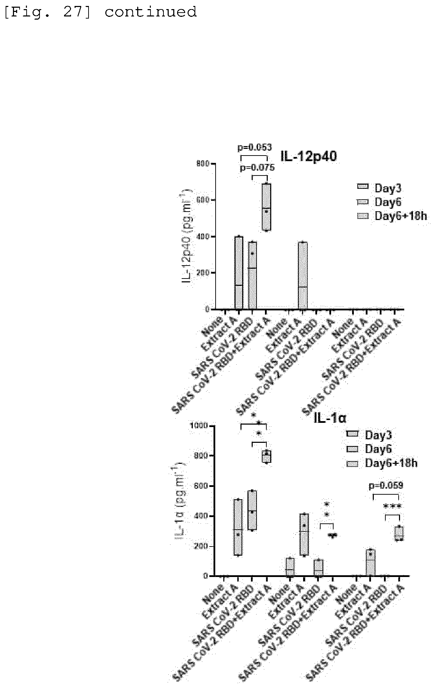

   Fig. 27 shows Bio-Plex analysis of time course PBMC stimulation. Human PBMCs derived from three healthy individuals were labeled with CTV and stimulated with Extract A (30 pg/ml), SARS CoV-2 RBD (1 pg/ml), or their combination for 3 days, after which the media was replaced with the fresh medium containing IL-2, and the cells were cultured for additional 3 days. On day 6, the cells were re-stimulated with SARS CoV-2 RBD antigen for 18 h (the final 4 h with protein transport inhibitor), and the supernatants derived from before and after re-stimulations were collected and cytokine levels were measured by 48-plex Bio-Plex assay.
[Fig. 28-1]

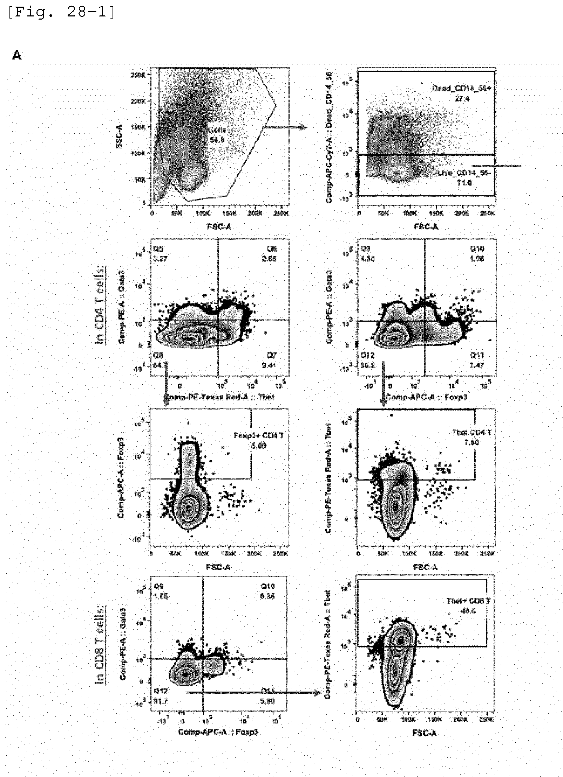

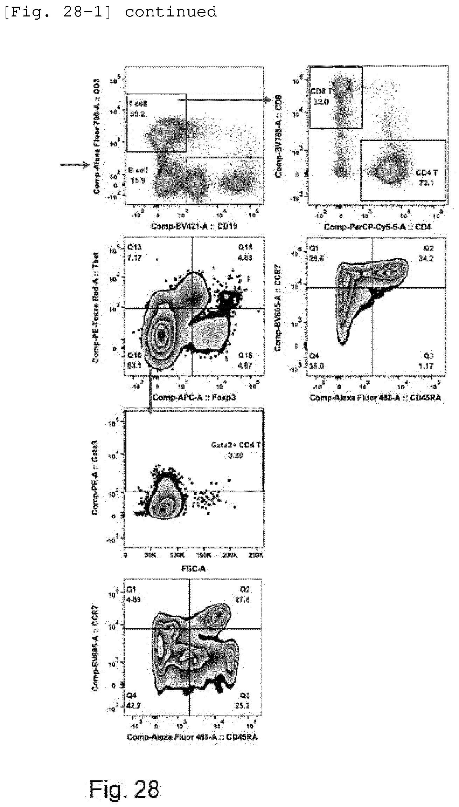

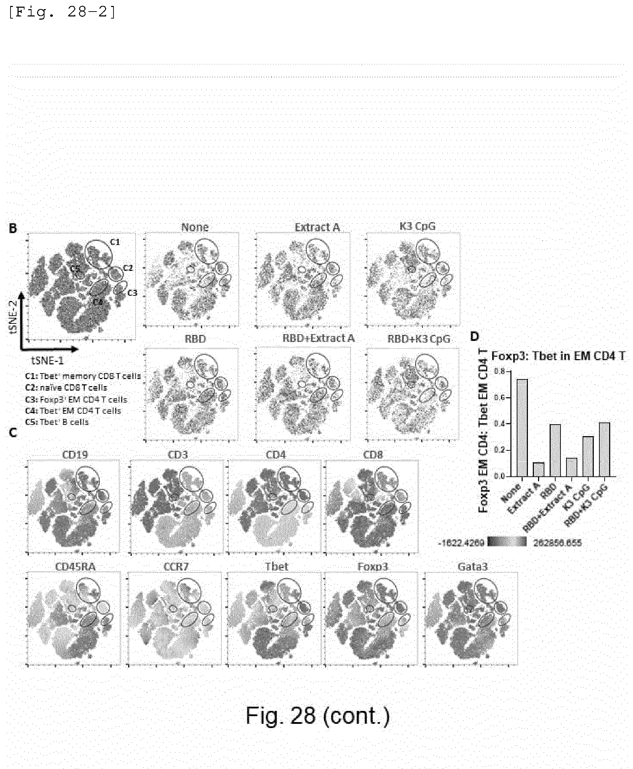

   Fig. 28 shows that Extract A-mediated SARS CoV-2-specific T cell enhancement cannot simply be attributed to the adjuvant effect (by transcription factor analysis). (Fig. 28A) Human PBMCs derived from a healthy individual were labeled and stimulated with Extract A (30 pg/ml), SARS CoV-2 RBD (1 pg/ml), or their combination for 3 days, after which the media was replaced with a fresh medium containing IL-2, and the cells cultured for additional 3 days. On day 6, the cells were re-stimulated with SARS CoV-2 RBD antigen for 18 h. Transcription factors were analyzed by FACS upon intracellular staining. After gating the cells based on FSC and SSC, live CD14/56-cells were gated from the single cell gate. T cells and B cells were gated based on CD3 and CD19 expression from live/CD14/56-gate. CD4 and CD8 T cells were gated from CD3+ cells. Tbet+, IFN-γ+ and Gata3+ cells were gated from CD4 or CD8 T cells, which are negative for other two transcription factors (e.g. Tbet+ cells are gated from Gata3-Foxp3- cells). In addition, memory phenotype was determined by CCR7 and CD45RA expression in proliferated CD4 and CD8 T cells.
[Fig. 28-2]
   (Fig. 28B) tSNE analysis was performed with live, CD14/56- cells by FlowJo after concatenation of all samples, and representative tSNE plots together with the tSNE plot of all concatenated samples are shown. (Fig. 28C) Heatmaps show the expression of surface makers and transcription factors in each specific population. Scale bar shows the expression level of each marker from low (dark blue) to high (red). (Fig. 28D) Foxp3/Tbet ratios in effector memory CD4 T cells are shown in bar graphs.
[Fig. 29-1]

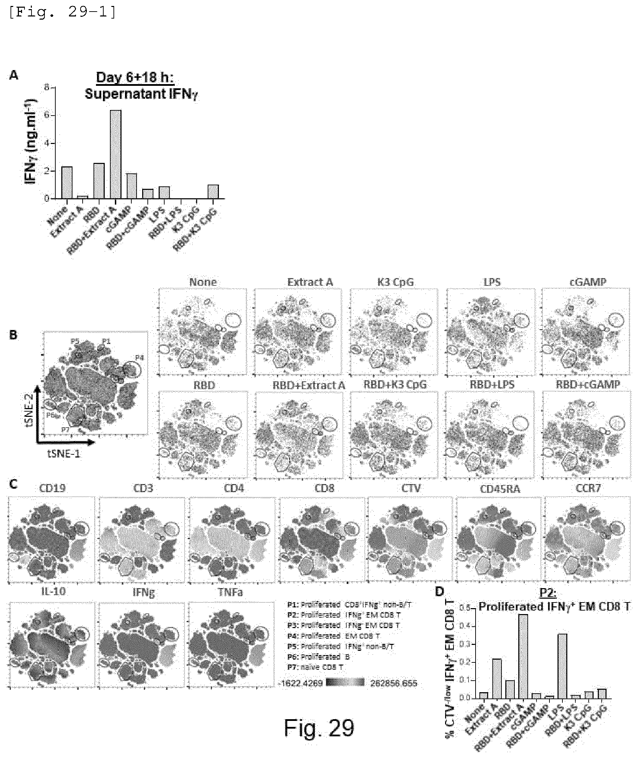

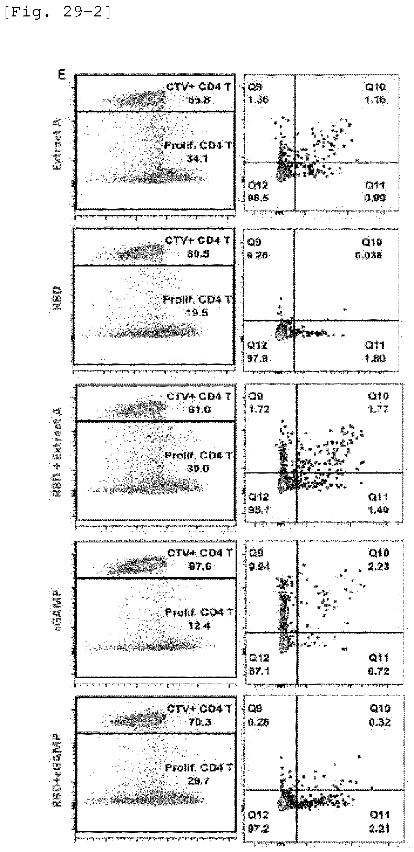

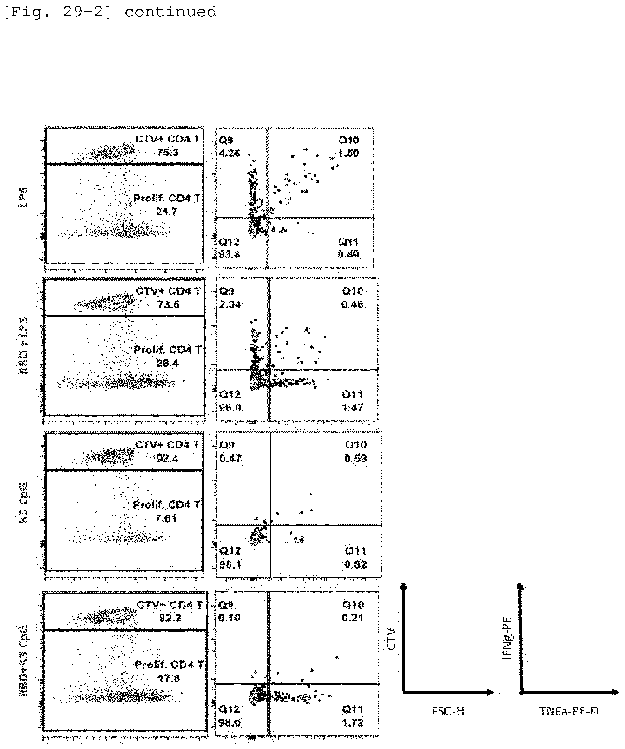

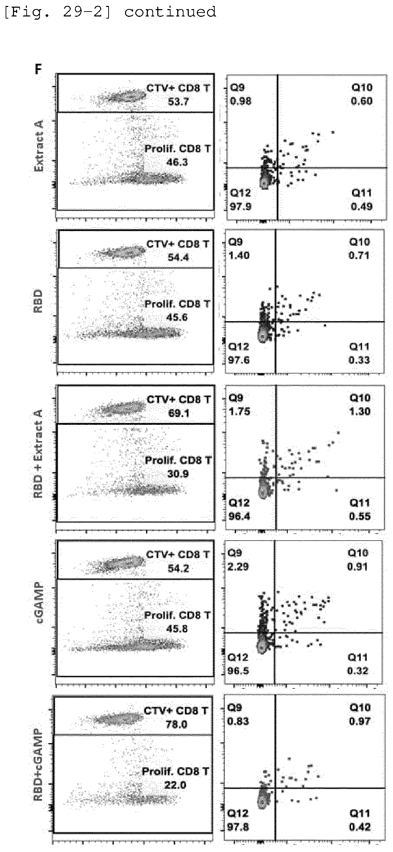

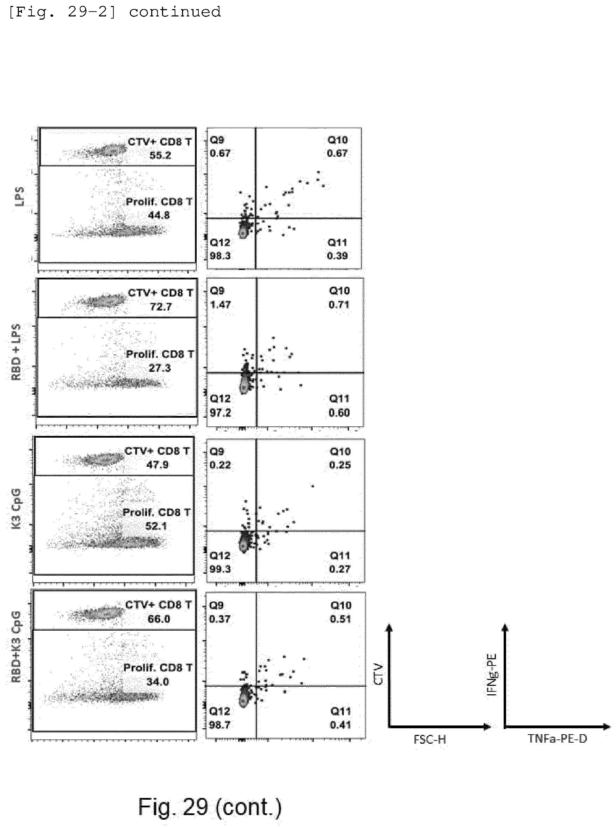

   Fig. 29 shows that Extract A-mediated SARS CoV-2-specific T cell enhancement cannot simply be attributed to the adjuvant effect. (Fig. 29A) Human PBMCs from two healthy individuals were labeled and stimulated with Extract A (30 pg/ml), SARS CoV-2 RBD (1 pg/ml), or their combination for 3 days after which the media was replaced with a fresh medium containing IL-2, and the cells were cultured for additional three days. On day 6, the cells were re-stimulated with SARS CoV-2 RBD antigen for 18 h. Supernatant IFN-γ levels were measured by ELISA. (Fig. 29B) Cytokine production was analyzed by FACS upon intracellular staining. tSNE analysis was performed with live CD14/56- cells using FlowJo^{™} after concatenation of all samples from two individuals, and representative tSNE plots from one individual together with the tSNE plot of all concatenated samples are shown. (Fig. 29C) Heatmaps show the expression of surface makers and transcription factors in each specific population. Scale bar shows expression levels of each marker from low (dark blue) to high (red). (Fig. 29D) Frequencies of P1 and P2 populations from tSNE analysis of the representative healthy control were plotted in bar graphs.
[Fig. 29-2]
   (Fig. 29E) FACS plots show CD4 T cell proliferation by CTV dye dilution and TNFα and IFN-γ production from the proliferated CD4 T cells (CTV^{low/negative} gate) . (Fig. 29F) FACS plots show CD8 T cell proliferation by CTV dye dilution and TNFα and IFN-γ production from the proliferated CD8 T cells.
[Fig. 30]
   Fig. 30 shows gating strategy for intracellular staining of cytokines in Fig. 20. After gating cells based on FSC and SSC, live CD14/56- cells were gated. T cells and B cells were gated based on CD3 and CD19 expression from live/CD14/56- gate. CD4 and CD8 T cells were gated from CD3+CD19- cells, upon which proliferated CD4 or CD8 T cells were gated based on the CTV intensity (e.g., CTV-diluted cells represent proliferated cells). IFN-γ+, IL-10+ or TNFα+ cells were gated from proliferated CD4 or CD8 T cells. In addition, memory phenotype was determined by CCR7 and CD45RA expression in proliferated CD4 and CD8 T cells.

### [Description of Embodiments]

The present disclosure is described hereinafter while showing some of the best modes thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

### (definition)

The terms used herein are described hereinafter.

In the present specification, the "causative factor" of a disease and the like refers to a factor to be the cause that induces the disease. In the case of infectious diseases, it refers to a pathogenic factor or an infectious factor such as viruses, bacteria, protozoa, and mycoplasma; in the case of autoimmune diseases such as allergy and the like, a substance to be the immunogen (causative substance) such as allergen and the like can be mentioned; and tumor cells, cancer genes, toxic substances and the like can be mentioned in the case of cancer, tumor, or neoplasm.

In the present specification, the "disease" is interpreted in a broader sense and refers to a state of disorder or inconvenience in the mind or body of a human or animal, and refers to any condition that cannot be said a healthy condition which is not specifically defined, such as an illness, disorder, or various symptoms.

In the present specification, the "antigen component" refers to a component capable of controlling (e.g., eliciting, enhancing, suppressing, eliminating, etc.) an antigen-antibody reaction in a subject, and is sometimes referred to as an "antigen" in the present specification. It may be a single component (substance) or a complex. The antigen components may be provided in plural different combinations, which is referred to as a "combination of antigen components". The antigen component in the present specification may be provided as an isolated one, a complex thereof, or an extract containing the same.

In the present specification, the "causative factor antigen component" is an antigen component that causes a disease (e.g., infectious disease, allergy or autoimmune disease), and refers to those derived from the causative factor, as well as antigen components that cross-react with the causative factor (preferably, but not limited to, all or a part of the causative factor). In the present specification, whether a certain component or substance is a "causative factor antigen component" can be confirmed, for example, by comprehensively identifying and comparing proteins or mRNAs that cause diseases (e.g., infectious disease, allergy autoimmune disease). For the identification, mass spectrometry, microarray, and next-generation sequencer are mainly used, and it can be confirmed by examining sequence information and the like. The "antibody" refers to a protein that recognizes and eliminates foreign substances. In this case, the foreign substance is called an "antigen". The causative factor antigen component may be, for example, a molecule that appears at least partially on the membrane surface (e.g., the spike protein of viruses such as coronaviruses), or a molecule that constitutes a virus particle (e.g., envelope protein E, membrane protein M, nucleocapsid protein N or rhinovirus capsid proteins VP1, VP2, VP3, VP4). Alternatively, the causative factor in the causative factor antigen component may be, in the case of previously unknown causative factors such as novel viruses (e.g., SARS-CoV-2), a causative agent known in the art (known and therefore may also have immune memory) such as a virus that cross-reacts therewith (e.g. previously known coronaviruses, for example, HCoV-HKU1, HCoV-OC43, SARS-CoV, MERS-CoV). This is because the causative factor antigen component may be urgently required as a target for treatment or prevention, and thus is expected to play a role as a molecule that is highly likely to elicit an immune response.

In the present specification, the "non-causative factor antigen component" refers to an antigen component based on factors that are neither derived from nor cross-reactive with causative factors that cause disease (e.g., infectious diseases, allergies, autoimmune diseases, etc.). Basically, the non-causative factor antigen component can be said to be a factor different from the causative factor antigen factor. In the present specification, whether a component or substance is a "non-causative factor antigen component" can be identified by methods similar to those for a "causative factor antigen component". In general, components such as proteins and the like that are generally specific to disease (e.g., infectious disease, allergy, autoimmune disease) or present in excess are sometimes referred to as "disease antigens". Therefore, non-causative factor antigen (component) can be defined as any component other than a disease antigen that does not cross-react with the antigen. In the present specification, the non-causative factor antigen component optionally has an immunostimulatory action (or adjuvant activity). Examples of the non-causative factor antigen component include a Mycobacterium tuberculosis hot water extract or a part thereof, and specific examples include a hot water extract of human Mycobacterium tuberculosis Aoyama B strain.

In the present specification, the "conditions under which (a certain component X exists)" refers to any condition that exists in a subject in a manner in which that component X acts upon administration of another component Y to the subject. For example, a combination of component Y and component X is administered simultaneously or at different times (the combination may be a combination drug or may be in the form of a combination administered as a combination of separate medicaments); component X is confirmed to be present in the subject (e.g., by antigen test or, where appropriate, genetic test such as PCR and the like), and component Y is administered to the subject in which the presence is confirmed, and the like. Without wishing to be bound by theory, to achieve one of the purposes of the present disclosure, it was found that, in a condition in which a causative factor antigen component derived from and/or cross-reactive with a causative factor of a target disease is present in a subject, by administering to the subject a non-causative factor antigen component that is neither derived from the causative factor of the disease nor cross-reactive with the causative factor, somatic immunity that a non-causative factor antigen component that is neither derived from nor cross-reactive with the causative factor of the disease may have against the subject can be directed against causative factor of the target disease, which provided one feature of the present disclosure.

In the present specification, the "pre-administration" refers to an act aimed at inducing an immune response by administering a non-causative factor antigen component or Mycobacterium tuberculosis hot water extract or a part thereof when a subject does not have an immune response to the aforementioned non-causative factor. After pre-administration, when the subject is confirmed to have an immune response, the composition and the like of the present disclosure can be administered to the subject.

In the present specification, with respect to any substance or component, the term "specific" means that the substance or component has the property of controlling a particular response in a subject. As a representative example in the present specification, the term "specific" particularly in the fields of treatment and prophylaxis means that the subject has immune memory. Particularly, the term can mean being specific for memory T cells of a subject.

In the present specification, whether a test subject "has immune memory " for a component or substance can be evaluated by measuring, in the subject or a biological component (e.g., cell, etc.) derived from the subject, whether the component or substance (i) enhances cytokine production in an antigen-dependent manner for memory CD4-positive T cells, or has a growth-promoting effect, (ii) alters the expression of surface antigens of memory-type regulatory T cells, (iii) changes the ratio of Treg and Th1, (iv) induces IFN-γ production from T-bet positive Th1 cells, (v) alters the ability to produce IFN-γ (vi) alters the ability to produce IL-2, (vii) alters the ability to produce TNF-α, and (viii) determines whether the blood has antibodies specific to a component or substance, and confirming that at least one of these yields a positive result. These tests can also be used to ascertain whether an immune response is currently displayed or was displayed in the past. Therefore, whether immune memory and immunity responsiveness are currently displayed or were displayed in the past, for example, can be tested and confirmed typically by performing an antibody test or other immune response activity test for the presence or absence of antibodies to the target antigen (e.g., any antigen derived from the virus as long as it is against SARS-CoV-2).

In the present specification, when a test subject has an "immune response" to a certain component or substance, it means that some kind of immune reaction occurs against the component or substance. In the test subject or a biological component (e.g., cell, etc.) derived from the test subject, it can be identified by observing changes in various immune cells, or increases or decreases in immune-related substances (e.g., cytokines, etc.) of components or substances thereof, or can also be judged by objective indices, and subjective judgment based on the experience of a doctor or the like can also be made. An immune response can be confirmed by a tuberculin reaction test or an interferon gamma release assay test, or by a mother and child health handbook or its equivalent, clinical records, or other medical information. In one embodiment, the objective confirmation of the immune response is performed by a tuberculin reaction test or an interferon gamma release assay test, or objective information contained in a mother and child health handbook or its equivalent, clinical records, or other medical information, preferably a tuberculin reaction test or an interferon gamma release assay test. In the present disclosure, objective confirmation may be preferred over subjectivity. Without wishing to be bound by theory, this is because it can be confirmed that the immune effects of the present disclosure are exhibited.

In the present specification, the "foreign substance to a subject" refers to any substance (e.g., proteins, amino acids, peptides, nucleic acid molecules, polysaccharides, toxins, etc.) that is not present in the body of the subject. In the present specification, the "subject" is interchangeable with and synonymous with "test object" or "test subject" and can be synonymous with "patient" when afflicted with a disease. In the present specification, the "target", "test object" or "test subject" refers to a human or a mammal, including non-human primates (e.g., one or more kinds of mouse, guinea pig, hamster, rat, mouse, rabbit, swine, sheep, goat, bovine, horse, cat, dog, marmoset, monkey, chimpanzee, and the like).

In the present specification, the "infectious disease" may be any infectious disease, and may be any infectious diseases such as a viral infectious disease (including any form of virus such as single-stranded or double-stranded DNA virus, RNA virus, etc.), bacterium infectious disease, protozoa infectious disease, Mycoplasma infection and the like, for example, tuberculosis, Coronavirus, malaria, yellow fever virus, smallpox virus, smallpox vaccination, measles/rubella, polio, epidemic parotitis (mumps)/MUMPS, rotaviral infection, varicella (possum), yellow fever, Ebola, West Nilefever, hib infection, pneumococcul infection, pertussis, Japanese encephalitis, meningococcal infection, Salmonella infection, pathogenic Escherichia coli, Toxoplasma, Zika virus, herpes virus type 1, EBV/Epstein-Barr virus (herpes virus type 4), CMV/cytomegalovirus (herpes virus type 5), influenza, MARS, rabies and diphtheria and the like. In the present disclosure, the "non-causative factor antigen component" and "causative factor antigen component" may be components derived from different infectious diseases. In one embodiment, when the infectious disease from which the "causative factor antigen component" is derived is COVID-19 and the causative factor is SARS-CoV-2, the infectious disease from which the "non-causative factor antigen component" is derived is tuberculosis, and the "non-causative factor antigen component" is a component derived from tuberculosis. In this case, it is preferable that the subject has a history of BCG vaccination, a history of tuberculosis infection, or antigen responsiveness to Mycobacterium tuberculosis (having immune memory).

In the present disclosure, the "virus" refers to an infectious structure that uses the cells of other organisms to self-replicate. Taxonomically, "virus" includes Rhinovirus family, adenovirus family, Coronaviridae family, RS virus family, influenza virus family, parainfluenza virus family, Enterovirus family and the like.

In the present specification, the "coronavirus-related infections" refers to infections caused by viruses belonging to the coronavirus family. Taxonomically, "Coronaviridae family" includes "Alphacoronavirus", "Betacoronavirus", "Deltacoronavirus", and "Gammacoronavirus". Coronaviruses that infect humans include, but are not limited to, HCoV-229E, HCoV-OC43, HCoV-NL63, HCoV-HKU1, SARS-CoV, SARS-CoV-2, and MARS-CoV.

In the present specification, the "immune abnormality" refers to any disease, disorder or condition caused or suspected to be caused, at least in part, by an abnormality of the immune system. It refers to condition in which the immune system is abnormal due to some cause, making it easier to get infections or causing allergic reactions. Immune abnormality include, but are not limited to, allergies, autoimmune diseases, and the like. When it is directed against one's own antigens, it is generally called an autoimmune disease, and when it is directed against foreign antigens, it is called an allergy. When the immune response is strong, it becomes an autoimmune disease state against self antigens, while being allergic to non-self antigens. It can be said that a person with a weak immune response develops a cancerous state against self antigens and an infectious disease against non-self antigens.

In the present specification, the "autoimmune disease" is an exaggerated immune response directed against a specific self antigen. Autoimmune diseases can be said to be diseases caused by the breakdown of immune tolerance, in which symptoms occur when the immune system, which is responsible for recognizing and eliminating foreign substances, overreacts and attacks even the own normal cells and tissues. Examples of the autoimmune diseases include autoimmune uveitis.

In the present specification, the "allergy" is a disease in which the immune response is excessively directed against a specific non-self antigen, and the immune response is directed against an "allergen". The "allergen" refers to an antigen that can react with the antibody of a subject with an allergic disease. Examples thereof include, but are not limited to, allergen derived from tree pollen (acacia, alder, velvet ash, beech, birch, maple, mountain cedar, red cedar, box elder, cypress, American elm, Chinese elm, ash, togasawara, gum tree, eucalyptus, enoki, hickory, sugar maple, mesquite, casinos, konara spp, Eucalyptus trees, enoki, hickory, American linden, sugar maple, mesquite, cashew, Quercus spp., olive, pecan, pepper, pine, Japanese knotweed, Russian olive, American spruce, Japanese elder, black walnut, black willow, etc.), allergen derived from plant pollen (cottonwood, gewgaw, nagahagrass, sparrowhawk, corn, hirsutake grass, savannah corn, crowfoot, chamogaya, cornucopia, perennial rye-grass, rice, Harugaya, Japanese knotweed, daylily, red spider lily, Japanese white fir, Common Sorrel, Solidago altissima, Iso borax, shiroza, calendula, nettle, green foxtail millet, spatula psyllium, Ambrosia trifida, ragweed, Perennial ragweed, Salsola tragus, Yamayomogi Japanese mugwort, broom, Sheep sorrel etc.), allergen derived from insects (Silkworm, mites, bees, wasps, ants, cockroaches etc.), allergen derived from fungi (Alternaria, Aspergillus, Botulinum, Candida, Cephalosporium, Carbularia, Epicoccum, Epidermophyton, Fusarium, Helminthosporium, Chain Cladosporium, Kekavi, Penicillium, Pharma, Pluralia pullulans, Beetle Mold, etc..), allergen derived from animal hair (dog, cat, bird, etc.), allergen protein derived from house dust, allergen derived from food (OVA etc.), and the like. Representative diseases of "allergy" include atopic dermatitis, allergic rhinitis (hay fever, etc.), allergic conjunctivitis, allergic gastroenteritis, bronchial asthma, childhood asthma, food allergy, drug allergy, or urticaria.

In the present specification, the "inflammatory diseases" refers to a disease or condition characterized by abnormal inflammation (e.g., elevated levels of inflammation compared to controls, such as healthy individuals who do not have the disease). As non-limiting examples of inflammatory diseases, atopy, asthma, autoinflammatory diseases, hypersensitivity, childhood allergic asthma, allergic asthma, inflammatory bowel disease, Celiac disease, Crohn's disease, colitis, ulcerative colitis, collagenous colitis, lymphocytic colitis, diverticulitis, irritable bowel syndrome, short bowel syndrome, blind loop syndrome, chronic sustainability diarrhea, refractory diarrhea in infancy, traveller's diarrhea, immunoproliferative small intestinal disease, chronic prostatitis, post-abnominal syndrome, tropical sprue, Whipple's disease, Wolman disease, arthritis, rheumatoid arthritis, Behcet's disease, uveitis, pyoderma gangrenosum, erythema nodosum, traumatic brain damage, psoriatic arthritis, juvenile idiopathic arthritis, multiple sclerosis, systemic lupus erythematosus (SLE), myasthenia gravis, young people onset diabetes, type 1 diabetes, Guillain-Barre syndrome, Hashimoto encephalitis, Hashimoto thyroiditis, ankylopoietic spondylarthritis, psoriasis, Sjogren's syndrome, vasculitis, glomerulonephritis, autoimmune thyroiditis, bullous pemphigoid, sarcoidosis, Ichthyosis, Graves' ophthalmopathy, Addison's disease, vitiligo, acne vulgaris, pelvic inflammatory disease, reperfusion injury, sarcoidosis, graft rejection, interstitial cystitis, atherosclerosis, and atopic dermatitis can be mentioned.

In the present specification, the "prophylaxis" or "prevention" is an act of administering an active ingredient in the present disclosure to an individual who has not developed the target disease, intended to for example prevent development of the disease. Vaccines are representative examples of medicines intended for prevention. In the present disclosure, prevention means that even if a causative factor of a disease is present in a subject, it is not usually judged to be a disease state unless it develops. Even in such a condition, it can be targeted for treatment and can be said to be prevented.

In the present specification, the "treatment" or "therapy" is, for example, an act of administering an active ingredient of the present disclosure to an individual (test subject, patient) diagnosed as having a developed disease by a physician or a similar practitioner, intended to, for example, alleviate the disease or condition, reduce the number of infectious disease-causing viruses or organisms in a subject, or revert back to the state before the development of the disease. Even if the objective of administration is to prevent aggravation of a disease or symptom, or to reduce the number of viruses or organisms that cause infectious diseases, if the person being administered is a patient, it is a therapeutic act.

Prevention or treatment of immune abnormality includes prevention of immune abnormality, recovery from immune abnormality, or prevention of immune abnormality.

In the present specification, the "memory T cells" refer to cells that exist in vivo for a long period of time and function to maintain immune memory. In the body, 90% of effector T cells are dead after 1-2 weeks unless exposure to the same antigen is sustained. Some of the remaining T cells are then largely divided into two cell populations. They function as "memory cells" because they function to maintain immune memory by existing in vivo for a long period of time. Memory cells are broadly classified into central memory T cells (T_{CM}) and effector memory T cells (T_{EM}). The long-term survival of these two types of memory T cells makes it possible to trigger a rapid immune response when the same pathogen invades again. It is said that not only do memory T cells generated at the first encounter with an antigen survive for a long time, but each time the same antigen is encountered again, a new pool of memory T cells is formed.

In the present specification, the "effector T cells" or "Teff" refers to T cells that positively control immune responses, and are immune cells that directly attack and destroy target factors or suppress proliferation thereof. Effector T cells include killer T cells, helper T cells, γδ (gamma delta) T cells, NK cells, NKT cells and the like. In the present specification, CD4-positive T cells, CD4+ cells and CD4 T cells are used interchangeably and refer to T cells that are positive for CD4. CD8-positive T cells, CD8+ cells and CD8 T cells are used interchangeably and refer to CD8-positive T cells. CD4-negative T cells and CD4-T cells are used interchangeably and refer to CD4-negative T cells. CD8-negative T cells and CD8-T cells are used interchangeably and refer to CD8-negative T cells.

T_{CM} cells are close to naive T cells in terms of cell surface markers, and express CCR7, one of the chemokine receptors, and CD62L, one of the adhesion factors, and mainly present in the T-cell area of secondary lymphoid tissue. When exposed to the same antigen again, they produce IL-2 and proliferate rapidly, and some of them are said to differentiate into TEM cells.

On the other hand, T_{EM} cells have decreased expression of adhesion factors such as CCR7 and CD62L, and are mainly present in the localized inflammation (e.g., lung, liver, intestinal tract, etc.) rather than secondary lymphoid tissue. Stimulation with the same antigen is said to produce large amounts of cytokines such as IL-4, IFN-γ and IL-5.

In a preferred embodiment, the memory T cells targeted by components of the present disclosure may be memory regulatory T cells (IL-2 producing). Regulatory T cells (Treg) are a type of T cell that block excessive immune responses. Treg cells are roughly divided into two types of endogenous Treg (natural Treg; nTreg) cells that are naturally generated in the thymus, and induced Treg (iTreg) cells that are generated by stimuli such as cytokines in peripheral tissues. The "Memory type regulatory T cells (IL-2-producing)" are cells having both characteristics of "memory T cells" and "regulatory T cells".

In the present specification, the "immune hyperreactivity" refers to an in vivo immune reaction acting more strongly than a normal reaction to target and attack a substance that does not adversely affect the living body. The "immune hyperreactivity" includes conditions such as allergies, autoimmune diseases, cytokine storms, and the like. Allergy is a disease in which the immune system is over-activated due to excessive production of IgE, IgG, and IgM antibodies that are produced when foreign substances such as allergens, bacteria, and viruses enter the body. Autoimmune diseases are diseases in which the immune system malfunctions and attacks its own cells and tissues. Cytokine storm is a disease in which excessive production of inflammatory cytokines induces an excessive immune response and causes inflammation in various cells and tissues. The body also has a mechanism for suppressing immune excess, such as the production of IL-10, which is an inhibitory cytokine. IL-10 is an inhibitory cytokine important for the induction and maintenance of immune tolerance and the control of excessive immune responses. It is considered to be involved in the control of excessive inflammatory responses by effector T cells in pathogen elimination and autoimmune reactions, and in the maintenance of homeostasis of the intestinal environment maintained by the symbiosis between intestinal bacteria and the intestinal immune system. It is said to calm immune reactions by acting on immune cells such as T cells and macrophages, directly suppressing cell activation, and weakening the antigen-presenting ability of macrophages.

In the present specification, the "cellular immunity" refers to a cellular response to cells characterized by antigen expression and/or antigen presentation with class I or class II MHC. The cellular response involves cells called T-cells or T-lymphocytes that act as "helpers" or "killers". Helper T cells (also called CD4+ T cells) play a central role in regulating the immune response, and killer cells (also called cytotoxic T cells, cytolytic T cells, CD8+ T cells or CTLs) kill cells, including diseased cells. As shown in the present disclosure, it can be said that it is a specific immune mechanism rather than innate immunity, and the combination of the two components of the present disclosure can freely control humoral immunity or humoral immune memory. This phenomenon was discovered for the first time by the present disclosure.

In the present specification, the "antigen responsive" has the same meaning as known in the art, and refers to the control of an antigen-antibody reaction of a subject to a specific substance or the like. Antigen responsiveness can typically be tested and confirmed by performing an antibody test for the presence or absence of antibodies to the target antigen (for example, one against SARS-CoV-2 can be any antigen derived from the virus)

In the present specification, when referring to a certain subject, the "antigen responsive profile" refers to a general term (profile) summarizing the antigen responsiveness that the subject has to various substances and the like. Antigen responsiveness profiles can be obtained by various techniques. For example, it can be obtained as a profile by confirming current or past physical conditions such as medical history (e.g., history of infection) and vaccination history, or by actually confirming antigen responsiveness using a panel of antigens. These can be realized, for example, by conducting medical interviews, medical history based on maternal and child handbooks or equivalents, vaccination history, and combinations thereof. In addition, whether or not there is responsiveness can be confirmed by collecting body fluid (e.g., blood) from the subject, separating peripheral blood cells, and determining whether the peripheral blood cells produce cytokines (such as IL-2, IFN-γ, TNF-α, and combinations of two or more thereof) in response to antigens related to the antigen profile, and can also be confirmed by measuring other biomarkers.

In the present specification, the "immunostimulatory action" refers to the action of non-specifically activating the immune function of the body and enhancing the weakened defense force. Whether or not a substance has an "immunostimulatory effect" can be confirmed by conducting a test that evaluates the activation of immune cells using the innate immune receptor reporter gene assay, lymphocytes, etc., as an index of the production of cytokines, and the like.

In the present specification, the "activation" refers to a state in which the functions of cells (e.g., T cells), proteins, polypeptides, genes, etc. are increased. The "activation" includes a state in which changes or increases in cell function are observed, a state of enhanced cell proliferation, a state in which the expression of proteins, polypeptides, genes, etc. is increased or a state in which the pattern of expression is changed, a state of suppressing suppressive cells, and a state in which a suppressed function is released. In particular, in the present disclosure, it refers to the activation of regulatory T cells (Treg). Activation of regulatory T cells (Treg) is sometimes referred to as release of suppression or conversion of regulatory T cells (Treg), but both are synonymous. In the present specification, the activation of Treg is sometimes referred to against a certain target, and in this case, it means that Treg is activated to the target and exerts a preventive or therapeutic effect on a disease. As an example, activation of Tregs includes conversion into or generation of effector T cells (Teff) against the causative factor or cells containing the causative factor. Targets in this case include, but are not limited to, causative agents of infections, allergies, autoimmune diseases, and the like.

Whether immune cells are "activated" can be confirmed by the test etc. described in "Immunostimulatory action". The "activation" of a gene can be quantified using a real-time PCR method, an RNA-Seq method, Northern hybridization, a hybridization method using a DNA array, or the like. The expression level of a polypeptide can be quantified using an antibody that recognizes the polypeptide, a staining compound that binds to the polypeptide, or the like. In addition to the quantitative methods mentioned above, conventional methods used in this technical field may also be used.

In the present specification, the "adjuvant" is a substance used to increase the effect (immunogenicity) of drugs such as vaccines (antigens) by administering it with the drugs, and is a term derived from the Latin word 'adjuvare', which means 'to help'. Whether a substance functions as an "adjuvant" to another substance (e.g. an antigen) can be confirmed by a test including administering it with the antigen to mice and assessing antigen-specific antibody production, or by performing a test described in the present specification. When used in infectious diseases in the present disclosure, it is not antigen-specific but activates innate immunity regardless of antigen, and is demonstrated in the present disclosure to be effective in preventing infection.

In the present specification, an "antigen dependent" "action" relating to a certain component means, when referring to a subject such as a T cell, that the component has an effect on the subject as a result of an antigen-antibody reaction. It can be confirmed by, for example, that the effect disappears when it inhibits the antigen-antibody reaction.

In the present specification, the "memory-CD4-positive T cells" refers to memory T cells that are CD4-positive. Here, as for CD4 positivity in the present specification, staining at a high level compared to the level of staining with a non-specific antibody used as a negative control is considered positive, by an immunostaining method using CD4-specific antibody labeled with fluorescent dye and the like.

In the present specification, the "Foxp3-positive Treg cell" refers to Treg cells that are Foxp3-positive. Here, as for Foxp3 positivity in the present specification, staining at a high level compared to the level of staining with a non-specific antibody used as a negative control is considered positive, by an immunostaining method using Foxp3-specific antibody labeled with fluorescent dye and the like.

In the present specification, the "IFN-γ-producing T cells" refers to T cells having interferon γ (IFN-γ)-producing ability. Here, IFN-γ-producing T cells in the present specification can be identified based on staining at a high level compared to the level of staining with a non-specific antibody used as a negative control, by an immunostaining method using IFN-γ-specific antibody labeled with fluorescent dye and the like.

In the present specification, the " type 1 helper T cell" is also indicated as "Th1 cell" and is a subgroup of CD4-positive T cells (so-called helper T cells) such as naive CD4-positive T cells matured in the thymus. It is a type of cell that rapidly enters the bloodstream, migrates to sites of infection, secretes cytokines such as IFN-γ and IL-2, and triggers macrophage activation and inflammatory responses. Th1 cells are responsible for cell-mediated immunity, which is an immune response that occurs locally and in which CTLs and macrophages directly attack cells. A subgroup of CD4-positive T cells also includes type 2 helper T cells (Th2 cells). Th2 cells secrete cytokines such as IL-4 and IL-5 and activate naive B cells that recognize the same antigens in secondary lymphoid tissues. Th2 cells are responsible for humoral immunity, an immune response centered on B cells and antibodies.

In the present specification, the "T-bet-positive Th1 cell" refers to Th1 cells that are T-bet-positive. Here, as for T-bet positivity in the present specification, staining at a high level compared to the level of staining with a non-specific antibody used as a negative control is considered positive, by an immunostaining method using T-bet-specific antibody labeled with fluorescent dye and the like. The transcription factor T-bet encoded by the Tbx21 gene in Th1 cells directly and positively feedforward regulate the production of interferon-γ as a phylogenetic transcription factor. Interferon γ is classified as a type II interferon as one of the interferon family with anti-pathogenic and anti-infective effects, and known to induce the expression of T-bet, a transcription factor that defines Th1 cells, and maintain interferon γ production by feedforward regulation.

In the present specification, that the ability such as IFN-γ-producing ability, IL-2-producing ability, and TNF-α-producing ability, are "enhanced" means a significant increase in the amount of IFN-γ and IL-2 produced and the number of producing cells due to stimulation with an antigen or the like compared to the negative control, which is judged by an immunostaining method using an antibody specific to IFN-γ, IL-2, TNF-α, labeled with a label (such as a substance) such as a fluorescent dye. The amount of cytokines produced can be measured by ELISA and the number of cytokine-producing cells can be measured by FACS.

In the present specification, the "biomarker" refers to a substance or event, such as a protein, measured in a biological sample such as blood, whose presence or absence, concentration, or level reflects the presence or progress of a particular physical condition, such as a disease. Therefore, in the present specification, the biomarker is understood to include events such as whether it acts on memory CD4-positive T cells in an antigen-dependent manner, whether it alters memory-type regulatory T cells, whether it alters the ratio of Treg to Th1, whether it induces IFN-γ production from T-bet positive Th1 cells, whether it modulates IFN-γ productivity, whether it alters IL-2 productivity, altering TNF-α productivity, and the like.

Whether or not the antibody acts on memory CD4-positive T cells in an antigen-dependent manner can be determined by FACS analysis based on a statistically significant increase in the number of positive cells of the antibody used for staining.

Whether memory type regulatory T cells are changed can be determined by FACS analysis based on a statistically significant increase in the number of antibody-positive cells used for staining.

Whether the ratio of Treg and Th1 is changed and whether IFN-γ production from T-bet-positive Th1 cells is induced can be determined by measuring by FACS after intracellular cytokine and transcription factor staining, and by measuring the produced cytokine by ELISA.

Whether or not IFN-γ productivity, IL-2 productivity and TNF-α productivity are changed can be determined by measuring by FACS after intracellular cytokine and transcription factor staining, and by measuring the produced cytokine by ELISA.

In the present specification, the "bias" of the "existence ratio" of cells means that the ratio of multiple types of cells is statistically significantly different from the ratio that exists in a normal state. Whether deviated or not can be determined by reference to cell analysis results obtained by any techniques for analyzing cells (e.g., FACS and the like).

In the present specification, the "human *Mycobacterium tuberculosis* hot water extract" is typically a substance produced from a human *Mycobacterium tuberculosis,* which is a mixture including polysaccharides with arabinose, mannose, and glucose as the primary ingredients. While anti-infectious disease effects due to human *Mycobacterium tuberculosis* hot water extracts have been studied for a long time, the detailed mechanism of action thereof is not necessarily elucidated. Further, the extract has not been used as a prophylactic drug. The extract can also comprise a trace amount of ingredients such as a protein, peptide, amino acid, nucleic acid, or lipid (glycolipid) when appropriate.

In the present specification, the "part of human Mycobacterium tuberculosis hot water extract" may be any part (component, combination of components, or a part of component, and the like) of human Mycobacterium tuberculosis hot water extract. For example, polysaccharides such as arabinose, mannose, glucose and the like, a trace amount of ingredients such as protein, amino acid, nucleic acid, lipid (glycolipid) and the like, for example, mitochondrial heat shock protein10(mHSP10; Primary Accession number:P9WPE5, Secondary Accession number(s):L0TFJ8, P09621), Mycobacterium tuberculosis low molecular weight antigen (MTB12; Primary Accession number:P9WIN7, Secondary Accession number(s):L0T9F9, O05822, P0A5P8) and/or lipoprotein LpqH(Primary Accession number:P9WK61, Secondary Accession number(s):L0TGP1, P0A5J0, P11572) can be mentioned. Any of these components or combinations thereof can be used as non-causative factor antigen components in the present disclosure. mHSP10, MTB12 and LpqH are antigen components contained in human Mycobacterium tuberculosis hot water extract, and are molecules having the activity of inducing IFNγ secretion.

In another embodiment, a part of the hot water extract of Mycobacterium tuberculosis may include a STING agonist derived from Mtb, alone or in combination with others.

The "STING", a (adapter molecule) stimulator of IFN genes (stimulator of interferon genes), identified as a membrane protein localized to the endoplasmic reticulum plays an important role in host defense mechanisms against various RNA and DNA virus infections. STING has also been reported to play an important role in inducing innate immune responses against DNA components derived from viruses and bacteria. Some of the present inventors clarified that STING can form complexes not only with genomic DNA derived from viruses, but also with synthetic double-stranded DNA of 45-90 base pairs called ISD, and further with self-DNA components derived from apoptotic cells. Analysis of the DNA interaction region in vitro indicated that the C-terminal region of STING is important. Recognition of various DNA components by STING induces changes in the dynamic localization of STING to peripheral regions of the nuclear envelope, and has been shown to induce interferon production through activation of TBK1. Furthermore, it has been suggested that STING may be involved in the regulation of chronic inflammatory responses through recognition of self DNA components as well as non-self DNA components derived from microorganisms.

In the present specification, the "STING ligand" and "STING agonist" are used interchangeably and is a ligand (agonist) of the (adaptor molecule) stimulator of interferon genes of IFN genes (stimulator of interferon genes), inducing type I IFN production and NF-κB-mediated cytokine production. STING agonists have been postulated to be membrane proteins localized to the endoplasmic reticulum. As STING agonists, in addition to cGAMP, cyclic dinucleotides of bacterial origin, c-di-AMP and c-di-GMP, are ligands for adapter molecule stimulators (STING) of IFN genes that signal through the TBK1-IRF3 axis to induce type I IFN production and NF-κB-mediated cytokine production [Burdette et al., Nature. (2011)478:515-8; Mcwhirter et al., J. Exp. Med. (2009)206:1899-1911]. Recent studies have shown that these cyclic dinucleotides function as potent vaccine adjuvants due to their ability to enhance antigen-specific T-cell and humoral immune responses. Some of the inventors have previously reported that the STING agonist DMXAA unexpectedly induces type 2 immune responses via STING-IRF3-mediated production of type I IFN [Tang et al., PLoS One. (2013)8:1-6]. The clinical utility of STING agonists, including cyclic dinucleotides, has been controversial, since a type 2 immune response may fail to induce a type 1 immune response. For example, the most common adjuvant, the aluminum salt (alum), lacks the ability to induce cell-mediated immunity, which is considered to protect against disease or cancer from intracellular pathogens [Hogenesch et al., Front. Immunol. (2013)3:1-13]. To overcome this limitation, alum has been combined with a number of different kinds of adjuvants, including monophosphoryl lipid A [Macleod et al., Proc. Natl. Acad. Sci. U. S. A. (2011)108:7914-7919] and CpG ODN [Weeratna et al., Vaccine. (2000)18:1755-1762]. For technologies related to STING, similar to microbial DNA, host DNA can also be a danger signal, particularly when host DNA is inappropriately present in the cytosol, thereby resulting in the production of interferons and inflammatory cytokines [Desmet et al., Nat. Rev. Immunol. (2012)12:479-491; Barber et al., Immunol. Rev. (2011)243:99-108]. One recently identified cytosolic DNA sensor is cyclic GMP-AMP synthase (cGAS), which catalyzes the production of the non-canonical cyclic dinucleotide cGAMP (2'3'-cGAMP) and contains non-canonical 2', 5' bond and 3', 5' bond between the purine nucleosides [Sun et al., Science. (2013)339:786-91]. Canonical cGAMP (3'3') is synthesized in bacteria and has more diverse bonds than mammalian 2'3'-cGAMP, GMP and AMP nucleosides are linked by a bis-(3',5') bond [Wu et al., Science. (2013)339:826-30; Zhang et al., Mol. Cell. (2013)51:226-35].

Therefore, STING agonists that may be used in the present disclosure are preferably Mtb-derived STING agonists. Cyclic dinucleotides (CDN) such as 2'3'-cGAMP, c-di-AMP, 3'3'-cGAMP, 2'3'-cGAMP and the like, xanthenone derivatives such as DMXAA, and the like can be mentioned. As shown in the present specification, examples of those derived from Mtb include c-di-AMP, c-di-GMP, 3'3'-cGAMP, 2'3'-cGAMP and/or dsDNA that can produce these via cGAS in the human cytoplasm. For example, it is a significant advantage that 3'3'-cGAMP can be used as an adjuvant for infectious diseases and as a therapeutic or prophylactic agent of the present disclosure. STING agonists are also described in WO2010/017248, the contents of which are incorporated by reference in their entirety.

The following is a representative manufacturing method of human *Mycobacterium tuberculosis* hot water extracts.

Human *Mycobacterium tuberculosis* is cultured for 3 to 7 weeks in a 37°C thermostatic vessel. The film of microbial cells formed on a medium is then filtered out. The moist microbial cells with medium components removed by washing with water are used as the extracted raw material. The microbial cells are floated in a distilled water at an amount that is 15 to 40-fold of the wet weight thereof, and heated for 80 to 180 minutes at 90 to 120°C for extraction. The residue of the microbial cells is removed with a sterilization filter, and the extracted solution is concentrated to 60% or less, and then acetone, trichloroacetic acid, ammonium sulfate, sulfosalicylic acid, or the like is added thereto so as to arrive at 0.5 to 3% (w/v), and the mixture is stirred and left standing. The deposited precipitate is then centrifuged and removed, and the supernatant is subjected to running water dialysis. Inner fraction of dialysis fluid is subjected to vacuum concentration to a 1/20 to 1/4 volume, and sodium chloride is added to the concentrate so as to arrive at 0.5 to 1% (w/v). 2 to 4-fold volume of ethanol is added and the mixture is left standing, and then the precipitate is removed by centrifugation. After adding an additional 2 to 6-fold volume of ethanol and the mixture being left standing, a precipitating polysaccharide is obtained by centrifugation or the like. Those skilled in the art can understand that each of the conditions described above can be appropriately changed to obtain the same product.

In the present specification, the "kit" refers to a unit provided with the parts to be provided (e.g., the composition of the present disclosure, additional components, buffers, instructions, etc.), generally divided into two or more compartments. For the purpose of providing a composition that should not be provided in a mixed form for the sake of stability, etc., and is preferably used by mixing immediately before use or by administering separately, this kit form is preferred. Such kits advantageously include instructions or directions describing how to use or dispose of the provided parts (e.g., compositions, additional components), and the like. When a kit is used herein, the kit typically includes instructions and the like describing how to use the components, compositions, etc. of the present disclosure.

In the present specification, the "instructions" describes instructions how to use the present disclosure. The instructions contain language that directs how to use the present disclosure. This instruction shall, if necessary, be prepared in accordance with the form prescribed by the regulatory authority of the country in which the present disclosure is implemented (for example, the Ministry of Health, Labor and Welfare in Japan, the Food and Drug Administration (FDA) in the United States, and it is specified that it has been approved by the regulatory agency. The instructions can be provided in paper form, but are not limited to it, can also be provided in the form such as electronic medium (e.g., homepage provided on the Internet, e-mail).

### (Explanation of preferred embodiment)

Preferred embodiments of the present disclosure are described below. The embodiments provided below are provided for a better understanding of the disclosure, and it is understood that the scope of the disclosure should not be limited to the following description. Therefore, it is clear that a person skilled in the art can make appropriate modifications within the scope of the present disclosure in light of the description in the present specification. It is also understood that the following embodiments of the disclosure can be used singly or in combination.

### <Prevention and treatment of diseases based on the combination of causative factor antigen components and non-causative factor antigen components>

The present disclosure is based in part on the discovery that, by combining a causative factor antigen component that is derived from and/or cross-reactive with a causative factor of a target disease in a subject with a non-causative factor antigen component that is neither derived from nor cross-reactive with the causative factor of the disease, cellular immunity useful for preventing or treating the disease can be controlled, and the disease can be specifically prevented or treated. In one embodiment, the subject has immune memory for a non-causative factor antigen component. Exposure of a combination with a causative factor antigen component different from the non-causative factor antigen component to the subject results in free regulation of humoral immune memory for causative factor antigen components. It has been found that prevention or cure of the disease of interest is achieved by up-regulating (e.g., causing or enhancing) or down-regulating (e.g., suppressing or eliminating) as appropriate. While not wishing to be bound by theory, some of these responses include the ability to regulate immune responses in ways that differ from innate immunity. Such a combination can be accomplished under any condition in which component X is present in a manner in which component X acts in the subject when another component Y is administered to the subject. For example, a combination of component Y and component X is administered simultaneously or at different times (the combination may be a combination drug or may be in the form of a combination administered as a combination of separate medicaments); component X is confirmed to be present in the subject (e.g., by antigen test or, where appropriate, genetic test such as PCR and the like), and component Y is administered to the subject in which the presence is confirmed, and the like. While not wishing to be bound by theory, based on a quartet method, for example, those that are strongly involved in immune reactions are classified as "allergy" and "autoimmune disease", those that are weakly involved are classified as "infectious diseases" and "cancer", those that are endogenous are classified as "autoimmune disease" and "cancer", and those that are foreign are classified as "allergy" and "infectious disease". The present disclosure has demonstrated that it is possible to deal with all of these, especially infectious diseases, allergies, and autoimmune diseases.

In the present disclosure, by using human peripheral blood mononuclear cells (PBMC) from uninfected/unexposed healthy donors, the present inventors found that Extract A was able to promote, through regulation of the Treg/Th1 balance, SARS CoV-2-specific T cell responses potentially derived from coronavirus-specific T cells associated with the general cold and cross-reactive with SARS CoV-2. Specifically, the stimulatory effect of Extract A on T cells is dependent on preexisting Mtb memory, and this effect is largely independent of the training immunity induced by Extract A. Therefore, both BCG and Extarct A may provide therapeutic efficacy against COVID-19 by accelerating heterologous T cell responses against SARS CoV-2. Particularly, in the present disclosure, in one preferred but not limiting embodiment, Mycobacterium tuberculosis extracts such as Extarct A and the like are shown to be advantageous. In this regard, regarding BCG, it has been shown that repeated administration can lead to excessive immune reactions and inflammatory responses, and it is recommended to refrain from repeated administration. On the other hand, Mycobacterium tuberculosis extracts such as Extract A have demonstrated the ability to be repetitively administered, as indicated by a significant increase in inhibitory cytokines, and have shown safety. Furthermore, a notable increase in effectiveness through repeated administration has also been observed. Therefore, while not limited to a specific embodiment, in one preferred embodiment, it can be stated that even compared to BCG, particularly remarkable unexpected effects have been brought about by Mycobacterium tuberculosis extracts such as Extract A. Especially, these effects, which were not previously anticipated, represent significant effects, particularly in infectious diseases (e.g. bacterial infections, viral infections), and were unattainable with conventional medicines and the like. Hence, preventive and therapeutic effects are anticipated for infectious diseases not only for viral infections like COVID-19 but also for infectious diseases that may newly emerge and could give rise to pandemics, and they are expected to play a role as tools for pandemic prevention.

In one embodiment, the aforementioned non-causative factor antigen component is Mycobacterium tuberculosis hot water extract or a part thereof, preferably, the aforementioned non-causative factor antigen component is a hot water extract of human Mycobacterium tuberculosis Aoyama B strain or a part thereof, more preferably, the aforementioned Mycobacterium tuberculosis hot water extract or a part thereof is Extract A.

As a part of a preferred embodiment, for example, the Mycobacterium tuberculosis hot water extract or a part thereof may include Mycobacterium tuberculosis-derived STING agonists, LpqH, Y1269, PPD, CMV pp65 overlap peptide, Mtb-related antigens, metabolites such as STING agonist c-di-AMP, components capable of biosynthesizing STING agonists within human cells, or NOD2 agonists, or others. In the present disclosure, it has been discovered that these sequences or portions thereof can be used as epitopes to elicit CD4 T-cell responses. In a preferred embodiment, the Mycobacterium tuberculosis hot water extract or a part thereof is a Mycobacterium tuberculosis-derived STING agonist, c-di-AMP, c-di-GMP, 3'3'-cGAMP, 2'3'-cGAMP, and/or dsDNA can produce these via cGAS in the cytoplasm of host cells such as human cells.

While not wishing to be bound by theory, as for the effective components, non-causative factor antigen components, or adjuvants used in the present disclosure, they could potentially be tuberculosis antigen-specific T-cell epitopes (peptide sequences) derived from these mycobacterial tuberculosis hot water extracts or parts thereof. Furthermore, when these were exposed to a biological entity, they were found to interact with CD4 T-cells. To the best knowledge of the inventor, the details regarding CD4 T-cells are not well-established, and the phenomenon induced by components of Mycobacterium tuberculosis hot water extract or a part thereof, or their constituents, leading to such an effect, has not been conventionally known. In particular, the discovery of the peptide sequence of the tuberculosis antigen-specific T-cell epitope among the components contained in Extract A was unexpected. It was also confirmed that these T-cells are CD4 T-cells. Additionally, synthetic peptides with the same sequence as the protein present in Extract A, when induced the activation of the same T-cells, indicating that the triggering could occur not only with naturally derived materials but also with synthetic or artificial substances, which was unexpected given the previous knowledge about MHC and its affinity.

In conventional immunology, only antigen-specific responses were emphasized, and it was not believed that immune reactions could be carried out by antibodies bearing antigens alone. However, the present disclosure reveals that T cells demonstrating antigen-specific responses also influence surrounding T cells and macrophages, and it has been discovered that non-causative antigen factors can trigger immune responses effectively in many diseases such as cancer and infections. While BCG was previously described as conferring long-lasting innate immunity rather than acquired immunity, the present disclosure has revealed that acquired immunity enhances innate immunity. Therefore, the present disclosure is understood to uncover new aspects of immune memory.

In the present specification, LpqH is a 19 kDa protein and is a lipoprotein of Mycobacterium tuberculosis (Mycobacterium tuberculosis lipoprotein) (Accession Number:P0A5J1). LpqH contributes to TLR2 activation. See, Sanchez A, Espinosa P, Garcia T, Mancilla R. The 19 kDa Mycobacterium tuberculosis lipoprotein (LpqH) induces macrophage apoptosis through extrinsic and intrinsic pathways: a role for the mitochondrial apoptosis-inducing factor. Clin Dev Immunol. 2012; 2012:950503. doi:10.1155/2012/950503.

In the present specification, Y1269 is a Mycobacterium tuberculosis-derived protein (Accession Number:P9WM44). Reference may also be made to https://www.uniprot.org/uniprot/P9WM44.

In the present specification, PPD is an antigen purified from Mycobacterium tuberculosis culture filtrate (PPD:Purified Protein Derivative) and is used for the tuberculin reaction.

In the present specification, CMV pp65 overlapping peptide is a cytomegalovirus antigen and is a structure protein detected from the initial stage of CMV infection. It is available from Miltenyi. See, Wills MR, Carmichael AJ, Mynard K, Jin X, Weekes MP, Plachter B, Sissons JG: The human cytotoxic T-lymphocyte (CTL) response to cytomegalovirus is dominated by structural protein pp65: frequency, specificity, and T-cell receptor usage of pp65-specific CTL. J Virol. 1996, 70: 7569-7579.

In the present specification, "Mtb-related antigen" is also referred to as "Mtb antigen", and it refers to any antigen related to tuberculosis. Representative examples are those described in Table 1B.

**[Table 1B]**

| database | deposit number | mass | score | **emPAI** | description | other name |
|---|---|---|---|---|---|---|
| **SwissProt** | **LPQH_MYCBO** | **15309** | **1046** | **1.63** | lipoprotein LpqH lipoprotein 20 kDa | **mb3789** |
| **SwissProt** | **MTB12_MYCBO** | **16625** | **643** | **2.53** | molecular weight antigen MTB12 | **CFP2** |
| **SwissProt** | **CUT1_MYCBO** | **21997** | **295** | **0.34** | MB2006c expected to be cutinase | **CFP21** |
| **SwissProt** | **CUT2_MYCBO** | **24139** | **284** | **0.56** | cut2 expected to be cutinase | **mb2323** |
| **SwissProt** | **CH10_MYCBO** | **10798** | **278** | **9.01** | 10 kDa chaperonin groS | |
| **SwissProt** | **PE15_MYCBO** | **9913** | **218** | **2.50** | uncharacterized PE family protein PE15 | **mb1421** |
| **SwissProt** | **ESXB_MYCBO** | **10787** | **207** | **5.82** | ESAT-6-like protein EsxB | **cfp10** |
| **SwissProt** | **RHO_MYCBO** | **65151** | **192** | **0.26** | transcription termination factor Rho | **mb1329** |
| **SwissProt** | **Y0148_MYCTU** | **29874** | **147** | **0.24** | 0.24 putative short-chain dehydrogenase/reductase Rv0148 | **Y0148** |
| **SwissProt** | **CH601_MYCBO** | **55843** | **126** | **0.31** | 60 kDa chaperonin GroEL | |
| **SwissProt** | **GARA.MYCTO** | **17240** | **124** | **0.68** | glycogen accumulation regulator GarA | **cfp17, mb1858** |
| **SwissProt** | **DAPD_MYCBO** | **32793** | **109** | **0.30** | 2,3,4,5-tetrahydropyridine-2,6-dicarboxylate N-succinyltransferase dapD | **mb1233c** |
| **SwissProt** | **LPQE_MYCBO** | **18921** | **92** | **0.56** | putative lipoprotein LpqE | **mb3615** |
| **SwissProt** | **MP64_MYCBO** | **25068** | **92** | **0.66** | immunogenic protein MPB64 | **mpt64** |
| **SwissProt** | **NUSG_MYCBO** | **25431** | **88** | **0.40** | transcription termination/anti-termination protein NusG | **mb0658** |
| **SwissProt** | **ACPM_MYCBO** | **12516** | **87** | **0.94** | meromycolate extension acyl carrier protein | **mb2268** |
| **SwissProt** | **Y2091_MYCTO** | **26118** | **84** | **0.63** | uncharacterized protein MT2152 | **Y_2118** |
| **SwissProt** | **RL6_MYCBO** | **19366** | **66** | **0.55** | 50S ribosomal protein L6 rplF | |
| **SwissProt** | **GRPE_MYCBO** | **24544** | **57** | **0.41** | protein GrpE | |
| **SwissProt** | **Y1269_MYCTO** | **12770** | **54** | **0.66** | protein MT1307 | **Y1300_MYCBO** |
| **SwissProt** | **ESPC_MYCTO** | **10845** | **47** | **0.46** | ESX-1 secretion-associated protein EpC | **BCG_3679c** |

In the present specification, a "NOD2 agonist" refers to certain peptide glycans having MDP (Muramyl Dipeptide) or MDP-like structures, which can mean as one example a substance that binds to the NOD2 receptor and activates NOD2, and an example thereof may be MDP (Muramyl Dipeptide).

The term "MDP" used in the present specification is Muramyl Dipeptide and can be used as an agonist to activate the PGE2 secretion in mesenchymal stem cells through activating the NOD2 pathway, in the present disclosure.

In the present specification, c-di-AMP is adenylyl-(3'->5')-3'-adenylic acid, cyclic nucleotide, disodium salt (adenylyl-(3'->5')-3'-adenylic acid, cyclic nucleotide, disodium salt). It is available from Cayman and the like.

In the present specification, c-di-GMP is guanylyl(3'→5')-3-guanylic acid, cyclic nucleotide, disodium salt (guanylyl-(3'->5')-3'-guanylic acid, cyclic nucleotide, disodium salt). It is available from Cayman and the like.

In the present specification, 3'3'-cGAMP is adenylyl-(3'->5')-3'-guanylic acid, cyclic nucleotide, disodium salt (adenylyl-(3'->5')-3'-guanylic acid, cyclic nucleotide, disodium salt), or alternatively known as c-GMP-AMP sodium salt, cyclic GMP-AMP sodium salt, cyclic AMP-GMP sodium salt. It is represented by 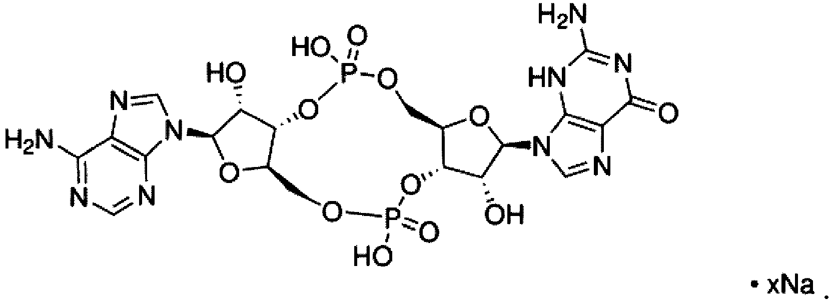 It is available from Cayman and the like.

In the present specification, 2'3'-cGAMP is 2'3'-adenylyl-(3'→5')-3'-guanylic acid, cyclic nucleotide, disodium salt (adenylyl-(3'->5')-3'-guanylic acid, cyclic nucleotide, disodium salt), or alternatively known as, c[G(2',5')pA(3',5')p] sodium salt, cyclic [G(2',5')pA(3',5')p], 2',5'-3',5'-cGAMP, cGAMP(2'-5')Empirical Formula (Hill Notation). 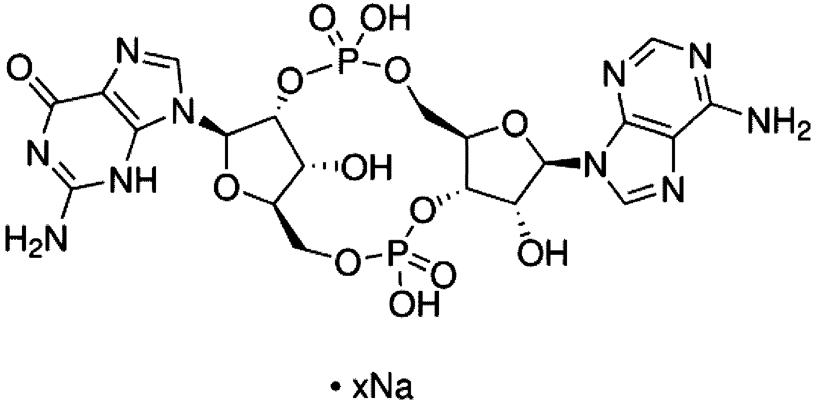 It is available from Cayman and the like.

A trace amount of Mtb antigen present in Extract A, a representative example, has an effect on diseases in immunized mice such as BCG-immunized mice through a mechanism involving the strong activation of IFNγ-producing Th1 cells. Furthermore, Extract A, a representative example, contains Mtb-derived STING agonists necessary to mediate the effects of Extract A on diseases. Th1 cell responsive to non-causative antigen components such as Extract A is also found in human PBMCs and is expected to respond to the anti-tumor effects of Extract A in humans. Non-causative antigen components like Extract A accelerate immune responses to causative factors (e.g., anti-SARS-CoV-2 immunity) in PBMCs naive to SARS-CoV-2 such as causative factors through the modulation of Treg/Th1 balance.

Extract A, a representative example of the present disclosure, is a hot water extract of Mycobacterium tuberculosis (Mtb). As demonstrated in the Examples, Extract A, a representative example of non-pathogenic antigen components, exhibits anti-tumor and antimicrobial activities (particularly against non-Mtb pathogens). Mechanisms underlying the anti-tumor and antimicrobial effects of Extract A are elucidated in the present disclosure. As shown in the present specification, immunization of animals such as mice with non-pathogenic antigen components like Extract A or non-pathogenic factors like BCG results in the realization of potent effects (e.g., anti-tumor effects) against diseases. In vivo studies with BCG-immunized mice, as illustrated in the present specification, revealed that IFNγ-secreting CD4 T cells and Mtb-derived STING agonists identified in Extract A are essential mediators for the effects (e.g., anti-tumor effects) of non-pathogenic antigen components like Extract A against diseases. Trace amounts of Mtb proteins can be detected in Extract A solutions, and the administration of one such protein to BCG-immunized mice through injection also demonstrated effects against diseases. Furthermore, PPD-responsive Th1 cells in human PBMCs also exhibit responsiveness to non-pathogenic antigen components like Extract A, implying the potential of these cells to respond to diseases in humans. Additionally, by utilizing human PBMCs derived from healthy donors naive to pathogens such as SARS-CoV-2, specific T cell responses to pathogens like SARS-CoV-2 can be enhanced by non-pathogenic antigen components like Extract A, through the modulation of the Treg/Th1 balance. Specifically, the boosting effect of T cells by non-pathogenic antigen components like Extract A relies on existing Mtb memory. Thus, non-pathogenic antigen components like Extract A potentially demonstrate therapeutic effects against various diseases (e.g., autoimmune diseases, allergies, cancer, and infections such as COVID-19) by boosting heterologous Th1 cell responses, or by training acquired immune cells in individuals with BCG immunity or a history of tuberculosis.

The present inventors have analyzed the mechanism underlying the anti-tumor and anti-infectious effects of non-causative factor antigen components such as Extract A. The activity (such as anti-tumor activity) of non-causative factor antigen components like Extract A against diseases was found to be observed only in mice with immune memory to BCG, and not in naive mice. Furthermore, mass spectrometry analysis of Extract A solution revealed the presence of Mycobacterium tuberculosis (Mtb)-related antigens, indicating their similar effect on diseases as Extract A. Additionally, through in vivo studies using disease models (e.g., tumor-bearing mice) and utilizing mice lacking various immune cells or molecules, it was discovered that both IFNγ-producing Th1-type CD4 T cells and the STING pathway mediated by Mtb-derived STING agonists present in Extract A contribute effectively to the effects of non-causative factor antigen components like Extract A against diseases. Moreover, a strong correlation was found between the T cell responses induced by Extract A and those mediated by Mtb antigens in human PBMCs obtained from healthy donors. This suggests that the anti-tumor effects of non-causative factor antigen components like Extract A in a clinical setting depend on T cell responses which can be a non-causative factor (e.g., Mtb) specific cross-reactiveness.

In order to evaluate the antiviral effects of non-causative factor antigen components such as Extract A, focusing on Th1 cytokine IFNγ, we have demonstrated that non-causative factor antigen components like Extract A enhance the production of IFNγ mediated by causative factors such as SARS CoV-2 in PBMC obtained from naive healthy subjects for causative factors such as SARS CoV-2, and this phenomenon is prominently detected in Th1 cells.

While not wishing to be bound by theory, as a mechanism supporting the present disclosure, it is understood that adjustment of Treg/Th1 balance is achieved through the mechanism that relies on existing MtB memory, and does not depend on training immunity induced by adjuvants, to promote specific IFNγ production from T cells towards causative factor antigens such as SARS CoV-2 by non-causative antigen components like Extract A. Consequently, the present disclosure indicates the potential of non-causative antigen components (e.g., Extract A) as effective therapeutic tools for various diseases, including cancer and infections (including COVID-19).

In one embodiment, the aforementioned causative factor antigen component may be infectious pathogenic factors, while the aforementioned non-causative antigen components may function as adjuvants when using the aforementioned causative factor antigens as vaccines.

By focusing on Th1 cytokine IFNγ, the present inventors have demonstrated that in non-infected healthy PBMC, non-causative antigen components (e.g., Extract A) can induce the production of IFNγ from CD4 and CD8 T cells through causative factors (e.g., SARS CoV-2). While not bound by theory, non-causative antigen components (e.g., Extract A) promote IFNγ production from T cells specifically towards causative factors (e.g., SARS CoV-2) by regulating Treg/Th1 balance through a mechanism that relies on existing MtB memory and does not depend on adjuvant-induced training immunity. Therefore, the experimental data provided in the present disclosure show that the non-causative factor antigen component (e.g., Extract A) could be an effective means of immunotherapy for viral infectious diseases (e.g., COVID-19).

In one embodiment of the present disclosure, it has been demonstrated that Extract A, a representative example of a non-causative factor antigen component, can induce anti-tumor immunity only in mice with pre-existing immunity induced by BCG immunization. While not bound by theory, as a mechanism in this embodiment of the present disclosure, the presence of several MtB-derived proteins in Extract A has been found to be crucial for the induction and activation of Th1-type memory T-cell responses induced by BCG immunization, in the tumor microenvironment. Furthermore, in the tumor microenvironment of BCG-immunized mice, the activation of the STING pathway mediated by IFNγ production from Tbet+CD4 T-cells induced by Extract A and the STING agonist derived from MtB found in Extract A is essential for mediating the anti-tumor effects of Extract A. Importantly, Extract A-reactive cells have been found to correlate with MtB-derived protein antigens including protein antigens detected in Extract A (e.g., as MHS-P10), suggesting that the mechanism mediating the anti-tumor effects of Extract A in humans immunized with BCG vaccine or with a history of tuberculosis could be similar to what has been observed in BCG-immunized mice. Furthermore, the present disclosure has revealed that MtB-derived Extract A can enhance the Th1-type T-cell response specific to SARS CoV-2 by shifting the Treg/Th1 balance towards the Th1-type effector T-cell phenotype, through a mechanism dependent on pre-existing MtB-specific T-cell memory in SARS CoV-2-naive hPBMC. The focus of this disclosure is not on a general innate immune response, but rather on an acquired immune response mediated by SARS CoV-2, and the observed phenomena in the present invention are believed to be not solely dependent on the training immunity induced by Extract A, as the results of the present invention were dependent on pre-existing MtB memory. Taken together, the data regarding the anti-tumor effects obtained under in vivo conditions and the anti-viral effects of Extract A obtained under in vitro conditions suggest that the protective effects mediated by Extract A against pathogens or tumors containing non-MtB-related antigens could be induced by two mechanisms. One involves MtB/Coronavirus-specific T-cell responses with cross-reactivity against SARS CoV-2/tumors, and the other involves induction of trained acquired immune responses against non-MtB pathogens or tumors through expansion proliferation and mobilization to the tumor/infection site of IFNγ-producing MtB-specific Th1 cells.

In one embodiment, as non-causative antigen components, Mycobacterium tuberculosis hot water extracts or parts thereof include Mycobacterium tuberculosis-derived STING agonists, LpqH, Y1269, PPD, CMV pp65 overlap peptide, MtB-related antigens, components capable of biosynthesizing STING agonists within human cells, NOD2 agonists (e.g., peptide glycans with MDP or MDP-like structures) or the like.

In one embodiment, the present disclosure provides specific and effective therapeutic and preventive effects against the target disease (e.g., infection, allergy, or autoimmune disease) by utilizing non-causative antigen components with immune memory in test subjects under the presence of causative factor antigen components derived from and/or cross-reacting with the causative factors of the target disease.

In one aspect, the present disclosure provides a composition for the prevention or treatment of a disease, characterized by being administered under conditions where a causative factor antigen component derived from and/or cross-reactive with the causative factor of the target disease is present in a subject, and further comprising a non-causative factor antigen component that is neither derived from the causative factor nor cross-reacts with the causative factor of the disease.

In another aspect, the present disclosure provides a pharmaceutical composition for the prevention or treatment of a disease in a subject, comprising a combination of a causative factor antigen component derived from and/or cross-reactive with a causative factor of the disease, and a non-causative factor antigen component that is neither derived from nor cross-reactive with the causative factor of the disease.

In yet another aspect, the present disclosure provides a kit for the prevention or treatment of a disease in a subject, comprising a causative factor antigen component derived from and/or cross-reactive with a causative factor of the disease, and a non-causative factor antigen component that is neither derived from nor cross-reactive with the causative factor of the disease.

By providing such compositions, pharmaceuticals, or kits, exposure of a combination with antigen components other than non-causative factor antigen components to a subject controls humoral immunity against causative factor antigen components, thereby achieving prevention or cure of the target disease.

In one embodiment, the causative factor includes foreign substances for the target. Alternatively, in another embodiment, the causative factor includes pathogens of infections, substances causing immunological abnormalities such as allergy, autoimmune disease, and the like,, or toxins.

In a preferable embodiment, the disease targeted by the disclosure is an infectious disease. The disease targeted by the disclosure can include, for example, bacteria, microorganisms, viruses, mycoplasmas, and the like, and preferably, viral infections. Viral infections can be caused by viruses belonging to the coronavirus family, or viruses belonging to the group selected from rhinoviruses, adenoviruses, coronaviruses, RS viruses, influenza viruses, parainfluenza viruses, and enteroviruses. Alternatively, the causal factor can be viruses belonging to the genus Betacoronavirus, specifically selected from the group consisting of HCoV-HKU1, HCoV-OC43, SARS-CoV, MERS-CoV, and SARS-CoV-2. As one of the specific embodiments, the causative factor can even be the SARS-CoV-2 virus. It is understood that any variant of the SARS-CoV-2 virus is included.

In one preferred embodiment, the subject has immune memory for non-causative factor antigen components. While not wishing to be bound by theory, subjects (e.g., those with a history of tuberculosis or vaccination) having immune memory for non-causative factor antigen components (e.g., tuberculosis extracts) may have their humoral immune response directed toward the non-causative factor antigen components by administration of the combination of components disclosed herein, thereby acting on the causative factor antigens that become the target of humoral immune response. It is considered that in this preferable embodiment, the effect is exerted by the induction or enhancement of humoral immunity.

In one embodiment, this composition, medicament, or kit disclosed herein may contain causative factor antigen components. By including additional causative factor antigen components along with non-causative factor antigen components in one composition, it is preferable because it allows for the administration of non-causative factor antigen components under the presence of causative factor antigen components at any time. Alternatively, by including additional causative factor antigen components along with non-causative factor antigen components in one composition, even if the subject already contains causative factor antigen components, the administration of the composition can enhance its effect when humoral immunity is not sufficiently controlled.

In one embodiment, the disclosed composition, medicament, or kit is intended for the prevention of the mentioned disease. In another embodiment, the disclosed composition, medicament, or kit, among others, is intended for the treatment of the mentioned disease. When the disclosed composition, medicament, or kit is directed towards prevention, it is preferable for them to contain causative factor antigen components. This preference arises from the fact that the targets requiring prevention often lack causative factor antigen components, and even when they are present, they are considered insufficient to achieve the desired effect.

In one embodiment, the disclosed composition, medicament, or kit prevents or treats the aforementioned disease without or with reduced immune hyperreactivity. While not bound by theory, it is often observed that immunomodulatory agents affecting the immune system can lead to immune hyperreactivities, such as cytokine storms, and instances where severe COVID-19 patients rapidly deteriorate and die due to immune hyperreactivities are well-documented. The disclosed technology, while not limited by theory, combines causative factor antigen components and non-causative factor antigen components to achieve treatment or prevention by controlling humoral immunity in a manner that does not involve or reduces immune hyperreactivities, thereby eliciting an immune response against the causative factor for the intended purpose. Those skilled in the art, based on the disclosure of this specification, can inspect whether immune hyperreactivities are occurring as needed and adjust the amounts of causative factor antigen components and non-causative factor antigen components appropriately. In one preferred embodiment of the present disclosure, Mycobacterium tuberculosis extracts like Extract A can prevent or treat the disease without causing or with reduced immune hyperreactivity. This is particularly advantageous in fields where repeated administration, such as during pandemics, is anticipated, compared to previously known methods that can lead to immune hyperreactivities upon repeated administration, making it especially favorable.

In one embodiment, the disclosed composition, medicament, or kit is administered in a form that effectively regulates cellular immunity against the causative factors of the mentioned disease (such as induction, enhancement, suppression, attenuation, etc.). Such modes of administration may include delivering all components simultaneously at once or at different time intervals. This could involve alternating administration of non-causative factor antigen components and causative factor antigen components, or initially administering non-causative factor antigen components and causative factor antigen components simultaneously, followed by subsequent administrations of non-causative factor antigen components alone. In this context, intentional administration of causative factor antigen components (such as in the form of a vaccine for the disease) is permissible, as well as administration in a natural form where the subject has contracted the disease (including cases where subjective symptoms are present or absent) is permissible. In the case of infections, this could encompass stages prior to the onset of symptoms. Natural contraction of the disease may also include instances where the subject has a history of the disease or is currently affected by the disease.

In one embodiment, it is characterized in that whether a causative factor antigen component derived from and/or cross-reactive with the causative factor of the target disease is present in the aforementioned subject is confirmed, and when it is not present, the causative factor antigen component is administered together with the aforementioned non-causative factor antigen component. The presence of causative factor antigen components can be determined by conducting any known antigen testing or similar methods in the field.

In one embodiment, the target disease is an infectious disease, an allergy, or an autoimmune disease.

In one embodiment, the disease is a viral infectious disease or bacterium infectious disease; in a specific embodiment, it may be a viral infectious disease, for example, an infectious disease related to Coronavirus.

The causative factor antigen component is a molecule that is at least partially present on the surface of the causative factor of the target disease. Therefore, if the target disease is a viral infection, the component could constitute the molecules forming virus particles; if the target disease is a bacterial infection, it could be a molecule present on the cell membrane or cell wall.

In another aspect, the present disclosure provides combination of a causative factor antigen component derived from and/or cross-reactive with a causative factor of a target disease in a subject and a non-causative factor antigen component that is neither derived from nor cross-reactive with the causative factor of the disease.

In one embodiment, causative factor antigen components encompass pathogens of infections, substances causing immunological abnormalities such as allergy autoimmune disease, and the like, or toxins. In specific instances, these components are bacteria, microorganisms, viruses, protozoa, or their constituents. The term "constituents" may also include substructures such as individual organelles or protein units within living organisms, as well as fragments. These components may represent the smallest functional units capable of regulating antigen-antibody reactions. In one embodiment, causative factor antigen components are viruses or their substructures. For instance, in specific examples, these components are viruses belonging to the coronavirus family or substructures thereof. Alternatively, in different embodiments, the causative factors are viruses belonging to a family selected from the group consisting of Rhinovirus, adenovirus, Coronavirus, RS virus, influenza virus, parainfluenza virus, and Enterovirus. Preferably, the causative factor are the genus Betacoronavirus, including examples such as HCoV-HKU1, HCoV-OC43, SARS-CoV, MERS-CoV, and ARS-CoV-2. In a particular embodiment, it are the SARS-CoV-2 virus.

In another embodiment of the present disclosure, the non-causative factor antigen component is a Mycobacterium tuberculosis hot water extract or a part thereof and may be, for example, human Mycobacterium tuberculosis Aoyama B strain hot water extract or a part thereof.

In one embodiment, the kit of the present disclosure includes an instruction manual that provides guidance on how to use each component contained in the disclosed kit. If the components included are medicaments or compositions, the manual provides instructions on how to administer them. If the kit includes reagents for antigen testing, the manual may also detail the methodology for conducting the antigen test.

In another aspect, the present disclosure provides a method for preventing or treating a disease in a subject, comprising administering an effective amount of a non-causative factor antigen component that is neither derived from nor cross-reactive with the causative factor of the disease to the subject, under conditions where a causative factor antigen component derived from and/or cross-reactive with the causative factor of the disease is present. This method allows for the adoption of any of the various embodiments described elsewhere, including the prevention and treatment of the disease based on the combination of causative factor antigen components and non-causative factor antigen components.

In another aspect, the present disclosure provides a method for preventing or treating a disease in a subject, comprising administering an effective amount of a causative factor antigen component derived from and/or cross-reactive with the causative factor of the disease to the subject, and administering an effective amount of a non-causative factor antigen component that is neither derived from nor cross-reactive with the causative factor of the disease to the subject. This method allows for the adoption of any of the various embodiments described elsewhere, including the prevention and treatment of the disease based on the combination of causative factor antigen components and non-causative factor antigen components.

In another aspect, the present disclosure provides a method for preventing or treating a disease in a subject. The method involves testing whether causative factor antigen components, which are derived from and/or cross-reactive with the causative factors of the disease, are present in the subject. Based on the results of the test, if effective amounts of causative factor antigen components are present in the subject, the method further comprises administering effective amounts of non-causative factor antigen components, which do not originate from the causative factors of the disease nor cross-react with them, to the subject. Alternatively, if effective amounts of causative factor antigen components are not present in the subject, the method involves administering effective amounts of both causative factor antigen components and non-causative factor antigen components, which do not originate from the causative factors of the disease nor cross-react with them, to the subject. This method allows for the adoption of any of the various embodiments described elsewhere, including the prevention and treatment of the disease based on the combination of causative factor antigen components and non-causative factor antigen components.

### <Prevention and Treatment of Diseases Based on Immune Memory Mechanism>

The present disclosure generally provides compositions or treatments, utilizing the immune memory mechanism, for the prevention or treatment of diseases in test subjects (such as infectious diseases, allergies, and autoimmune diseases). While not bound by theory, the disclosure involves compositions, medicaments, or kits that combine causative factor antigen components, derived from and/or cross-reactive with the causative factors of the target disease, with non-causative factor antigen components to control the immune response against the target disease. This is based on the presence of immune memory in the test subject. These compositions, medicaments, or kits aim to achieve these outcomes.

In one embodiment, the disclosed composition, medicament, or kit containing non-causative factor antigens is characterized by the administration of non-causative factor antigens to the subject under the condition of the presence of causative factor antigen components, derived from and/or cross-reactive with the causative factors of the target disease. Specifically, for subjects already possessing the causative factor of the target disease (such as infectious diseases, allergies, or autoimmune diseases), the disclosed composition, medicament, or kit may be administered with non-causative factor antigens alone. On the other hand, for subjects not possessing the causative factor of the target disease, the disclosed composition, medicament, or kit may be administered with non-causative factor antigens in combination with causative factor antigen components of the target disease (such as infectious diseases, allergies, or autoimmune diseases).

In one embodiment, the causative factors of the target disease in the subject encompass foreign substances for the subject. In a more specific embodiment, the aforementioned causative factors include pathogenic factors of infectious diseases, allergens, causative agents of autoimmune diseases, or toxins associated with immunological abnormalities.

In one embodiment, the disclosed composition, medicament, or kit is intended to prevent or treat the disease in the subject without inducing or with reduced immune hyperreactivity. While not bound by theory, the disclosed composition, medicament, or kit is believed to not induce immune hyperreactivity in the subject, as it not only activates the immune response against the target disease in the subject but also enhances the production of IL-10, which functions to suppress the immune response.

In one embodiment, the disclosed composition, medicament, or kit is administered in a form effective for controlling cellular immune responses (including upregulation or downregulation) against diseases (such as infectious diseases, allergies, or autoimmune diseases) in the subject.

In one embodiment, the disclosed composition, medicament, or kit is used for the treatment of infectious diseases, allergies, or autoimmune diseases. In a specific embodiment, the disease may involve viral infections, bacterial infections (such as bacterial conjunctivitis), or parasitic infections (such as Acanthamoeba keratitis). In a more specific embodiment, the disease may specifically pertain to viral infections. In an even more specific embodiment, the disease may relate to infections associated with coronaviruses.

In further embodiments, the present disclosure provides compositions comprising a non-causative factor antigen component that is neither derived from nor cross-reactive with an infectious disease, an allergy, and/or an autoimmune disease to be the target, which activates (or removes, converts, or transforms inhibition) a regulatory T cell (Treg) against the target, wherein a test subject has immune memory for the antigen component, and, in the test subject, an activity of the regulatory T cell (Treg) against cells infected with the causative factor of the infectious disease, the allergy, and/or the autoimmune disease or cells containing the causative factor is suppressed. Alternatively, the present disclosure provides methods involving administering an effective amount of non-causative factor antigen components to a subject under the presence of the causative factors of the target infection, in order to activate (or relieve inhibition, conversion, or transformation of) regulatory T cells (Treg) suppressed in the test subject, and having immune memory to the non-causative antigen components, against the target.

In this embodiment, preferably, the aforementioned Treg is converted to an effector T cell (Teff) for the causative factor of the disease (infectious disease, allergy, and/or autoimmune disease) or the cell containing the causative factor of the disease in the aforementioned test subject, by further presence of a causative factor antigen component derived from and/or cross-reactive with the causative factor of the target disease in the aforementioned test subject. In a more preferred embodiment, the aforementioned test subject has immune memory for the aforementioned causative factor and/or a factor cross-reactive with the causative factor.

In the present disclosure, apart from non-specific immune actions, it was found that the included components (non-causative components) act on superior antigen causative factor immunity under the presence of the target (causative factor). This was also confirmed with other components, and it was found that non-causative factor antigen components specific to a test subject (or test subject has immune memory) can be employed for the treatment or prevention of targets (e.g., infectious diseases, allergies, or autoimmune diseases). For example, it was demonstrated that a human type Mycobacterium tuberculosis hot water extract containing the antigen shows preventive and therapeutic effects against the onset of diseases other than tuberculosis (e.g., infectious diseases, allergies, or autoimmune diseases) in the presence of the causative factors of these diseases, in BCG-infected cells used as the model (that is, cells that can be assessed as having immune memory against components of Mycobacterium tuberculosis as antigens). Furthermore, for the therapeutic and/or preventive effect of the specific components (human type Mycobacterium tuberculosis hot water extract in the case of BCG) in test subjects who have immune memory for a specific component such as BCG-infected cells, antigen responsiveness of the specific component (e.g., Mycobacterium tuberculosis hot water extract) is also important. As the mechanism thereof, it was newly found that the antigenic response are triggered by immune memory resulting from past vaccinations (antigens), etc., and an immune response to antigens other than the target enhances the immune effects against the target. While not bound by theory, it can be inferred that the presence of causative factor antigen components derived from and/or cross-reactive with the causative factors of the target disease in the test subject can further induce the conversion of Treg into effector T cells (Teff) against the causative factors of the disease (infectious disease, allergy, and/or autoimmune disease) or cells containing the causative factors of the infectious disease in the test subject.

In one embodiment, the present disclosure provides a composition comprising non-causative antigen components other than the causative factors of the target infectious disease. The composition is designed to activate regulatory T cells (Treg) in which a test subject possesses immune memory to the antigen components, and activity against the causative factors of the infectious disease or cells infected with the causative factors of the infectious disease is suppressed in the test subject. Alternatively, the present disclosure encompasses a method including administering an effective amount of non-causative factor antigen components to a test subject to activate regulatory T cells (Treg), which possess immune memory for the non-causative factor antigen components and are suppressed in the subject. In the present disclosure, the activation of Treg can confer cytotoxicity against infected cells or an immune-potentiating effect against causative factors of the infectious disease. Particularly, by further including causative antigen components originating from and/or cross-reacting with causative factors of the target disease in the subject, the aforementioned Treg cells are preferably converted into effector T cells (Teff) against the causative factors of the disease (infectious disease, allergy, and/or autoimmune disease) or cells containing the causative factors of the disease (e.g., cells infected with causative factors of an infectious disease if the disease is infectious).

In one embodiment, the aforementioned Treg cells are memory T cells and/or CD4-positive cells. While not bound by theory, this is due to the relationship with immune memory and the observed activity via CD4-positive cells, notwithstanding any theoretical constraints.

In another embodiment, the aforementioned antigen component contains a protein, a part thereof, or a peptide.

In one embodiment, the non-cause antigen component includes antigens selected from a group consisting of pathogens or their components related to infections that are not the target disease, antigens related to medical history, and antigens related to vaccination history. In one specific embodiment, the non-cause antigen component includes human type Mycobacterium tuberculosis heat water extract.

The technology disclosed herein allows for the adoption of various embodiments of similar structural features as described in this specification, and their combinations within the scope of the present disclosure, are understood.

In one aspect, the present disclosure provides a method for producing or providing in other manner a composition for the prophylaxis or treatment of an infectious disease, an allergy, or an autoimmune disease in a test subject, the method comprising A) a step of specifying a specific antigen component for the test subject that is neither derived from nor cross-reactive with a causative factor of the infectious disease, the allergy, or the autoimmune disease, B) a step of specifying whether the test subject has immune memory for the antigen component and selecting one with the immune memory, and C) a step of producing or providing in other manner the selected antigen component.

Thus, the present disclosure identifies the responsiveness of a test subject to components effective as vaccines for the target disease based on the past medical history of the test subject. By administering the responsive components to the test subject in relation to their medical history, the disclosure aims to prevent or treat the disease.

Therefore, in another aspect, the present disclosure provides a method for preventing or treating the disease comprising: a) obtaining the past medical history of the test subject; b) identifying the responsiveness of the subject to components effective as vaccines for the target disease based on the obtained medical history; and c) administering the responsive components to the test subject in relation to their medical history.

In one embodiment, in the present disclosure, it is preferable that effective components such as non-causative antigen components are specific to the memory T cells of the test subject. Being specific to memory T cells means that the test subject has immune memory for a particular antigen.

In one embodiment, a specific example found in the present disclosure is the therapeutic drug for infectious diseases, wherein under the presence of the causative factor of the infectious disease, a Mycobacterium tuberculosis hot water extract exhibits anti-infectious effects as discovered in the present disclosure. Conventionally, human type Mycobacterium tuberculosis hot water extract is considered to have non-specific immunotherapeutic effects and the immune-activating effect is considered to be the primary action mechanism thereof. However, the present inventors have intensively studied and found that, in the presence of the causative factor of the infectious disease and apart from non-specific immune action, the included components work towards anti-infectious immunity that is similar to or superior to that by the infectious disease causative factor antigen. This was also confirmed with other components, and it was found that non-causative factor antigen components specific to a test subject (or test subject has immune memory) can be employed for the treatment or prevention of infectious diseases. For example, it was demonstrated that a human type Mycobacterium tuberculosis hot water extract containing the non-causative factor antigen shows preventive and therapeutic effects against the onset of infectious diseases in the presence of the causative factor of the infectious disease, in BCG-infected cells used as the model (that is, cells that can be assessed as having immune memory against components of Mycobacterium tuberculosis as antigens). Furthermore, for the therapeutic and/or preventive effect of the specific components (human type Mycobacterium tuberculosis hot water extract in the case of BCG) in subjects who have immune memory for a specific component such as BCG-infected cells, antigen responsiveness of the specific component (e.g., Mycobacterium tuberculosis hot water extract) is also important. As the mechanism thereof, it was newly found that the antigenic response is triggered by immune memory resulting from past vaccinations (antigens), etc., and an immune response to the non-causative factor antigen enhances the anti-infectious immune action.

In one embodiment, the memory T cells related to immune memory which are targeted by the present disclosure are memory regulatory T cells. As an index thereof, IL-2 production can be observed. Indices thereof besides IL-2 production include IFN-γ production, TNF-α production, combinations of two or three thereof, and the like. In other words, when determining the presence of immune memory, a target antigen is added to a test system containing T cells, and enhancement in IL-2 production, IFN-γ production, TNF-α production, or a combination of two or three thereof is observed, whereby the presence of immune memory to the antigen is determined.

In one embodiment, specific components such as the non-antigenic factors disclosed herein have immune adjuvant activity (e.g., adjuvant activity). Immune adjuvant activity can be confirmed using any known methods in the art. For instance, it can be assessed through actions on natural immune receptors or by evaluating the ability to induce specific antibody production, among other methods.

In one embodiment, specific components such as the non-antigenic factors disclosed herein act antigen-dependently on memory CD4-positive T cells. Whether these components act antigen-dependently on memory CD4-positive T cells can be confirmed using any known methods in the field. For example, flow cytometry analysis using MHCII tetramers and specific antigen peptides, or analyzing IFN-γ-producing cells by flow cytometry after stimulation with specific antigens, can be employed to determine antigen-dependent action on memory CD4-positive T cells.

In one embodiment, specific components such as the non-antigenic factors disclosed herein have the activity to bias the ratio between Foxp3-positive Treg cells and IFN-γ-producing T cells. The bias in the ratio between Foxp3-positive Treg cells and IFN-γ-producing T cells can be confirmed using any suitable techniques for cell analysis (e.g., FACS), and a commonly used approach is to measure it by FACS after intracellular cytokine staining and transcription factor staining. The criteria for determination in this case may include, for example, increasing IFN-γ-producing T cells compared to Foxp3-positive Treg cells, but are not limited to this. The IFN-γ-producing T cells may include Th1 helper T cells. Alternatively, in another embodiment, specific components such as the non-antigenic factors disclosed herein have the activity to bias the ratio between Foxp3-positive Treg cells and type 1 helper T cells.

While not bound by theory, an important aspect of the biasing of the ratio between Foxp3-positive Treg cells and IFN-γ-producing T cells is considered, and its mechanism of action is contemplated. In this regard, specific components such as the heat extract of human type Mycobacterium tuberculosis (immunomodulatory components) act antigen-dependently on memory CD4-positive T cells, thereby biasing the ratio between Foxp3-positive Treg cells and IFN-γ-producing T cells including type 1 helper T cells (Th1 cells). The present inventors have discovered that this leads to a shift towards an immune response resembling an anti-infectious immunity. In other words, the disclosure provides the potential to enhance the antigen-dependent anti-infectious immunity within a living organism by using non-antigen components contained in the disclosed composition to activate dormant T cells and change the balance between suppressive T cells and anti-infectious immunity. In one embodiment, the disclosure encompasses antigen components that can control immune responses through CD4-positive T cells. In one embodiment, the disclosure involves confirming whether a test subject can regulate an anti-infectious immune response through CD4-positive T cells, and if the test subject can regulate an anti-infectious immune response through CD4-positive T cells, administering the composition. In further embodiments, the immune response is not mediated by CD8-positive T cells.

In one embodiment, within the context of the present disclosure, the targeted IFN-γ-producing T cells may include type 1 helper T cells (Th1 cells). While not confined to any particular theory, it is possible to act antigen-dependently on memory CD4-positive T cells and induce a shift towards an anti-infectious immune response, including type 1 helper T cells (Th1 cells). Moreover, increasing IFN-γ-producing T cells within individuals with infectious diseases can enhance the anti-infectious effect.

In another embodiment, within the context of the present disclosure, the IFN-γ-producing T cells may include T-bet-positive Th1 cells. While not bound by specific theories, it is known that in host defense, interferon-γ induces the expression of T-bet, a transcription factor that regulates Th1 cell development, and that interferon-γ production is maintained through feedforward control. T-bet has been studied for its novel roles in Th1 cell recognition of autocrine interferon and its potential to induce abnormal type I interferon responses in the absence of T-bet. T-bet preferentially suppresses genes and pathways activated by autocrine type I interferon, thereby limiting abnormal amplification of type I interferon signaling. Thus, T-bet not only actively drives Th1 cell differentiation but also plays a role in suppressing abnormal autocrine type I interferon and downstream signaling pathways within Th1 cells. (see, for example, Harms Pritchard, G., Hall, A. O., Christian, D. A. et al.: Diverse roles for T-bet in the effector responses required for resistance to infection. J. Immunol., 194, 1131-1140 (2015) and the like).

In one embodiment, within the scope of the present disclosure, the specific (or memory-inducing) non-causative antigen components are preferably capable of enhancing at least one of the following abilities in a sample derived from the subject: IFN-γ production, IL-2 production, and TNF-α production. While not confined to specific theories, the capabilities of enhancing IFN-γ, IL-2, and TNF-α production are advantageous in promoting anti-infectious activity.

In the present disclosure, the use of the BCG infection model can be based on established data, as this model is similar to vaccination history or past infection models. It is suggested that memory T cells established and lying dormant due to prior vaccinations can be reactivated by specific antigens, leading these cells to promote an anti-infectious immune response under the presence of disease-causing antigen. This hypothesis has been demonstrated through experimentation in other immune memory models. Therefore, the therapeutic approach based on the immune memory model of the present disclosure can be applied not only to the examples provided but also to various diseases. Furthermore, similar effects could potentially be observed at the clinical trial level.

While not bound by theory, as a mechanism of action in the present disclosure, it is considered that in animal models, apart from the importance of biasing the ratio of Th1 cells and Foxp3-positive Treg cells, and the production of IFN-γ, other aspects include the initial response of specific components of the present disclosure in peripheral blood-derived cells (such as humans), which are due to memory-type regulatory T cells based on vaccination history or past history, characterized by IL-2 production. Specific components of the present disclosure are believed to function as a crucial mechanism, controlling IFN-γ production and inducing changes in the ratio of Foxp3-positive regulatory T cells (Tregs) and T-bet-positive Th1 cells, as well as inducing IFN-γ production from T-bet-positive Th1 cells, as evidenced by time-course experiments. Insights such as IFN-γ production, IL-2 production, and TNF-α production, each from memory-type regulatory T cells based on test subjects' immune memory such as vaccination history, medical history, and the like are novel and were discovered through diligent research by the inventors of this invention. Therefore, it can be asserted that IFN-γ production, IL-2 production, and TNF-α production using peripheral blood-derived cells from humans and the like have been demonstrated as biomarkers for responder selection through the present disclosure. Additionally, in conjunction with this, by further incorporating antigen components derived from causative factors of the target disease and/or causative factors that cross-react with said causative factors in test subjects, it is noteworthy that Tregs can be converted into effector T cells (Teff) against causative factors of said disease (infectious diseases, allergies, and/or autoimmune diseases) or cells containing the causative factors of the infectious disease within said test subjects.

That is, it indicates the ability to pre-confirm, on an individual basis, the IFN-γ production capability, IL-2 production capability, and TNF-α production capability of this drug using human peripheral blood-derived cells, suggesting the potential for becoming a companion diagnostic. Furthermore, since these biomarkers do not correspond to responses to causative factor antigens of the disease, they can also be utilized for healthy individuals without causative factor antigens, enabling preventive measures.

In this way, novel insights based on scientific evidence have been gained regarding the anti-infectious immunomodulatory effects of heat-extracted materials from human type Mycobacterium tuberculosis, which have already been confirmed as safe. This has led to the invention of a new anti-infectious immunotherapy and preventive method that can be administered to appropriately selected subjects. Furthermore, this mechanism is not limited solely to Mycobacterium tuberculosis, and it is anticipated that in antigen-specific interventions based on infection history and vaccine records for other infectious diseases, the anti-infectious effects can be enhanced, thereby offering individually tailored treatments.

### <Vaccine adjuvant>

In another aspect, the present disclosure provides an adjuvant or adjuvant composition for vaccines against infectious diseases, which includes heat-extracted materials from Mycobacterium tuberculosis or a portion thereof, or offers a therapeutic or preventive method utilizing such adjuvants, whether used as adjuvants or for use as adjuvants.

In another aspect, the present disclosure provides an adjuvant or adjuvant composition for vaccines against infectious diseases, which includes STING agonists, or offers a therapeutic or preventive method utilizing such adjuvants, whether used as adjuvants or for use as adjuvants.

In another aspect, the present disclosure provides an adjuvant or adjuvant composition for vaccines against viral infectious diseases, which includes STING agonists, or offers a therapeutic or preventive method utilizing such adjuvants, whether used as adjuvants or for use as adjuvants.

In one embodiment, heat-extracted materials from Mycobacterium tuberculosis or a portion thereof are derived from human-type tuberculosis Aoyama B strain or a portion thereof, and in a preferred embodiment, heat-extracted materials from Mycobacterium tuberculosis or a portion thereof are Extract A.

In one embodiment, heat-extracted materials from Mycobacterium tuberculosis or a portion thereof include tuberculosis-derived STING agonists, LpqH, Y1269, PPD, CMV pp65 overlapping peptide, MtB-related antigens, components capable of biosynthesis of STING agonists within human cells, or NOD2 agonists, wherein heat-extracted materials from Mycobacterium tuberculosis or a portion thereof include c-di-AMP, c-di-GMP, 3',3'-cGAMP, 2',3'-cGAMP, and/or dsDNA producible via cGAS within the human cytoplasm.

In one embodiment, the causative factors of the infectious diseases include bacteria, viruses, or protozoa, and preferably, the causative factors of the infectious diseases are viruses. In a preferred embodiment, the causative factors of the infectious diseases belong to the family Coronaviridae, wherein the causative factors of the infectious diseases are viruses belonging to the family selected from the group consisting of Rhinoviruses, Adenoviruses, Coronaviruses, RS viruses, Influenza viruses, Parainfluenza viruses, and Enteroviruses. In one specific embodiment, the causative factors of the infectious diseases are viruses belonging to the genus Betacoronavirus. In specific embodiments, the causative factors of the infectious diseases are viruses selected from the group consisting of HCoV-HKU1, HCoV-OC43, SARS-CoV, MERS-CoV, and SARS-CoV-2. In one specific embodiment, the causative factors of the infectious diseases are SARS-CoV-2 viruses. In one aspect of one embodiment of the present disclosure, while enhancing immunity, the disclosure is capable of suppressing excessive reactions (improvement and maintenance of the body's intrinsic defense functions).

### (Companion diagnostics, treatment)

"In another aspect, the present disclosure provides treatment based on companion diagnostics for infectious diseases, allergies, and/or autoimmune disorders. In this regard, the disclosure includes a composition for prevention or treatment of said infectious diseases, allergies, and/or autoimmune disorders, which contains non-causative factor antigen components not derived from or cross-reacting with causative factor antigens of said infectious diseases, allergies, and/or autoimmune disorders. The composition is characterized by: 1) confirming an immune response to said non-causative factor in the subject, and 2) administering the composition to the subject if the subject possesses the immune response.

In another aspect, the present disclosure provides a method for prevention or treatment of infectious diseases, allergies, and/or autoimmune disorders in a subject. The method comprises: confirming an immune response to said non-causative factor in the subject and administering an effective amount of non-causative factor antigen components that do not originate from or cross-react with causative factor antigens of said infectious diseases, allergies, and/or autoimmune disorders to the subject requiring the same, if the subject possesses the immune response.

Furthermore, the present disclosure provides non-causative factor antigen components for prevention or treatment of infectious diseases, allergies, and/or autoimmune disorders. The components do not originate from or cross-react with causative factor antigens of said infectious diseases, allergies, and/or autoimmune disorders. The components are characterized by: 1) confirming an immune response to said non-causative factor in the subject, and 2) administering the composition to the subject if the subject possesses the immune response.

Additionally, the present disclosure provides a use for manufacturing medicaments for prevention or treatment of infectious diseases, allergies, and/or autoimmune disorders, which includes non-causative factor antigen components that do not originate from or cross-react with causative factor antigens of said infectious diseases, allergies, and/or autoimmune disorders. The medicaments are characterized by: 1) confirming an immune response to said non-causative factor in the subject, and 2) administering the composition to the subject if the subject possesses the immune response.

In one embodiment, infectious diseases, allergies, and/or autoimmune disorders include infectious diseases. Although not bound by theory, regarding infectious diseases, the disclosure confirms that the composition, in the presence of 1) an immune response to said non-causative factor in the subject, and 2) administration of the composition to the subject with the immune response, demonstrates the conversion of acquired immunity through immune memory to natural immunity, resulting in a favorable vaccine effect, thus providing applications not conventionally considered. The subject possesses immune memory to non-causative factor antigen components, which, upon further exposure, controls humoral immunity to causative factor antigen components (e.g., those different from tuberculosis-derived components), leading to prevention or healing of the target disease.

In one embodiment, the present disclosure provides compositions, methods, uses, or non-causative factor antigen components for prevention or treatment of non-tuberculosis infectious diseases in the subject, wherein the compositions or non-causative factor antigen components contain heat-extracted materials from Mycobacterium tuberculosis or a portion thereof. In a preferred embodiment, heat-extracted materials from Mycobacterium tuberculosis or a portion thereof are derived from human-type tuberculosis Aoyama B strain or a portion thereof, and in a preferred embodiment, heat-extracted materials from Mycobacterium tuberculosis or a portion thereof are Extract A."

In one embodiment, heat-extracted materials from Mycobacterium tuberculosis or a portion thereof are derived from human-type tuberculosis Aoyama B strain or a portion thereof, and in a preferred embodiment, heat-extracted materials from Mycobacterium tuberculosis or a portion thereof are Extract A.

In one embodiment, the compositions or components of the present disclosure are administered in an effective amount for the production of IL-10.

In another embodiment, the infectious disease in the present disclosure is a viral infectious disease, and in specific embodiments, the infectious disease is related to coronavirus viral infectious diseases.

In one embodiment, the immune response can be confirmed subjectively or objectively, preferably by objective confirmation. In certain embodiments, objective confirmation of the immune response may be performed through tuberculin reaction tests or interferon-γ release assays, or from objective information contained in maternal and child health records or equivalent documents, medical records, or other medical information. Preferably, the immune response is examined through tuberculin reaction tests or interferon-γ release assays.

In one embodiment, if the subject does not possess an immune response, non-causative factor antigen components or heat-extracted materials from Mycobacterium tuberculosis or a portion thereof may be administered to the subject in advance, and subsequently, if it is confirmed that the subject possesses the immune response, the composition or non-causative factor antigen components or heat-extracted materials from Mycobacterium tuberculosis or a portion thereof may be administered to the subject. Thus, even in subjects lacking or having lost immune memory, an immune response can be induced, enabling the achievement of the intended therapeutic or preventive effects of the present disclosure. If necessary, the pre-administration may be repeated until the subject possesses the immune response."

In one embodiment, the pre-administration is administration before or after developing the aforementioned infectious disease.

In another embodiment, the pre-administration is administration after developing the aforementioned infectious disease.

In one embodiment, it is administered under conditions where causative factor antigen component derived from and/or cross-reactive with the causative factor is present.

### <Production method, provision method in other manner>

In another aspect, the present disclosure provides a method for producing or providing in other manner a composition for the prevention or treatment of a certain disease or disorder in a test subject. The method includes the steps of: identifying antigen components that are distinct from the causative factors of the infectious disease and specific to the test subject; B) determining whether the test subject has immune memory for the antigen components and selecting those with immune memory; and C) producing or providing in other manner the selected antigen components. The term "or providing in other manner" as used herein refers to any method of providing, other than producing a new entity, and may include, for example, isolating, purifying, or obtaining from an appropriate source of the entity. In step C), it is permissible to produce novel non-causative factor antigen components or provide them by isolating from other sources.

### <Treatment/prophylaxis method, companion diagnosis/treatment, and medicament>

In one aspect, the present disclosure provides a method for the prophylaxis or treatment of an infectious disease, allergy, or autoimmune disease, including a) a step of obtaining an antigen responsiveness profile of a test subject, b) a step of specifying an antigen component or a combination of antigen components from the antigen responsiveness profile, wherein the antigen component or the combination of the antigen components is specified based on the immune responsiveness presented now or in the past by the test subject to the antigen component or the combination of the antigen components, and c) a step of administering the antigen component or the combination of the antigen components identified in step b) to the test subject in an amount sufficient to regulate an immune response in the test subject.

In specific embodiments, obtaining the antigen responsiveness profile in the present disclosure can be confirmed through methods that involve verifying the subject's past physical conditions and determining whether one or more antigen candidates are responsive in samples derived from the subject, or both. For example, when confirming immune responsiveness to tuberculosis, methods such as the tuberculin skin test and interferon-γ release assays can objectively indicate immune responsiveness.

In another embodiment, obtaining the antigen responsiveness profile in the present disclosure includes identifying antigen components or combinations thereof that control the immune response via CD4-positive T cells.

In specific embodiments, relevant past medical conditions that could be utilized include medical history and vaccination history. Medical history and vaccination history can be obtained by referencing materials such as maternal and child health records or equivalent documents, medical records, and other medical information (including electronically recorded information of the subject stored in clouds or electronic chips, for instance). Maternal and child health records (MCH handbook) refer to essential notebooks (books) containing information maintained by families, intended to promote and maintain the health of both mothers and children.

In specific embodiments, the applicable physical conditions may include a history of infection, and the usable non-causative factor antigen may encompass an antigen related to the infectious disease. The types of infectious disease that can be utilized are optional. For example, tuberculosis, malaria, yellow fever virus, smallpox virus, smallpox vaccination, measles/rubella, polio, epidemic parotitis (mumps)/MUMPS, rotaviral infectious disease, varicella (possum), yellow fever, Ebola, West Nile fever, hib infection, pneumococcul infection, pertussis, Japanese encephalitis, meningococcal infection, Salmonella infection, pathogenic Escherichia coli, Toxoplasma, Zika virus, herpes virus type 1, EBV/Epstein-Barr virus (herpes virus type 4), CMV/cytomegalovirus (herpes virus type 5), influenza, MARS, rabies, and diphtheria and the like can be mentioned.

The diseases such as infectious diseases, allergies, or autoimmune diseases derived from non-causative factors used in the present disclosure may include any optional diseases such as infectious diseases, allergies, or autoimmune diseases as described in this specification. Therefore, in the context of describing physical conditions in this specification, when terms such as "BCG vaccination history", "tuberculosis infection history", or "antigen responsiveness to Mycobacterium tuberculosis" are used, these physical conditions may be replaced with any optional infectious diseases as described in this specification.

In a preferred embodiment, diseases such as infectious diseases, allergies, or autoimmune diseases derived from non-causative factors are tuberculosis. In this case, the physical conditions may involve BCG vaccination history, tuberculosis infection history, or antigen responsiveness to Mycobacterium tuberculosis. In a preferred embodiment where the infectious disease is tuberculosis, it may be advantageous for the non-causative antigen component to include human type Mycobacterium tuberculosis hot water extract. This disclosure can be employed for treatment and additionally, for preventive purposes.

The non-causative factor antigens of the present disclosure may be provided in the dosage form of a pharmaceutical composition. In specific embodiments, the pharmaceutical composition, medicament, or kit may comprise one or more compounds and at least one pharmaceutically acceptable carrier, wherein one or more compounds may be converted into at least one compound (e.g., a prodrug) in the test subject, for example, at least one compound of Extract A (see examples). In certain embodiments, the pharmaceutical composition, medicament, or kit may comprise one or more compounds and at least one pharmaceutically acceptable carrier, wherein one or more compounds may be converted into at least one (non-infectious) antigen component in the test subject, i.e., a prodrug. In cases involving multiple drugs, they may be included within a single composition (combination formulation), or they may be contained in separate compositions. When formulated as a single composition, the formulation may utilize known forms in the art, including those exemplified in the present disclosure.

The term "carrier" as used herein refers to a pharmaceutically acceptable substance, composition, or excipient such as, for example, a liquid or solid bulking agent, diluent, additive, solvent, or capsule forming agent, which is associated with or enables the transport or carriage of a target pharmaceutical compound from an organ or portion of the body to another organ or portion of the body. "Pharmaceutically acceptable" refers to being compatible with other raw materials in a formulation and being harmless to patients. Non-limiting examples of pharmaceutically acceptable carriers, carriers, and/or diluents can include sugars such as lactose, glucose, and sucrose, starch such as corn starch and potato starch, cellulose and derivatives thereof such as carboxymethylcellulose sodium, ethyl cellulose, and cellulose acetate, excipients such as powdered tragacanth, malt, gelatin, talc, cocoa butter, and suppository wax, oil such as peanut oil, cotton seed oil, safflower oil, sesame oil, olive oil, corn oil, and soybean oil, glycols such as propylene glycol, polyols such as glycerin, sorbitol, mannitol, and polyethylene glycol, esters such as ethyl oleate and ethyl laureate, buffering agents such as agar, magnesium hydroxide, and aluminum hydroxide, alginic acid, pyogenic substance-free water, isotonic saline, Ringer's solution, ethyl alcohol, phosphate buffer, and other nontoxic compatible substances used in a pharmaceutical formulation. A humectant, emulsifier, and lubricant such as sodium lauryl sulfate, magnesium stearate, or polyethylene oxide-polypropylene oxide copolymer, as well as a colorant, releasing agent, coating agent, sweetener, flavoring agent, fragrance, preservative, and antioxidant can also be included in a composition.

As used herein, "parenteral administration" refers to a dosage form for any route that is not oral administration. Any mode for administration in a mode and level that are effective for treating or preventing a disease intended for infectious disease treatment, prevention, or the like is employed. Examples of means of parenteral administration include administration through transdermal absorption or transmucosal absorption, as well as injection, infusion, and combinations thereof. For example, administration through transdermal absorption or transmucosal absorption exerts an effect by contacting a transdermally absorbed formulation such as a paste agent, adhesive formulation, or spray with the skin or mucous membrane so that a drug in the formulation migrates into the body through the skin or mucous membrane. Examples of administration via injection or infusion include intravenous, intradermal, subcutaneous, intramuscular, and enteral administration (intestinal infusion), which can also be administered as a bolus and/or sustained infusion. Injection or infusion can use a suspension, liquid agent, emulsion, or implanted agent in an oily or aqueous medium, comprising another formulation substance such as a suspending agent, stabilizer, and/or a dispersant. For intradermal administration, it can be combined with a device such as a microneedle to form a less invasive dosage form. Enteral administration (intestinal infusion) can provide sustained drug delivery to the proximal small intestine by using a tube or portable infusion pump by percutaneous endoscopic gastrostomy. More preferably, the administration can be subcutaneous administration, intradermal administration, or intramuscular administration. Parenteral administration (e.g., transdermal administration) can be performed with a tape/patch agent or powder, spray, ointment, paste, cream, lotion, gel, solution, or the like. A composition, medicament, kit, or the like suitable for parenteral administration can comprise at least one type of a pharmaceutically acceptable aseptic isotonic aqueous or non-aqueous solution, dispersant, suspension, emulsion, implanted agent, or aseptic powder that can be reconstituted in an aseptic injection solution or dispersant immediately before use.

The compositions, medicament, kit, or the like disclosed herein that are suitable for oral administration can be in a dosage form of a capsule, cachet, pill, tablet, lozenge (generally using a fragrance base tragranth or acacia and sucrose), powder, granule, aqueous or non-aqueous liquid solution, aqueous or non-aqueous liquid suspension, oil-in-water emulsion, water-in-oil emulsion, elixir, syrup, troche (using inactive base such as gelatin, glycerin, sucrose, and/or acacia), and/or mouthwash, which each comprises a predetermined amount of at least one type of compound in the present disclosure.

A composition, medicament, kit, or the like disclosed in the present specification can be administered as a bolus, an electuary or paste.

The causative factor antigen and non-causative factor antigen of the present disclosure can be administered in any dosage form. Any dosage form, whether oral administration or parenteral administration, can be used, as long as the effect can be exerted. Preferably, parenteral administration is used.

When administering causative and non-causative factor antigens of the present disclosure as medicaments, various delivery systems are known, and using such systems, it is possible to administer the therapeutic agents of the present disclosure to appropriate sites. Examples of such systems include liposomes, lipid particles, micro-particles, and microcapsules for encapsulation; for polypeptides, the use of recombinant cells capable of expressing them, and the use of endocytosis mediated by receptors. Constructing therapeutic nucleic acids as part of retroviral vectors or other vectors is also possible. Methods of introduction include, but are not limited to, intradermal, intramuscular, intraperitoneal, intravenous, subcutaneous, intranasal, extradural, and oral routes. By any suitable route, for example, administration can be accomplished through injection, bolus injection, absorption through epithelial or mucous membranes (e.g., oral, rectal, and intestinal mucous membranes), and if necessary, using an aerosolizer for inhalation or a nebulizer, and coadministration with other biologically active agents is also possible. Administration can be systemic or local. Furthermore, if there is a lesion, it can be administered through an appropriate route, such as direct injection into the lesion or other suitable routes.

A solid dosage form for oral administration (capsule, tablet, pill, sugar coated tablet, powder, granule, or the like) can be mixed with any of one or more types of pharmaceutically acceptable carrier such as sodium citrate or dicalcium phosphate, and/or a filler or bulking agent such as starch, lactose, sucrose, glucose, mannitol, and/or silicic acid, binding agent such as carboxymethyl cellulose, alginic acid salt, gelatin, polyvinyl pyrrolidone, sucrose, and/or acacia, a moisturizing agent such glycerol, a disintegrant such as agar, calcium carbonate, potato or tapioca starch, alginic acid, specific silicic acid salt, sodium carbonate, and sodium starch glycolate, a dissolution delaying agent such as paraffin, an absorption promotor such as a quaternary ammonium compound, humectant such as cetyl alcohol, glycerol monostearate, and polyethylene oxide-polypropylene oxide copolymer, an absorbent such as kaolin and bentonite clay, a lubricant such as talc, calcium stearate, magnesium stearate, solid polyethylene glycol, sodium lauryl sulfate, and mixture thereof, and a colorant. For a capsule, tablet, and pill, a pharmaceutical composition, medicament, kit, or the like can also comprise a buffering agent. A similar type of solid composition, medicament, kit, or the like can also be used as a filler in a soft and hard filled gelatin capsule using an additive such as lactose and high molecular weight polyethylene glycol.

A liquid dosage form for oral administration can comprise a pharmaceutically acceptable emulsion, microemulsion, solution, suspension, syrup, and elixir. In addition to the active ingredient, a liquid dosage form can comprise an inactive diluent used in conventional art, such as water or other solvent, solubilizing agent, and emulsifying agent, e.g., ethyl alcohol, isopropyl alcohol, ethyl carbonate, ethyl acetate, benzyl alcohol, benzyl benzoate, propylene glycol, 1,3-butylene glycol, oil (especially cotton seed oil, peanut oil, corn oil, germ oil, olive oil, castor oil, and sesame oil), glycerol, tetrahydrofuryl alcohol, polyethylene glycol, sorbitan fatty acid ester, mixture thereof, and the like. Furthermore, a compound can be dissolved using cyclodextrin such as hydroxypropyl-β-cyclodextrin.

The component of the present disclosure can comprise an adjuvant such as a humectant, emulsifying and suspending agent, sweetener, flavoring agent, colorant, fragrance, or preservative. In addition to one or more types of compounds according to the present disclosure, a suspension can comprise a suspending agent such as ethoxylated isostearyl alcohol, polyoxyethylene sorbitol and sorbitan ester, microcrystalline cellulose, aluminum methhydroxide, bentonite, agar, tragacanth, or mixture thereof.

The composition, medicament, kit, and the like disclosed in the present specification can be a suppository for rectal or vaginal administration, which can be prepared by mixing one or more types of compounds according to the present disclosure with one or more types of suitable non-stimulatory additives or carriers including cocoa butter, polyethylene glycol, suppository wax, salicylate, or the like. The composition is a solid at room temperature, but a liquid at body temperature. Thus, the composition melts in the rectal or vaginal cavity and releases the compound of the present disclosure. A pharmaceutical composition, medicament, kit, and the like suitable for vaginal administration can also comprise a pessary, tampon, cream, gel, paste, foam, or spray formulation comprising a carrier known to be suitable in conventional art.

The dosage form for topical or transdermal administration of the composition of the present disclosure can comprise powder, spray, ointment, paste, cream, lotion, gel, solution, patch, and inhalant. A pharmaceutical composition or pharmaceutical tablet can be mixed with a pharmaceutically acceptable carrier and a preservative, buffering agent, or high pressure gas that may be required under aseptic conditions.

An ointment, paste, cream, and gel can comprise, in addition to the composition of the present disclosure, an additive such as animal or vegetable fat, oil, wax, paraffin, starch, tragacanth, cellulose derivative, polyethylene glycol, silicone, bentonite, silicic acid, talc, zinc oxide, or mixture thereof.

Powder or spray can comprise, in addition to the pharmaceutical composition or pharmaceutical tablet of the present disclosure, an additive such as lactose, talc, silicic acid, aluminum hydroxide, calcium silicate, or polyamide powder, or a mixture thereof. Furthermore, spray can comprise common high pressure gas such as chlorofluorohydrocarbon and volatile unsubstituted hydrocarbon such as butane and propane.

Ophthalmic formulations, optical ointment, powder, solution, and the like are also understood to be within the scope of the present disclosure.

A composition, medicament, kit and the like suitable for parenteral administration can comprise at least one type of pharmaceutically acceptable aseptic isotonic aqueous or non-aqueous solution, dispersant, suspension, emulsion, or aseptic powder that can be reconstituted in an aseptic injection solution or dispersant immediately before use.

Typically, compositions for injection administration are solutions in sterile, isotonic buffer solutions. If necessary, the composition can also include solubilizing agents and local anesthetics such as lidocaine to alleviate pain at the injection site. Generally, the components can be supplied separately or mixed together in a unit dosage form for supply. For example, they can be provided in sealed containers such as ampoules or sachets indicating the quantity of the active agent, in the form of freeze-dried powder or aqueous concentrate. If the composition is to be administered by injection, it can be distributed using vials containing sterile pharmaceutical-grade water or physiological saline. For injection administration of the composition, it is also possible to provide ampoules of sterile water or physiological saline for injection prior to administration, to ensure that the components are mixable before injection.

The components of the present disclosure, if applicable and as needed, can also be formulated as neutral, salt forms, or other prodrugs (such as esters). Pharmaceutically acceptable salts can include those formed with free carboxyl groups derived from hydrochloric acid, phosphoric acid, acetic acid, citric acid, tartaric acid, etc., as well as those formed with free amine groups derived from isopropylamine, triethylamine, 2-ethylaminoethanol, histidine, procaine, etc. Additionally, salts derived from sodium, potassium, ammonium, calcium, and ferrous hydroxide can also be included.

The term "salt" as used in the present specification includes acid and/or base salt formed with inorganic and/or organic acid and base. As used herein, the term "pharmaceutically acceptable salt" refers to a salt which is suitable for use in contact with tissue of a subject without excessive toxicity, stimulation, allergic reaction, and/or similar events with a reasonable balance of effect/risk ratio within the scope of a definitive medical judgment. Pharmaceutically acceptable salts are well known in the art. For example, pharmaceutically acceptable salts are described in detail in Berge et al., J. Pharmaceutical Sciences (1977) 66: 1-19.

Pharmaceutically acceptable salts can be produced with an inorganic or organic acid. Non-limiting examples of suitable inorganic acids include hydrochloric acid, hydrobromic acid, phosphoric acid, sulfuric acid, and perchloric acid. Non-limiting examples of suitable organic acids include acetic acid, oxalic acid, maleic acid, tartaric acid, citric acid, succinic acid, and malonic acid. Other non-limiting examples of suitable pharmaceutically acceptable salts include adipic acid salt, alginic acid salt, ascorbic acid salt, aspartic acid salt, benzenesulfonic acid salt, besilate, benzoic acid salt, bisulfuric acid salt, boric acid salt, butyric acid salt, camphoric acid salt, camphorsulfonic acid salt, citric acid salt, cyclopentanepropionic acid salt, digluconic acid salt, dodecylsulfuric acid salt, ethanesulfonic acid salt, formic acid salt, fumaric acid salt, glucoheptonic acid salt, glycerophosphoric acid salt, gluconic acid salt, hemisulfuric acid salt, heptanoic acid salt, hexanoic acid salt, hydroiodic acid salt, 2-hydroxy-ethanesulfonic acid salt, lactobionic acid salt, lactic acid salt, lauric acid, lauryl sulfate, malic acid salt, maleic acid salt, malonic acid salt, methanesulfonic acid salt, 2-naphthalenesulfonic acid salt, nicotinic acid salt, nitric acid salt, oleic acid salt, oxalic acid salt, palmitic acid salt, pamoic acid salt, pectinic acid salt, persulfuric acid salt, 3-phenylpropionic acid salt, phosphoric acid salt, picric acid salt, pivalic acid salt, propionic acid salt, stearic acid salt, succinic acid salt, sulfuric acid salt, tartaric acid salt, thiocyanic acid salt, p-toluenesulfonic acid salt, undecanoic acid salt, and valeric acid salt. In some embodiments, examples of organic acids that can produce a salt include acetic acid, propionic acid, glycolic acid, pyruvic acid, oxalic acid, lactic acid, trifluoroacetic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, and salicylic acid.

A salt can be prepared at the time of separation and purification of a disclosed compound or separately by reacting the compound with a suitable base or acid. Non-limiting examples of pharmaceutically acceptable salts obtained from a base include alkali metals, alkaline earth metals, ammonium, and N+(C₁₋₄ alkyl) 4 salts. Non-limiting examples of suitable alkali or alkaline earth metal salts include sodium, lithium, potassium, calcium, magnesium, iron, zinc, copper, manganese, and aluminum salt. Furthermore, non-limiting examples of suitable pharmaceutically acceptable salts optionally include nontoxic ammonium, quaternary ammonium, and amine cation formed using a counter ion such as a halide ion, hydroxide ion, carboxylic acid ion, sulfuric acid ion, phosphoric acid ion, nitric acid ion, lower alkyl sulfonic acid ion, and aryl sulfonic acid ion. Non-limiting examples of suitable organic bases that can produce a salt include primary amine, secondary amine, tertiary amine, substituted amine including naturally-occurring substituted amine, cyclic amine, and basic ion exchange resins such as isopropylamine, trimethylamine, diethylamine, triethylamine, tripropylamine, and ethanol amine. In a specific embodiment, a pharmaceutically acceptable base addition salt can be selected from ammonium, potassium, sodium, calcium, and magnesium salt.

In an embodiment of the present disclosure, a target subject can be a patient in a state before onset of a disease (e.g., infectious disease, allergy, or autoimmune disease), after treatment of the disease, or an early stage of onset of the disease. Alternatively, a target subject can be a healthy individual. If a healthy individual is a subject, the disclosure is performed as a preventive method.

In the present disclosure, a process is carried out to identify antigens or combinations of antigens that elicit a response in the subject based on the antigen responsiveness profile. Here, the responsiveness to the subject may be related to disease treatment or prevention, and in particular, if it is indicative of an association with immune memory. For example, the responsiveness may include a response to the production of IFN-γ, IL-2, TNF-α, or any combination of two or three of these cytokines.

In another aspect of the present disclosure, a method is provided for producing or providing a companion diagnostic agent or diagnostic method for the prevention or treatment of diseases (e.g., infectious diseases, allergies, or autoimmune diseases), as well as treatment or prevention methods or medicaments based thereon.

In one specific embodiment, the confirmation of responsiveness is characterized by conducting preliminary companion diagnostic based on medical history, vaccination history, and testing of immune responsiveness to peripheral blood mononuclear cells (PBMCs) isolated from the subject. The feasibility of such companion diagnostics is demonstrated in the examples of the present disclosure. Those skilled in the art can understand that based on such examples or other specific descriptions, similar companion diagnostics can also be carried out in advance for other diseases or disorders.

In the present specification, "preliminary companion diagnostic based on medical history, vaccination history, and testing of immune responsiveness to peripheral blood mononuclear cells (PBMCs) isolated from the subject" is preferably confirmed using objective indicators. However, if objectivity can be assured, confirmation can also be done using subjective indicators. When confirming using objective indicators, for example, the following steps can be taken: ·Isolate peripheral blood mononuclear cells (PBMCs) from human subjects who desire vaccination effects or treatments according to the present disclosure (including those who have developed viral infections, those who have not developed viral infections (those who have viral antigen but are asymptomatic or have recovered), and normal test subjects). ·Objectively confirm the immune memory (immunoresponsiveness) of the subjects using tests such as tuberculin reaction tests or interferon-γ release assays to examine tuberculosis infections.

When the above tests are not used, while ensuring objectivity, alternative methods such as the following can also be used: ·Acquire the antigen responsiveness profile based on medical history or vaccination history using non-invasive methods, interviews, maternal and child health handbooks or their equivalents, medical records, or other medical information (may rely on electronically recorded information of test subjects stored in clouds or electronic chips).

Here, the vaccination history and infection history can include, but are not limited to, tuberculosis, malaria, yellow fever virus, smallpox virus, vaccinia, measles/rubella, polio, mumps, rotavirus infection, chickenpox, yellow fever, Ebola, West Nile fever, Hib infection, pneumococcal infection, whooping cough, Japanese encephalitis, meningococcal infection, salmonella infection, pathogenic E. coli, toxoplasma, Zika virus, herpes simplex virus type 1, EBV/Epstein-Barr virus (herpes simplex virus type 4), CMV/Cytomegalovirus (herpes simplex virus type 5), influenza (virus), MARS, rabies, diphtheria, among others.

When objectively confirming the statement "confirming immunoresponsiveness to peripheral blood mononuclear cells (PBMCs) isolated from the subject based on tests for immunoresponsiveness", the confirmation can be conducted as follows:
·In one embodiment, the confirmation of responsiveness involves isolating PBMCs (e.g., 1.0 × 10⁴ to 1.0 × 10⁶ cells) from human subjects desiring vaccination effects or treatments according to the present disclosure. The isolated PBMCs are then stimulated for about 6 to 24 hours with an extract such as Extract A (0.01 ug/mL to 1.0 mg/mL) or an extract containing Extract A (0.01 ug/mL to 1.0 mg/mL). The production of cytokines (e.g., IFN-γ, IL-2, TNF-α, etc.) is measured using commonly used detection methods in the art (e.g., ELISA, qPCR, and/or FACS). Subjects whose post-stimulation cytokine production exceeds a reference threshold based on pre-stimulation cytokine production (e.g., 1.1-fold or more, 1.2-fold or more, 1.3-fold or more, etc., preferably 2-fold or more) are selected as responsive subjects for administration.

As an example of such companion diagnostics or treatments, it can be mentioned that one could confirm whether the subject has antigens against the causative factors of the disease and determine whether the use of a human type Mycobacterium tuberculosis hot water extract can be employed as an antigen component, utilizing the subject's past medical history, and particularly their history of tuberculosis infection. Although not bound by theory, using the subject's past medical history, such as their history of tuberculosis infection, one could identify subjects who can be administered with a human type Mycobacterium tuberculosis hot water extract as an antigen component for preventing diseases (e.g., infectious diseases, allergies, or autoimmune diseases). This could enable the prevention of the disease. Alternatively, using the subject's past medical history, such as their history of tuberculosis infection, one could identify subjects who can be administered with a human type Mycobacterium tuberculosis hot water extract as an antigen component for treating diseases (e.g., infectious diseases, allergies, or autoimmune diseases), thus enabling the treatment of the disease. Furthermore, to determine whether the subject has antigens against the causative factors of the disease, antigen testing can be conducted using methods such as antigen testing in the subject's blood.

The aforementioned physical condition in the past and the aforementioned component may be one or more selected from tuberculosis, malaria, yellow fever virus, smallpox virus, smallpox vaccination, measles/rubella, polio, epidemic parotitis (mumps)/MUMPS, rotaviral infectious disease, varicella (possum), yellow fever, Ebola, West Nile fever, hib infection, pneumococcul infection, pertussis, Japanese encephalitis, meningococcal infection, Salmonella infection, pathogenic Escherichia coli, Toxoplasma, Zika virus, herpes virus type 1, EBV/Epstein-Barr virus (herpes virus type 4), CMV/cytomegalovirus (herpes virus type 5), influenza virus, MARS, rabies, and diphtheria.

In one embodiment, the subjects to which the present disclosure is applicable include patients with a history of BCG vaccination or tuberculosis infection, as well as healthy individuals in whom antigen responsiveness has been confirmed. The method disclosed herein constitutes a personalized therapeutic approach. In this context, the subjects of interest could be individuals with a history of vaccination, those with a history of infection, individuals with a history of Mycobacterium tuberculosis infection, or individuals with a history of BCG vaccination.

Furthermore, Mycobacterium tuberculosis extracts that can be used in the present disclosure may be administered preventively before onset, for recurrence prevention after treatment, or during the early stages of the disease (e.g., infectious disease, allergy, or autoimmune disease), and such extracts produced by methods exemplified in this specification or modified methods thereof, can also be administered subcutaneously, intradermally, or intramuscularly during the early stages of disease onset.

In a specific embodiment, the present disclosure provides a method for the prevention or treatment of a test subject's disease (e.g., infectious disease, allergy, or autoimmune disease) using non-causative factor components. The component used here is an antigen or extract identified by means of an interview and/or through patient history and vaccination records. The test subjects are individuals with a confirmed history of infection or vaccination, and the component is administered for recurrence prevention after treatment, preventive administration before onset, or during the early stages of disease onset, and during the early stages of disease onset as needed. This method is characterized by employing non-causative components. Furthermore, the present disclosure also offers non-causative factor antigen components for use in the prevention or treatment of a test subject's disease (e.g., infectious disease, allergy, or autoimmune disease). The component used here is an antigen or extract identified by means of an interview and/or through patient history and vaccination records. The test subjects are individuals with a confirmed history of infection or vaccination, and the component is administered for recurrence prevention after treatment, preventive administration before onset, or during the early stages of disease onset, and during the early stages of disease onset as needed.

In one aspect, the present disclosure provides a vaccine formulation comprising at least one antigen of the present disclosure and an adjuvant. In one embodiment, this vaccine formulation is utilized for personalized medicine. In this personalized medicine approach, appropriately tailored vaccines can be provided to individual patients or subjects based on companion therapies provided as disclosed herein. In specific embodiments of the present disclosure, the target diseases may include infectious diseases, allergies, and autoimmune diseases.

In one embodiment, the identification of a test subject's responsiveness in the treatment or prevention of the present disclosure may be carried out using a history of prior infections, vaccination records, and responsiveness to IFN-γ production, IL-2 production, TNF-α production, or any two or three of these using peripheral blood. The "responsiveness to IFN-γ production, IL-2 production, TNF-α production, or any two or three of these using peripheral blood" may be implemented as follows:
- Isolation of peripheral blood mononuclear cells from the test subject's blood.
- Culturing peripheral blood mononuclear cells in the presence of the substance, or stimulators such as PPD (purified protein derivative of tuberculin), tuberculosis antigens, and the like, and protein transport inhibitors such as Brefeldin A,, monensin, and the like.
- After a certain period of cultivation, recovering peripheral blood mononuclear cells, staining them with fluorescent-labeled antibodies specific to cell surface markers and intracellular cytokines, and analyzing stained cells using a flow cytometer.
- Determining the increase of cytokine-producing cells upon stimulation with Extract A or tuberculosis antigen in memory CD4 T cells (CD3+ CD4+ CD45RA-). Extract A is exemplified, for instance, in Example 1, and described in detail.

In a specific embodiment, the tuberculosis antigen extract used in the present disclosure is a human type Mycobacterium tuberculosis hot water extract or an extract from other Mycobacterium tuberculosis (safe extract). Compositions similar to those used for tuberculin, for example, are also preferred.

In one aspect, the present disclosure provides a vaccine formulation comprising an antigen of the present disclosure and an adjuvant (e.g., a substance that stimulates a Th1-type immune response). In a specific embodiment, this vaccine formulation is utilized for personalized medicine. In this personalized medicine approach, appropriate vaccines are prepared for each patient or subject based on companion therapies provided as disclosed herein. In a specific embodiment, the medicament or antigen component provided in the present disclosure comprises a human type Mycobacterium tuberculosis hot water extract or an extract from other Mycobacterium tuberculosis (safe extract) or an extract component, along with an adjuvant. Such vaccine formulations can be prepared by mixing each material substance at any concentration, as exemplified in Example 4. In certain embodiments, this vaccine formulation is intended for use in methods of personalized medicine.

The following materials can be mentioned as adjuvant materials that can be used as vaccine formulations:
- Adjuvant materials that activate natural immune receptors (e.g., TLR agonists such as bacterial membrane-derived substances, DNA, RNA, dinucleotides, NOD1, NOD2 agonists)
- Aluminum-containing adjuvant materials such as hydroxide aluminum
- Adjuvant materials capable of creating emulsions such as ISA51 or ISA720
- Adjuvant materials containing cytokines (IL-2, IL-12, IFN-α, GM-CSF). Preferably, the adjuvant agent includes a substance (e.g., nucleic acid-based substances like CpG) that stimulates a Th1-type immune response.

In one aspect, the present disclosure provides compositions, medicaments, kits, etc., for treating or preventing a subject's disease, disorder, or symptom. These compositions, medicaments, kits, etc., include causative factor antigens and non-causative factor antigen components (which may be isolated or included in an extract or other form) and are administered at appropriate dosages and usage (e.g., administered subcutaneously, intradermally, or intramuscularly once a day in the first week and approximately once a week in subsequent weeks). The present disclosure provides a composition characterized by such features. Additionally, prevention and treatment methods related to this composition are also provided. In one embodiment, the antigen component is present at about 0.001 µg or more per unit dose. Dosage, dosing intervals, and administration methods may be appropriately selected based on the patient's age, weight, symptoms, target organs, etc. It is also preferable for a therapeutic agent to contain an effective amount of an active ingredient to exhibit a therapeutic effective dose or a desired effect. Effective doses can be estimated from dose-response curves obtained from in vitro or animal model test systems.

In one preferred embodiment, causative factor antigens and non-causative factor antigens can be administered multiple times (e.g., two or more times, three or more times, any integer value) and the frequency of multiple administrations can be varied, increased, or decreased as appropriate. In one embodiment, causative factor antigens and non-causative factor antigens can be administered once a day, three times a week, twice a week, once a week, once every two weeks, once every three weeks, once every four weeks, once every five weeks, once every six weeks, once every seven weeks, once every eight weeks, once every nine weeks, once a month, once every two months, once every three months, once every four months, once every five months, once every six months, once every seven months, once every eight months, once every nine months, once every ten months, once every eleven months, once a year, once every two years, once every three years, once every four years, once every five years, once every six years, once every seven years, once every eight years, once every nine years, or once every ten years, and even more frequently, for over ten years. The initial administration may be once a day, followed by increasing or decreasing frequency (e.g., once a week for subsequent administrations).

Multiple administrations of non-causative factor antigens can maintain the subject's immune response moderately over an extended period, and it is anticipated that a state of high resistance to the subject's disease, disorder, or symptom can be maintained.

In one aspect, the composition, medicament, or kit of the present disclosure for treating or preventing the target disease, disorder, or symptom can be administered to subjects who do not have a history of BCG vaccination or tuberculosis infection and whose antigen responsiveness has not been confirmed. In this case, non-causative factor antigen components or Mycobacterium tuberculosis hot water extracts or parts thereof as described in this specification may be administered to the test subjects prior to administering the compositions, medicaments, or kits of the present invention. Subsequently, if it is confirmed that the subjects have acquired immune responsiveness, the compositions, medicaments, or kits of the present invention may be administered to achieve the treatment or prevention of the disease, disorder, or symptom. While not bound by theory, it is believed that pre-administration of non-causative factor antigen components or Mycobacterium tuberculosis hot water extracts or parts thereof to test subjects can form immune memory against tuberculosis in these test subjects, thereby acquiring immune responsiveness to tuberculosis similar to test subjects with a history of BCG vaccination or tuberculosis infection. Therefore, pre-administration of non-causative factor antigen components or Mycobacterium tuberculosis hot water extracts or parts thereof as described in this specification may be performed once or repeated multiple times until the test subject acquires immune responsiveness. Additionally, pre-administration can occur before or after the test subject is affected by the disease, disorder, or symptom (e.g., infection). Furthermore, the compositions of the present disclosure may be administered under conditions where causative factor antigen components originating from and/or cross-reacting with causative factors of the disease, disorder, or symptom (e.g., infection) are present.

In one embodiment, the dosage of the compositions of the present disclosure may vary depending on the nature of the disease, disorder, or condition, and can be determined by those skilled in the art based on the teachings of this specification and established clinical techniques. In some cases, the use of in vitro assays may assist in identifying the optimal dosing range. The precise dosage to be used in the formulation can also vary depending on the route of administration and the severity of the disease or disorder, and should be determined based on the judgment of the attending physician and the circumstances of each patient. In another embodiment, causative factor antigens and non-causative factor antigens in the present disclosure may be administered at doses ranging from about 0.1 pg/1 dose to about 1 mg/1 dose.

In specific embodiments, pharmaceutical compositions containing the components of the present disclosure can be administered through liposomes, lipid particles, micro particles, or microcapsules. In various aspects of the present invention, the use of such compositions may be useful for achieving sustained release of the components of the present disclosure.

Formulation procedures for medicaments, etc., in the present disclosure are known in the art, as documented in sources such as the Japanese Pharmacopoeia, United States Pharmacopeia, and pharmacopeias of other countries. Therefore, those skilled in the art can determine the embodiments, such as the amounts to be used, without conducting excessive experimentation, based on the teachings of this specification.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure described hereinafter is based on the Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### [Example]

The Examples are described hereinafter. When necessary, animals used in the following Examples were handled in compliance with The University of Tokyo Animal Experiment Practice Regulations and other relevant ethical standards and guidelines, based on the Declaration of Helsinki. While the specific products described in the Examples were used, reagents can be substituted with an equivalent product from another manufacturer (Sigma-Aldrich, Wako Pure Chemical, Nacalai Tesque, R & D Systems, USCN Life Science INC, or the like).

### (Manufacturing Example)

Extract A used in this Example was manufactured in the following manner.

Human *Mycobacterium tuberculosis* strain Aoyama B which had been lyophilized and stored (-20°C) was subjected to seed culture at 37 ± 1°C in a Sauton potato medium⁽¹⁾. The seed-cultured bacteria were transferred to a production medium⁽²⁾ and cultured (primary culture) for 5 to 7 weeks at 37 ± 1°C. The resulting cells were washed with water for injection. To the cells, water for injection was then added in an amount 20-fold of the weight of the wet cells. The mixture was heated at 100°C for 120 minutes to obtain an extract. The extract was filtered with a 0.45 µm-membrane filter and then concentrated under reduced pressure so that the saccharide content (in terms of D-arabinose by the phenol-sulfuric acid method) would be 4.0 to 6.0 mg/ml to obtain a concentrate. Subsequently, in order to remove proteins, 1 w/v% of sulfosalicylic acid was added to the concentrate. The mixture was left standing for 15 to 20 minutes at 10°C or lower. Precipitates were then removed by centrifugation (10°C or lower, 1,150 x G, 10 minutes) to recover the supernatant. The protein concentration of the supernatant is 0.30 mg/ml or lower (Lowry method, in terms of tyrosine). The supernatant was further processed to remove sulfosalicylic acid until the concentration was at or below the detection limit (10 ppm or less, method using ferric chloride solution). The resultant solution was concentrated under reduced pressure so that the saccharide content would be 1.8 to 2.2 mg/ml, and the concentrate was combined with sodium chloride (0.9 w/v%) and cold ethanol at the same volume as the concentrate. The mixture was left standing for 40 hours or longer at 10°C or lower, and then the precipitates (polysaccharide of high molecular weight region) were removed by centrifugation (10°C or lower, 2,040 x G, 10 minutes). Subsequently, the supernatant was combined with four times the amount of cold ethanol, and the mixture was left standing for 40 hours or longer at 10°C or lower and centrifuged (10°C or lower, 2,040 x G, 10 minutes) to recover precipitates. The precipitates were dissolved in water for injection. After the saccharide content was adjusted to 1.8 to 2.2 mg/ml, the solution was filtered with a 0.45 um membrane filter and sterilized with high pressure steam (121°C, 20 minutes) to prepare an Extract A solution.
(1) Sauton-potato medium Washed potato slices were soaked in a Sauton medium, sterilized for 15 minutes at 115°C, and then used as a Sauton-potato medium.
   Sauton Medium
   L-asparagine (monohydrate) 4.0 g
   Citric acid (monohydrate) 2.0 g
   Magnesium sulfate (heptahydrate) 0.5 g
   Potassium monohydrogenphosphate (anhydride) 0.5 g
   Ammonium iron citrate 0.05 g
   Glycerol 60 ml
   The above ingredients were dissolved in water to prepare a 1,000 ml solution. pH was adjusted to 7.0 to 7.3 by using a sodium hydroxide solution.
(2): Production medium
   L-asparagine (monohydrate) 4.0 g
   Citric acid (monohydrate) 2.0 g
   Magnesium sulfate (heptahydrate) 0.5 g
   Potassium monohydrogenphosphate (anhydride) 0.5 g
   Ammonium iron citrate 0.05 g
   Glycerol 60 ml
   The above ingredients were dissolved in water to prepare a 1,000 ml solution and sterilized with high pressure steam (121°C, 20 minutes). pH was adjusted to 7.0 to 7.3 by using a sodium hydroxide solution.

The physicochemical properties of the resulting Extract A solution were as follows.
(1) Appearance:
   Pale yellow clear liquid
(2) pH:
   4.50 to 5.30
(3) Protein content:
   3.5 wt.% (as an amino acid) in a lyophilized product
(4) Nucleic acid content:
   0.1 wt.% in a lyophilized product
(5) Primary constituent monosaccharides of polysaccharide:
   Mannose 43.4 wt.%, arabinose 18.2 wt.%, and glucose 10.4 wt.% (hydrolyzed with 2N trifluoroacetic acid for two hours at 100°C, and then subjected to liquid chromatography using 2-cyanoacetamide fluorescent derivative (S. Honda, et al., Anal. Chem., 52, 1079 (1980)).

The Extract A solution prepared by the method described in the Manufacturing Example described above can be appropriately diluted prior to use. In the following Examples, the Extract Z solution was diluted 1 to 50,000-fold and adjusted to a suitable concentration for use.

### (General Experimental Method)

Unless otherwise specified, the examples disclosed in this document were carried out using the following experimental techniques and reagents, if not explicitly stated.

### Human PBMCs

PBMCs were isolated from healthy volunteer blood donors using gradient separation with Ficoll-Paque Plus (GE Healthcare). Additionally, frozen PBMCs obtained prior to the COVID-19 pandemic, with distinct reactivity against PPD and CMV antigens, were purchased from Cellular Technology Limited (CTL). Freshly isolated PBMCs were cultured in RPMI containing 1% penicillin/streptomycin (P/S), 10% FCS, and 5% autologous plasma. Frozen PBMCs were thawed according to the protocol provided by CTL, briefly involving thawing cells in a 37°C water bath, washing with CTL Wash Medium, and subsequently culturing cells in CTL Test Medium supplemented with 1% P/S, 10% FCS, 5% autologous plasma, and 1% L-glutamine.

### (Human PBMC Labeling and Stimulation)

After cell counting, PBMCs were labeled with 2.5 µM CTV (Thermo Fisher Scientific) at 37°C in a water bath for 20 minutes while gently shaking in RPMI. Following labeling, cells were washed with PBS containing 5% FBS and then cultured in RPMI or CTL Test Medium containing 1% penicillin/streptomycin (P/S), 10% FCS, and 5% autologous plasma in 96-well round-bottom plates. To assess the efficiency of CTV staining, samples from Day 0 were analyzed by flow cytometry (FACS). The cells were first cultured for 3 days with SARS-CoV-2 RBD antigen (GenScript), PPD (Japan BCG Manufacturing), and CMVpp65 antigen (Miltenyi Biotec) in the presence or absence of Extract A (Zeria Pharmaceutical Co., Ltd.). Following the initial 3-day culture, the cell culture medium was replaced with a medium containing IL-2 and autologous plasma, and stimulation for antigen-specific T cell expansion proliferation was conducted for another 3 days. After this expansion proliferation procedure, cells were restimulated for 18 hours with SARS-CoV-2 RBD, PPD, and CMV antigens alone (with the addition of a protein transport inhibitor in the final 4 hours). The T cell response was then analyzed using intracellular staining and FACS.

### (Cytokine Measurement)

On the 3rd and 6th days following re-stimulation, the levels of IL-10 and IFNγ in the culture supernatants were measured using the Human IL-13 and IFNγ DuoSet ELISA Kit (R&D Systems), following the instructions provided in the kit's manual. Cytokine levels in the culture supernatants from PBMCs stimulated on the 6th day were measured using the Bio-Plex Pro Human Cytokine Screening 48-plex Panel (Bio-Rad). This enabled the quantification of cytokines present in the culture supernatants, providing insights into the immune response and cytokine profiles of the stimulated PBMCs.

### (FACS Analysis)

After stimulation, cells were harvested and incubated with Human TruStain FcX Fc Receptor Blocking Solution (BioLegend) for 10 minutes. Following this, cells were stained with the following cell surface molecule staining antibodies along with LIVE/DEAD Fixable Near-IR stain (Thermo Fisher Scientific):
·CD19-APC/Cy-7(clone :HIB19, Biolegend)
·CD14 APC/Cy-7(clone :M5E2, Biolegend)
·CD56 APC/Cy-7(clone :HCD56, Biolegend)
·CD3-AF700(clone :SK7, Biolegend)
·CCR7-BV605(clone :G043H7, Biolegend)
·CD45RA-AF488(clone :HI100, Biolegend)
·CD4-PerCpCy5.5(clone :OKT4, Biolegend)
·CD8-BV785(clone :RPA-T8, Biolegend)antibody in Brilliant Stain Buffer(BD Biosciences)

Following staining and washing, cells were fixed and permeabilized for at least 30 minutes using the eBioscience Foxp3/Transcription Factor Staining Buffer Set (Thermo Fisher Scientific). Subsequently, cells were washed with permeabilization/wash buffer and stained with the following intracellular staining antibodies.
·T-bet-PE Dazzle 594(clone :4B10, Biolegend)
·Foxp3-PE(clone :206D, Biolegend)
·IFNγ-PE/Cy-7(clone :4S.B3, Biolegend)
·IL-10-APC(clone :JES3-19F1, Biolegend)

Following this, cells were washed and analyzed using the LSR Fortessa flow cytometer (BD Biosciences). The gating strategy for FACS in this experimental setup is illustrated in Fig. 2.

### (t-SNE Analysis)

For each experiment, all samples from healthy PBMC donors were analyzed using t-distributed Stochastic Neighbor Embedding (t-SNE) analysis in the FlowJo software. During this analysis, a maximum event count of 20,000 was set for concatenating samples and excluding dead cells and CD14/CD19/CD56-negative cells. The t-SNE analysis was conducted using the default settings within the FlowJo software.
iterations:1000, perplexity: 30, KNN algorithm, Exact(vantage point tree), gradient algorithm:Barnes-Hut.

The heatmap t-SNE plot was generated from concatenated samples.

### (Statistical analysis)

Statistical analysis was performed using GraphPad Prism Software version 8 (GraphPad). Unless otherwise specified, Student's t-test was employed to determine statistical significance (*p<0.05; **p<0.01; ***p<0.001) .

### (Example 1: Verification of Correlation between Mycobacterium tuberculosis Infection and Memory T Cells)

In this particular case, the effectiveness of personalized therapy was validated by analyzing the response of memory T cells through antigen stimulation in patients with a history of BCG vaccination or tuberculosis infection, as well as in healthy individuals who demonstrated antigen responsiveness.

### (method)

To investigate heterologous T cell responses against SARS-CoV-2 mediated by Extract A, human PBMCs were obtained from healthy and non-infected individuals. The PBMCs were labeled with Cell Trace Violet (CTV) and initially cultured with SARS-CoV-2 receptor-binding domain (RBD) antigen (1 µg/ml) in the presence or absence of Extract A (30 µg/ml) for three days. Subsequently, the cell culture medium was replaced with a medium containing IL-2 and autologous serum, and antigen-specific T cells were further stimulated for three days to induce expansion. Six days after this expansion step, cells were restimulated for 18 hours with SARS-CoV-2 RBD antigen alone, during which a protein transport inhibitor was added to the medium for the final four hours. Subsequent intracellular staining was performed, and cell proliferation, intracellular cytokines, and transcription factors were analyzed by flow cytometry (Fig. 1A). To identify unique T cell populations induced by the combination of Extract A and SARS-CoV-2 RBD antigen, dead cells and CD14/CD19/CD56- cells were excluded from the analysis. Samples from all three healthy PBMC donors were concatenated, and t-distributed Stochastic Neighbor Embedding (t-SNE) analysis was conducted (Fig. 1B and 1C).

### (Results and Discussion)

The populations gated by the t-SNE map appeared to exhibit distinct inductions by each stimulus (Fig. 1B). Compared to the control stimulus in representative individuals, the t-SNE plots revealed that Extract A stimulates the expansion of the C5 group (proliferating Tbet⁺ effector memory (EM) CD8 T cells), while leading to a reduction in the proportion of the C4 group (terminally differentiated EMRA CD8 T cells) (Fig. 1B and C). Furthermore, RBD stimulation resulted in an increase in the C1 (IFNγ⁺ Tbet⁺ proliferating EM CD4 T cells) and C2 (proliferating Foxp3⁺ EM CD4 T cells) populations, but led to a decrease in the C3 (proliferating CD3⁻CD4⁺Foxp3⁺ Tbet⁺ IL-2⁺ cells) group (Fig. 1B and C). In contrast, RBD+Extract A stimulation resulted in a notable increase in the C1 group while causing a reduction in the C2 population (Fig. 1B and C). This observation suggests that Extract A has the potential to modulate the Treg/Th1 balance. Similar outcomes were observed in an additional two healthy donors (Fig. 3).

### (Example 2: Validation of SARS-CoV-2-Specific Th1 Immune Response in Non-Infected PBMCs via Regulation of Treg/Th1 Balance by Extract A)

In this example, we investigated the SARS-CoV-2-specific Th1-type immune response mediated by Extract A in human PBMCs derived from non-infected individuals. The purpose of this study was to validate the modulation of the Treg/Th1 balance through Extract A in non-infected human PBMCs.

### (Method)

PBMCs obtained from three healthy individuals were labeled with CTV and then stimulated for three days with Extract A (30µg/ml), SARS-CoV-2 RBD (1µg/ml), or their combination of both. Following this initial stimulation, the cell culture medium was replaced with fresh medium containing IL-2, and the cells were further cultured for an additional three days. On the sixth day, the cells were re-stimulated with SARS-CoV-2 RBD antigen for 18 hours, with the addition of a protein transport inhibitor during the final four hours of stimulation. The levels of IFNγ in the supernatants after antigen re-stimulation on the sixth day were measured using ELISA (Fig. 4A). Subsequently, intracellular staining was performed to analyze cell proliferation, intracellular cytokines, and transcription factors using FACS. After concatenating all samples of the three individuals, t-SNE analysis was conducted on the living CD14/CD19/CD56- cells using FlowJo (Fig. 4B). The proportional representation of the C1 population from t-SNE analysis was plotted as a boxplot using data from all three individuals (Fig. 4C). Tbet expression and IFNγ production were analyzed by FACS in proliferated CD4 and CD8 T cells (CTV^{low/negative} gate) (Fig. 4D) . The proportional representation of the C2 population from t-SNE analysis and the Foxp3 MFI in proliferated CD4 T cells (CTV^{low/negative} gate) were plotted as boxplots using data from all three individuals (Fig. 4E).

### (Results and Discussion)

The cumulative induction of IFNγ production from human PBMCs by both SARS-CoV-2 antigen and Extract A was observed (Fig. 4A). Correlating with the IFNγ data in the supernatant, t-SNE and FACS analyses revealed that IFNγ-producing Tbet⁺ CD4 T cell proliferation (C1 population) was cumulatively induced by the combined stimuli (Fig. 4B and 4C). This suggests that Extract A enhances the SARS-CoV-2-specific Th1-type T cell response. Furthermore, Extract A was found to enhance the proliferation of SARS-CoV-2 antigen-specific IFNγ+ Tbet+ CD8 T cells in non-infected human PBMCs (Fig. 4D and 5).

### (Example 3: Verification of Induction of Expanded Proliferation of Innate Immune Cells with Suppressive Function Capable of Producing IL-10 at an Earlier Time Point by Extract A)

In this example, we performed t-SNE analysis on immune cell populations at an earlier time point, specifically three days after stimulation with SARS-CoV-2 antigen, to examine the induction of immune cell expansion and proliferation in the presence or absence of Extract A.

### (method)

PBMCs derived from three healthy individuals were labeled with CTV and stimulated for three days with Extract A (30 µg/ml), SARS-CoV-2 RBD antigen (1 µg/ml), or their combination. Afterward, the culture medium was replaced with fresh medium containing IL-2, and the cells were further cultured for an additional three days. On the third day, cells were restimulated with SARS-CoV-2 RBD antigen for six hours in the presence of a protein transport inhibitor. Subsequently, intracellular staining was performed, and cell proliferation, intracellular cytokines, and transcription factors were analyzed using FACS. After concatenating all samples from three individuals, t-SNE analysis was conducted on living CD14/CD19/CD56- cells using FlowJo (Fig. 6A). Heatmaps were generated to visualize the expression of surface and intracellular markers, including cytokines and transcription factors, in specific cell populations (Fig. 6B). The relative proportions of the P1 and P2 populations derived from t-SNE analysis were plotted using box plots, utilizing data from all three individuals (Fig. 6C). The levels of IL-10 in the supernatant on the third day were measured using ELISA (Fig. 6D).

### (Results and Discussion)

Due to the relatively short culture period, significant T-cell proliferation through CTV dye dilution was not observed. However, some degree of natural immune cell proliferation in response to Extract A stimulation was detected (P1 and P2) (Fig. 6). Upon further detailed analysis of these populations, Extract A was found to induce the proliferation of CD3⁻ CD4⁺Tbet⁺Foxp3⁺IL-2⁺ and CD3⁻CD4⁺Tbet⁺IL-2⁺ natural immune cell subsets. CD3⁻CD4⁺Tbet⁺Foxp3⁺IL-2⁺ showed a significant increase in IL-10 production mediated by SARSCoV-2 in response to Extract A, indicating a potential suppressive activity (Fig. 6C and 6D). However, the IL-10 levels induced by Extract A did not correlate with the increased presence of the CD3⁻ CD4⁺Tbet⁺Foxp3⁺IL-2⁺ subset, suggesting the existence of additional sources of IL-10, possibly from other natural immune cells (such as DCs or non-proliferating Tregs, since Treg proliferation was not observed on day 3 of CTV dilution) (Fig. 6C and 6D). Furthermore, Bio-Plex analysis of supernatants from PBMC cultures revealed that RBD and Extract A potentially induced IL-12p40 and IL-1α on the third day of re-stimulation, potentially induced IL-2, GM-CSF, and TNFα on the third and sixth days of re-stimulation, and induced IL-17A on the sixth day of re-stimulation. This suggests a potential contribution of Extract A in regulating the Th1/Treg balance and implicates a potential involvement of Th1-driven cytokines (Fig. 8).

Furthermore, Extract A contains adjuvants (e.g., NOD2) capable of activating Mtb-related antigens and natural immune receptors. Therefore, Extract A was compared to other adjuvants (such as K3 CpG and LPS, known for inducing Th1, as well as cGAMP, known for inducing a balanced Th1/Th2 immune response). Intracellular staining for transcription factors seven days after stimulation with SARS-CoV-2 antigen, with or without Extract A or K3 CpG, in human PBMCs derived from healthy donors revealed that Extract A reduced the proportion of Tregs with an effector memory phenotype ((Foxp3⁺CCR7⁻CD45RA⁻ CD4 T cells) and decreased the Foxp3/Treg ratio in EM CD4 T cells. However, similar effects were not observed with K3 CpG. This indicates that the Treg/Th1 regulatory effect of Extract A is not solely due to its adjuvant effects (Fig. 9A). Furthermore, the IFNγ production from human PBMCs induced by RBD was unique to Extract A and not observed with other tested adjuvants (including K3 CpG, LPS, and cGAMP) (Fig. 8). Specifically, the proliferation of IFNγ+TNFα+CD4 and CD8 T cells mediated by RBD was enhanced by the addition of Extract A, but not by other adjuvants (Figs. 9B-F). This suggests that the enhancement of SARS-CoV-2-specific T cell responses by Extract A is not attributed to the induction of trained immunity by adjuvants present in Extract A.

### (Example 4: Verification that pre-existing Mtb-specific T cell memory is necessary for the augmentation of anti-SARS-CoV-2-specific T cell responses through Extract A)

In this study, we investigated whether pre-existing Mycobacterium tuberculosis (Mtb) memory is required for the enhancement of T cell responses to SARS-CoV-2 antigen by Extract A observed in non-infected human PBMCs.

### (Method)

We analyzed the effect of Extract A on SARS-CoV-2-specific T cell responses in PBMCs with distinct reactivity to Mtb antigen PPD or Cytomegalovirus (CMV) antigen (using Mtb non-related antigen as a negative control). Human PBMCs derived from healthy individuals with high/low PPD/CMV reactivity were labeled with CTV and stimulated with CMV pp65, PPD (1 µg/ml), Extract A (30 µg/ml), SARS-CoV-2 RBD (1 µg/ml), or their combinations for three days. The culture medium was then replaced with fresh medium containing IL-2 and further cultured for three days. On the 6th day, cells were restimulated with SARS-CoV-2 RBD antigen for 18 hours, during which a protein transport inhibitor was added to the medium for the final 4 hours. IFNγ levels in the supernatant were measured using ELISA, and intracellular staining was performed to analyze cell proliferation, intracellular cytokines, and transcription factors by FACS. The percentage of IFNγ-producing RBD/CMV-expanded CD4 T cells was plotted against the percentage of IFNγ-producing RBD+Extract A-expanded CD4 T cells, or the Foxp3:Tbet ratio in RBD+Extract A-expanded CD4 T cells (Fig. 11A). PPD/CMV spot counts were plotted against the percentage of IFNγ-producing RBD+Extract A-expanded CD4 T cells or CD8 T cells (Fig. 11B).

### (Results and Discussion)

Not only between the IFNγ+ PPD-induced proliferating CD4 T cells and RBD+Extract A-induced CD4 T cell proliferation, but also between RBD+Extract A-induced CD4 T cell proliferation and PPD spot count or PPD-induced CD4 T cell proliferation, a strong positive correlation was observed (Fig. 11). However, there was a tendency for IFNγ+ PPD-induced proliferating CD4 T cells to have a negative correlation with the Foxp3/Tbet ratio in RBD+Extract A-induced CD4 T cells (p=0.075). These results suggest that pre-existing Mtb memory is required for the enhancement of SARS-CoV-2-specific T cell responses through Extract A-mediated modulation of the Treg/Th1 balance (Fig. 11). On the other hand, a strong correlation was not observed between CMV-induced T cell responses and RBD+Extract A-induced T cell responses. This reinforces that the Extract A-mediated enhancement of SARS-CoV-2-specific T cell responses requires Mtb memory but not CMV memory.

**[Table 1]**

| | Table 1. (A) information on the reactivity of commercial frozen PBMCs to PPD provided by CTL (B) information on the reactivity of commercial frozen PBMCs to CMV provided by CTL | |
|---|---|---|
| **A** | **PPD ELISPOT** | **PBMC ID:** |
| | 1043 | 259 |
| | 1000 | 275 |
| | 600 | 318 |
| | 903 | 172 |
| | 717 | 274 |
| | 27 | 198 |
| | 32 | 454 |
| | 10 | 387 |
| | 24 | 472 |
| | 8 | 393 |
| | 12 | 439 |
| | | |
| **B** | **CMV ELISPOT** | **PBMC ID:** |
| | 827 | 267 |
| | 616 | 334 |
| | 707 | 335 |
| | 722 | 172 |
| | 1 | 438 |
| | 1 | 439 |

Based on the above results, the enhancing effect of Extract A on SARS-CoV-2-specific T cell responses has been demonstrated. This suggests that administration of Extract A could potentially lead to therapeutic benefits against viral infections such as SARS-CoV-2.

### (Example 5) Effects in Other Diseases)

In this embodiment, we conducted in vivo validation tests for treatment involving non-antigen components that are neither derived from nor cross-react with causative factors of the disease, under conditions where causative factor-derived and/or cross-reactive antigen components are administered.

### (Material and method) mouse

All animal experiments were conducted based on the Guidelines for the Care and Use of Animals approved by National Institutes of Biomedical Innovation, Health, and Nutrition (approval number: DS27-25R1) and the Institute of Medical Science the University of Tokyo (IMSUT) (approval number: PM-21-81). Female C57BL/6J mice were purchased from Clea Japan, Inc. (Tokyo, Japan). Mice deficient for CD4, MHC class II, Batf3, IL12p40, CD1d1, NOD2, or γδ T cells were purchased from the Jackson Laboratory (Bar Harbor, Maine, USA). Embryonic stem cells of the STING (Tmem173)-deficient mice (Tmem173^{tm1Camb} (KOMP) Mbp ES cell line (JM8A3.N1)) were purchased from Knockout Mouse Project (KOMP) Repository (California, U.S.A.), and STING deficient mice were generated at the animal facility of National Institutes of Biomedical Innovation, Health and Nutrition. Almost all experiments for assessing the anti-tumor effect were performed with female mice.

### cell line

B16-BL6 melanoma cells were obtained from Tohoku University (Sendai, Japan). B16-F10 cells were purchased from RIKEN BRC (Tsukuba, Japan). These cell lines were maintained in the R10 medium, which comprises RPMI medium 1640 (Nacalai tesque, Kyoto, Japan) supplemented with 10% fetal bovine serum (Nichirei Biosciences Inc. Tokyo, Japan), 100 U/mL of penicillin (Meiji Seika Pharma, Tokyo, Japan), and 100 ug/mL streptomycin (Meiji Seika Pharma).

Freeze-dried glutamate BCG vaccine and purified protein derivative (PPD) was obtained from Japan BCG laboratory (Tokyo, Japan). Freund's incomplete adjuvant (FIA) was purchased from Sigma Aldrich (St. Louis MO, USA). Extract A solution was purchased from Zeria Pharmaceutical Co., Ltd., (Tokyo, Japan). Sodium chloride was added to Extract A solution to obtain an isotonic Extract A solution (stock solution concentration: 2 mg/ml). For in vivo experiments, isotonic Extract A solution was diluted ten-fold by using physiological saline. Anti-CD4 (GK1.5), anti-CD8 (53-6.7), anti-IFN-γ (XMG1.2), and anti-NK1.1 (PK136) antibodies and corresponding isotype control antibodies were purchased from Biolegend (San Diego CA, USA). Recombinant Mtb LpqH and Mtb Hsp10 were obtained from LIONEX GmbH (Braunschweig, Germany). Human Hsp10 protein was obtained from ATGen (Los Angeles CA, USA). Oligodeoxynucleotide K3 (5 -ATC GAC TCT CGA GCG TTC TC-3) was synthesized by GeneDesign, Inc. (Osaka, Japan).

### animal experiment

Freeze dried glutamate BCG vaccine was re-suspended in physiological saline solution (1 mg/mL). A total of 100 µl of BCG solution was subcutaneously injected to into the mice twice (five and two weeks prior to tumor inoculation). In the case of Extract A and IFA immunizations, Extract A was emulsified with the same volume of IFA using the GP syringe connecter (GreenPeptide, Kurume, Japan) and Norm-Ject (HENKE SASS WOLF, Tuttlingen, Germany). A total of 100 µl of Extract A emulsion was intradermally injected to the mice at base of the tail four weeks and two weeks before tumor inoculation. Subsequently, LpqH, Extract A, or saline were injected into the mice every other day starting from eight days prior to tumor inoculation until euthanization. B16-BL6 (3.0×10⁴ cells/mouse) and B16-F10 (3.0×10⁴ cells/mouse) cells were subcutaneously inoculated into the naive mice, BCG-immunized mice, and Extract A emulsion-immunized mice, and the tumor size was measured using a digital caliper (Mitutoyo Corporation, Kanagawa, Japan), and the length (L), width (W), and height (H) were assessed. Tumor volume (V) was calculated as V=L × W × H.

For depletion of CD4+ cells, a total of 200 µg of anti-CD4 antibody was injected intraperitoneally (ip) 1 day before and 3, 7, and 11 days after tumor inoculation. In the case of CD8 depletion, 200 µg of antibody was injected ip 3, 2 and 1 days before tumor inoculation and 2, 5, 8, and 11 days after tumor inoculation. In the case of IFN-γ, 200 µg of antibody was injected ip immediately before tumor inoculation, and 3, 6, 9, and 12 days after tumor inoculation.

### cytokine and c-di-AMP ELISA

Upon harvesting the cells from the spleens of pre-immunized mice, splenocytes were resuspended in R10 medium, and the cell numbers were determined using the Z1 particle counter (Beckman coulter, Inc, CA, USA). Subsequently, the cell numbers were adjusted to 2 × 10 7 cells/mL in the R10 medium.

A total of 100 µl of this cell suspension was dispensed into 96 well flat bottom microplates (Asahi Techno Glass, Chiba, Japan), and the cells were stimulated with 100 µl of the R10 medium containing Extract A. After 48 h, the microplates were centrifuged, and the supernatants of the cell cultures were collected. The concentrations of the cytokines were quantified using enzyme-linked immunosorbent assay (ELISA) kit (R&D systems (Minneapolis, MN, USA)) according to the manufacturer's instructions.

The levels of IL-10 and IFN-γ in hPBMC culture supernatants from day 3 and day 6 cultures (after re-stimulation) were measured using human IL-13 and IFN-γ DuoSet ELISA kits (R&D Systems, USA) according to the instructions of the kit.

The cytokines in the culture supernatant derived from PBMCs stimulated for 6 days were analyzed using Bio-Plex Pro Human Cytokine Screening 48-plex panel (Bio-Rad, California, USA).

The levels of c-di-AMP in the BCG vaccine with or without boiling, Extract A solution or PPD were determined using c-di-AMP ELISA kit from Cayman Chemical Company (Michigan, USA) according to the manufacturer's instructions. The boiling of BCG vaccine solution to extract the intracellular contents of the live attenuated Mycobacterium bovis bacteria was performed as follows: Lyophilized BCG vaccine was resuspended in PBS at the final concentration of 1 mg/ml, and a part of the BCG solution was boiled at 95 °C for 30 minutes. Upon cooling down the boiled BCG vaccine solution, it was used in ELISA to determine c-di-AMP levels.

### Protease treatment of Extract A

EDTA - 0.5% Trypsin solution was obtained from Nacalai tesque Inc. (Kyoto, Japan). and Subtilisin (P5380) was obtained from Sigma-Aldrich Co. LLC (Tokyo, Japan). These proteases were heat inactivated (HI) by incubation at 96 °C for 15 minutes. Intact or HI proteases were mixed with Extract A and incubated for 16 hours. Next, these solutions were incubated at 96 °C for 15 minutes to inactivate intact enzymes.

### in vitro depletion (MACS)

Experiments were performed according to the manufacturer's instructions using AUTOMACS (Miltenyi Biotec, Japan).

### Isolation of tumor infiltrating leucocytes (TIL)

Tumor tissues were harvested from mice on day 14 after tumor inoculation and cut into small pieces with scissors. Small fragments of tumors were further digested using Tumor dissociation kit (Miltenyi Biotec) and gentleMACS dissociator (Miltenyi Biotec). After digestion, the cell suspensions were filtered using a mesh with a size of 70 um. Density gradient centrifugation was performed using Lympholyte-M (Cedarlane) to separate TILs from other cells including tumor cells, the remaining red blood cells and dead cells. After centrifugation, the intermediate layer containing TILs was collected, and the cells were stimulated with Extract A (100 ug/mL) or LpqH (10 ug/mL). Golgi Plug and Golgi Stop were added simultaneously as stimulants. After 10 h of stimulation, cells were collected and washed with PBS. Following staining with Live/Dead fixable blue dead cell stain kit (Thermo Fisher Scientific) and blocking with anti-mouse CD16/32 antibody, cells were fixed and permeabilized with BD Cytofix/Cytoperm (BD) according to the manufacturer's instructions and stained with the following fluorescence-conjugated antibodies: anti-CD3 (17A2), anti-CD4 (GK1.5), anti-CD8 (53-6.7), anti-CD44 (IM7), anti-CD45 (30-F11), anti-T-bet (4B10), anti-Foxp3 (150D), and IFN-γ (XMG1.2). Fluorescence-conjugated antibodies were purchased from BioLegend. The stained cells were analyzed using FACS LSRII (BD), and the data were analyzed using the FlowJo software (FlowJo, USA).

### Flow cytometric analysis of splenocytes

Splenocytes were stimulated with Extract A (20 gg/mL) together with Golgi Stop and Golgi Plug (BD Biosciences, USA) for 6 h. After stimulation, the cells were stained using LIVE/DEAD Fixable Blue Dead Cell Stain Kit (Thermo Fisher Scientific) for 5 min and then stained with anti-CD4 (RM4-5; BD), anti-CD8α (53-6.7; Biolegend) and anti-CD44 (IM7; Biolegend) antibodies. After fixation and permeabilization by BD Cytofix/Cytoperm solution (BD), intracellular cytokines were stained using anti-CD3 (17A2; Biolegend), anti-IFN-γ (XMG1.2; Biolegend), anti-IL-13 (ebio13A; eBioScience, USA), and anti-IL-17 (TC11-18H10.1; Biolegend) antibodies.

### Human PBMC stimulation and intracellular cytokine staining (ICS)

All experiments performed using human PBMCs were approved by the Institutional Review Board of the National Institutes of Biomedical Innovation, Health, and Nutrition (approval number: 146-05) or Institutional Review Board of the Institute of Medical Science the University of Tokyo (IMSUT) (approval number: 2019-25-0919). For fresh PBMC studies, PBMCs were isolated from healthy volunteer blood donors via gradient separation performed with Ficoll-Paque Plus (GE Healthcare). Cryopreserved Human PBMCs were purchased from CTL Europe GmbH. Frozen PBMCs were thawed in 37°C water bath and washed using CTL anti-aggregate wash (CTL Europe GmbH), after which they were resuspended in RPMI1640 medium containing 10% FBS. Cells were cultured in 96-well plates at a density of 1 × 10⁶ cells/well and allowed to stand in a CO₂ incubator overnight. Subsequently, Extract A (100 µg/mL), PPD (3 µg/mL), Cytomegalovirus (CMV) pp65 overlapping peptides (3 µg/mL) or recombinant Mtb proteins (each 10 µg/mL) were added. A total of 1 ug/mL of anti-CD28 (CD28.2), Golgi Plug and Golgi Stop were also added simultaneously as stimulants, and the cells were stimulated for 6 h. After the stimulation, PBMCs were harvested and washed with PBS. Following staining with Live/Dead fixable blue dead cell stain kit (Thermo Fisher Scientific) and blocking with Human TruStain FcX (Biolegend), surface staining was performed with the following fluorescence-conjugated antibodies: anti-CD4 (OKT4), anti-CD8 (RPA-T8), anti-CD45RA (HI100), and anti-CCR7 (G043H). After fixing and permeabilizing the cells with BD Cytofix/Cytoperm solution, the cells were stained with fluorescence-conjugated anti-CD3 (SK7), anti-CD154 (24-31), anti-IL-2 (MQ1-17H12), anti-TNF-α (MAb11), and anti-IFN-γ (4SB3) antibodies. All aforementioned antibodies were purchased from BioLegend. The stained cells were analyzed using FACS LSRII (BD), and the data were analyzed using the FlowJo software.

### CTV Labeling and processing of human PBMCs for long term culture

After cell counting, PBMCs were labeled by incubating with 2.5 µM CTV (Thermo Fischer Scientific, USA) for 20 min in 37°C water bath with gentle shaking in RPMI. After labeling, cells were washed with PBS containing FBS (5%) and cultured in RPMI or CTL Test medium containing 1% penicillin/streptomycin (P/S), 10% FCS and 5% autologous plasma in 96-well round bottom plates. Samples collected on day 0 were analyzed by FACS for evaluating the CTV staining efficiency. Cells were initially stimulated with SARS CoV-2 RBD antigen (GenScript, Japan), PPD (Japan BCG Laboratory, Japan), and CMVpp65 (Miltenyi Biotec, Germany) in the presence or absence of Extract A (Zeria Pharmaceutical, Japan) for three days, upon which the cell culture medium was replaced with the medium containing IL-2 and autologous plasma to stimulate expansion of antigen-specific T cells for additional three days. After this expansion procedure, the cells were re-stimulated with SARS CoV-2 RBD, PPD, and CMV antigen for 18 h (protein transport inhibitor was added for the last 4 h), and T cell responses were analyzed by intracellular staining and FACS.

Upon stimulation, cells were harvested, incubated with Human TruStain FcX Fc Receptor Blocking Solution (Biolegend, USA) for 10 minutes, and stained with LIVE/DEAD Fixable Near-IR stain (Thermo Fischer Scientific, USA) together with cell surface molecule staining antibodies, including CD19-APC/Cy-7 (clone: HIB19, Biolegend), CD14 APC/Cy-7 (clone: M5E2, Biolegend), CD56 APC/Cy-7 (clone: HCD56, Biolegend), CD3-AF700 (clone: SK7, Biolegend), CCR7-BV605 (clone: G043H7, Biolegend), CD45RA-AF488 (clone: HI100, Biolegend), CD4-PerCpCy5.5 (clone: OKT4, Biolegend), CD8-BV785 (clone: RPA-T8, Biolegend) antibodies in Brilliant Stain Buffer (BD Biosciences, USA). After staining and washing, cells were fixed and permeabilized using the fixation and permeabilization reagent of eBioscience Foxp3/Transcription Factor Staining Buffer Set (Thermo Fischer Scientific) for at least 30 minutes. Then, cells were washed with perm/wash buffer and stained with intracellular staining antibodies, including T-bet-PE Dazzle 594 (clone: 4B10, Biolegend), Foxp3-PE (clone: 206D, Biolegend), IFN-γ-PE/Cy-7 (clone: 4S.B3, Biolegend) and IL-10-APC (clone: JES3-19F1, Biolegend) antibodies. Subsequently, they were washed and analyzed by LSR Fortessa (BD Biosciences).

### tSNE Analyses

All samples obtained from healthy PBMC donors in each experiment were concatenated by setting maximum event number to 30000 upon exclusion of dead and CD14/19/56- cells from the analysis, and t-Distributed Stochastic Neighbor Embedding (t-SNE) analysis was performed in FlowJo Software with the following default settings: iterations:1000, perplexity: 30, KNN algorithm, Exact (vantage point tree), gradient algorithm: Barnes-Hut. Heatmap tSNE plots were generated from concatenated samples.

### Statistical Analysis

Data are represented as mean ± standard error (S.E.), and the data was analyzed by using Graphpad Prism (Graphpad Prism, USA). Student's unpaired t-test was used to determine the statistical confidence unless stated otherwise in the figure legends. * p < 0.05; ** p < 0.01; *** p < 0.05.

### Extract A exerts anti-tumor effect only in pre-immunized mice

Extract A is the hot water extract of M. tuberculosis, and it contains various molecules, such as NOD2 agonists, which can stimulate innate immunity (Katsunuma et al., 2015). Therefore, we initially investigated whether innate immunity is responsible for mediating the anti-tumor effect of Extract A. Starting from two days after tumor inoculation, Extract A or K3 CpG, which is a TLR9 agonist capable of inducing anti-tumor type 1 immunity (Klinman, 2004), was intratumorally injected into mice every other day. The results showed that the intratumoral administration of Extract A or saline failed to suppress the tumor growth (Fig. 1A and B), whereas K3 CpG almost completely suppressed B16-BL6 melanoma tumors in WT naive mice (Fig. 1C). In addition, even when Extract A treatment started from 8 days before tumor inoculation, no anti-tumor effect of Extract A was observed in B16-BL6 or B16-F10 tumor-bearing mice (Fig. 1D and G).

One of the main differences between mice and humans, with Extract A demonstrating promising results in clinical trials, is that mice could be raised in a specific pathogen-free (SPF) environment whereas humans are exposed to various pathogens and vaccinations including BCG (Sugiyama et al., 2014). Especially, most Japanese test subjects involved in clinical trials had history of tuberculosis or had been vaccinated with BCG (Yamamoto and Yamamoto, 2007). Therefore, we next examined the anti-tumor effect of Extract A in BCG-immunized animals. The results showed that Extract A exerted anti-tumor effect in explanted tumor models using subcutaneous injection of B16-BL6 melanoma (Fig. 13E), B16-F10 melanoma (Fig. 13H) and Lewis Lung cancer (LLC) cells (Fig. 13J) in BCG-immunized mice. These data indicate that BCG-induced acquired immune responses play an important role in mediating the anti-tumor effect of Extract A. It was also demonstrated by further studies that Extract A exerted strong anti-tumor effect in animal models for B16-BL6 melanoma (Fig. 13F) and B16-F10 melanoma (Fig. 13I) when mice were immunized with Extract A emulsified in IFA. Moreover, we also found that the anti-tumor effect of Extract A varied depending on the type of adjuvant used for priming immunization. When K3 CpG + cGAMP, which induces a strong Th1 response (Temizoz et al., 2015) was used for the priming immunization, Extract A demonstrated an anti-tumor effect (Fig. 13K). In contrast, when the Th2 aluminum adjuvant (Brewer et al., 1999) was used, Extract A demonstrated no anti-tumor effect (Fig. 13L).

CD4 T but not CD8-positive T cells are essential for the anti-tumor effect of Extract A in BCG-immunized tumor-bearing mice

Former results indicated that anti-tumor effect of Extract A requires immunization, which concerns acquired immunity (Fig. 13). In order to confirm this hypothesis, we examined the anti-tumor effect of Extract A in Rag2 deficient mice, which lack CD4 T, CD8-positive T, B, γδ T, and NKT cells due to deficiency in the Rag2 recombination activity required for V(D)J reconstitution (Shinkai et al., 1992, and Tatsumi et al., 1993). Extract A exerted no anti-tumor effect in tumor-bearing BCG-immunized RAG2 deficient mice (Fig. 14A). These results strongly suggests that the anti-tumor effect of Extract A is dependent on acquired immunity. Therefore, we further evaluated the involvement of CD4 and CD8-positive T cells by using specific antibodies for depletion of CD4- and CD8-expressing cells. The results demonstrated that the anti-tumor effect of the Extract A was lost in CD4-positive cell-depleted mice (Fig. 14B); however, the anti-tumor effect was observed in mice depleted of CD8-positive cells (Fig. 14C). Furthermore, the necessity of CD4 T cells for the anti-tumor activity of Extract A was also confirmed by additional experiments performed using CD4-deficient mice (Fig. 14D) and MHC class II-deficient mice (Fig. 14E). In contrast, since the effect of Extract A was also observed in Batf3-deficient mice lacking CD8α+ DC, the findings indicated that CD8+ cells, including CD8-positive T cells, are not essential for the anti-tumor effect of Extract A (Fig. 21B).

The involvement of antibody-dependent cell-mediated cytotoxicity (ADCC) in mechanisms underlying the anti-tumor effect of Extract A was also examined using FcγR deficient mice and NK1.1+ cell-depleted mice. Extract A maintained its anti-tumor effect in both FcγR-deficient and NK1.1+ cell-depleted mice (Fig. S1C and D). Moreover, Extract A also showed anti-tumor effect in CD1d1-deficient mice lacking CD1d-restricted NKT cells (Sköld and Behar, 2003) and mice lacking γδ T cells (Fig. S1E and G). Taken together, these results suggested that the anti-tumor effect of Extract A in BCG-immunized mice depends on CD4 T cells rather than CD8 T, NKT, γδ T cells, or ADCC.

### Th1 responses and STING are necessary for mediating the anti-tumor effect of Extract A

Because CD4 T cells are required for the anti-tumor effect of Extract A in BCG-immunized mice, we next examined the types of these T cell responses. After BCG infection and multiple subcutaneous Extract A injections administered in a similar way as that used in our tumor studies, the spleen cells of the mice were harvested and stimulated with Extract A in order to assess the type of T cell responses induced by Extract A treatment (Fig. S2A). Our results revealed that Extract A robustly induced the secretion of Th1 cytokine IFN-γ from the spleen cells of the immunized mice (Fig. S2B). The induction of IL-13 and IL-10 was also observed with Extract A administration, even in naive mice (Fig. S2C and D). In addition, a lower amount of IL-17 secretion was observed in BCG-immunized mice even without the administration of Extract A (Fig. S2). Furthermore, intracellular staining and FACS analysis of the spleen cells obtained from mice confirmed that the number of IFN-γ producing memory CD4 T cells was remarkably increased with the administration of Extract A in BCG-immunized mice. Nevertheless, no significant IL-17 or IL-13 production was observed upon administration of Extract A with or without BCG immunization (Fig. 2F).

Current results suggested that IFN-γ plays an important role in mediating the anti-tumor effect of Extract A. To confirm this interpretation, we examined the anti-tumor effect of Extract A in mice treated with IFN-γ neutralizing antibodies. The key role of IFN-γ in mediating the anti-tumor effect of Extract A was confirmed by our results showing that the anti-tumor effect of Extract A was abolished in IFN-γ neutralizing antibody-treated mice (Fig. 2G). Furthermore, Extract A-induced IFN-γ secretion from splenocytes of BCG immunized mice was not observed in both CD4-deficient and MHC class II-deficient mice (Fig. 2H). Moreover, IFN-γ secretion from the splenocytes in response to Extract A was diminished after CD4+ cell depletion but not after CD8+ cell depletion (Fig. 2I). These results suggested that Extract A elicited potent Th1 type CD4 T cell responses capable of producing IFN-γ, which is necessary for mediating the anti-tumor effect of Extract A in BCG-immunized mice.

Cytosolic nucleic acid sensing cGAS-STING pathway is one of the pathways playing a key role in mediating the anti-tumor immunity, and it is possible that the hot water extract of Mtb may contain bacteria-derived STING agonists, such as cyclic dinucleotides c-di-AMP, c-di-GMP or 3'3'cGAMP (Temizoz et al., 2016, Dey et al., 2015 and Bai et al., 2012). Therefore, we evaluated the anti-tumor effect of Extract A in STING-deficient mice. The results showed that the anti-tumor effect of Extract A that was clearly observed in heteromice for STING was not observed in STING-deficient mice, in which only host cells are deficient for STING but not the tumor cells (Fig. 14J). These data suggests that STING ligands derived either from tumor cells or found in Extract A could be responsible for mediating the anti-tumor effect of Extract A. To analyze the source of STING ligands, we next measured the concentrations of cyclic di-nucleotide c-di-AMP in the Extract A solution and discovered that c-di-AMP, which can be produced by Mtb (Bai et al., 2012), was observed in the Extract A solution, and its levels increased upon boiling BCG vaccine suggesting that Mycobacterium bovis of BCG vaccine also produces c-di-AMP that can be released by killing the bacteria by boiling (Fig. 14K). Taken together, it is revealed that Extract A induces anti-tumor immunity via IFN-γ-secreting CD4 T cells and STING pathway activation induced by Mtb-derived STING in BCG-immunized tumor-bearing mice.

### Injection of tumor-unrelated protein contained in Extract A suppresses tumor growth in BCG-immunized mice

To assess whether protein antigens contained in Extract A are responsible for the induction of IFN-γ from Th1 type CD4 T cells, we treated Extract A with proteases. Protease treatments, in contrast to heat inactivated (HI) enzymes, tended to reduce the activity of Extract A responsible for inducing the expression of IFN-γ (Fig. 3A). In addition, mass spectrometry analysis revealed at least twenty proteins that were present in the Extract A solution (Table.1). [Table 1 A]

By screening these candidate proteins in the splenocytes of Extract A emulsion-injected mice, we found that the protein number-1 (LpqH) and protein number-18 managed to induce IFN-γ from the splenocytes of mice injected with Extract A emulsion (Fig. 3B). Then, we synthesized the peptide library of these proteins and screened the splenocytes of mice injected with Extract A emulsion for the activity responsible for the induction of IFN-γ. The results showed that two peptides from the LpqH peptide pool induced the expression of IFN-γ (Fig. 3C). In addition, stimulation of splenocytes derived from BCG-immunized mice with LpqH induced the secretion of IFN-γ (Fig. 3D). Besides, the injection of recombinant LpqH protein demonstrated anti-tumor activity in Extract A emulsion-immunized and BCG-immunized mice but not in naive mice (Fig. 3, E-G) similar to the anti-tumor effect of Extract A. These results suggested that administration of Extract A-derived antigen to BCG-immunized mice can suppress tumor growth and that one of the active ingredients of Extract A is a protein antigen.

### A challenge with tumor-unrelated antigen can suppress the tumor growth via specific Th1 cells

In order to elucidate the relationship between tumor-unrelated antigen-specific T cell responses and anti-tumor effect, immunizations were performed with a model antigen ovalbumin (OVA). Consequently, mice that were immunized with OVA plus Extract A emulsified in IFA (water-in-oil emulsion adjuvant) adjuvant resulted in the suppression of B16-BL6 tumor growth (Fig. 3H and I). In addition, administration of Flu antigen also mediated tumor suppression in Flu (PR8 strain)-infected mice but not in naive mice, similar to that observed with Extract A, whose anti-tumor effect required BCG immunization (Fig. 3J and K).

### Extract A increases the number of Th1 cells in the tumor microenvironment

We next analyzed the tumor microenvironment to elucidate how Extract A exerts its anti-tumor effect. The results showed that the administration of Extract A to BCG-immunized mice, but not naive mice, significantly increased the number of CD3-positive CD45-positive cells in the tumor microenvironment (Fig. 16A). Further investigations revealed that these TILs mostly comprised CD4 T cells rather than CD8 T cells in the BCG-immunized mice and Extract A-treated mice (Fig. 16B). In addition, analysis of the CD4-positive T cell phenotypes performed by examining the expression of the transcription factors T-bet (Th1) and Foxp3 (Treg) revealed that the number of T-bet-positive Th1 type CD4 T cells was significantly increased (Fig. a6C and D). A significant Extract A-mediated increase in the number of T-bet-negative Foxp3-positive cells was also observed, albeit the difference was lower compared to that observed with T-bet-positive cells (Fig. 16C). Moreover, in addition to increased proportion of T-bet-positive Foxp3-negative and T-bet-positive Foxp3-positive CD4 T cells in the tumor microenvironment of BCG-immunized mice and Extract A-treated mice, a significant negative correlation was observed between the tumor weight and the number of T-bet-positive CD4 T cells that infiltrated the tumor (Fig. 16E). In contrast, no correlation was observed between the tumor weight and Tbet-negative Foxp3-positive CD4 T cell number in the TILs (Fig. 4E). These changes were observed only in the tumor microenvironment but not in the draining lymph nodes or spleen (Fig. 23). Therefore, Extract A-mediated increase in the number of T-bet-positive T cells in the tumor microenvironment is a tumor-specific phenomenon rather than a systemic change.

Subsequently, to investigate whether IFN-γ was produced from TILs, we measured the intracellular levels of IFN-γ from TILs with or without Extract A stimulation (Fig. 4F and G). IFN-γ-producing memory CD4 T cells were detected both in the presence or absence of stimulation, suggesting that Extract A-derived antigen independent T cells were activated (Fig. 4G). Under these experimental conditions, since tumor-derived cells and debris were probably present in the culture medium, it is possible that the T cell response was detected against tumor antigens. Moreover, IFN-γ producing CD4 T cells in the TILs responded to Extract A and LpqH in the tumor microenvironment of the BCG-immunized and Extract A-treated mice (Fig. 4G). Indeed, IFN-γ was produced primarily by T-bet+Foxp3- CD44+ memory T cells and some T-bet+Foxp3+ CD44+ memory T cells in the tumor microenvironment (Fig. 4H). Nevertheless, the numbers of IFN-γ+ CD4 T cells were negatively correlated with tumor weight after stimulation with Extract A or Lpqh in BCG-immunized Extract A-treated mice, suggesting that Extract A exerts an anti-tumor effect via recruitment of Extract A-reactive Th1 cells to the tumor microenvironment, thereby culminating in IFN-γ-mediated tumor regression (Fig. 16 I).

### Extract A-induced IFN-γ and IL-2 secretion from human PBMCs correlated with Mtb antigen reactivity

To elucidate whether Extract A can stimulate similar Th1 type T cell responses in humans, we assessed Extract A-induced IFN-γ secretion from human PBMCs. Human PBMCs were stimulated with Extract A, PPD, CMV peptide, or some recombinant proteins present in Extract A (MHSP10, HSP60.1, LpqH, Rv2376), and then cytokine secretion was analyzed by intracellular staining and FACS. The results demonstrated that Extract A could induce the secretion of IFN-γ, IL-2 and TNF-α from CD154+ activated CD4+CD45RA-CCR7+ cells (central memory T cell; TCM) in human PBMCs derived from some donors. IFN-γ secretion was also induced upon stimulation with CMV pp65 peptides such as PPD and MHSP10 (used as Mtb-unrelated negative control) or Mtb antigens, in some donors. More importantly, a strong positive correlation was observed between responses of human PBMCs to Extract A and those to Mtb antigens PPD and MHSP10 (also detected in Extract A), while no correlation was observed between the response of hPBMCs to Extract A and that to CMV pp65 overlapping peptides (Fig. 17). In addition, a significant correlation was also observed between IL-2 induced by Extract A and IL-2 induced not only by PPD but also by the proteins present in Extract A (MHSP10, HSP60.1, LpqH, Rv2376) (Table 2). Therefore, these results indicate that Extract A-reactive central memory T cells that are found in the PBMCs derived from individuals with a history of tuberculosis infection or BCG vaccination could contribute to the anti-tumor effect of the Extract A in humans.

**[Table 2]**

| **Table 2. correlation between Extract A and antigen vs. Extract A** | | | | | | |
|---|---|---|---|---|---|---|
| | IL-2 | | IFN-*γ* | | TNF-*α* | |
| | r | *p* | r | *p* | r | *p* |
| PPD | 0.849 | <0.0001 | 0.854 | <0.0001 | 0.839 | <0.0001 |
| CMV | 0.224 | 0.3433 | 0.0977 | 0.6818 | 0.184 | 0.4362 |
| MHSP10 | 0.519 | 0.019 | 0.707 | 0.0005 | 0.495 | 0.0266 |
| LpqH | 0.45 | 0.0467 | -0.0582 | 0.8074 | 0.44 | 0.052 |
| HSP60.1 | 0.437 | 0.0537 | 0.268 | 0.2528 | 0.134 | 0.572 |
| Rv2376 | 0.6 | 0.0052 | 0.104 | 0.6613 | 0.492 | 0.0277 |

### (Example 6: Example of Demonstration in Viral Infections)

This example demonstrates the effectiveness of the disclosed techniques in viral infections. Unless otherwise specified, the experimental methods follow those described in Example 5.

(The combination of Extract A and SARS-CoV-2 antigen induces expansion and proliferation of specific T cell populations in SARS-CoV-2 naive PBMCs.)

Next, we investigated whether cross-protective T cell responses induced by Extract A can mediate protective effects against COVID-19. Previous study suggests that common coronavirus-specific T cells in SARS CoV-2-naive or unimmunized/unexposed individuals may provide cross-protection against SARS-CoV-2 (Mateus et al., 2020). In contrast, BCG vaccination is capable of inducing cross-protective immune responses against several non-Mtb pathogens via mechanisms involving induction of trained immunity and heterologous T cell responses. Therefore, in order to analyze Extract A (containing Mycobacterium tuberculosis antigens)-mediated enhancement of heterologous T cell responses against SARS CoV-2, CTV-labeled human PBMCs derived from healthy and SARS CoV-2-naive individuals were cultured initially with SARS CoV-2 receptor binding domain (RBD) antigen in the presence or absence of Extract A for three days. Subsequently, the cell culture medium was replaced with a medium containing IL-2 to stimulate the expansion proliferation of antigen-specific T cells for three days. After this expansion proliferation procedure, the cells were re-stimulated with SARS CoV-2 RBD antigen-only, and the T cell responses were analyzed by intracellular staining and FACS (Fig. 18A). In order to identify unique T cell subsets activated by the combination of Extract A and SARS CoV-2 RBD antigen, all samples derived from three healthy PBMC donors were concatenated upon exclusion of dead and CD14/19/56- cells from the analysis, and t-Distributed Stochastic Neighbor Embedding (t-SNE) analysis was performed. Populations gated on the tSNE maps seemed to be differentially induced upon each stimulation (Fig. 18B). tSNE plots compared to control stimulation in a representative individual revealed that while Extract A stimulated expansion proliferation of C5 population (proliferated Tbet+ effector memory (EM) CD8 T cells), it induced a decline in the frequency of C4 population (highly differentiated EM (TEMRA) CD8 T cells) (Fig. 18 B and C). Furthermore, RBD stimulation mediated an increase in C1 (IFN-g+ Tbet+ proliferated EM CD4 T cells) and C2 (proliferated Foxp3+ EM CD4 T cells) cell populations but reduced C3 (proliferated CD3-CD4+Foxp3+Tbet+IL-2+ cells) populations (Fig. 18 B and C). In contrast, stimulation with RBD+Extract A mediated a remarkable increase in the C1 population while decreasing C2 population (Fig. 18 B and C), raising the hypothesis that Extract A could be modulating the Treg/Th1 balance. Similar findings were also observed in two additional healthy donors (Fig. 24).

### (Example 7: Boosting effect in viral infections)

In this embodiment, the disclosed compositions and the like are demonstrated to have a boosting effect in viral infections. The experimental methods follow those described in Example 5, unless otherwise specified.

(Extract A boosts the anti-SARS-CoV-2 specific T-cell response in SARS-CoV-2 naive PBMCs by regulating the Treg/Th1 balance.)

### Extract A boosts anti-SARS-CoV-2-specific T cell responses in SARS CoV-2-naive PBMCs via modulation of Treg/Th1 balance

Based on our recent findings demonstrating that IFN-γ-producing CD4 T cells mediate the anti-tumor effect of Extract A in murine tumor models (Oka et al., 1999, Oka et al., 2003, Oka et al., 2004), we investigated the Extract A-mediated induction of SARS CoV-2-specific Th1 type immune responses in human PBMCs derived from SARS CoV-2-naive individuals. We found that the combination of SARS CoV-2 antigen and Extract A significantly enhanced the production of IFN-γ compared to that observed with RBD- or Extract A-only in human PBMCs (Fig. 7A). In correlation with the supernatant IFN-γ data, tSNE and FACS analyses also revealed that the proliferation of IFN-γ-producing Tbet+ CD4 T cells (C1 population) was synergistically induced by stimulation with the combination, suggesting that Extract A enhances SARS CoV-2-specific Th1 type T cell responses (Fig. 7B and C). Moreover, Extract A also enhanced SARS CoV-2-specific IFN-γ+ Tbet+ CD8 T cell proliferation in SARS CoV-2-naive human PBMCs (Fig. 19D and 25).

Despite the reported beneficial effect of Tregs for viral infections including SARS CoV-2, Treg-mediated suppression of anti-microbial T cell responses can interfere with development of potent anti-viral T cell responses (Meckiff et al., 2020, Bhela et al., 2017, Anghelina et al., 2009, Peng et al., 2008). Therefore, we also analyzed Extract A-mediated modulation of Tregs and found that Extract A suppressed SARS CoV-2 RBD-induced Treg activation, indicating that Extract A may induce potent anti-SARS CoV-2 T cell responses that can potentially control virus replication via modulation of Treg/Th1 balance (Fig. 7E). Moreover, Foxp3+ median fluorescence intensity (MFI) in the total CD4 T cells was enhanced upon stimulation with SARS CoV-2 antigen, which was significantly decreased to comparable levels as those observed with stimulation with Extract A, demonstrating that regulatory T cells activated by SARS CoV-2 antigen can be suppressed by Extract A to potentiate SARS CoV-2 specific T cell responses (Fig. 24C).

To further analyze anti-SARS CoV-2 specific T cell responses mediated by Extract A, we examined immune cell populations at earlier time points on day three after stimulation with SARS CoV-2 antigen with or without Extract A by tSNE analysis. Although significant T cell proliferation was not observed with CTV dye dilution owing to the short duration of culture, some proliferating innate immune cell populations were observed upon stimulation with Extract A (P1 and P2) (Fig. 26). More detailed analysis of those populations revealed that Extract A stimulated proliferation of CD3-CD4+Tbet+Foxp3+IL-2+ and CD3-CD4+Tbet+IL-2+ innate immune subsets. The former subset may possess a suppressive activity as we observed high levels of IL-10 production upon stimulation with the combination of RBD and Extract A (Fig. 26B-E). However, since the induction of IL-10 expression mediated by Extract A does not correlate with the increased frequency of CD3-CD4+Tbet+Foxp3+IL-2+ subset, it is possible that additional sources of IL-10 were present, including other innate immune cells, such as DCs or even acquired immune cells, such as unproliferated Tregs (as no Treg proliferation was observed on day three upon CTV dilution) (Fig. 26D and E). Furthermore, Bio-Plex analysis of the supernatants obtained from PBMC cultures revealed that RBD and Extract A potently induced the production of IL-12p40 and IL-1α on day three and that of IL-2, GM-CSF and TNFα on days three and six after re-stimulation in addition to induction of IL-17A production on day six, suggesting the potential contribution of these Th1-driving cytokines to Extract A-mediated modulation of the Th1/Treg balance (Fig. 27).

Adjuvants have the ability to disrupt immune-tolerance and stimulate certain T cell responses (e.g. Th1/2/17) depending on the downstream pathways activated by that specific adjuvant (Temizoz et al., 2018). As Extract A contains both Mtb-related antigens and adjuvants capable of activating innate immune receptors, such as NOD2, we compared the effects of Extract A with those of other adjuvants, including Th1-inducing adjuvants K3 CpG and LPS, in addition to cGAMP, which can induce a balanced Th1/2 type immune response (Temizoz et al., 2016). Intracellular staining of human PBMCs derived from healthy donors for analysis of transcription factors seven days after stimulation with SARS CoV-2 antigen with or without Extract A or K3 demonstrated that Extract A, but not K3 CpG, decreased the ratio of Tregs with the EM phenotype (Foxp3+ CCR7-CD45RA- CD4 T cells) in addition to mediating a reduction in the ratio of Foxp3/Treg in EM CD4 T cells, suggesting that the modulating effect of Extract A on Treg/Th1 can't simply be attributed to its adjuvant effect (Fig. 28). Furthermore, RBD-induced IFN-γ production from human PBMCs was enhanced by Extract A-only unlike other tested adjuvants, including K3 CpG, LPS and cGAMP (Fig. 29). Specifically, RBD-mediated proliferation of IFN-γ+TNFα+ CD4 and CD8 T cells was enhanced by the addition of Extract A but not with the addition of other adjuvants. These results support that Extract A-mediated enhancement of SARS CoV-2-specific T cell responses does not merely depend on the induction of trained immunity mediated by adjuvants included in Extract A (Fig. 29B-F).

### (Example 8: Role of Immune Memory in Viral Infections)

This embodiment demonstrates the significant role of immune memory in substantiating the efficacy of the disclosed technique. Experimental methods, unless specified otherwise, adhere to those outlined in Example 5.

(Pre-existing Mtb-specific T cell memory is crucial for facilitating the enhancement of the anti-SARS-CoV-2 specific T cell response mediated by Extract A.)

Considering the results about Extract A indicating that the anti-tumor effect of Extract A requires pre-existing Mtb memory and data showing that anti-SARS CoV-2 specific T cell response enhancing effect of Extract A is not simply due to its adjuvant effect, we next examined the requirement of pre-existing Mtb memory for the T cell response-enhancing effect of Extract A observed in SATS CoV-2-naive human PBMCs against SARS CoV-2 antigens. We analyzed the effect of Extract A on SARS CoV-2 specific T cell responses in PBMCs with different reactivity to Mtb antigen PPD or CMV antigen (an Mtb unrelated antigen used as negative control). Consequently, we found a strong positive correlation not only between the expression of IFN-γ induced by PPD and RBD+Extract A on day three and day but also between Tbet/Foxp3 MFI ratio in RBD+Extract A-proliferated EM CD4 T cells and PPD spot count (Fig. 20). Taken together, these data suggest that pre-existing Mtb memory is required for Extract A-mediated enhancement of SARS CoV-2 specific T cell responses via modulation of Th1/Treg ratio (Fig. 20). In contrast, no strong correlation was observed between CMV-induced T cell responses and T cell responses induced by RBD+Extract A, confirming that anti-SARS CoV-2 specific T cell response enhancement of Extract A specifically requires Mtb memory (Fig. 20). Please refer to Table 3 and Fig. 30 for information on the reactivity of commercial frozen PBMCs to PPD and CMV provided by CTL.

**[Table 3]**

| Table 3. information on the reactivity of commercial frozen PBMCs to PPD and CMV provided by CTL | | |
|---|---|---|
| PBMC ID | PPD IFNγ⁺ ELISPOT count | CMV class I/II peptide IFNγ⁺ ELISPOT count |
| 314 | 32 | unavailable |
| 388 | 47 | 177 |
| 393 | 8 | 3 |
| 427 | 39 | 140 |
| 444 | 41 | 439 |
| 454 | 32 | 3 |
| 472 | 24 | 3 |
| 479 | 17 | 206 |
| 273 | 501 | 2 |
| 274 | 717 | 1 |
| 315 | 369 | 43 |
| 318 | 600 | 5 |
| 320 | 422 | 216 |
| 351 | 329 | 2 |
| 438 | 331 | 1 |
| 469 | 406 | 124 |
| 366 | 67 | 4 |
| 406 | 125 | 6 |
| 453 | 177 | 2 |
| 268 | 178 | 531 |
| 343 | 62 | 748 |
| 365 | 297 | 600 |
| 428 | 103 | 402 |
| 434 | 104 | 430 |
| 481 | 132 | 263 |

While not bound by theory, the following interpretations can be drawn from the aforementioned examples.

Our in vivo studies in the present disclosure performed using depletion antibodies and several KO mice, such as RAG2 deficient mice lacking acquired immune cells, including T cells, and suggested that the anti-tumor effect of a non-causative factor antigen component (e.g., the Extract A) was primarily dependent on the induction of heterologous CD4 T cell responses rather than trained immunity, which scarcely depends on innate immune cells (Fig. 14 and 21). Lardone et al. and Kalafati et al. considered BCG and β-glucan as trained immunity agonists, and they showed that β-glucan induced anti-tumor immune responses via epigenetic modulation of granulopoiesis and neutrophil function in mice while BCG trained tumor-infiltrating M2 macrophages to enhance IFN-γ production from CD4 T cells in patients with melanoma (Lardone et al., 2017, Kalafati et al., 2020). Since it was not in the present disclosure observed that any anti-tumor effect of Extract A in RAG2-deficient mice with neutrophils and macrophages, we do not believe that such trained immunity-related mechanisms play important role in mediating the anti-tumor effect of Extract A in the experimental model of the present disclosure. Furthermore, although Extract A was administered subcutaneously in the vicinity of the tumor in this study, it is possible that a stronger anti-tumor effect may be observed with intratumoral administration as various studies demonstrated anti-tumor effect of the intratumoral injection of BCG against solid tumors, such as bladder cancer (Biot et al., 2012). In the study performed by Biot et al., nonetheless, discovery of the protein antigens as active ingredients of Extract A is crucial because it allows us to use the reactivity against these protein antigens as a predictive marker for responsiveness to Extract A immunotherapy in patients with various diseases. Although our finding that Mtb-derived antigen can exert effects to diseases is in contrast with the above-mentioned findings, it is possible that the combination of causative factor antigen and Th1-stimulating causative factor tumor-unrelated antigen can exert effects to diseases more effectively compared to conventional antigen-based immunotherapies.

It is well-known that live attenuated vaccines and infections, such as smallpox, elicit a robust immune response lasting for many years, or even offer a life-long immunity via the activation of long-lasting memory B or T cells (Hammarlund et al., 2003). It is now clear that Mtb-reactive T cells persist even in adulthood due to childhood BCG vaccination or tuberculosis infection (Fig. 17). Furthermore, we demonstrated that anti-tumor immunity can be induced by utilizing the immune response generated by inoculation with a live attenuated vaccine. It is an interesting question whether immune memory acquired by other infectious diseases can be applied for treatment application. In this study, regression of tumors was observed after administration of Flu vaccine after influenza virus infection. Hence, we expect that such heterologous immune memory responses may be extrapolated for many other infectious diseases, including COVID-19. Here, we conducted primarily experiments with BCG-immunized animals; however, it is also possible to use other live vaccines, such as yellow fever and vaccinia.

Although data obtained from CD4 and MHC class II-deficient mice clearly showed that CD4 T cells are necessary for the anti-tumor effect of Extract A, depletion of CD4+ cells suppressed tumor growth efficiently even without treatment with Extract A (Figs. 2 B, D and E). The slower tumor growth (compared to other WT mice) in CD4⁺ cell-depleted mice may be attributed to the anti-CD4 antibody-mediated depletion of suppressive Tregs, which are also CD4+.

As shown in the present disclosure, expression of T-bet in mice is induced when Foxp3⁺ cells are exposed to IFN-γ(see Lighvani et al., 2001). Nevertheless, Overacre-Delgoffe et al. reported that a population of Foxp3⁺ Tregs, named fragile Tregs, in the tumor microenvironment are capable of producing IFN-γ, which drives the development of more IFN-γ-producing fragile Tregs, thereby eliciting anti-tumor immunity (Overacre-Delgoffe et al., 2017). According to the present disclosure, the immuno-suppressive activity of Tbet⁺Foxp3⁺ cells seems to be lower than that of Tbet⁻Foxp3⁺ cells. In the present disclosure, in one embodiment, the population of total Foxp3⁺ cells increased in the group treated with Extract A. Specifically, although the population of Tbet⁻Foxp3⁺ cells, with potentially high immuno-suppressive activity, increased slightly in the group treated with Extract A, a significant increase was observed in the number of Tbet⁺ Foxp3⁺ cells. This explains the anti-tumor effect of Extract A do not depend on the increase in the number of Treg cells. In contrast, Levine et al. defined T-bet⁺Foxp3⁺ T cells as stable Tregs that specifically suppress Th1 but not Th2/17 responses (Levine et al., 2017). On the contrary, a Foxp3⁺ T-bet⁺ IFN-g⁺ Treg population with reduced suppressive activity, therefore exhibiting a fragile phenotype, was identified in patients with autoimmune diseases, such as multiple sclerosis (MS) and type I diabetes (McClymont et al., 2011, Dominguez-Villar et al., 2011). Thus, when fragile Tregs are activated systemically, there is a risk of the development of autoimmunity. Therefore, when applied to cancer immunotherapy, it is desirable to specifically activate fragile Tregs within the tumor microenvironment. Since Extract A dose not induce significant changes with respect to systemic percentages of Tbet⁺ Foxp3⁺ T cells, Extract A is considered safe in terms of systemic risk for induction of fragile Treg-dependent autoimmunity (Fig. 23).

In the present disclosure, although the effects to disease could be successfully induced upon administration of Extract A-derived protein to BCG-immunized mouse, the dose used was at least several hundred times more than the dose present in Extract A. A potent anti-tumor effect mediated by Extract A despite the low concentrations of protein antigens can be attributed to the fact that other components of Extract A, including several other Mtb-related antigens, metabolites, such as the STING agonist c-di-AMP, or some innate immune stimulators, such as NOD2 agonists could be synergizing with each other to boost Extract A specific-Th1 responses that can efficiently eradicate tumors. Indeed, the experimental data in the present specification obtained using NOD2-deficient mice show that Extract A has a significant anti-tumor effect, suggesting that NOD2 agonists present in Extract A or BCG are required for mediating the anti-tumor effect of Extract A (Fig. 21H). However, it is important that tumor growth in NOD2-deficient mice is slow compared to that in WT or other KO mice, suggesting that NOD2 itself supports tumor growth. In agreement with this hypothesis, Biswas et al. demonstrated that NOD2 can suppress Th1-driven inflammation in the ileum (Biswas et al., 2010). Since Th1 responses are important for tumor eradication, it is possible that elevated Th1 responses in the NOD2-deficient mice halt tumor development. In contrast, the experimental data in the present specification clearly show that Extract A contains Mtb-derived STING agonist c-di-AMP, and it is important for mediating the anti-tumor effect of Extract A in BCG-immunized mice (Fig. 14J and K). Although c-di-AMP is produced by Mtb, there are no reports showing that Extract A, the hot water extract of Mtb, contains c-di-AMP (Bai et al., 2012). In addition, the experimental data in the present specification shows that boiling the BCG enables release of the intracellular c-di-AMP content of live-attenuated M. bovis bacteria, and that the hot water extract of Mtb is expected to contain c-di-AMP that is released by heat-killing of the bacteria for the extraction of water-soluble contents (Fig. 2K). Despite the low concentrations of c-di-AMP (approximately 0.4 ng/injection) found in the Extract A solution injected at the concentration used in in vivo experiments in the present specification, it is likely that repeated injections of low amounts of STING agonists can synergize with low amounts of other pathogen-associated molecular patterns (PAMPs) or damage-associated molecular patterns (DAMPs), such as microbial or tumor-derived nucleic acids to induce robust anti-tumor immune responses.

Current PBMC culturing protocol for examining the effect of Extract A involved in enhancing anti-SARS CoV-2 T cell response observed in this study involves three days of antigen stimulation for expanding the population of SARS CoV-2 cross-reactive T cells, which may include common coronavirus-specific T cells. The number of these cells is low in healthy individuals without any stimulation (Grifoni et al., 2020, Weiskopf et al., 2020, Le Bert et al., 2020, Mateus et al., 2020). Nevertheless, we added IL-2 to the PBMC culture after three days of antigen stimulation to keep antigen-specific proliferating T cells alive via co-stimulation without inducing high background proliferation, which is in agreement with the data reported by Kennell et al. who clearly showed that addition of IL-2 at later intervals reduced background proliferation while increasing the sensitivity of the proliferation assay (Kennell et al., 2014).

In one embodiment, based on the data showing that Extract A reduced the number of SARS CoV-2-induced EM Tregs while increasing the proliferation of IFN-γ producing Tbet+ CD4 T cells, we hypothesize that Mtb/common coronavirus-specific Tregs that are cross-reactive to SARS CoV-2 may convert to Th1 type T cells capable of producing IFN-γ in response to SARS CoV-2. Although we have no direct evidence for this hypothesis, IL-12 and IL-6-mediated conversion of CD25^{low} Tregs, which are termed Foxp3-unstable, into Th1 cells compared to CD25^{high} Tregs that are resistant to Th1 conversion has been reported in mouse models of ocular herpes simplex virus-1 (HSV-1) infection (Bhela et al., 2017). Nonetheless, these Foxp3-unstable Tregs are pathogenic for chronic HSV-1 infection. Considering these data and the beneficial effects of Tregs in SARS CoV-2 infection (Meckiff et al., 2020, Bhela et al., 2017), it can be presumed that Extract A may exacerbate SARS CoV-2 pathology. However, since Extract A reduces the frequency of proliferated, potentially antigen-specific Tregs rather than completely depleting Tregs, and treatment of mice with Extract A did not decrease systemic number of Tregs (Fig. S3 and S4C), we believe that such toxicity is unlikely. In contrast, Su et al. demonstrated that a reduction in lymphocytic choriomeningitis virus (LCMV)-specific Tregs was correlated with viral clearance in mice in addition to the negative correlation between percentages of Treg and antigen specific memory T cells in hPBMCs (Su et al., 2016). Hence, we believe that the suppression of SARS CoV-2-mediated activation of Tregs could be used to support induction of potent anti-SARS CoV-2 T cell responses capable of aiding in efficient viral clearance. In addition, our hypothesis is supported by epidemiological observations and clinical trials showing that BCG vaccination is safe and could mediate protective effects against respiratory tract infections with non-Mtb pathogens and might improve COVID19 outcomes by decreasing the severity of symptoms along with the disease incidence (Hensel et al., 2020, Moorlag et al., 2020, Giamarellos-Bourboulis et al., 2020). Another hypothesis in addition or as an alternative to the Treg-to-Th1 conversion hypothesis explained above is that Mtb/common coronavirus-specific memory T cells that are cross-reactive to SARS CoV-2 are mediated by SARS CoV-2 antigen to a higher extent than Tregs due to Th1-inducing adjuvants, such as MDP, within Extract A (Girvan et al., 2011, Caruso et al., 2014, Prescott et al., 2020).

In one example of the present disclosure, the stimulation of the hPBMCs with Extract A for three days induced proliferation of CD3-CD4+Tbet+Foxp3+IL-2+ (P1) and CD3-CD4+Tbet+IL-2+ (P2) innate immune subsets. The inventors speculated that the P1 cells could demonstrate suppressive potential due to both Foxp3 expression and elevated levels of IL-10 by combination of SARS CoV-2 antigen and Extract A stimulation in supernatants of human PBMCs (Fig. 26). In spite of the significant increase in the proliferation of P1 population mediated by Extract A on day three, no significant IL-10 production was observed by ELISA or FACS upon stimulation with Extract A only, suggesting the presence of additional Extract A-activated IL-10 sources that are yet to be identified and potentially include other immune cells, such as DCs, ILCs, or even acquired immune cells such as common coronavirus/Mtb-specific Tregs. Indeed, immune-regulatory cells, such as MDSCs, play dual roles in the regulation of viral infections and development of subsequent collateral tissue damage (Dorhoi et al., 2019, Amodio et al., 2019). For instance, MDSCs were reported to halt anti-viral immunity against dengue fever, LP-BM5 retrovirus (in mouse AIDS model), and SARS CoV-2 via suppression of efficient anti-viral T cell response (Green et al., 2013, Guo et al., 2019, Sacchi et al., 2020, Reizine et al., 2021). In the case of SARS CoV-2, elevated levels of MDSCs correlated with disease severity because of enhanced arginase activity and subsequent impaired T cell proliferation and lymphopenia, which was improved by arginine supplementation (Reizine et al., 2021). In contrast to the findings of the study conducted in Europe where COVID-19 mortality rates are high compared to countries such as Japan, a Japanese study conducted by Takano et al. reported that transiently expanding and proliferating polymorphonuclear (PMN)-MDSCs are observed in severe COVID-19 survivors but not in non-survivors, suggesting that transient PMN-MDSCs aid in regulating severe inflammation in patients with COVID-19 (Takano et al., 2021). In contrast, the activation of immune-regulatory cells and induction of the expression of IL-10 mediated by Extract A, as observed with the experiments in the present disclosure conducted with PBMCs, are found to be generated at earlier time points before potent memory Th1 responses develop. Therefore, it is important for such immune-regulatory pathways to be activated in addition to potent Th1 responses in order to prevent collateral tissue damage, which may occur if those potent inflammatory responses are left uncontrolled. In agreement with the inventors' hypothesis, Lessard et al. found that the treatment of mice with MDP can convert inflammatory Ly6C^{high} monocytes into Ly6C^{low} patrolling monocytes, which can produce immune-regulatory cytokines IL-10 and TGFβ in a NOD2-dependent manner and alleviate LPS-mediated inflammation in vivo (Lessard et al., 2017). In addition, Bio-Plex analyses of the PBMC culture supernatants in the present disclosure revealed that RBD and Extract A can robustly induce the expression of Th1-inducing cytokines TNFα, IL-1α and IL-12 on day three (Fig. 27). Indeed, by using in vitro differentiation systems and TCRαβ transgenic mice specifically recognizing OVA or hen egg lysozyme, Shibuya et al. reported that IL-1α and TNFα are necessary for the elicitation of IL-12-induced Th1 responses (Shibuya et al., 1998). In addition, the expression of Th1-related cytokines IL-2 and IFN-γ was robustly induced by the combination of Extract A and RBD on day six, indicating elicitation of robust Th1 responses (Fig. 7 and S7). Nonetheless, Extract A and RBD also mediated the production of IL-17A from hPBMCs on day six after RBD re-stimulation, suggesting the induction of Th17 responses (Fig. 27). Since BCG vaccine inoculation or BCG-derived Mtb vaccines are known to induce Th17 responses, and Extract A contains Mtb-derived antigens, an expected outcome involves the development of Th17 responses mediated by Extract A (Gopal et al., 2012, De Cassan et al., 2010). Besides, BCG vaccine-induced expression of IL-17A is one of the mechanisms required for development of Th1 responses via suppression of Mtb-mediated inhibitory factors, such as IL-10 secretion, in vivo (Gopal et al., 2012).

Bacteria and extracts of mushrooms, including Coley toxin, are thought to exhibit anti-tumor effect by immuno-potentiation via stimulation of innate immune receptors such as TLR4 (Thotathil and Jameson, 2007, Mourits et al., 2018). On the contrary, the present inventors showed that the hot water extract of Mtb induces potent effects to diseases in the mice with pre-existing BCG memory via mechanisms involving a Th1-type T-cell response specific to Mtb antigen which is mediated by Mtb antigen, a non-causative factor antigen molecule derived from Extract A, that are also cross-reacting to other diseases. In addition, Mtb-derived STING agonist found in Extract A is also important for mediating the effects to diseases of Extract A. Moreover, observation of the effect to the diseases by Flu vaccination in Flu-immunized mice also revealed that heterologous T cell responses induced by non-causative factor antigen molecule may provide protection against not only specific tumors or diseases but also infections with various types of unrelated pathogens. Consistently, the inventors' data also revealed that Mtb-derived Extract A can express or enhance heterologous T cell responses capable of cross-reacting to SARS CoV-2 in SARS CoV-2-naive individuals with pre-existing Mtb memory, which also supports the hypothesis that vaccination with matter derived from Mycobacterium tuberculosis might induce protective immune responses against SARS CoV-2. Therefore, it is considered that rather than employing BCG vaccine to fight against pandemic such as COVID-19, immunization (vaccination) with pandemic causative factor (e.g., SARS CoV-2 vaccine) with Extract A as an adjuvant could be a safer approach, because even multiple systemic injections of Extract A are safer than those of BCG. BCG history due to tuberculosis infection or vaccination history may lead to immune local or systemic adverse events, including disseminated BCG infections in the immunocompromised individuals (Venkataraman et al., 2015, Whelan et al., 2009). However, nevertheless, as a potent inducer of type 1 immunity, Extract A is an advantageous tool for immunotherapy administered for allergy, because it is capable of alleviating or avoiding allergy-associated type 2 immunity (Gieseck et al., 2018).

### (Example 9: Prevention, re-infection prevention, and treatment of bacterial infections)

This example demonstrates the verification of prevention, re-infection prevention, and/or treatment of bacterial infections.

### (Method)

In the prevention of bacterial infections (such as bacterial conjunctivitis, for example), bacterial antigen components responsible for the causative factors of bacterial infections, along with Extract A, are administered to subjects without bacterial infections.

In the prevention of recurrence and/or treatment of bacterial infections (such as bacterial conjunctivitis, for example), Extract A is administered to subjects who have had or currently have bacterial infections.

Following administration, the prognosis of bacterial infections in the subjects is observed using techniques commonly employed in the art of this field.

### (Example 10: Prevention, prevention of reinfection, and treatment of parasitic infections)

This example validates the prevention, prevention of reinfection, and/or treatment of parasitic infections.

### (Method)

In the prevention of parasitic infections, such as Acanthamoeba keratitis, for instance, the causative antigen of the parasitic infection and Extract A are administered to subjects without parasitic infections.

In the prevention of recurrent parasitic infections, such as Acanthamoeba keratitis, or in the treatment thereof, Extract A is administered to subjects who have had or currently have parasitic infections.

After administration, the post-treatment prognosis of parasitic infections in subjects is observed using techniques commonly employed in the art of this field.

### (Example 11: Prevention, prophylaxis, re-infection prevention, and treatment of viral infections)

This embodiment conducts clinical trials for the prevention of viral infections, prevention of disease onset, prevention of re-infection, and/or treatment of viral infections.

### (Method)

### (Identification of non-causative antigen)

Peripheral blood mononuclear cells (PBMCs) are isolated from individuals who are symptomatic with viral infections, individuals who are asymptomatic but have detectable viral antigen (indicating asymptomatic or resolved viral infections), and normal individuals. The antigen response profile is determined by confirming the vaccination history and/or infection history of the above individuals. Vaccination history and infection history may include but are not limited to tuberculosis, malaria, yellow fever virus, smallpox virus, vaccinia, measles/rubella, polio, mumps, rotavirus infection, varicella (chickenpox), yellow fever, Ebola, West Nile fever, Hib infection, pneumococcal infection, whooping cough, Japanese encephalitis, meningococcal infection, salmonellosis, pathogenic E. coli, toxoplasmosis, Zika virus, herpes simplex virus type 1, EBV/Epstein-Barr virus (herpesvirus type 4), CMV/cytomegalovirus (herpesvirus type 5), influenza virus, MARS, rabies, diphtheria, etc. This is not an exhaustive list and may include other viral infections.

The isolated PBMCs (e.g., 1.0×10⁴ to 1.0×10⁶ cells) mentioned above are stimulated with Extract A (0.01 µg/mL to 1 mg/mL) or an extract containing Extract A (0.01 µg/mL to 1 mg/mL) for approximately 6 to 24 hours. The production of cytokines (e.g., IFN-γ, IL-2, and/or TNF-α) is measured using commonly used detection methods in the field (such as ELISA, qPCR, and/or FACS). Individuals whose post-stimulation cytokine production is more than twice that of pre-stimulation cytokine production are selected as Responders. However, the selection of individuals is not necessarily required.

### (Administration of Extract A in test subjects who have developed viral infections or have recovered from viral infections)

In test subjects with active viral infections, test subjects with asymptomatic viral infections those who have recovered from viral infections (where viral antigens are detected but no symptoms or active infection are present), as well as in normal test subjects, Extract A is administered according to the following dosing regimen for the evaluation of clinical efficacy. ·Group Composition: Placebo or Extract A (approximately 0.001 µg/single dose to approximately 1 mg/single dose) or an extract containing Extract A (equivalent to approximately 0.001 µg/single dose to approximately 1 mg/single dose of Extract A) for two doses
·Number of Test Subjects: Approximately 100 to 1000 participants.
·Route of Administration: SC (Subcutaneous), IM (Intramuscular), or ID (Intradermal) administration.
·Frequency of Administration: Approximately once per day (Week 1), and approximately once per week (Subsequent weeks).
·Duration of Administration: Single dose up to approximately 1 year.
·Evaluation Criteria: Virus clearance rate, time to virus negativity confirmation, trend in virus genome quantity, duration of fever, time to improvement in symptoms (fever, cough, sore throat, headache, muscle or joint pain, chills or sweating, malaise or fatigue, shortness of breath, altered consciousness, runny nose, chest pain, diarrhea, nausea/vomiting, etc.), rate of severe cases and mortality, percentage change in serum antibodies, use of mechanical ventilation/ECMO, improvement in imaging findings, temporal changes in WBC count and differential, hemoglobin, platelet, creatinine, glucose, total bilirubin.
·Evaluation criteria include improvement in test values such as ALT, AST, PT, percentage of patients with each severity level assessed by ordinal scale, time to improvement in oxygenation, type/frequency/severity of adverse events, number and proportion of infected individuals, time from infection to onset, time from symptom appearance to PCR positivity disappearance, time to symptom appearance or symptom improvement, presence of severe cases (hospitalization with oxygen supplementation), presence of critical cases (requiring mechanical ventilation, shock vitals, or ICU management due to organ failure other than lungs), variation from baseline in serum antibody titers against the antigen, variation from baseline in IFN-γ production against the antigen, and one or more items from the type/frequency/severity of adverse events category as evaluation criteria.

### (Results)

All evaluation criteria demonstrate that the group administered with the non-antigenic factor antigen has significantly better outcomes compared to the placebo group, and examples of success within the tested dose range can be observed.

### (Administration of causative factor antigens and Extract A in healthy test subjects)

In the case of healthy test subjects, causative factor antigens of viral infections and Extract A are administered according to the following dosing regimen to evaluate clinical effectiveness.
·Group Composition:
   (1) Placebo
   (2) Extract A (approximately 0.001 µg/1 dose - approximately 1 mg/1 dose) or an extract containing Extract A (equivalent to approximately 0.001 µg/1 dose - approximately 1 mg/1 dose of Extract A) administered in two doses
   (3) Causative factor of viral infection alone
   (4) Causative factor of viral infection and Extract A (approximately 0.001 µg/1 dose to approximately 1 mg/1 dose) or an extract containing Extract A (equivalent to approximately 0.001 µg/1 dose to approximately 1 mg/1 dose of Extract A) administered in two doses
·Number of test subjects: Approximately 100 - 30,000 participants
·Route of administration: SC (subcutaneous), IM (intramuscular), or ID (intradermal)
·Dosage frequency: Once (Week 1) and subsequently once every 1-4 weeks from Week 2 onwards, with additional doses as needed at intervals of 1-4 weeks
·Administration period: 1 to 10 doses (for preventive purposes, follow-up period after administration completion ranges from 1 month to over 1 year).

Evaluation criteria: Number and proportion of infected individuals, time from infection to onset, time from symptom appearance to PCR positivity disappearance, time to symptom appearance or symptom improvement, presence of severe cases (hospitalization with oxygen supplementation), presence of critical cases (requiring mechanical ventilation, shock vitals, or ICU management due to organ failure other than lungs), variation from baseline in serum antibody titers against the antigen, variation from baseline in IFN-γ production against the antigen, and one or more items from the type/frequency/severity of adverse events category as evaluation criteria.

### (Results)

All evaluation criteria show that the group administered with a combination of non-causative antigen and virus infection causative factors, significantly outperforms the placebo or the group administered solely with virus infection causative factors, with observed instances of significant efficacy within the tested dosage range.

### (Example 12: Prevention, recurrence prevention, and treatment of allergies)

This embodiment verifies the prevention, recurrence prevention, and/or treatment of allergies.

### (Method)

In the prevention of allergies, such as allergic conjunctivitis, in individuals without allergenic causative factors, the allergenic causative factors and Extract A are administered.

In the prevention of recurrent allergies, such as allergic conjunctivitis, and/or in the treatment of allergies, individuals with allergenic causative factors and/or individuals who have allergenic causative factors are administered Extract A.

After administration, follow-up observations of the prognosis of allergies in the subjects are conducted using techniques commonly employed in the field.

### (Example 13: prophylaxis, recurrence prophylaxis, and treatment of autoimmune diseases)

In this Example, prophylaxis, recurrence prophylaxis, and/or treatment of autoimmune diseases are verified.

### (Method)

In the prevention of autoimmune diseases (such as autoimmune uveitis), individuals without the causative antigen of the autoimmune disease are administered the causative antigen of the autoimmune disease and Extract A.

In the prevention of recurrence and/or treatment of autoimmune diseases (such as autoimmune uveitis), individuals who had the causative antigen of the autoimmune disease and/or individuals who have the causative antigen of the autoimmune disease are administered Extract A.

After administration, the prognosis of autoimmune diseases in the subjects is observed using techniques commonly used in the field.

### (Example 14: preparation)

(1) In the case of a monotherapy consisting solely of non-causative antigen components that are neither derived from the causative factors of the disease nor cross-react with the said causative factors
   Following the method outlined in the <Manufacturing Example>, the crude solution of Extract A is prepared. The prepared crude solution of Extract A is diluted to 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, 200-fold, 300-fold, 400-fold, 500-fold, 600-fold, 700-fold, 800-fold, 900-fold, 1,000-fold, 2,000-fold, 3,000-fold, 4,000-fold, 5,000-fold, 6,000-fold, 7,000-fold, 8,000-fold, 9,000-fold, 10,000-fold, 20,000-fold, 30,000-fold, 40,000-fold, 50,000-fold, 60,000-fold, 70,000-fold, 80,000-fold, 90,000-fold, or 100,000-fold to achieve a suitable concentration for administration. Subsequently, sodium chloride is added to achieve a final concentration of 0.9%, and the mixture is sterilized using methods such as autoclaving. Finally, the formulation is prepared into an appropriate dosage form.
(2) In the case of a combination comprising antigen components derived from the causative factors of the disease of interest, and/or antigen components that cross-react with the said causative factors, along with antigen components that neither originate from the causative factors of the disease nor cross-react with the said causative factors.

Following the method described in the case of a monovalent formulation comprising only antigen components that neither originate from the causative factors of the disease nor cross-react with the said causative factors, the original solution of Extract A is diluted to a concentration suitable for administration. Subsequently, sodium chloride is mixed to achieve a final concentration of 0.9%. After sterilization using techniques such as autoclaving, the final concentration is adjusted appropriately by adding the spike protein of the coronavirus. The formulation is then prepared into the appropriate dosage form.

### (Example 15: Treatment and prevention, including companion diagnostics)

An example of treatment and prevention with companion diagnostics for individuals (humans) seeking vaccination is provided.

Peripheral blood mononuclear cells (PBMCs) are isolated from human subjects (individuals) who are interested in vaccine efficacy or treatment as disclosed herein, including those with active viral infections, those without active viral infections (with detectable viral antigens but asymptomatic or recovered from viral infections), and also potentially including healthy individuals.

The antigen response profile is specified by confirming the immune memory (immune responsiveness) of the above-mentioned subjects through tests to assess tuberculosis infection such as tuberculin skin tests, interferon-γ release assays, and the like, maternal and child health records or equivalent documents, medical records, or other medical information (which may also rely on electronically recorded information of the test subjects stored in clouds or electronic chips.

The isolated PBMCs mentioned above (for example, 1.0 × 10⁴ to 1.0 × 10⁶ cells) are stimulated with Extract A (0.01 ug/mL to 1.0 mg/mL) or an extract containing Extract A (0.01 ug/mL to 1.0 mg/mL) for approximately 6 to 24 hours. The production of cytokines (such as IFN-γ, IL-2, and/or TNF-α) is measured using detection methods commonly used in the art (such as ELISA, qPCR, and/or FACS). Human subjects with post-stimulation cytokine production exceeding a predetermined threshold based on the pre-stimulation cytokine production (e.g., 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2-fold or more, 3-fold or more, 4-fold or more, 5-fold or more, 6-fold or more, 7-fold or more, 8-fold or more, 9-fold or more, or 10-fold or more, preferably 2-fold or more) are selected as vaccination candidates.

The vaccination schedule can be performed as needed, for example, following an initial administration, additional immunizations can be given with an appropriate interval, such as one week or other suitable period.

### (Results)

It is confirmed that the likelihood of contracting the infectious disease is reduced compared to before vaccination.

### (Example 16: Treatment and prevention, including companion diagnostics)

An example of treatment and prevention with companion diagnostics for individuals (humans) desiring vaccination is provided.

Peripheral blood mononuclear cells (PBMCs) are isolated from human subjects (including those who desire vaccine efficacy or treatment based on the present disclosure and may have virus infection, may have had virus infection without symptoms, or may be normal).

The immune memory (immune responsiveness) of the above-mentioned subjects is identified through tests such as tuberculin skin tests, interferon-γ release assays, and other examinations to assess tuberculosis infection. Additionally, antigen response profiles are determined using sources such as maternal and child health records or their equivalents, medical records, and other medical information (which may also rely on electronically recorded information stored in clouds or electronic chips).

The isolated PBMCs mentioned above (for example, 1.0 × 10⁴ to 1.0 × 10⁶ cells) are stimulated with Extract A (0.01 ug/mL to 1.0 mg/mL) or an extract containing Extract A (0.01 ug/mL to 1.0 mg/mL) for approximately 6 to 24 hours. The production of cytokines (such as IFN-γ, IL-2, and TNF-α) is measured using detection methods commonly used in the art (such as ELISA, qPCR, and/or FACS). Human subjects with post-stimulation cytokine production exceeding a predetermined threshold based on the baseline cytokine production (e.g., 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2-fold or more, 3-fold or more, 4-fold or more, 5-fold or more, 6-fold or more, 7-fold or more, 8-fold or more, 9-fold or more, or 10-fold or more, preferably 2-fold or more) are selected as vaccination candidates.

The vaccination schedule can be administered as needed, for example, following an initial administration, additional immunizations can be given with an appropriate interval, such as one week or other suitable period.

### (Results)

It is confirmed that the likelihood of contracting the infectious disease is reduced compared to before vaccination.

### (Example 17: Treatment and prevention including companion diagnostics)

Providing an example of treatment and prevention with companion diagnostics for individuals (humans) seeking vaccination.

Peripheral blood mononuclear cells (PBMCs) are isolated from human subjects (including those who desire vaccine efficacy or treatment based on the present disclosure and may have virus infection, may have had virus infection without symptoms, or may be normal).

The antigen response profile is determined by objectively confirming the immune memory (immune responsiveness) of the aforementioned subjects through the tuberculin skin test.

The isolated PBMCs mentioned above (for example, 1.0 × 10⁴ to 1.0 × 10⁶ cells) are stimulated with Extract A (0.01 ug/mL to 1.0 mg/mL) or an extract containing Extract A (0.01 ug/mL to 1.0 mg/mL) for approximately 6 to 24 hours. The production of cytokines (such as IFN-γ, IL-2, and TNF-α) is measured using detection methods commonly used in the art (such as ELISA, qPCR, and/or FACS). Human subjects with post-stimulation cytokine production exceeding a predetermined threshold based on the baseline cytokine production (e.g., 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2-fold or more, 3-fold or more, 4-fold or more, 5-fold or more, 6-fold or more, 7-fold or more, 8-fold or more, 9-fold or more, or 10-fold or more, preferably 2-fold or more) are selected as vaccination candidates.

The vaccination schedule can be administered as needed, for example, following an initial administration, additional immunizations can be given with an appropriate interval, such as one week or other suitable period.

### (Results)

It is confirmed that the likelihood of contracting the infectious disease is reduced compared to before vaccination.

### (Results)

### (Example 18: Treatment and prevention, including companion diagnostics)

An example of treatment and prevention with companion diagnostics for individuals (humans) seeking vaccination is provided.

Peripheral blood mononuclear cells (PBMCs) are isolated from human subjects (including those who desire vaccine efficacy or treatment based on the present disclosure and may have virus infection, may have had virus infection without symptoms, or may be normal).

The antigen response profile is specified by confirming the immune memory (immune responsiveness) of the aforementioned subjects by Interferon-γ release assays.

The isolated PBMCs mentioned above (for example, 1.0 × 10⁴ to 1.0 × 10⁶ cells) are stimulated with Extract A (0.01 ug/mL to 1.0 mg/mL) or an extract containing Extract A (0.01 ug/mL to 1.0 mg/mL) for approximately 6 to 24 hours. The production of cytokines (such as IFN-γ, IL-2, and TNF-α) is measured using detection methods commonly used in the art (such as ELISA, qPCR, and/or FACS). Human subjects with post-stimulation cytokine production exceeding a predetermined threshold based on the baseline cytokine production (e.g., 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2-fold or more, 3-fold or more, 4-fold or more, 5-fold or more, 6-fold or more, 7-fold or more, 8-fold or more, 9-fold or more, or 10-fold or more, preferably 2-fold or more) are selected as vaccination candidates.

The vaccination schedule can be administered as needed, for example, following an initial administration, additional immunizations can be given with an appropriate interval, such as one week or other suitable period.

### (Results)

It is confirmed that the likelihood of contracting the infectious disease is reduced compared to before vaccination.

### (Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein. The present application claims priority to International Application PCT/JP2021/005951 filed on February 17, 2021 with the WIPO, and Japanese Patent Application No. 2021-208598 filed on December 22, 2021 with Japan Patent Office. It is understood that the entire content thereof is incorporated herein by reference.

### [Industrial Applicability]

The present disclosure provides a method of preventing and treating a disease such as infectious disease on a conventionally unavailable mechanism.

## Claims

1. A composition for the prophylaxis or treatment of an infectious disease, an allergy, and/or an autoimmune disease in a subject, comprising a non-causative factor antigen component that is neither derived from nor cross-reactive with a causative factor of the infectious disease, the allergy, and/or the autoimmune disease, wherein
the composition is **characterized in that**
1) the subject is confirmed to have an immune response to the non-causative factor,
2) the composition is administered to the subject when the subject has the immune response.

2. A composition for the prophylaxis or treatment of an infectious disease in a subject, comprising a non-causative factor antigen component that is neither derived from nor cross-reactive with a causative factor of the infectious disease, wherein
the composition is **characterized in that**
1) the subject is confirmed to have an immune response to the non-causative factor,
2) the composition is administered to the subject when the subject has the immune response.

3. A composition for the prophylaxis or treatment of an infectious disease other than tuberculosis in a subject, comprising a Mycobacterium tuberculosis hot water extract or a part thereof, wherein
the composition is **characterized in that**
1) the subject is confirmed to have an immune response to tuberculosis,
2) the composition is administered to the subject when the subject has the immune response.

4. The composition according to claim 3, wherein the aforementioned Mycobacterium tuberculosis hot water extract or a part thereof is a hot water extract of Mycobacterium tuberculosis Aoyama B strain.

5. The composition according to claim 3 or 4, wherein the aforementioned Mycobacterium tuberculosis hot water extract or a part thereof is Extract A.

6. The composition according to any one of claims 1 to 5, wherein the aforementioned composition is administered in an amount effective for the production of IL-10.

7. The composition according to any one of claims 1 to 6, wherein the aforementioned infectious disease is a viral infectious disease.

8. The composition according to any one of claims 1 to 7, wherein the aforementioned infectious disease is a viral infectious disease related to Coronavirus.

9. The composition according to any one of claims 1 to 8, wherein the aforementioned immune response is objectively confirmed.

10. The composition according to claim 9, wherein the aforementioned immune response is objectively confirmed by a tuberculin reaction test or interferon γ release assay, or by objective information contained in the mother and child health handbook or its equivalent, clinical records, or other medical information.

11. The composition according to any one of claims 1 to 10, wherein the aforementioned immune response is tested by a tuberculin reaction test or an interferon γ release assay.

12. The composition according to any one of claims 1 to 10, wherein the aforementioned non-causative factor antigen component or a Mycobacterium tuberculosis hot water extract or a part thereof is pre-administered to the test subject when the aforementioned subject does not have the aforementioned immune response, and thereafter, the aforementioned composition is administered to the subject when the subject is confirmed to have the immune response.

13. The composition according to claim 11, wherein the aforementioned pre-administration is repeated until the aforementioned subject has the aforementioned immune response.

14. The composition according to claim 11 or 12, wherein the aforementioned pre-administration is administration before or after the onset of the aforementioned infectious disease.

15. The composition according to claim 13, wherein the aforementioned pre-administration is administration after the onset of the aforementioned infectious disease.

16. The composition according to any one of claims 1 to 14, which is administered under conditions where a causative factor antigen component derived from and/or cross-reactive with the aforementioned causative factor is present.

17. A composition for the prophylaxis or treatment of a target disease in a subject, comprising a non-causative factor antigen component that is neither derived from nor cross-reactive with a causative factor of the disease, wherein the composition is administered under conditions where a causative factor antigen component derived from and/or cross-reactive with the causative factor of the disease is present.

18. The composition according to claim 17, wherein the aforementioned causative factor comprises an exogenous substance for the aforementioned subject.

19. The composition according to claim 17 or 18, wherein the aforementioned causative factor of the disease comprises a pathogenic factor of an infectious disease, a causative substance of an allergy, a causative substance of an autoimmune disease, or a toxic substance.

20. The composition according to any one of claims 17 to 19, wherein the aforementioned disease is an infectious disease.

21. The composition according to any one of claims 17 to 20, wherein the aforementioned subject has immune memory for the aforementioned non-causative factor antigen component.

22. The composition according to any one of claims 17 to 21, comprising the aforementioned causative factor antigen component.

23. The composition according to any one of claims 17 to 22, which is for the prophylaxis of the aforementioned disease.

24. The composition according to any one of claims 17 to 23, wherein the aforementioned composition prevents or treats the aforementioned disease without or with reduced immune hyperreactivity.

25. The composition according to any one of claims 17 to 24, wherein the aforementioned composition is administered in a form effective for regulating cellular immunity against the aforementioned causative factor of the disease.

26. The composition according to any one of claims 17 to 25, which is **characterized in that** whether a causative factor antigen component derived from and/or cross-reactive with the causative factor of the target disease is present in the aforementioned subject is confirmed, and when it is not present, the causative factor antigen component is administered together with the aforementioned non-causative factor antigen component.

27. The composition according to any one of claims 17 to 26, wherein the aforementioned disease is an infectious disease, an allergy, or an autoimmune disease.

28. The composition according to any one of claims 17 to 27, wherein the aforementioned disease is a viral infectious disease or a bacterium infectious disease.

29. The composition according to any one of claims 17 to 28, wherein the aforementioned disease is a viral infectious disease.

30. The composition according to any one of claims 17 to 29, wherein the aforementioned disease is an infectious disease related to Coronavirus.

31. The composition according to any one of claims 17 to 30, wherein the aforementioned causative factor antigen component is a molecule that appears at least partially on the surface of the causative factor of the target disease.

32. The composition according to any one of claims 17 to 31, wherein the aforementioned non-causative factor antigen component is a Mycobacterium tuberculosis hot water extract or a part thereof.

33. The composition according to any one of claims 17 to 32, wherein the aforementioned non-causative factor antigen component is a hot water extract of Mycobacterium tuberculosis Aoyama B strain or a part thereof.

34. The composition according to any one of claims 17 to 33, wherein the aforementioned non-causative factor antigen component is Extract A.

35. The composition according to any one of claims 17 to 34, wherein the aforementioned non-causative factor antigen component comprises a Mycobacterium tuberculosis-derived STING agonist, LpqH, Y1269, PPD, CMV pp65 overlapping peptide, a Mtb-associated antigen, a component capable of biosynthesizing STING agonist in human cells, or a NOD2 agonist.

36. The composition according to any one of claims 17 to 35, wherein the aforementioned non-causative factor antigen component comprises c-di-AMP, c-di-GMP, 3'3'-cGAMP, 2'3'-cGAMP, which are Mycobacterium tuberculosis-derived STING agonists, and/or dsDNA capable of producing 2'3'-cGAMP via cGAS in human cytoplasm.

37. The composition according to any one of claims 17 to 36, wherein the aforementioned non-causative factor antigen component is administered two times or more.

38. The composition according to any one of claims 17 to 37, which is **characterized in that**
1) the aforementioned subject is confirmed to have an immune response to the aforementioned non-causative factor,
2) the aforementioned composition is administered to the aforementioned subject when the subject has the aforementioned immune response.

39. The composition according to claim 38, wherein the aforementioned immune response is objectively confirmed.

40. The composition according to claim 38 or 39, wherein the aforementioned immune response is objectively confirmed by a tuberculin reaction test or interferon γ release assay, or by objective information contained in the mother and child health handbook or its equivalent, clinical records, or other medical information.

41. The composition according to any one of claims 38 to 40, wherein the aforementioned immune response is tested by a tuberculin reaction test or an interferon γ release assay.

42. The composition according to any one of claims 38 to 41, wherein the aforementioned non-causative factor antigen component or a Mycobacterium tuberculosis hot water extract or a part thereof is pre-administered to the test subject when the aforementioned subject does not have the aforementioned immune response, and thereafter, the aforementioned composition is administered to the subject when the subject is confirmed to have the immune response.

43. The composition according to claim 42, wherein the aforementioned pre-administration is repeated until the aforementioned subject has the aforementioned immune response.

44. The composition according to any one of claims 42 to 43, wherein the aforementioned pre-administration is administration before or after the onset of the aforementioned infectious disease.

45. The composition according to any one of claims 42 to 44, wherein the aforementioned pre-administration is administration after the onset of the aforementioned infectious disease.

46. A combination of a causative factor antigen component derived from and/or cross-reactive with a causative factor of a target disease in a subject and a non-causative factor antigen component that is neither derived from nor cross-reactive with the causative factor of the disease.

47. The combination according to claim 46, wherein the aforementioned causative factor antigen component comprises a pathogenic factor of an infectious disease, a causative substance of an allergy, a causative substance of an autoimmune disease, or a toxic substance.

48. The combination according to claim 46 or 47, wherein the aforementioned causative factor antigen component is a bacterium, a virus or protozoa, or a part thereof.

49. The combination according to any one of claims 46 to 48, wherein the aforementioned causative factor antigen component is a virus or a part thereof.

50. The combination according to any one of claims 46 to 49, wherein the aforementioned causative factor antigen component is a virus belonging to the Coronaviridae family or a part thereof.

51. The combination according to any one of claims 46 to 50, wherein the aforementioned causative factor is a virus belonging to a family selected from the group consisting of Rhinovirus, adenovirus, Coronavirus, RS virus, influenza virus, parainfluenza virus, and Enterovirus.

52. The combination according to any one of claims 46 to 51, wherein the aforementioned causative factor is a virus belonging to the genus Betacoronavirus.

53. The combination according to any one of claims 46 to 51, wherein the aforementioned causative factor is a virus selected from the group consisting of HCoV-HKU1, HCoV-OC43, SARS-CoV, MERS-CoV, and SARS-CoV-2.

54. The combination according to any one of claims 46 to 51, wherein the aforementioned causative factor is SARS-CoV-2 virus.

55. The combination according to any one of claims 46 to 49, wherein the aforementioned non-causative factor antigen component is a Mycobacterium tuberculosis hot water extract or a part thereof.

56. The combination according to any one of claims 46 to 55, wherein the aforementioned non-causative factor antigen component is a hot water extract of Mycobacterium tuberculosis Aoyama B strain or a part thereof.

57. The combination according to any one of claims 46 to 56, wherein the aforementioned non-causative factor antigen component is a Mycobacterium tuberculosis hot water extract or a part thereof.

58. The combination according to any one of claims 46 to 57, wherein the aforementioned non-causative factor antigen component is a hot water extract of Mycobacterium tuberculosis Aoyama B strain or a part thereof.

59. The combination according to any one of claims 46 to 57, wherein the aforementioned Mycobacterium tuberculosis hot water extract or a part thereof is Extract A.

60. The combination according to any one of claims 46 to 57, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.

61. The combination according to any one of claims 46 to 57, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.

62. The combination according to any one of claim 55 or 56, wherein the aforementioned non-causative factor antigen component is administered two times or more.

63. The combination according to any one of claims 55 to 62, wherein the aforementioned causative factor antigen component is a pathogenic factor of an infectious disease, and the aforementioned non-causative factor antigen component functions as an adjuvant when the aforementioned causative factor antigen component is used as a vaccine.

64. The combination according to any one of claims 46 to 63, wherein 1) the aforementioned subject is confirmed to have an immune response to the aforementioned non-causative factor, and
2) when the subject has the aforementioned immune response, one or both of the aforementioned combinations is/are administered to the subject.

65. The combination according to claim 64, wherein the aforementioned immune response is objectively confirmed.

66. The combination according to claim 64 or 65, wherein the aforementioned immune response is objectively confirmed by a tuberculin reaction test or interferon γ release assay, or by objective information contained in the mother and child health handbook or its equivalent, clinical records, or other medical information.

67. The combination according to any one of claims 64 to 66, wherein the aforementioned immune response is tested by a tuberculin reaction test or an interferon γ release assay.

68. The combination according to any one of claims 64 to 67, wherein the aforementioned non-causative factor antigen component or a Mycobacterium tuberculosis hot water extract or a part thereof is pre-administered to the test subject when the aforementioned subject does not have the aforementioned immune response, and thereafter, the aforementioned combination is administered to the subject when the subject is confirmed to have the immune response.

69. The combination according to claim 68, wherein the aforementioned pre-administration is repeated until the aforementioned subject has the aforementioned immune response.

70. The combination according to any one of claims 68 to 69, wherein the aforementioned pre-administration is administration before or after the onset of the aforementioned infectious disease.

71. The combination according to any one of claims 68 to 70, wherein the aforementioned pre-administration is administration after the onset of the aforementioned infectious disease.

72. A medicament for the prophylaxis or treatment of a target disease in a subject, comprising a combination of a causative factor antigen component derived from and/or cross-reactive with a causative factor of the target disease and a non-causative factor antigen component that is neither derived from nor cross-reactive with the causative factor of the disease.

73. The medicament according to claim 72, further comprising one or more features described in any of claims 64 to 71.

74. A kit for the prophylaxis or treatment of a target disease in a subject, comprising a causative factor antigen component derived from and/or cross-reactive with a causative factor of the target disease and a non-causative factor antigen component that is neither derived from nor cross-reactive with the causative factor of the disease.

75. The kit according to claim 74, further comprising one or more features described in any of claims 64 to 71.

76. A method for preventing or treating a disease in a subject, comprising administering an effective amount of a non-causative factor antigen component that is neither derived from nor cross-reactive with the causative factor of the disease to the subject, under conditions where a causative factor antigen component derived from and/or cross-reactive with the causative factor of the disease is present.

77. The method according to claim 76, further comprising one or more features described in any of claims 64 to 71.

78. A method for preventing or treating a disease in a subject, comprising administering an effective amount of a causative factor antigen component derived from and/or cross-reactive with the causative factor of the disease to the subject, and administering an effective amount of a non-causative factor antigen component that is neither derived from nor cross-reactive with the causative factor of the disease to the target.

79. The method according to claim 78, further comprising one or more features described in any of claims 64 to 71.

80. A composition comprising a non-causative factor antigen component that is neither derived from nor cross-reactive with an infectious disease, an allergy, and/or an autoimmune disease to be the target, which removes or converts suppression on a regulatory T cell (Treg), wherein a test subject has immune memory for the antigen component, and, in the test subject, an activity of the regulatory T cell (Treg) against a causative factor of the infectious disease, the allergy, and/or the autoimmune disease, or a cell infected with the causative factor or containing the causative factor is suppressed.

81. The composition according to claim 80, wherein the aforementioned Treg is converted to an effector T cell (Teff) for the causative factor of the disease or the cell containing the causative factor of the infectious disease in the aforementioned test subject, by further presence of a causative factor antigen component derived from and/or cross-reactive with the causative factor of the target disease in the aforementioned test subject.

82. The composition according to claim 80 or 81, wherein the aforementioned test subject has immune memory for the aforementioned causative factor and/or a factor cross-reactive with the causative factor.

83. The method according to any one of claims 80 to 82, further comprising one or more features described in any of claims 64 to 71.

84. A composition for the prophylaxis or treatment of an infectious disease in a test subject, comprising an antigen component that is specific in the test subject for a component that is different from the causative factor of the infectious disease.

85. A composition for the prophylaxis or treatment of an infectious disease in a test subject, which is substantially free of a causative factor of the infectious disease or a part thereof and comprises an antigen component from a pathogen of a pre-existing infectious disease different from the infectious disease of the test subject.

86. A composition for the prophylaxis or treatment of an infectious disease other than tuberculosis, comprising a Mycobacterium tuberculosis extract.

87. A composition comprising a Mycobacterium tuberculosis extract for regulating immunity enhancement against a causative factor of an infectious disease other than tuberculosis in a subject when the subject is exposed to the causative agent.

88. A composition for the prophylaxis or treatment of an infectious disease, an allergy, or an autoimmune disease in a test subject, comprising a specific antigen component in the test subject that is neither derived from nor cross-reactive with a causative factor of the infectious disease, the allergy, or the autoimmune disease, wherein
the antigen component is a protein or a part thereof, or a peptide, and capable of regulating an immune response via CD4-positive T cells,
the infectious disease, the allergy, or the autoimmune disease comprises those that can be prevented or treated by an immune response via CD4-positive T cells, and wherein
whether the test subject is capable of regulating a CD4-positive T cell-mediated immune response to the infectious disease, the allergy, or the autoimmune disease is confirmed, and the composition is administered when the test subject is capable of regulating a CD4-positive T cell-mediated immune response to the infectious disease, the allergy, or the autoimmune disease.

89. A composition for the prophylaxis or treatment of an infectious disease in a test subject, comprising a specific antigen component in the test subject for a component different from a causative factor of the infectious disease, wherein
the infectious disease comprises a viral infectious disease,
the antigen component is a protein or a part thereof, or a peptide, and capable of regulating an immune response via CD4-positive T cells,
the viral infectious disease comprises those that can be prevented or treated by an immune response via CD4-positive T cells, and wherein
whether the test subject is capable of regulating an antivirus immune response via CD4-positive T cells is confirmed, and when the test subject is capable of regulating an antivirus immune response via CD4-positive T cells, the composition is administered.

90. The composition according to any one of claims 84 to 89, further comprising one or more features described in any of claims 64 to 71.

91. A method for producing or providing in other manner a composition for the prophylaxis or treatment of an infectious disease, an allergy, or an autoimmune disease in a test subject, the method comprising:
A) a step of specifying a specific antigen component for the test subject that is neither derived from nor cross-reactive with a causative factor of the infectious disease, the allergy, or the autoimmune disease,
B) a step of specifying whether the test subject has immune memory for the antigen component and selecting one with the immune memory, and
C) a step of producing or providing in other manner the selected antigen component.

92. A method for determining whether a specific antigen component for a test subject that is neither derived from nor cross-reactive with a causative factor of an infectious disease, an allergy, or an autoimmune disease can prevent or treat the infectious disease, the allergy, or the autoimmune disease in the test subject, the method comprising:
B) a step of specifying whether the test subject has immune memory for the antigen component, and specifying that the infectious disease, the allergy, or the autoimmune disease in the test subject can be prevented or treated when the immune memory is present.

93. A method for the prophylaxis or treatment of an infectious disease, an allergy, or an autoimmune disease, comprising a) a step of obtaining an antigen responsiveness profile of a test subject,
b) a step of specifying an antigen component or a combination of antigen components from the antigen responsiveness profile, wherein the antigen component or the combination of the antigen components is specified based on the immune responsiveness presented now or in the past by the test subject to the antigen component or the combination of the antigen components, and
c) a step of administering the antigen component or the combination of the antigen components identified in step b) to the test subject in an amount sufficient to regulate an immune response in the test subject.

94. The method according to any one of claims 91 to 93, further comprising one or more features described in any of claims 64 to 71.

95. An adjuvant for a vaccine against an infectious disease, comprising a Mycobacterium tuberculosis hot water extract or a part thereof.

96. An adjuvant for a vaccine against an infectious disease, comprising a STING agonist.

97. An adjuvant for a vaccine against a viral infectious disease, comprising a STING agonist.

98. The adjuvant according to claim 95, wherein the aforementioned Mycobacterium tuberculosis hot water extract or a part thereof is a hot water extract of Mycobacterium tuberculosis Aoyama B strain or a part thereof.

99. The adjuvant according to claim 95 or 98, wherein the aforementioned Mycobacterium tuberculosis hot water extract or a part thereof is Extract A.

100. The adjuvant according to any one of claims 95 to 99, wherein the aforementioned Mycobacterium tuberculosis hot water extract or a part thereof comprises a Mycobacterium tuberculosis-derived STING agonist, LpqH, Y1269, PPD, CMV pp65 overlapping peptide, a Mtb-associated antigen, a STING agonist, or a NOD2 agonist.

101. The adjuvant according to any one of claims 95 to 100, wherein the aforementioned Mycobacterium tuberculosis hot water extract or a part thereof comprises c-di-AMP, c-di-GMP, 3'3'-cGAMP, 2'3'-cGAMP, which are Mycobacterium tuberculosis-derived STING agonists, and/or dsDNA capable of producing these via cGAS in human cytoplasm.

102. The adjuvant according to any one of claims 95 to 101, wherein a causative factor of the aforementioned infectious disease is a bacterium, a virus, or a protozoa.

103. The adjuvant according to any one of claims 95 to 102, wherein the aforementioned causative factor of the infectious disease is a virus.

104. The adjuvant according to any one of claims 95 to 103, wherein the aforementioned causative factor of the infectious disease is a virus belonging to the Coronaviridae family.

105. The adjuvant according to any one of claims 95 to 104, wherein the aforementioned causative factor of the infectious disease is a virus belonging to a family selected from the group consisting of Rhinovirus, adenovirus, Coronavirus, RS virus, influenza virus, parainfluenza virus, and Enterovirus.

106. The adjuvant according to any one of claims 95 to 105, wherein the aforementioned causative factor of the infectious disease is a virus belonging to the genus Betacoronavirus.

107. The adjuvant according to any one of claims 95 to 106, wherein the aforementioned causative factor of the infectious disease is a virus selected from the group consisting of HCoV-HKU1, HCoV-OC43, SARS-CoV, MERS-CoV, and SARS-CoV-2.

108. The adjuvant according to any one of claims 95 to 107, wherein the aforementioned causative factor of the infectious disease is a SARS-CoV-2 virus.

109. The adjuvant according to any one of claims 95 to 108, further comprising one or more features described in any of claims 64 to 71.

110. The adjuvant according to any one of claims 95 to 108, which converts innate immunity to acquired immunity.
